# EUROPEAN PATENT SPECIFICATION

(11) **EP 2 783 631 B1**
(45) Date of publication and mention of the grant of the patent: **01.01.2020**
(21) Application number: 12850826.4
(22) Date of filing: 15.11.2012
(51) Int. Cl.: A61B 5/16, A61B 5/0245, A61B 5/18

(54) **BIOLOGICAL STATUS ESTIMATION DEVICE AND COMPUTER PROGRAM**
VORRICHTUNG ZUR LEBENSSTATUSMESSUNG UND COMPUTERPROGRAMM DAFÜR
DISPOSITIF D'ESTIMATION D'UN ÉTAT BIOLOGIQUE ET PROGRAMME D'ORDINATEUR

(30) Priority: 25.11.2011 JP 2011257330
(43) Date of publication of application: 01.10.2014
(73) Proprietor: Delta Tooling Co., Ltd., Hiroshima-shi, Hiroshima 736-0084 (JP)
(72) Inventor: FUJITA Etsunori, Hiroshima-shi Hiroshima 736-0084 (JP); OGURA Yumi, Hiroshima-shi Hiroshima 736-0084 (JP); UCHIKAWA Ryuichi, Hiroshima-shi Hiroshima 736-0084 (JP)
(74) Representative: Hoffmann Eitle
(86) International application number: PCT/JP2012/079703
(87) International publication number: WO 2013/077253

(56) References cited:
- EP-A1- 2 537 468
- WO-A1-2011/046178
- JP-A- 2008 264 138
- JP-A- 2011 167 362
- US-A1- 2006 235 315
- US-A1- 2010 228 139

## Description

### Technical Field

The present invention relates to a technique of detecting biological signals including indices of an autonomic nervous system and reaction information of the autonomic nervous system to estimate a biological state (in particular, a normal fatigued state where fatigue accumulates due to activities, a function recovery state realized by a predetermined function recovery means, or a slump state) from a relative change in a sympathetic nerve function in relation to a predetermined state of a parasympathetic nerve function controlled by the sympathetic nerve function or a relative change in a sympathetic nerve function in relation to a predetermined state of a parasympathetic nerve function.

### Background Art

In Patent Literature 1, the present applicant has disclosed a biological state estimation device including a means that obtains a time-series waveform of frequencies from a time-series waveform of a biological signal which is mainly a pulse signal of a cardiovascular system detected from the upper body of a person, and obtaining a time-series waveform of a frequency gradient and a time-series waveform of a frequency fluctuation to analyze the frequency of the time-series waveforms. During frequency analysis, power spectra of respective frequencies corresponding to predetermined functional adjustment signal, fatigue reception signal, and activity adjustment signal are obtained. Then, the state of a person is determined from a time-series change in the power spectra. Since the fatigue reception signal indicates the degree of progress of fatigue in a normal active state, when the degrees of predominance of the functional adjustment signal and the activity adjustment signal are compared with the fatigue reception signal as distribution ratios thereof, it is possible to determine the state of a person (relaxed state, fatigued state, sympathetic nerve predominant state, parasympathetic nerve predominant state, or the like) more accurately.

Moreover, in Patent Literature 2, the present applicant has proposed a technique of determining whether a driver is drunk or not more accurately. Specifically, a device disclosed in Patent Literature 2 includes:
a frequency-dynamic information processing means that obtains a tendency of a time-series fluctuation regarding the frequency of pulse waves detected from the back by an airpack; and
a drunk state determining means that determines that the driver is in a drunk state when the tendency of the time-series fluctuation regarding the frequency obtained by the frequency-dynamic information processing means diverges from a tendency of the time-series fluctuation regarding the frequency in a non-drunk state. The device determines whether the driver is in the drunk state by comparing with the time-series fluctuation regarding the frequency in the non-drunk state. Since the device determines the state of the driver using the time-series fluctuation as well as analyzing the frequency of the pulse waves changing depending on the condition of the person, it is possible to determine whether the driver is drunk or not more accurately than the conventional technique.

Patent Literatures 3 to 5 disclose techniques in which a pressure sensor is disposed in a seat cushion portion to detect and analyze buttock pulse waves to determine a dozing symptom of a driver during driving. Specifically, maximum values and minimum values of a time-series waveform of the pulse waves are obtained according to a Savitzky-Golay smoothing and differentiation method. The maximum values and the minimum values are divided every five second to obtain the average values thereof. The square of the difference between the obtained average values of the maximum and minimum values is used as a power value, and this power value is plotted every five second to create a time-series waveform of the power values. In order to read a global change in the power values from the time-series waveform, the gradient of power values in a certain time window Tw (180 seconds) is obtained according to a least-square method. Subsequently, similar calculation is performed in the next time window Tw with an overlap period T1 (162 seconds) and the results are plotted. This calculation (slide-calculation) is sequentially repeated to obtain a time-series waveform of the gradient of power values. On the other hand, the time-series waveform of the pulse waves is subjected to chaos analysis to obtain maximum Lyapunov exponents, and maximum values are obtained according to smoothing and differentiation similarly to the above, and a time-series waveform of the gradient of the maximum Lyapunov exponents is obtained by performing slide-calculation.

The time-series waveform of the gradient of the power values has phases opposite to the phases of the time-series waveform of the gradient of the maximum Lyapunov exponents. Moreover, a low-frequency and high-amplitude waveform among the time-series waveform of the gradient of the power values is determined as a characteristic signal indicating the dozing symptom, and a point at which the amplitude decreases is determined as a dozing point.

### Citation List

### Patent Literature

Patent Literature 1: Japanese Patent Application Publication No. 2011-167362
Patent Literature 2: WO2010/134525A1
Patent Literature 3: Japanese Patent Application Publication No. 2004-344612
Patent Literature 4: Japanese Patent Application Publication No. 2004-344613
Patent Literature 5: WO2005/092193A1

According to US 2010/228139 A1, in a living body inspection apparatus to inspect RLS (Restless Legs Syndrome), a pulse interval is obtained from a pulse wave signal, thereby performing a frequency analysis of the obtained pulse interval using COM. From a result of the frequency analysis, the low frequency components ranging from 0.04 to 0.15 Hz and the high frequency components ranging from 0.15 to 0.4 Hz are extracted. As an index posterior to an age amendment, low frequency components (LF)/high frequency components (HF) is obtained, for instance. It is then determined whether LF/HF is equal to or greater than a predetermined determination value indicating RLS. For example, it is determined whether LF/HF is equal to or greater than 0.65, which suspects RLS. It is determined whether a signal is accurately calculated which indicates an activity of autonomic nerve. A state of RLS is determined using LF/HF.

### Summary of Invention

### Problems to be solved by the invention

By the way, for example, a breath-alcohol meter used for detection of a drunk state can naturally detect whether the driver is drunk at that point in time. However, for example, even if a long-distance truck driver or the like is determined not to be drunken by a breath-alcohol meter when checked by a management company before departure, the management company cannot check whether the driver has drunk if the driver has drunk during a rest before arriving at a destination. When the driver has returned to the management company without having an accident, it is difficult for the management company to check whether the driver has drunk unless the driver voluntarily admits to having drunk. However, when the driver has actually drunk during driving duties, if the driver self-determines having become sober after several tens of minutes although the driver has drunk in a rest time, for example, and goes on driving, this may result in a severe accident. Thus, it is very important to detect and monitor drunken driving.

Thus, if the biological signal obtained during driving as well as before starting driving duties is collected, and the state of the driver after completion of the duties can be checked retrospectively using the data, the analysis results can be used for guidance of safe driving and accidents and violations can be suppressed. Naturally, a system that transmits the biological signal during the driving as well as after completion of duties using a communication means to check the driver's state during the driving duties in real-time may be employed. More preferably, if it is possible to analyze whether the driver's state is caused by normal fatigue resulting from driving, the driver is in a sick state (including an ahead sick state), or the quality of sleep of the driver (whether the driver had sleep appropriate for recovering functional damage due to fatigue, that is, whether the driver had high quality sleep without nocturnal awakening which includes so-called REM sleep and non-REM sleep) as well as detecting alcohol, it is possible to contribute to safe driving of the driver. Naturally, such analysis is helpful to health maintenance of a person to lead daily life without limiting to driving. Although the techniques disclosed in Patent Literatures 1 to 5 can detect the degree of fatigue and alcohol, it is always desirable to further improve the accuracy of the analysis results. In particular, as for detection of alcohol, it is preferable to improve the analysis accuracy from the perspective of management of safe driving. It is further preferable to comprehensively determine whether the state of a person is in a normal state or a slump state (including a sick state, a very fatigued state, and the like) without limiting to detection of alcohol using one system.

With the foregoing in view, the present applicant aims to provide a technique of detecting various states of a person more accurately. In particular, the present applicant aims to provide a technique of specifying whether the person's state results from alcohol intake by digitizing a fluctuation waveform obtained through frequency analysis and determining whether main resonance indicating heart rate fluctuation obtained from the biological signal is a harmonic oscillation system or an irregular vibration system.

### Means for solving the problem

In order to solve the problems, the present applicant has focused on the following facts and made the present invention.

First, the homeostasis of a person is maintained by fluctuation, and the frequency band of the fluctuation is in an ultra-low-frequency band of 0.001 to 0.04 Hz. On the other hand, in atrial fibrillation that is one of heart diseases, it is said that the characteristic of fluctuation of a cardiovascular system is switched at 0.0033 Hz. Moreover, there is a report that an abnormal power value is observed at a frequency band of 0.01 to 0.04 Hz in the heart rate fluctuation during sleep of a sleep apnea syndrome (SAS) patient. Thus, by monitoring a change in the fluctuation in such a ultra-low-frequency band or a frequency band near 0.0033 Hz or near 0.01 to 0.04 Hz, it is possible to detect the degree of homeostasis control.

Moreover, a biological signal in a drunk state, an imminent sleeping state or a transitional state occurring due to medical activities such as injections or medications is an irregular vibration system having different disturbance from a harmonic oscillation system showing states that are controlled by a homeostasis maintenance function inside the body such as a wakeful state, a drowsy state, or a sleeping state. The biological signal has two or three peak points of resonance frequency. A frequency that is predominant among these frequencies is called a dominant frequency. As for the way of the resonance frequency and the dominant frequency move, a finger plethysmogram and a surface pulse wave (in the present invention, an aortic pulse wave (APW)) show the same trend. On the other hand, a relaxed state and a tense state show clear peaks like the resonance curve of the harmonic oscillation system. Thus, since a number of resonance frequency peaks are present, it is possible to detect a drunk state and a transitional state and to distinguish both states. Further, since the recovery function of homeostasis is highly correlated with a change in fluctuation in the ultra-low-frequency band, the present applicant has focused on the resonance frequency of the harmonic oscillation system, the dominant frequency of the irregular vibration system, the trend of change thereof, and a change in a fluctuation expressed by a gradient in the fractal analysis that can detect these change appropriately. Thus, when a change in the fluctuation waveform in the low-frequency band is scored according to predetermined criteria, and the scores are plotted on a coordinate system to display vectors, the resonance frequency of the harmonic oscillation system, the dominant frequency of the irregular vibration system, and the state of these fluctuations can be magnified and highlighted, and the state close to the sense of a human can be detected more accurately. That is, the present applicant focuses on A, ω, and φ of a harmonic function Acos(ωt+φ) to express whether the biological signal is the harmonic oscillation system or the irregular vibration system as the function of A, ω, and φ to estimate the state of a person (that is, the state of a person is estimated using a fluctuation function indicating the control of the autonomic nervous system).

Moreover, after alcohol is absorbed from the stomach, the alcohol is carried by blood to stimulate the brain to give a feeling of elation and euphoria and to cause a vasodilatory effect on the skin. In this case, the liver degrades alcohol to produce acetaldehyde. Thus, a disturbance occurs in the heat rate depending on the degree of alcohol absorption and the trend of change in the fluctuation waveform and the trend of convergence and divergence change. Thus, by detecting the degree of alcohol absorption, it is possible to monitor the transition direction (physical condition change trend) of change in the physical condition. Moreover, since the effect of alcohol lasts for a long period and degradation thereof takes a considerable amount of time, the trend of change in the fluctuation waveform on the time axis changes depending on the degree of degradation. That is, since the duration and the effect of alcohol are different depending on the effectiveness of external stress, the fluctuation waveform changes for a longer period of time than that which becomes effective in a short period as when a person drinks a nutrient, for example. Thus, the trend of change in the on-spot physical condition state (analysis physical condition state) during state analysis is different with the lapse of time. Once a state of change where alcohol becomes effective is created, the state maintains and a fluctuation of homeostasis control for stabilizing the state is reduced. On the other hand, the effect on the degree of change of the physical condition, of a normal fatigue resulting from daily activities or works or drinking of a nutrient drink, as compared to alcohol is not too small as compared to a normal state but shows the same amplitude of fluctuation as a normal healthy state or a temporary abrupt change. However, the change shows a certain fluctuation width. Moreover, in the case of nutrient drinks, it is thought that the medicinal properties last for a short period of time and few nutrient drink has a remarkably long-lasting effect on both physical conditions and senses like alcohol. Moreover, in a slump state (including a sick state, an ahead sick state, a very bad physical condition), few people has a feeling of elation and euphoria and shows different indices from those of the alcohol intake state from the perspective of physical conditions and senses, and a fluctuation of change shows a different trend from any one of the alcohol intake state and the normal state.

A biological state estimation device of the present invention is a biological state estimation device that estimates a biological state using a biological signal of an autonomic nervous system, collected by a biological signal measuring means, the biological state estimation device including:
a frequency analysis means that analyzes frequencies of the biological signal to obtain a fluctuation waveform in a ultra-low-frequency band of 0.001 Hz to 0.04 Hz; and
a state estimation means that substitutes and displays the fluctuation waveform obtained by the frequency analysis means with index values regarding a sympathetic nerve and a parasympathetic nerve based on predetermined criteria to estimate the biological state based on a change with time in the index values.

According to the present invention the state estimation means is a means that obtains the fluctuation waveform obtained by the frequency analysis means as coordinate points on a four-quadrant coordinate system in which respective indices regarding the sympathetic nerve and the parasympathetic nerve are illustrated on vertical and horizontal axes based on the predetermined criteria to display vectors and estimates the biological state based on a change with time of the coordinate points.

The state estimation means preferably includes a first analysis determination means that estimates whether the biological state is a normal fatigued state where fatigue accumulates due to activities, a slump state, or a function recovery state where a predetermined function recovery means is performed based on a position of a coordinate point in a target analysis time segment in relation to a coordinate point in a reference analysis time segment.

The first analysis determination means preferably determines that the biological state is an alcohol intake state that corresponds to a refresh state in drunkenness degree classification corresponding to the function recovery means when the coordinate point in the target analysis time segment is in a predetermined range in relation to the coordinate point in the reference analysis time segment.

The first analysis determination means preferably classifies the slump state into a state where a person endures a slump factor and a state where a person resists against a slump factor.

The first analysis determination means preferably includes at least one of:
an analysis determination means A that estimates a transition direction of an overall change in physical conditions after a change factor of a predetermined biological state is added in a reference analysis time segment based on the degree of change in the fluctuation waveform as a physical condition change trend; and an analysis determination means B that estimates a physical condition state in a predetermined analysis period when a predetermined period has passed after a change factor of the predetermined biological state is added based on the degree of change of the fluctuation waveform as an analysis physical condition state.

The state estimation means preferably further includes a second analysis determination means that substitutes the positions on the coordinate system of the coordinate points in the target analysis time segment with trigonometric representations to plot the positions again in a new coordinate system and estimates the biological state based on the replotted positions of the coordinate points.

The second analysis determination means is preferably a means that creates trigonometric representation coordinates with respect to each of the respective coordinate points obtained by the analysis determination means A and B of the first analysis determination means, the trigonometric representation coordinates being plotted using an angle corresponding to the trigonometric representations of the coordinate points obtained by the analysis determination means A as one axis and an angle corresponding to the trigonometric representations of the coordinate points obtained by the analysis determination means B as the other axis, and the second analysis determination means preferably estimates the biological state based on the positions of the coordinate points of the trigonometric representation coordinates.

The second analysis determination means preferably includes:
a means that obtains a sine angle of each of the respective coordinate points obtained by the analysis determination means A and B of the first analysis determination means to create sine-representation coordinates plotted using the sine angle of the respective coordinate points obtained by the analysis determination means A as one axis and the sine angle of the respective coordinate points obtained by the analysis determination means B as the other axis; and a means that obtains a tangent angle of the respective coordinate points obtained by the analysis determination means A and B of the first analysis determination means to create tangent-representation coordinates plotted using the tangent angle of the respective coordinate points obtained by the analysis determination means A as one axis and the tangent angle of the respective coordinate points obtained by the analysis determination means B as the other axis, and the second analysis determination means preferably estimates the biological state based on the positions of the coordinate points of the sine-representation coordinates and the tangent-representation coordinates.

The state estimation means preferably further includes a sleep quality estimation means that estimates the quality of sleep as the function recovery means.

The state estimation means preferably further includes:
a physical condition map creation means that sequentially obtains coordinate points based on predetermined criteria using a difference between analysis periods which are different in respective analysis time segments to create a time-series change line indicating a time-series change in physical conditions in the analysis time segment; and a sensory map creation means that sequentially obtains coordinate points based on criteria different from those of the physical condition map creation means using a difference between analysis periods that are different in respective analysis time segments to create a time-series change line indicating a time-series change in senses in the analysis time segment, and the sleep quality estimation means preferably estimates the quality of sleep by taking a transition trend of the respective time-series change lines of the physical condition map creation means and the sensory map creation means.

A computer program of the present invention is a computer program set in a biological state estimation device that estimates a biological state using a biological signal of an autonomic nervous system, collected by a biological signal measuring means, the computer program causing a computer to execute:
a frequency analysis procedure that analyzes frequencies of the biological signal to obtain a fluctuation waveform in a ultra-low-frequency band of 0.001 Hz to 0.04 Hz; and a state estimation procedure that substitutes and displays the fluctuation waveform obtained by the frequency analysis procedure with index values regarding a sympathetic nerve and a parasympathetic nerve based on predetermined criteria to estimate the biological state based on a change with time in the index values.

According to the invention the state estimation procedure is a procedure that obtains the fluctuation waveform obtained by the frequency analysis means as coordinate points on a four-quadrant coordinate system in which respective indices regarding the sympathetic nerve and the parasympathetic nerve are illustrated on vertical and horizontal axes based on the predetermined criteria to display vectors and estimating the biological state based on a change with time of the coordinate points.

The state estimation procedure preferably includes a first analysis determination procedure that estimates whether the biological state is a normal fatigued state where fatigue accumulates due to activities, a slump state, or a function recovery state where a predetermined function recovery procedure is performed based on a position of a coordinate point in a target analysis time segment in relation to a coordinate point in a reference analysis time segment.

The first analysis determination procedure preferably includes at least one of:
an analysis determination procedure A that estimates a transition direction of an overall change in physical conditions after a change factor of a predetermined biological state is added in relation to a reference analysis time segment based on the degree of change in the fluctuation waveform as a physical condition change trend; and an analysis determination procedure B that estimates a physical condition state in a predetermined analysis period when a predetermined period has passed after a change factor of the predetermined biological state is added based on the degree of change of the fluctuation waveform as an analysis physical condition state.

The state estimation procedure preferably further includes a second analysis determination procedure that substitutes the positions on the coordinate system of the coordinate points in the target analysis time segment with trigonometric representations to plot the positions again in a new coordinate system and estimates the biological state based on the replotted positions of the coordinate points.

The state estimation procedure preferably further includes:
a physical condition map creation procedure that sequentially obtains coordinate points based on predetermined criteria using a difference between analysis periods which are different in respective analysis time segments to create a time-series change line indicating a time-series change in physical conditions in the analysis time segment; and a sensory map creation procedure that sequentially obtains coordinate points based on criteria different from those of the physical condition map creation procedure using a difference between analysis periods that are different in respective analysis time segments to create a time-series change line indicating a time-series change in senses in the analysis time segment, and the sleep quality estimation procedure preferably estimates the quality of sleep by taking a transition trend of the respective time-series change lines of the physical condition map creation procedure and the sensory map creation procedure.

### Effects of Invention

According to the present invention, a fluctuation waveform in an ultra-low-frequency band of 0.001 Hz to 0.04 Hz is obtained from a biological signal including reaction information of an autonomic nervous system index and an autonomic nervous system, collected from a biological signal measuring means, the fluctuation waveform is plotted as coordinate points on a four-quadrant coordinate system including an axis representing a sympathetic nerve function and an axis representing a parasympathetic nerve function controlled by a sympathetic nerve or a four-quadrant coordinate system including an axis representing a sympathetic nerve function and an axis representing a parasympathetic nerve function based on predetermined criteria, and a biological state is estimated based on a change with time of the coordinate points. According to the method of the present invention in which the fluctuation waveform is plotted as the coordinate points on the four-quadrant coordinate system based on predetermined criteria, the degree of predominance of the sympathetic nerve function or the parasympathetic nerve function and a change in the degree of fluctuation as the result of the control thereof, appearing as a change in the total sum of the two fluctuation waveforms in the ultra-low-frequency band can be detected in a magnified or highlighted manner.
Thus, the present invention is ideal for detecting the change in the state of a person. That is, the present invention is ideal for estimating whether the biological state is a normal fatigued state where fatigue accumulates due to activities, a slump state due to illness or the like, or a function recovery state realized by a predetermined function recovery means.

In particular, an alcohol intake state corresponding to a refresh state in drunkenness degree classification can be classified to a function recovery state resulting from an appropriate amount of alcohol intake. The alcohol intake state shows characteristics that a separation distance between the coordinate point in the reference analysis time segment and the coordinate point in the target analysis time segment falls within a predetermined range in the four-quadrant coordinates in which the fluctuation waveform in the ultra-low-frequency band is magnified and highlighted so as to be approximated to an sensory amount that is expressed by logarithmic axes and is close to a human's perception amount. Thus, the state estimation means can determine whether the biological state is an alcohol intake state corresponding to the refresh state in the drunkenness degree classification based on whether the position (separation distance) of the coordinate point is in the predetermined region. In this case, it is preferable for both a means that analyzes the physical condition change transition direction (physical condition change trend) and a means that analyzes an on-spot physical condition state (analysis physical condition state) during the analysis to estimate the alcohol intake state corresponding to the refresh state in the drunkenness degree classification when the position of the coordinate point is plotted in the predetermined region. Since a predetermined amount of alcohol intake causes a change in physical condition in a short period and the state after change continues for a certain amount of time, the use of these two indices enables the alcohol intake state corresponding to the refresh state in the drunkenness degree classification to be estimated more accurately.

The use of the means that estimates whether a change in the physical condition results from alcohol intake enables the biological signal collected during work from a long-distance truck driver or the like to be analyzed to detect when the driver has drunk during the work. Although this analysis is generally made after the driver returns to a management company, when the biological signal of the driver during work is transmitted to a management device of the management company, the management company can monitor the driver's state in real-time.

In the present invention, it is preferable that the biological state estimation device includes a frequency-gradient time-series analysis means that obtains a frequency-gradient time-series waveform from the biological signal and frequency analysis is performed using the frequency-gradient time-series waveform. A frequency fluctuation appearing as the result of the control of the autonomic nervous system and the fluctuation in homeostasis for controlling the frequency fluctuation generally do not show characteristics unless data of a long period (for example, 24 hours) is present. According to the present invention, this fluctuation can be estimated from the indices of the sympathetic nerve function, the indices in which the control of the sympathetic nerve function is superimposed on the parasympathetic nerve, or the indices of the parasympathetic nerve function separated from the sympathetic nerve function, collected from the measurement data in a short period as a fluctuation in the ultra-low-frequency band with the aid of the zero-cross detection means and the peak detection means from the biological signals of Surface Pulse Wave (APW).

### Brief Description of Drawings

Fig. 1 is a diagram illustrating a seat cushion-type biological signal measuring means used in an embodiment of the present invention.
Fig. 2 is a diagram illustrating a state where the biological signal measuring means is attached to a seat structure.
Fig. 3 is a central cross-sectional view of Fig. 2.
Fig. 4(a) is a partially notched view illustrating the structure of the biological signal measuring means attached to the seat structure, and Fig. 4(b) is a cross-sectional view along line A-A of Fig. 4(a).
Fig. 5 is an exploded perspective view of the biological signal measuring means.
Fig. 6 is the exploded perspective view of Fig. 5 when seen from the opposite side.
Fig. 7 is a diagram illustrating an arrangement of a pelvis and waist supporting member, a sensing mechanism unit, and a base cushion member.
Fig. 8 is an exploded perspective view of the sensing mechanism unit.
Fig. 9 is a diagram for describing the configuration of a biological signal estimation device according to an embodiment of the present invention.
Fig. 10 is a diagram for describing a method of obtaining a time-series waveform with the aid of a zero-cross detection means from an output signal obtained from the biological signal measuring means and a method of obtaining a time-series waveform with the aid of a peak detection means.
Figs. 11(a) to 11(d) are diagrams for describing regression lines created by a fluctuation waveform analying means and a method of obtaining a determination criterial score.
Fig. 12 is a diagram for describing an analysis method by an analysis determination means A of a state estimation means.
Fig. 13 is a diagram for describing an analysis method by the analysis determination means A of the state estimation means similarly to Fig. 12.
Fig. 14 is a diagram for describing an analysis method by the analysis determination means A of the state estimation means similarly to Figs. 12 and 13.
Fig. 15 is a diagram for describing an analysis method by an analysis determination means B of the state estimation means.
Fig. 16 is a diagram for describing an analysis method by the analysis determination means B of the state estimation means similarly to Fig. 15.
Fig. 17 is a diagram for describing an analysis method by the analysis determination means B of the state estimation means similarly to Figs. 15 and 16.
Fig. 18 is a diagram illustrating all analysis results obtained based on all conditions of Test Example 1 using the analysis determination means A on the same coordinates.
Fig. 19 is a diagram illustrating the analysis results of fatigue and non-medicinal drugs including alcohol among the analysis results of Test Example 1.
Fig. 20 is a diagram illustrating the state during drinking (alcohol intake) among the analysis results of Test Example 1.
Fig. 21 is a diagram illustrating the measurement results of a breath-alcohol concentration.
Fig. 22 is a diagram illustrating the state of taking non-medicinal products other than alcohol among the analysis results of Test Example 1.
Fig. 23 is a diagram illustrating the fatigued state among the analysis results of Test Example 1.
Fig. 24 is a diagram illustrating the state of bad physical condition among the analysis results of Test Example 1.
Fig. 25 is a diagram illustrating the analysis results obtained by the analysis determination means A analyzing biological signals of Subject K being in bad mental condition.
Fig. 26 is a diagram illustrating the analysis results of the state during drinking (alcohol intake) of Test Example 2 using the analysis determination means B.
Fig. 27 is a diagram illustrating the analysis results when taking non-medicinal drugs including alcohol in Test Example 2.
Fig. 28 is a diagram illustrating the analysis results during the fatigued state in Test Example 2.
Fig. 29 is a diagram illustrating the analysis results during the fatigued state in Test Example 2.
Fig. 30 is a diagram illustrating the analysis results obtained by the analysis determination means B analyzing biological signals of Subject K being in bad mental condition.
Figs. 31(a) and 31(b) are diagrams illustrating the analysis results obtained by the analysis determination means A and B in Test Example 3.
Figs. 32(a) and 32(b) are diagrams illustrating the analysis results of Subject A obtained by the analysis determination means A and B in Test Example 4.
Figs. 33(a) and 33(b) are diagrams illustrating the analysis results of Subject B obtained by the analysis determination means A and B in Test Example 4.
Figs. 34(a) and 34(b) are diagrams illustrating the analysis results of Subject C obtained by the analysis determination means A and B in Test Example 4.
Figs. 35(a) and 35(b) are diagrams illustrating the analysis results of Subject "Fujita Yoshito" obtained by the analysis determination means A and B in Test Example 5.
Figs. 36(a) and 36(b) are diagrams illustrating the analysis results of Subject YA obtained by the analysis determination means A and B in Test Example 5.
Fig. 37 is a diagram illustrating the analysis results of Subject HO obtained by the analysis determination means A and B in Test Example 5.
Fig. 38 is a diagram illustrating the analysis results of Subjects WA and SA as well as the subject of Fig. 37.
Figs. 39(a) and 39(b) are diagrams illustrating the analysis results of Subject KA suffering from depression, Subject HY suffering from diabetes, and Subject NI suffering from SAS (sleep apnea syndrome), obtained by the analysis determination means A and B.
Figs. 40(a) and 40(b) are diagrams illustrating the analysis results of Test Example 6.
Fig. 41 is a diagram illustrating the test results of Test Example 7.
Fig. 42 is a diagram illustrating representative examples of the FFT analysis results of original waveforms of aortic pulse waves (APW) in a wakeful state, a drowsy state, an imminent sleeping state, a sleeping state and after drinking (a period where an alcohol concentration is highest) and when taking a nutrient (when the nutrient starts working after taking the nutrient).
Fig. 43 is a diagram illustrating log-log graphs obtained by analyzing the frequencies of frequency-gradient time-series waveforms obtained by a zero-cross detection means processing the original APW waveforms used in Fig. 42.
Fig. 44 is a diagram illustrating log-log graphs obtained by analyzing the frequencies of frequency-gradient time-series waveforms obtained by a peak detection means processing the original APW waveforms used in Fig. 42.
Fig. 45 is a diagram illustrating analysis results obtained by the analysis determination means A (Method A) and the analysis determination means B (Method B) using the data illustrated in Figs. 43 and 44.
Fig. 46 is a diagram illustrating the coordinate points of respective subjects obtained from the measurement data of the states when the subjects were subjected to an automobile drive test after drinking Nutrient Drinks A to C among the test examples.
Fig. 47 is a diagram illustrating the coordinate points of respective subjects obtained from the data when the subjects took in alcohol among the test examples.
Fig. 48 is a diagram illustrating the coordinate points of respective subjects obtained from the sitting postures in a slump state (including in sickness) among the test examples.
Fig. 49 is a diagram illustrating the coordinate points obtained from the data of healthy and sick subjects in recumbent postures among the test examples.
Fig. 50 is a diagram for describing the configuration of a second analysis determination means.
Fig. 51 is a diagram illustrating the data of Subject JY and other subjects, obtained by the analysis determination means A (Method A) and the analysis determination means B (Method B).
Fig. 52 is a diagram illustrating sine-representation coordinates and tangent-representation coordinates of Fig. 51.
Fig. 53 is a diagram illustrating an example of a determination method.
Fig. 54 is a diagram illustrating breath-alcohol concentrations of respective subjects.
Fig. 55 is a diagram illustrating a zone showing a tendency that alcohol is absorbed quickly.
Fig. 56 is a diagram illustrating the zone in the coordinate systems of the analysis determination means A and B.
Fig. 57 is a diagram illustrating the sine-representation coordinates and the tangent-representation coordinates of all coordinate points plotted in Fig. 56.
Fig. 58 is a diagram illustrating an example of a determination method.
Fig. 59 is a diagram illustrating an analysis time segment.
Fig. 60 is a diagram illustrating the data obtained by the analysis determination means A (Method A) and the analysis determination means B (Method B) in all analysis time segments.
Fig. 61 is a diagram illustrating the sine-representation coordinates and the tangent-representation coordinates of all coordinate points plotted in Fig. 60.
Fig. 62 is a diagram illustrating the analysis results in each analysis time segment of the data obtained by the analysis determination means A (Method A) and the analysis determination means B (Method B).
Fig. 63 is a diagram illustrating the analysis results in each analysis time segments of the data obtained by the analysis determination means A (Method A) and the analysis determination means B (Method B).
Fig. 64 is a diagram illustrating analysis time segments.
Fig. 65 is a diagram illustrating breath-alcohol concentrations of respective subjects.
Fig. 66 is a diagram illustrating the data obtained by the analysis determination means A (Method A) and the analysis determination means B (Method B) in all analysis time segments.
Fig. 67 is a diagram illustrating the sine-representation coordinates and the tangent-representation coordinates of all coordinate points plotted in Fig. 66.
Fig. 68 is a diagram illustrating the analysis results in each analysis time segment, of the data obtained by the analysis determination means A (Method A) and the analysis determination means B (Method B).
Fig. 69 is a diagram illustrating the analysis results in each analysis time segment, of the data obtained by the analysis determination means A (Method A) and the analysis determination means B (Method B).
Fig. 70 is a diagram illustrating the data of respective subjects, obtained by the analysis determination means A (Method A) and the analysis determination means B (Method B).
Fig. 71 is a diagram illustrating the sine-representation coordinates and the tangent-representation coordinates of Fig. 70.
Fig. 72 is a diagram illustrating an example of a determination method.
Fig. 73 is a diagram illustrating the data and the like of Subject A in a second analysis time segment, obtained by the analysis determination means A and B.
Fig. 74 is a diagram illustrating the sine-representation coordinates and the tangent-representation coordinates of Fig. 73.
Fig. 75 is a diagram illustrating the data of Subject B in a second analysis time segment, obtained by the analysis determination means A and B.
Fig. 76 is a diagram illustrating the sine-representation coordinates and the tangent-representation coordinates of Fig. 75.
Fig. 77 is a diagram illustrating the data and the like of Subject C, obtained by the analysis determination means A and B.
Fig. 78 is a diagram illustrating the sine-representation coordinates and the tangent-representation coordinates of Fig. 77.
Fig. 79 is a diagram illustrating the processing results of the data of Subject JY.
Fig. 80 is a diagram illustrating the processing results of the data of Subject "Fujita Yoshito".
Fig. 81 is a diagram illustrating the processing results of the data of Subject YA.
Fig. 82 is a diagram illustrating the processing results of the data of Subject HO.
Fig. 83 is a diagram illustrating the processing results of the data of Subjects KA (depression), HY (diabetes), and NI (SAS (sleep apnea syndrome)).
Fig. 84 is a diagram illustrating analysis time segments.
Fig. 85 is a diagram illustrating the data in an outward trip, obtained by the analysis determination means A (Method A) and the analysis determination means B (Method B).
Fig. 86 is a diagram illustrating the sine-representation coordinates and the tangent-representation coordinates of the coordinate points plotted in Fig. 85.
Fig. 87 is a diagram illustrating the data in a return trip, obtained by the analysis determination means A (Method A) and the analysis determination means (Method B).
Fig. 88 is a diagram illustrating the sine-representation coordinates and the tangent-representation coordinates of the coordinate points plotted in Fig. 87.
Fig. 89 is a diagram illustrating the data in a return trip, obtained by the analysis determination means A (Method A) and the analysis determination means B (Method B).
Fig. 90 is a diagram illustrating the sine-representation coordinates and the tangent-representation coordinates of the coordinate points plotted in Fig. 89.
Fig. 91 is a diagram illustrating sleep polygraphs and HF and LF/HF time-series waveforms in Conditions 1 to 6 in "Sleep Quality Estimation 1" Test.
Fig. 92 is a diagram illustrating the sine-representation coordinates and the tangent-representation coordinates.
Fig. 93 is a diagram for describing computation conditions in nocturnal sleep when the analysis determination means B (Method B) is used.
Fig. 94 illustrates analysis results using the analysis determination means A in "Sleep Quality Estimation 2" Test.
Fig. 95 illustrates analysis results using the analysis determination means B in "Sleep Quality Estimation 2" Test.
Fig. 96 is a diagram illustrating the analysis results obtained by the sine-representation coordinates.
Fig. 97 is a diagram illustrating the analysis results obtained by the tangent-representation coordinates.
Fig. 98(a) is a diagram illustrating a physical condition map and a sensory map using the measurement results of 20111003 and a log-log graph of a power spectral density obtained by analyzing the frequencies of a frequency-gradient time-series waveform, and Fig. 98(b) is a diagram illustrating a physical condition map and a sensory map using the measurement results of 20111004 and a log-log graph of a power spectral density.
Fig. 99 is a diagram illustrating the log-log graphs and regression lines in an initial analysis time segment.
Fig. 100 is a diagram illustrating the log-log graphs and regression lines in the next analysis time segment.
Fig. 101 is a diagram illustrating the log-log graphs and regression lines in another next analysis time segment.
Fig. 102 is a diagram illustrating the log-log graphs and regression lines in another next analysis time segment.
Fig. 103 is a diagram illustrating representative examples of a log-log graph.
Fig. 104 is a diagram illustrating the analysis results of Subject MU in a wakeful state.
Fig. 105 is a diagram illustrating the sine-representation coordinates and the tangent-representation coordinates of Fig. 104.
Fig. 106 is a diagram illustrating the analysis results of Subject MU during daytime napping.
Fig. 107 is a diagram illustrating the sine-representation coordinates and the tangent-representation coordinates of Fig. 106.
Fig. 108 is a diagram illustrating the results of Subject MU in a wakeful state.
Fig. 109 is a diagram illustrating the results of Subject MU during daytime napping.
Fig. 110 is a diagram illustrating the results of Subject MU during nocturnal sleep.
Fig. 111 is a diagram illustrating the results of Subject KT during nocturnal sleep.
Fig. 112 is a diagram illustrating the analysis time segments of Test for Bed A.
Fig. 113 is a diagram illustrating a physical condition map and a sensory map of the analysis results of Bed A.
Fig. 114 is a diagram illustrating the analysis results of the analysis determination means A (Method A) and the analysis determination means B (Method B).
Fig. 115 is a diagram illustrating the sine-representation coordinates and the tangent-representation coordinates.
Fig. 116 is a diagram illustrating the measurement results obtained by an existing means that measures the state of a person during sleeping on Bed A.
Fig. 117 is a diagram illustrating the measurement results obtained by an existing means that measures the state of a person during sleeping on Bed A.
Fig. 118 is a diagram illustrating the analysis time segment of Test for Bed B.
Fig. 119 is a diagram illustrating a physical condition map and a sensory map of the analysis results of Bed B.
Fig. 120 is a diagram illustrating the analysis results of the analysis determination means A (Method A) and the analysis determination means B (Method B).
Fig. 121 is a diagram illustrating the sine-representation coordinates and the tangent-representation coordinates.
Fig. 122 is a diagram illustrating the measurement results obtained by an existing means that measures the state of a person during sleeping on Bed B.
Fig. 123 is a diagram illustrating the measurement results obtained by an existing means that measures the state of a person during sleeping on Bed B.
Fig. 124 is a diagram illustrating the measurement results obtained by an existing means that measures the state of a person during sleeping on Bed B.
Fig. 125 is a diagram illustrating the measurement results obtained by an existing means that measures the state of a person during sleeping on Bed B.
Fig. 126 is a diagram illustrating the analysis time segment of Test for Bed C.
Fig. 127 is a diagram illustrating a physical condition map and a sensory map of the analysis results of Bed C.
Fig. 128 is a diagram illustrating the analysis results of the analysis determination means A (Method A) and the analysis determination means B (Method B).
Fig. 129 is a diagram illustrating the sine-representation coordinates and the tangent-representation coordinates.
Fig. 130 is a diagram illustrating the measurement results obtained by an existing means that measures the state of a person during sleeping on Bed B.
Fig. 131 is a diagram illustrating the measurement results obtained by an existing means that measures the state of a person during sleeping on Bed B.
Fig. 132 is a diagram illustrating the measurement results obtained by an existing means that measures the state of a person during sleeping on Bed B.
Fig. 133 is a diagram illustrating the measurement results obtained by an existing means that measures the state of a person during sleeping on Bed B.
Fig. 134 is a diagram illustrating a breath-alcohol concentration.
Fig. 135 is a diagram illustrating the analysis results before and after drinking.
Fig. 136 is a diagram illustrating the analysis results when a person has performed a task in a sitting posture.
Fig. 137 is a diagram illustrating the analysis results when a person has drunk Nutrient Drink A.
Fig. 138 is a diagram illustrating the HF and LF/HF results obtained from finger plethysmograms.
Fig. 139 is a diagram illustrating a peak-frequency time-series waveform, a 0x-frequency time-series waveform, and a peak/0x-frequency time-series waveform of the data of Subject "Uchikawa".
Fig. 140 is a diagram illustrating frequency-gradient time-series waveforms obtained from the frequency time-series waveforms of Fig. 139.
Fig. 141 is a diagram illustrating analysis time segments used for analyzing 0x-frequency-gradient time-series waveforms and peak/Ox-frequency-gradient time-series waveforms.
Fig. 142 is a diagram illustrating the fluctuation waveforms of log-log graphs in respective analysis time segments of peak/Ox-frequency-gradient time-series waveforms.
Fig. 143 is a diagram illustrating the fluctuation waveforms of log-log graphs in respective analysis time segments of peak/Ox-frequency-gradient time-series waveforms.
Fig. 144 is a diagram illustrating the fluctuation waveforms of log-log graphs in respective analysis time segments of peak/Ox-frequency-gradient time-series waveforms.
Fig. 145 is a diagram illustrating the fluctuation waveforms of log-log graphs in respective analysis time segments of peak/Ox-frequency-gradient time-series waveforms.
Fig. 146 is a diagram illustrating the analysis results obtained by the analysis determination means A (Method A) and the analysis determination means B (Method B).
Fig. 147 is a diagram illustrating coordinate points plotted among the coordinate points of Fig. 146 using the determination criterial score of the peak/Ox-frequency-gradient time-series waveforms as a vertical axis.
Fig. 148 is a diagram illustrating the display results in respective analysis time segments of the data obtained by the analysis determination means A (Method A) and the analysis determination means B (Method B) illustrated in Fig. 146.
Fig. 149 is a diagram illustrating the display results in respective analysis time segments of the data obtained by the analysis determination means A (Method A) and the analysis determination means B (Method B) illustrated in Fig. 146.
Figs. 150(i) to 150(iv) are diagrams illustrating comparison between a verification finger plethysmogram and a surface pulse wave (APW) obtained by removing noise components of an original waveform of an output signal obtained from a biological signal measuring means and then emphasizing and filtering the fluctuation components in respective beats.
Fig. 151 is a diagram illustrating comparison between a surface pulse wave and a heart rate waveform calculated from the finger plethysmogram.
Fig. 152 is a diagram illustrating APW and electrocardiogram (ECG) of a subject.
Fig. 153 is a diagram illustrating the active levels of sympathetic nerves and parasympathetic nerves during a sleep induction test, obtained from a finger plethysmogram of a male subject in 20's.
Figs. 154A to 154D are diagrams illustrating log-log graphs of the frequency analysis results for ten minutes of an original APW wave and the frequency analysis results of a time-series waveform obtained by a zero-cross detection method and a peak detection method in respective states (wakeful, drowsy, imminent sleeping, and sleeping states).
Fig. 155 is a diagram illustrating an example of a scoring rule in the analysis using a frequency fluctuation computation means.
Fig. 156 is a diagram illustrating the scores obtained based on the rule of Fig. 155 in a time-series order (in the order of analysis time segments).
Fig. 157 is a diagram illustrating transitions of the balance of the autonomic nervous system, obtained by calculating a difference in scores between adjacent analysis time segments when the score of an initial analysis time segment is "0".
Fig. 158 illustrates four-quadrant coordinates in which the score calculated by a zero-cross detection means is on the horizontal axis and the score calculated by a peak detection means is on the vertical axis.

### Description of Embodiments

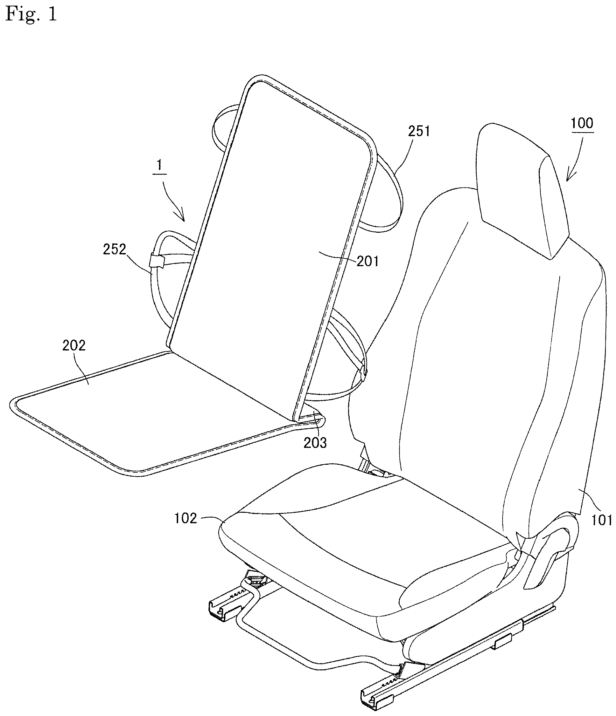

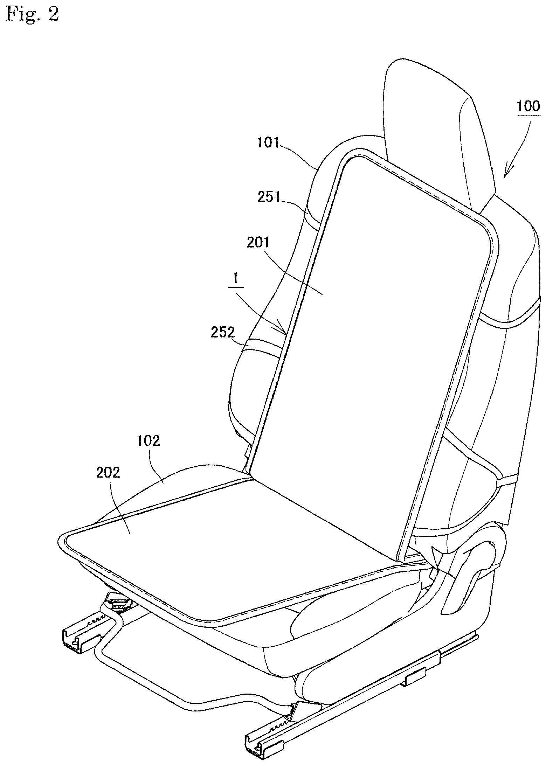

Hereinafter, the present invention will be described in further detail based on the embodiments of the present invention illustrated in the drawings. Figs. 1 and 2 are diagrams illustrating a biological signal measuring means 1 that collects a surface pulse wave (in this example, an aortic pulse wave (APW)) which is a biological signal to be analyzed by a biological state estimation device 60 according to the present embodiment. The aortic pulse wave is a pressure vibration generated from a movement of the heart and the aorta, detected from the back of the upper body of a person and carries information on the systolic and diastolic phases of the ventricles and elasticity information of the vascular wall which serves as an auxiliary pump of circulation. Moreover, a signal waveform caused by a heart rate fluctuation includes nervous activity information of the sympathetic nervous system and the parasympathetic nervous system (activity information of the parasympathetic nervous system including the compensatory mechanism of the sympathetic nerve), and a signal waveform caused by pulsation of the aorta includes activity information of sympathetic nerves. The analysis of APW enables to collect information on the movement of the aorta, the movement of the heart, and the activity of the autonomic nerves based on these movements, which will be described in detail later.

The biological signal measuring means 1 of the present embodiment is a seat cushion-type biological signal measuring means mounted to be superimposed on a seat structure 100 which is a human support mechanism. The seat cushion-type biological signal measuring means 1 of the present embodiment includes a back support cushion member 201 and a seat support cushion member 202, and a protruding piece 203 is formed at the boundary between the back support cushion member 201 and the seat support cushion member 202 so as to protrude backward. The protruding piece 203 is inserted in the gap between a seatback portion 101 and a seat cushion portion 102 of the seat structure 100, and the back support cushion member 201 is pulled and stretched to a back support portion (the seatback portion 101) of a seat structure by a stretching means described later.

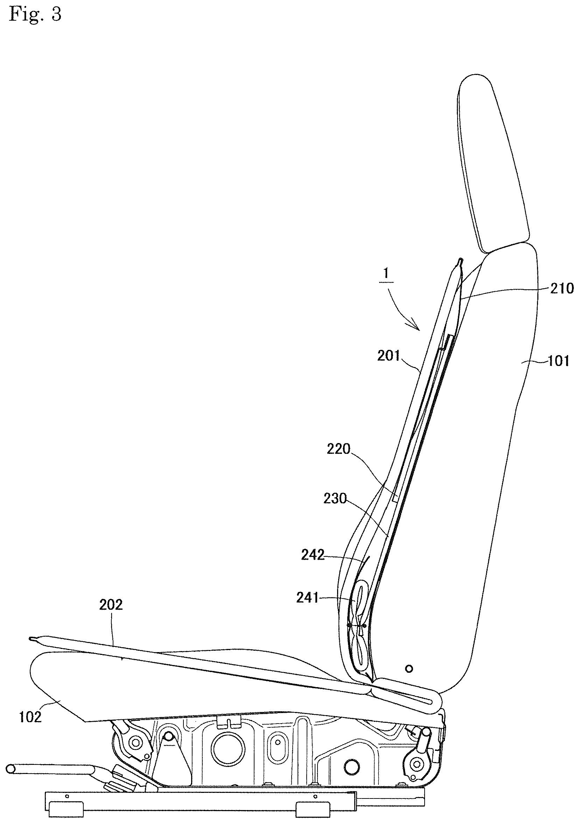

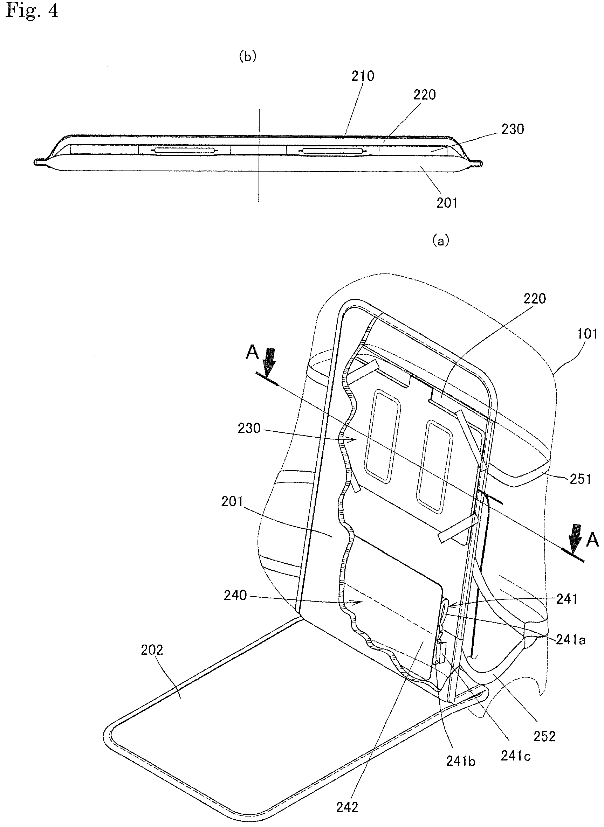

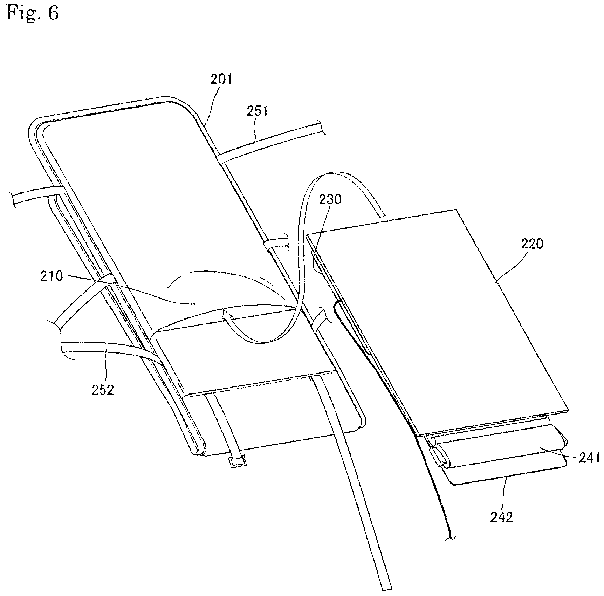

As illustrated in Figs. 3 to 6, a sensing mechanism unit 230 and a base cushion member 220 are arranged on the rear side of the back support cushion member 201. Specifically, both lateral portions of a bag-shaped member 210 formed of a cloth member are bonded to the peripheral portions of the back support cushion member 201, and the base cushion member 220 and the sensing mechanism unit 230 are inserted inside the bag-shaped member 210. Thus, the base cushion member 220 and the sensing mechanism unit 230 are not fixed to the back support cushion member 210 but are configured to be displaced in an up-down direction in the bag-shaped member 210.

The back support cushion member 210 and the base cushion member 220 are preferably formed of a 3-dimensional solid knitted member that is highly rigid in a tension direction. The 3-dimensional solid knitted member is a knitted fabric having a solid three-dimensional structure having a pair of ground knitted fabrics arranged to be spaced from each other and a large number of connecting strands reciprocating between the pair of ground knitted fabrics to connect both ground knitted fabrics as disclosed in Japanese Patent Application Publication No. 2002-331603 and Japanese Patent Application Publication No. 2003-182427, for example. The 3-dimensional solid knitted member has such a spring constant obtained from a load-deflection characteristic when the knitted member is stretched with an elongation ratio of 0% and is pressed approximately vertically to a planar direction that a spring constant obtained from a load-deflection characteristic when pressed by a pressure plate having a diameter of 98 mm is higher than a spring constant obtained from a load-deflection characteristic when pressed by a pressure plate having a diameter of 30 mm. With this configuration, the 3-dimensional solid knitted member has the same characteristics as the load characteristics of the muscle of a human and can increase the sense of fit and improve posture supporting properties.

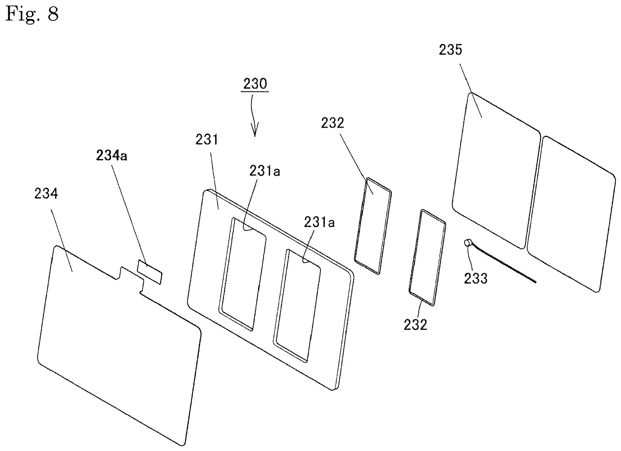

The sensing mechanism unit 230 includes a core pad 231, spacer pads 232, a sensor 233, a front film 234, and a rear film 235 as illustrated in Fig. 8.

The core pad 231 is formed in a planar form and has two vertically long through-holes 231a formed at symmetrical positions with a portion corresponding to the spinal column interposed. The core pad 231 is preferably formed of foam beads that is formed in a planar form. When the core pad 231 is formed of foam beads, the core pad 231 preferably has a thickness that is equal to or smaller than an average diameter of the beads with an expansion ratio being in the range of 25 and 50. For example, when the average diameter of beads having an expansion ratio of 30 is approximately 4 to 6 mm, the core pad 231 is sliced to a thickness of approximately 3 to 5 mm. With this configuration, the core pad 231 is given soft elasticity, emphasizes a fluctuation component (high-frequency component around 20 Hz) in respective beats of APW to detect the same as resonant solid vibration to thereby extract a frequency band of the heart rate component near 1 Hz to 3 Hz.

The spacer pads 232 are filled in the through-holes 231a of the core pad 231. The spacer pads 232 are preferably formed of a 3-dimensional solid knitted member. When the 3-dimensional solid knitted member is pressed by the back of a person, the connecting strands of the 3-dimensional solid knitted member are compressed, and a tension is generated in the connecting strands, whereby vibration of the body surface caused by the biological signal is transmitted via the muscle of a person. Moreover, the spacer pads 232 formed of a 3-dimensional solid knitted member is preferably thicker than the core pad 231. With this configuration, when the peripheral portions of the front film 234 and the rear film 235 are attached to the peripheral portions of the through-holes 231a, since the spacer pads 232 formed of a 3-dimensional solid knitted member are pressed in a thickness direction, tension is generated by the reactive force of the front film 234 and the rear film 235 and solid vibration (membrane vibration) is likely to be generated in the front film 234 and the rear film 235. On the other hand, auxiliary compression is generated in the spacer pads 232 formed of a 3-dimensional solid knitted member, and tension caused by the reactive force is generated in the connecting strands holding the form of the 3-dimensional solid knitted member in the thickness direction is generated, whereby string vibration is likely to be generated. A hook-and-loop fastener 234a is attached to the upper portion of the front film 234 and is attached to a hook-and-loop fastener 220a attached to the upper portion of the base cushion member 220, whereby the sensing mechanism unit 230 is held on the base cushion member 220. Moreover, the four corners of the sensing mechanism unit 230 is held on the base cushion member 220 by a tape member 230a.

The sensor 233 is fixed to any one of the spacer pads 232 before the front film 234 and the rear film 235 described above are stacked. Although the 3-dimensional solid knitted member that forms the spacer pad 232 includes a pair of ground knitted fabrics and connecting strands, since the string vibration of the connecting strands is transmitted to the front film 234 and the rear film 235 via the node points between the connecting strands and the ground knitted fabrics, the sensor 233 is preferably attached to a surface (the surface of the ground knitted fabric) of the spacer pad 232. A microphone sensor (especially, a capacitive microphone sensor) is preferably used as the sensor 233.

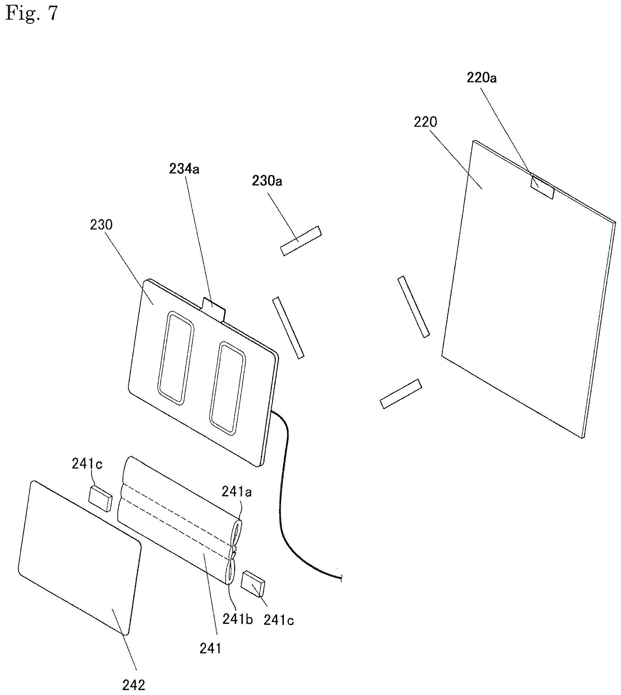

A pelvis and waist supporting member 240 is arranged on a rear surface side of the back support cushion member 201 below the sensing mechanism unit 230. As illustrated in Fig. 7, the pelvis and waist supporting member 240 includes a biasing member 241 in which the upper and lower edges of the 3-dimensional solid knitted member are folded inward and the central portion is sewn to form swelling portions 241a and 241b on the upper and lower sides and a flexible planar member (or a planar member formed of hard felt) 242 formed of a synthetic resin, which is formed in an approximately rectangular form having an area covering the entire surface of the upper and lower swelling portions 241a and 241b and which is bent to exhibit elasticity. The biasing member 241 is folded and sewn to form the swelling portions 241a and 241b on both sides to thereby increase the elasticity and the supporting pressure and generate the sense of stroke. The flexible planar member 242 covers the entire surface of the biasing member 241 to diminish the sense of contact of the biasing member 241. Thus, the pelvis and waist supporting member 240 of the present embodiment can exhibit high supporting pressure in the pelvis and waist supporting region with a simple configuration.

In the present embodiment, an urethane foam 241c is inserted in the inner space of the lower swelling portion 241b. The base cushion member 220 has a lower edge having such a size that the lower edge covers the upper swelling portion 241a, and the lower swelling portion 241b and the urethane foam 241c are not covered with the base cushion member 220. Due to this, when load is applied, the lower swelling portion 241b and the urethane foam 241c perform the role of the starting point when the flexible planar member 242 is bent to apply force that supports a region extending from the pelvis to the waist of a person in an obliquely upward direction.

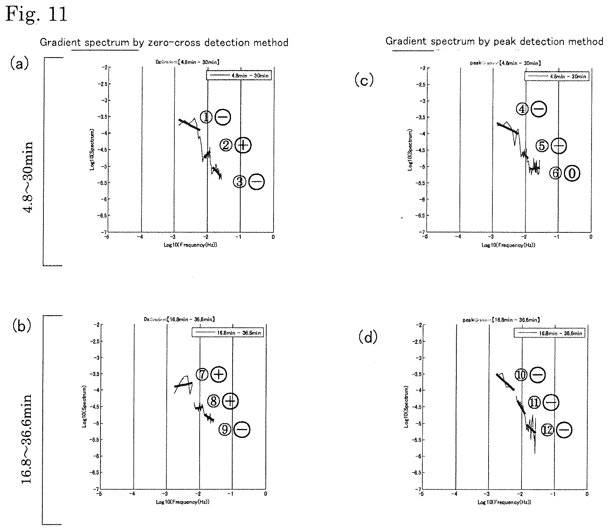

Here, the pelvis and waist supporting region is a region in which predetermined supporting pressure is applied to the region extending from the pelvis to the waist of a person by the elasticity of the pelvis and waist supporting member 240 and the tension of the back support cushion member 201. In Test Example 1 described later, the position of the pelvis and waist supporting region is set such that the pelvis and waist supporting region extends 350 mm upward from the seating surface of the seat support cushion member 202, a range 100 mm above the region is a middle region, and a region above the middle region is a scapula supporting region (see Fig. 11). In the present embodiment, when the seat cushion biological signal measuring means 1 is set on the seat structure 100, a person sits on the seat structure 100 in a static state, and body pressure distributions on the back support cushion member 201 are measured, a load sharing ratio to the entire load of the sitting person applied to the back support cushion member 201 is preferably set to 50% or higher in the pelvis and waist supporting region. More preferably, the load sharing ratio of the middle region to the entire load of the sitting person applied to the back support cushion member 201 is set to 20% or lower, and the load sharing ratio of the pelvis and waist supporting region and the scapula supporting region is set to 80% or higher. Further preferably, the load sharing ratio of the middle region is 10% or lower, and the load sharing ratio of the pelvis and waist supporting region and the scapula supporting region is 90% or higher. The load sharing ratio can be set to be in the above-described range by adjusting the thickness and material of the flexible planar member 242 such as the thickness of the 3-dimensional solid knitted member that forms the biasing member 241 of the pelvis and waist supporting member 240 and the size of the swelling portions 241a and 241b to thereby adjust the elasticity of the pelvis and waist supporting member 240.

The sensing mechanism unit 230 is arranged so that the position of the sensor 233 is in the range of the middle region and the sensing mechanism unit 230 is spaced by a predetermined distance from the upper edge of the pelvis and waist supporting member 240 when seen from the front side. This is to prevent the movement of the pelvis and waist supporting member 240 from affecting the sensing mechanism unit 230, and the separation distance is set to 10 mm or larger and preferably 30 mm or larger, and more preferably 50 mm or larger.

The pelvis and waist supporting member 240 supports the portion near the pelvis and the waist of a person, and in this case, preferably presses the portion in an obliquely upward direction as described above. Thus, the pelvis and waist supporting member 240 is preferably attached so that a line extending along the front surface of the flexible planar member 242 is separated from an outer line of the back of a supporting target person as the line advances toward the upper edge and that the angle between the line extending along the front surface and the outer line of the back of a supporting target person is in the range of 5 degrees to 45degrees. More preferably, the angle between the line extending along the front surface and the outer line of the back of a supporting target person is in the range of 5 degrees to 20 degrees.

The biological signal measuring means 1 of the present embodiment has a stretching means and is stretched by attaching the stretching means to the seatback portion 101 of the seat structure 100. As the stretching means that stretches the back support cushion member 201 to the seatback portion 101, a structure which can be pulled out from the peripheral portions and which includes a first belt member 251 provided on both sides of the scapula supporting region and a second belt member 252 provided on both sides of the pelvis and waist supporting region can be used. When the first and second belt members 251 and 252 surround the seatback portion 101 and is fixed with the length adjusted, the back support cushion member 201 is arranged as a tension structure. Moreover, the protruding piece 203 at the boundary between the back support cushion member 201 and the seat support cushion member 202 is inserted and sandwiched between the seatback portion 101 and the seat cushion portion 102.

With this arrangement, the load applied to the pelvis and waist supporting region in which the pelvis and waist supporting member 240 is arranged is relatively high and the load applied to the middle region is relatively low. That is, according to the present embodiment, even when the general seat structure 100 which uses an urethane material is used as a cushion member is used, by arranging the seat cushion biological signal measuring means 1, it is possible to easily create a structure in which the supporting load of the pelvis and waist supporting region of the back support cushion member 201 is relatively high and the supporting load of the middle region is relatively low. This supporting state induces a relaxed state of the anti-gravity muscle for maintaining the posture of the upper body higher than the waist. Thus, by arranging the sensor 233 in the middle region of the back support cushion member 201, it is possible to detect the biological signal with high sensitivity. Moreover, in the present embodiment, the sensing mechanism unit 230 is disposed between the back support cushion member 201 and the base cushion member 220 to create a three-layer structure which includes the back support cushion member 201, the sensing mechanism unit 230, and the base cushion member 220. Moreover, since the sensing mechanism unit 230 is disposed in the bag-shaped member 210, the sensing mechanism unit 230 and the base cushion member 220 can be displaced in the up-down direction. Thus, vibration transmitted from the seat structure 100 is removed by the base cushion member 220 and the displacement thereof. Further, since the sensing mechanism unit 230 is spaced by a predetermined distance from the pelvis and waist supporting member 240, the sensing mechanism unit 230 is rarely affected by external vibration. In particular, in the present embodiment, although the seat cushion biological signal measuring means collects the surface pulse wave (a biological signal (aortic pulse wave (APW)) generated by pulsation of the atrium, the ventricle, and the aorta) from the back of a person, since the seat cushion biological signal measuring means has the above-described configuration, it is possible to suppress the influence of other vibration (external vibration, body motion components, and the like) close to the frequency component of the APW and to detect the APW accurately.

The seat cushion biological signal measuring means 1 according to the embodiment can detect the biological signal (in particular, APW) more accurately regardless of the type of the target seat structure 100 (that is, whether an urethane material is used as a cushion member). The seat structure itself may be used as a biological signal detection mechanism which is ideal for collection of biological signals.

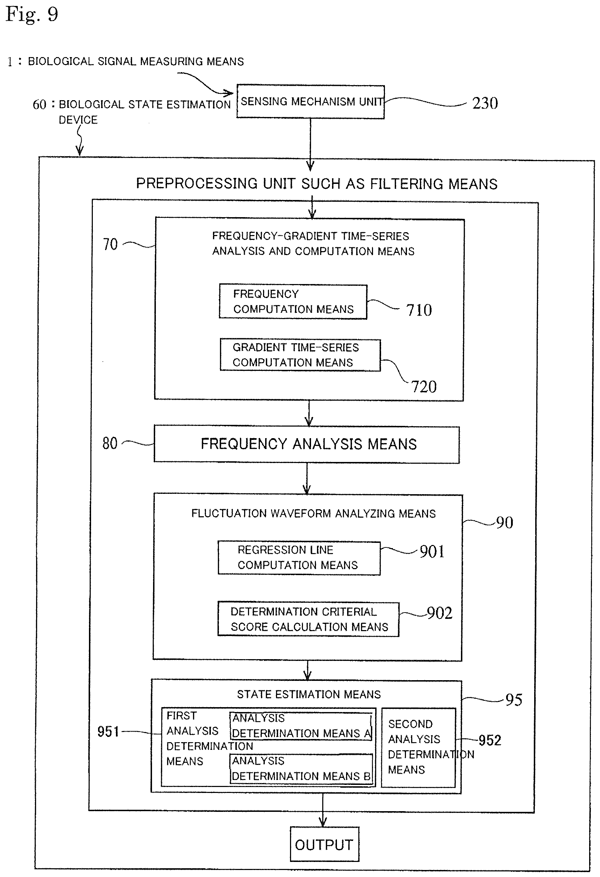

Next, the configuration of the biological state estimation device 60 will be described based on Fig. 9. The biological state estimation device 60 includes a frequency-gradient time-series analysis and computation means 70, a frequency analysis means 80, a fluctuation wave form analyzing means 90, and a state estimation means 95. The biological state estimation device 60 is configured as a computer, in which the frequency-gradient time-series analysis means 70 executes frequency-gradient time-series procedures, the frequency analysis means 80 executes frequency analysis procedures, the fluctuation waveform analyzing means 90 executes fluctuation waveform analysis procedures, and the state estimation means 95 executes state estimation procedures. A computer program may be provided in a state of being stored in a storage medium such as a flexible disk, a hard disk, a CD-ROM, a magneto-optical (MO) disk, a DVD-ROM, and a memory card and may be transmitted via a transmission line.

The frequency-gradient time-series analysis and computation means 70 that obtains frequency-gradient time-series waveforms includes a frequency computation means 710 and a gradient time-series computation means 720. The frequency computation means 710 obtains a frequency time-series waveform from an original waveform (preferably, filtered time-series data of a predetermined frequency region (for example, frequency components resulting from a body motion or the like are removed)) of the output signal obtained from the sensing mechanism unit 230 of the biological signal measuring means 1.

The frequency computation means 710 employs two methods: a method (hereinafter referred to as a "zero-cross detection means") of obtaining frequency time-series waveforms using switching points (hereinafter referred to as "zero-cross points") at which the positive and negative signs of the original waveform change and a method (hereinafter referred to as a "peak detection means") of smoothing and differentiating the original waveform to obtain time-series waveforms using maximum values (peak points).

Here, since it is believed that APW shows the systolic phase (intracardiac pressure) and the diastolic phase (intra-arterial pressure) of the heart (that is, the pulse pressure (the difference between the diastolic phase and the systolic phase)) and the pulse pressure decreases with sleep, it may be possible to estimate information on sleep and drowsiness from the 1st component and the 0.5th component obtained by analyzing the frequencies of APW. A portion corresponding to the T wave of an electrocardiogram is the 0.5th component and corresponds to the notch referred in a finger plethysmogram. The peak detection means detects the 1st component and the 0.5th component by analyzing the frequencies of APW and the zero-cross detection means detects points close to the 0.5th component. Thus, when the peak detection means and the zero-cross detection means are used, the peak detection means detects data corresponding to both the diastolic phase and the systolic phase, which is information on the behavior of both the heat and the aorta, and the zero-cross detection means detects data corresponding to the diastolic phase which is information on the behavior of the aorta. When APW and electrocardiogram (ECG) are compared, which will be described in detail later, the notch positions of APW are substantially identical to the T wave of ECG appearing in an ejection period where the semilunar valve of the heart is closed and the cardiac output stops. Thus, the zero-cross detection means collects the data of the diastolic phase of vessels and the peak detection means collects the data of both the diastolic phase and the systolic phase. That is, the zero-cross detection means detects the function of the sympathetic nervous system from the data of the elasticity of the aorta itself. The peak detection means detects the movements of both the aorta and the heart (that is, the function of the parasympathetic nervous system and the sympathetic nervous system).

Thus, it is possible to cancel information on a control state of the sympathetic nerve by looking at the difference (the difference obtained by subtraction and division) between them and to obtain information on the behavior when the sympathetic compensatory mechanism does not appear (that is, the control state of the parasympathetic nerve). Since the behavior of the aorta can be detected by the zero-cross detection means, it is possible to detect the control state of the sympathetic nerve. Moreover, it is possible to detect the behavior of the parasympathetic nerve in which the compensatory mechanism of the sympathetic nerve is added with the aid of the peak detection means. Further, it is possible to detect the behavior of the parasympathetic nerve by detecting the difference between the time-series waveforms of the frequency fluctuations detected by the peak detection means and the zero-cross detection means. Further, since the 1st component and the 0.5th component can be calculated with quick measurement, it is also possible to detect an ultra-low-frequency component by applying gradient time-series analysis to the time-series waveforms obtained from these components.

In other words, APW is a biological signal (a biological signal including the autonomic nervous system index and the composite reaction information of the sympathetic and parasympathetic nervous systems) including information on both the control state of the peripheral nervous system and the control state of the aorta similarly to the finger plethysmogram. A waveform obtained by extracting the absolute values of the gradient time-series waveforms of the biological signal obtained by the zero-cross detection means reflects an emergence state of the sympathetic nervous system. The peak detection means detects the emergence state of both sympathetic and parasympathetic nervous systems (that is, the behavior of the parasympathetic nervous system in which the compensatory mechanism of the sympathetic nerves is added). The waveform obtained by the peak detection means taking the absolute values of the gradient time-series waveform is relatively approximate to the behavior (in which the sympathetic compensatory mechanism is added) of the parasympathetic nerve obtained by the wavelet analysis of the finger plethysmogram. Thus, the zero-cross detection means can be used for indices indicating stress adaptation realized by the control of the autonomic nervous system and the physical condition which is the result of the control. On the other hand, the aortic behavior component of the frequency fluctuation time-series waveform obtained by the peak detection means, mainly associated with a frequency fluctuation of the heart rate and a fluctuation waveform obtained by analyzing the frequencies of the gradient time-series waveform, obtained by the zero-cross detection means, associated with the sympathetic nerves can be used as a waveform that is associated with feelings (pleasant and unpleasant feelings) such as excitement and sedation or satisfaction and dissatisfaction resulting from the feeling of comfort or discomfort.

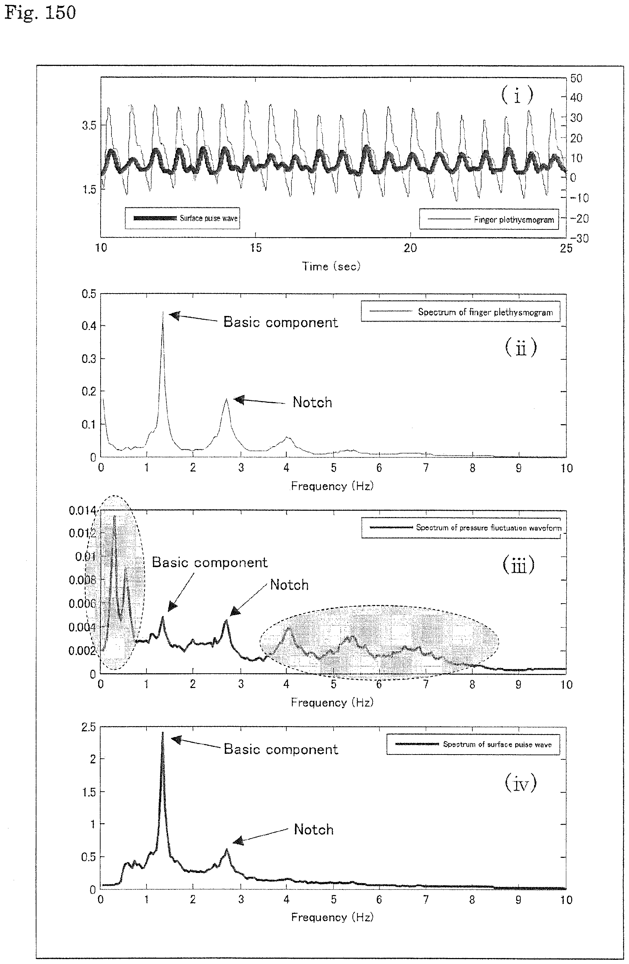

The relation between the APW and the finger plethysmogram and the relation between the APW and the autonomic nervous system will be described in further detail in Test Examples of Figs. 150 to 154.

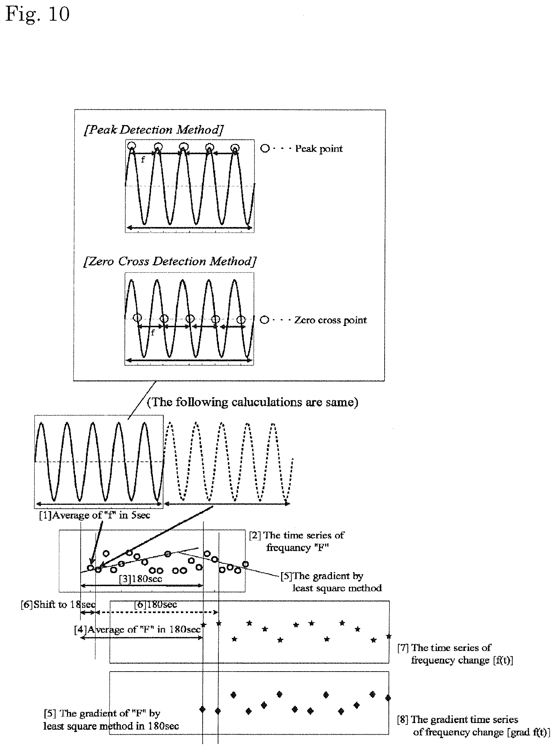

First, when zero-cross points are obtained, the zero-cross detection means (zero-cross procedure) divides the zero-cross points every five second, for example, obtains the reciprocals of the time intervals between the zero-cross points of a time-series waveform included in the five seconds of period as individual frequencies f, and employs the average value of the individual frequencies f in the five seconds of period as the value of the frequency F in the five seconds of period (step [1] of Fig. 10). The frequencies F obtained every five second are plotted to obtain a time-series waveform of frequencies (step [2] of Fig. 10). The peak detection means (peak detection procedure) obtains maximum values according to the Savitzky-Golay smoothing and differentiation method, for example. Subsequently, the maximum values in every five seconds are divided, for example, to obtain the reciprocals of the time intervals between the peak points (peak-side apexes of a waveform) which are the maximum values of the time-series waveform included in the five seconds of period as individual frequencies f, and the average value of the individual frequencies f in the five seconds of period is employed as the value of the frequency F in the five seconds of period (step [1] of Fig. 10). Then, the frequencies F obtained every five second are plotted to obtain a time-series waveform of frequencies (step [2] of Fig. 10).

The gradient time-series computation means 720 is configured such that the frequency computation means 710 sets a time window having a predetermined time width from the time-series waveform (APW) of frequencies of the output signal of the sensor of the biological signal measuring means 1 obtained using the zero-cross detection means or the peak detection means, obtains the gradient of the frequencies of the output signal of the sensor in respective time windows according to the least-square method, and outputs the time-series waveform of the gradient. Specifically, first, the gradient of frequencies in a certain time window Tw1 is obtained and plotted according to the least-square method (steps [3] and [5] of Fig. 10). Subsequently, the next time window Tw2 is set with an overlap period Tl (step [6] of Fig. 10), and the gradient of frequencies in this time window Tw2 is obtained and plotted according to the leat-square method in the same manner. This calculation (slide-calculation) is sequentially repeated and a time-series change of the gradients of frequencies of the output signal is output as a frequency-gradient time-series waveform (step [8] of Fig. 10). The time width of the time window Tw is preferably set to 180 seconds, and the overlap period T1 is preferably set to 162 seconds. These values are selected as values at which a characteristic signal waveform appears with highest sensitivity when sleep tests were conducted while changing the time width of the time window Tw and the overlap period T1 as described in Patent Literature 3 (WO2005/092193A1) filed by the present applicant.

The frequency analysis means 80 is a means that analyzes the frequencies of the frequency-gradient time-series waveform obtained from the frequency-gradient time-series analysis and computation means 70 and outputs the frequency analysis results as a log-log graph of which the horizontal axis represents the frequency and the vertical axis represents a power spectral density.

The fluctuation waveform analyzing means 90 is a means that performs analysis for plotting the fluctuation waveform of power spectra which are the frequency analysis results of the frequency analysis means 80 on a four-quadrant coordinate system based on predetermined criterion and includes a regression line computation means 901 and a determination criterial score calculation means 902.

The regression line computation means 901 is a means that obtains regression lines in respective predetermined cyclic regions (frequency ranges) of the analysis waveform (fluctuation waveform) output by the frequency analysis means 80.

In the predetermined frequency region used in the regression line computation means 901, a fluctuation that maintains the homeostasis of a human is present in an ultra-low-frequency band (VLF region) of 0.001 to 0.04 Hz.
Among these frequencies, a frequency band of 0.001 to 0.006 Hz (in particular, 0.001 to 0.0053 Hz) contains information indicating a macroscopic regulatory function (that is, a generally significant trend). A frequency band of 0.006 to 0.04 Hz contains information on a microscopic regulatory function (that is, a local fluctuation within the entire fluctuation) and is associated with a stress adaptive state like the reaction of a barrier in relation to the peripheral nervous system and a pleasant and unpleasant state. Among these frequencies, the influence of a local fluctuation is significant in a frequency band of 0.01 to 0.04 Hz. A so-called heart rate disturbance occurring in 0.3 to 2 minutes appears as irregular vibration. The sleep apnea syndrome (SAS) appears remarkable in the frequency near 0.01 Hz, which is an example. Thus, in the present embodiment, the cyclic region is divided into three regions of a long-cyclic region (low-frequency band) of 0.001 Hz to 0.006 Hz, a mid-cyclic region (mid-frequency band) of 0.006 Hz to 0.015 Hz, and a short-cyclic region (high-frequency band) of 0.015 Hz to 0.04 Hz. Thus, the mid-cyclic region and the short-cyclic region includes the frequency band of 0.01 to 0.04 Hz, and changes in these cyclic regions are associated with events which result in changes in the physical condition of a person (for example, whether the person has taken alcohol, whether the person has taken other drug components, and whether the person is in a sick state), and are ideally used for specifying the state of a person.

The regression line computation means 901 obtains regression lines in the respective cyclic regions according to the least-square method using the central frequencies as median values.

The determination criterial score calculation means 902 assigns a regional score to the respective regression lines obtained in the respective cyclic regions by the regression line computation means 901 based on the gradient thereof, obtains a shape score of the entire regression lines, and calculates a determination criterial score for estimating a biological state using at least one of the regional score and the shape score.

The regional score is the score corresponding to the gradient of each regression line. The score is assigned such that the gradient of each regression line is classified into three categories of an approximately horizontal state, an upward gradient state, and a downward gradient state. The upward gradient state is a state where the control of the autonomic nervous system accelerates, and the downward gradient state is a wakeful state where the control of the autonomic nervous system is stable or a sleeping state. Thus, the scores assigned to the upward and downward gradient states are changed using the approximately horizontal state as a reference. The gradient of the regression line may be determined as the approximately horizontal state, for example, if the gradient falls within the range of ±10 degrees with respect to the horizontal. The approximately horizontal state is considered to be a chaos state where the direction of the control of the autonomic nervous system is not determined or a resisting state where forced mind control is performed.

The shape score is the score of the entire shape created by the respective regression lines obtained by the regression line computation means 901. When the ends of adjacent regression lines are connected by an imaginary connection line, two adjacent regression lines may be approximately on one straight line, and a break point may appear between at least one of the regression lines and the imaginary connection line due to a difference in the gradients of the two adjacent regression lines and the difference in the values of the power spectral density. This break point is a bifurcation phenomenon occurring in an irregular vibration system, and this phenomenon changes depending on a time width in which disturbance changes and appears when a fluctuation of the biological state changes.
Moreover, this break point appears when a disturbance occurs as a large irregular vibration system, in particular, and when the degree of disturbance becomes stronger, the number of break points increases and the way of change changes. According to the test results described in Japanese Patent Application No. 2011-43428 filed by the present applicant, the number of break points is 1 or 0 when a person is healthy and in wakeful, relaxed, and stable states, the number of break points increases between fluctuations in the long-cyclic region where the overall trend appears and the short-cyclic region where the local regulatory state appears more remarkably, and similarly, the number of break points increases in a sick state. Thus, when a difference in the values of power spectral density between two regression lines in the adjacent cyclic regions is equal to or smaller than a predetermined value, and when the difference in the values of power spectral density between two regression lines in the adjacent cyclic regions is equal to or larger than a predetermined value and a difference in the angles of the gradients of the two regression lines is equal to or larger than a predetermined angle, the intersection of the two regression lines is counted as a break point. When the difference in the values of the power spectral density of two adjacent regression lines is equal to or smaller than a predetermined value and the difference in the angles of the gradients of the two regression lines is smaller than a predetermined angle, the two regression lines are regarded as one straight line, and it is determined that there is no break point between the regression lines.

In the present embodiment, the shape score is set such that the smaller the number break points, the higher the shape score. For example, the shape score is set to 0 when the number of break points is 3, 1 when the number of break points is 2, 2 when the number of break points is 1, and 3 when there is no break point. This is an example only, and this setting means that a higher score is assigned when the person is in a relaxed and stable state. The setting may be changed so that a lower score is assigned when the person is in a relaxed and stable state, for example.

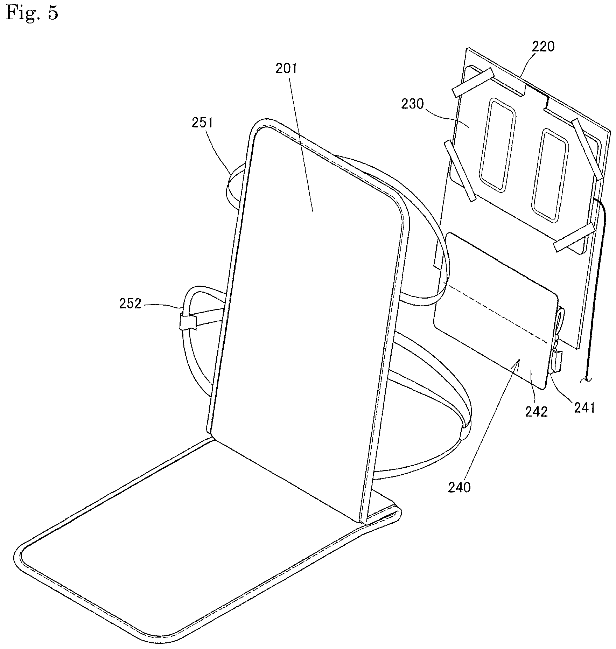

Figs. 11(a) to 11(d) are diagrams for describing examples of regression lines drawn by the regression line computation means 901 of the fluctuation waveform analysing means 90 and examples of determination criterial scores assigned by the determination criterial score calculation means 902. In this example, first, in order to calculate the regional score, +2 scores are assigned when the regression line has a downward gradient, +1 score is assigned when the regression line has a horizontal gradient, and 0 score is assigned when the regression line has an upward gradient. In Fig. 11(a), when the gradient in the long-cyclic region is downward, the gradient in the mid-cyclic region is upward, and the gradient in the short-cyclic region is downward, the regional score becomes 4 (=2+0+2). In Fig. 11(b), when the gradient in the long-cyclic region is upward, the gradient in the mid-cyclic region is upward, and the gradient in the short-cyclic region is downward, the regional score becomes 2 (=0+0+2). In Fig. 11(c), when the gradient in the long-cyclic region is downward, the gradient in the mid-cyclic region is downward, and the gradient in the short-cyclic region is horizontal, the regional score becomes 5 (=2+2+1). In Fig. 11(d), when the gradient in the long-cyclic region is downward, the gradient in the mid-cyclic region is downward, and the gradient in the short-cyclic region is downward, the regional score becomes 6 (=2+2+2). The shape score is 0 for Figs. 11(a) to 11(c) because the number of break points is 3 and 3 for Fig. 11(d) because there is no break point. Thus, the determination criterial score is the sum of the regional score and the shape score and is 4 (=4+0) for Fig. 11(a), 2 (=2+0) for Fig. 11(b), 5 (=5+0) for Fig. 11(c), and 9 (=6+3) for Fig. 11(d).

The state estimation means 95 obtains a time-series change in the determination criterial scores of the analysis waveforms (fluctuation waveforms) obtained by the determination criterial score calculation means 902 to estimate a biological state. In the present embodiment, the state estimation means 95 includes a first analysis determination means 951. The first analysis determination means 951 is a means that plots the coordinate points in a target analysis time segment with respect to the coordinate points in a reference analysis time segment and estimates the biological state based on the positions of the coordinate points in the analysis time segment, and includes two analysis determination means A and B. The determination criterial score is the score assigned based on predetermined criteria, for the frequencies of the fluctuation waveform regarding maintenance of homeostasis, a stress adaptive state, and physical conditions such as a pleasant and unpleasant state, a fatigued state, an ahead sick state, a sick state, and a normal state. Thus, a time-series change in the score enables to estimate the transition direction of the change in the physical conditions (that is, a state toward which the physical conditions move).

Here, a state appearing due to alcohol intake shows an abrupt change as compared to a state change resulting from other factors (fatigue, sickness, or the like) and shows a characteristic symptom that the changed state maintains for a long period due to the long degradation time of acetaldehyde. Although excessive drinking is exceptional, it is relatively easy to determine moderate drinking of a predetermined amount of alcohol (an alcohol intake state specifically corresponding to a refresh state in drunkenness degree classification). Other state that do not belong to the refresh state may be determined as a general fatigued state or a slump state due to sickness or the like. Here, the zero-cross detection means collects the behavior of the aorta (that is, the data in the diastolic phase), and is associated with the elastic modulus of the aorta itself and is thus rarely affected by pharmacological effects of alcohol. Thus, the zero-cross detection means detects enhancement in the level of sympathetic nerves by an increase in the blood pressure resulting from drinking. On the other hand, the peak detection means collects the data of both the heart and the aorta (that is, the data in both the diastolic phase and the systolic phase) and monitors a heart rate fluctuation, a behavior of the change in the activities of the sympathetic and parasympathetic nerves, and a behavior of the activity of the parasympathetic nerve in which a sympathetic compensatory mechanism is performed. Thus, the peak detection means is likely to be affected by alcohol. Thus, the fluctuation detected by the zero-cross detection means and the fluctuation detected by the peak detection means is observed, relatively, and analysis determination means A and B described later and a trigonometric function display means that displays the data obtained from the analysis determination means are estimated together. The trigonometric function display means detects the degree of change in the state (that is, a state transition speed) to estimate the direction of the change in state based on strength such as phase difference and amplitude. A difference in the fluctuation detected by the zero-cross detection means and the fluctuation detected by the peak detection means may be obtained and the data obtained from the difference can be observed by substitution with the data obtained using the peak detection means, which will be described later.

### (Analysis Determination Means A)

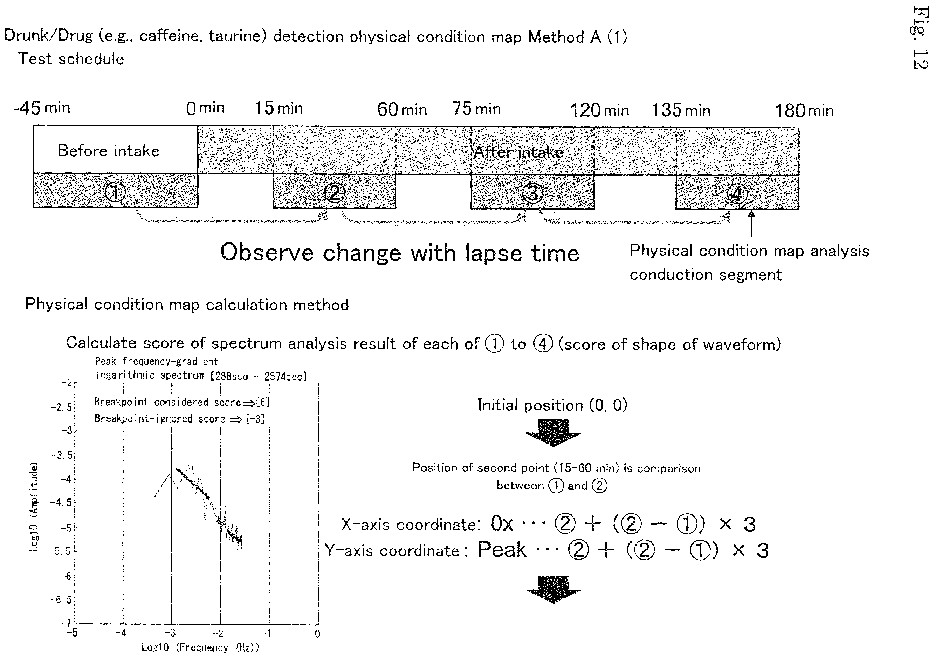

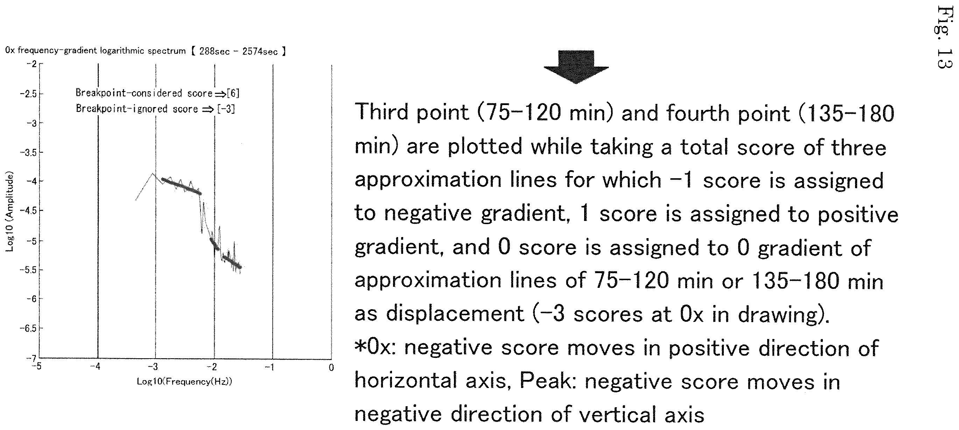

The analysis determination means A estimates a transition direction of an overall change in physical conditions after a change factor of a predetermined biological state is added in a reference analysis time segment from the degree of change in the fluctuation waveform as a physical condition change trend.
When the change factor of the biological state is alcohol intake, although the physical condition changes greatly in short period with absorption of alcohol, an overall transition direction of the physical condition is retrieved as a "physical condition change trend". Specifically, as illustrated in Figs. 12 and 13, the analysis determination means A is a means that estimates a physical condition change trend from the position (separation distance) of the coordinate point obtained in the entire target analysis time segment in relation to the coordinate point obtained in the entire reference analysis time segment. More specifically, the analysis determination means A uses a coordinate system of which the horizontal axis represents the indices indicating the behavior of the aorta, obtained by the zero-cross detection means detecting the state of the sympathetic nervous system and the vertical axis represents the indices indicating the behavior of both the heart and the aorta, obtained by the peak detection means detecting the state of both the sympathetic and parasympathetic nervous systems (the state of the parasympathetic nervous system in which the compensatory mechanism of the sympathetic nerves is performed). When a certain state change occurs (for example, a person takes alcohol), the analysis determination means A performs analysis of obtaining a difference indicating how much the coordinate point of the analysis time segment when the state change occurs is separated from the reference coordinate point plotted in this coordinate system as a vector representation. The analysis determination means A obtains basic information on how the overall physical condition changes with a state change and compares the target analysis time segments to obtain the difference between both. Specifically, the analysis determination means A performs the following procedures.

### (Procedure A1)

A physical condition change score is obtained using the determination criterial scores of a reference analysis time segment (initial position) and the next analysis time segment (second point) according to the following equation (see Fig 12).
Physical condition change score= (Determination criterial score of subsequent analysis time segment (second point)) + ((determination criterial score of subsequent analysis time segment (second point)) - (determination criterial score of previous analysis time segment (initial position)) × n (n is a correction coefficient) The n (correction coefficient) is determined according to the number of analysis target frequency regions (frequency bands). In the present embodiment, since the change in the three frequency regions of a long-cyclic region, a mid-cyclic region, and a short-cyclic region is detected, the correction coefficient is set to n=3 (in measurement periods of each analysis time segment, the frequency-gradient time-series waveform in all measurement periods (approximately 38 minutes) excluding a data-free period is used).

When the frequency time-series waveform is obtained by the zero-cross detection means, a change state regarding homeostasis maintenance associated with the aorta (that is, the sympathetic nerves) is obtained and the value thereof is plotted on the X-axis coordinate. When the frequency time-series waveform is obtained by the peak detection means, a behavior (in particular, a state change regarding the homeostasis maintenance associated with the parasympathetic nerve) of the fluctuation (the fluctuation in the parasympathetic nervous system in which the compensatory mechanism of the sympathetic nerves is performed) of both the sympathetic and parasympathetic nervous systems associated with the behavior of both the heart and the aorta is obtained and the value thereof is plotted as the Y-axis coordinate. The behavior of the heart is depicted using the information on these two-axes.

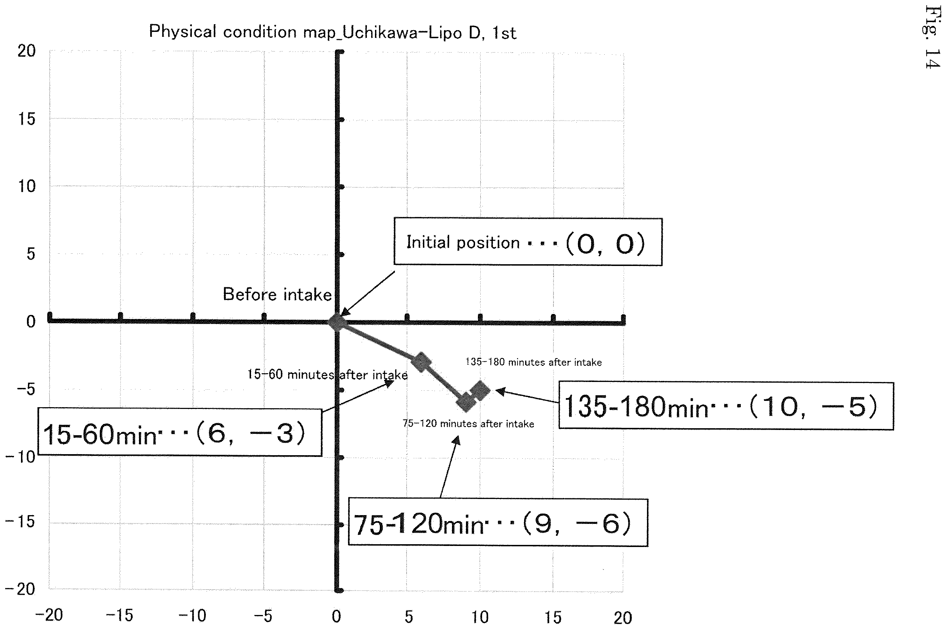

Since the physical condition change score obtained in the manner as described above uses the analysis time segment of the initial position and the analysis time segment of the second point, it detects the change in the physical condition between these points. Here, in what manner the state of the next analysis time segment changes with respect to the analysis time segment of the initial position is grasped enlargedly by multiplication with the correction coefficient. The analysis time segment of the initial position which is a reference is in a state before drinking or intake of drug components, and when the next analysis time segment is analyzed with respect to the reference analysis time segment, it is inferable that the state suddenly changes due to the influence of the alcohol or pharmacological effects of other drug components, and then gradually changes. Thus, as illustrated in Fig. 14, the initial position is set as the origin (0, 0) of the coordinate system, and the physical condition change score calculated by the above equation is plotted as the coordinate position of the second point and represented as a vector representation.

The determination criterial score used herein is a score combining a regional score and a shape score. The regional score represents the degree of stability of fluctuation for maintaining homeostasis, and the shape score represents a behavior of local control, and a healthy state or a sick state is estimated and identified, for example, from a bifurcation phenomenon. Hence, a relative variation is desirably determined by using a score combining these scores.

### (Procedure A2)

Once the initial position and the coordinate position of the second point are decided by the Procedure A1, the coordinate point of the analysis time segment which is to be determined is then determined by movement from the coordinate point of the previous analysis time segment (see Fig. 13). That is, the third point is moved from the coordinate point of the second point, and the fourth point is moved from the coordinate points of the third point.

At this time, motion is made in the X-axial direction and the Y-axial direction according to the gradients of the respective regression lines. In the present embodiment, regardless of whether the determination is made by the zero-cross detection means or the peak detection means, -1 score is assigned to the motion score when the gradient of the regression line is negative, +1 score is assigned to the motion score when the gradient is positive, and 0 score is assigned to the motion score when the gradient is horizontal. When the motion score determined by using the zero-cross detection means is negative, the movement is made in the positive direction of the horizontal axis of the coordinate system, and when the motion score determined by using the peak detection means is negative, the movement is made in the negative direction of the vertical axis of the coordinate system. The example illustrated in Fig. 13 is a fluctuation waveform determined in the analysis time segment of the third point by the zero-cross detection means, and in this example, since all of the long-cyclic region, mid-cyclic region, and short-cyclic region are negative, the score is -3 scores. In this case, movement is made by +3 in the X-axial direction from the coordinate point of the second point. In other words, as illustrated in Fig. 14, since the X coordinate of the analysis time segment (20-60 min) of the second point is +6, the X coordinate of the analysis time segment (80-120 min) of the third point is +9. In a similar manner, Y coordinates are determined by using fluctuation waveforms obtained by the peak detection means.

Fig. 14 illustrates an example of vector representation of coordinate points that are plotted on the four-quadrant coordinate system from the analysis determination means A, and connected to each other. The data include the plots according to the aforementioned Procedure 1 and Procedure 2 when a period of 45 minutes before taking Nutrient Drink A having a high taurine content is set as the initial analysis time segment (first analysis time segment) (using the frequency-gradient time-series waveform of about 38 minutes in this period), a period of 40 minutes after 20 to 60 minutes from the intake (using the frequency-gradient time-series waveform of about 38 minutes in this period) is set as the second analysis time segment, a period of 40 minutes after 80 to 120 minutes from the intake (using the frequency-gradient time-series waveform of about 38 minutes in this period) is set as the third analysis time segment, and a period of 40 minutes after 140 to 180 minutes from the intake (using the frequency-gradient time-series waveform of about 38 minutes in this period) is set as the fourth analysis time segment. In this manner, it is possible to grasp in what manner the physical condition changes with the lapse of time in each analysis time segment.

### (Procedure A3)

As can be seen from Fig. 14, the coordinate point significantly moves in the second analysis time segment with respect to the first analysis time segment (initial position) due to the pharmacological action of taurine or the like and the influence of taurine arises. Then coordinate points of the third analysis time segment and the fourth analysis time segment gradually change with respect to the coordinate point of the second analysis time segment. The analysis determination means A principally aims at retrieving a basic physical condition change trend according to difference between the initial position and the second analysis time segment. This is because generally, effects of alcohols and drug components emerge greatly at the point when a predetermined time has lapsed after the intake, and then gradually reduce. The fluctuation trend on and after the third analysis time segment indicates the manner of change from the second analysis time segment (generally, the time after about 20 to 40 minutes from the initial position) where influence of alcohol or the like is significantly exhibited.

### (Analysis determination means B)

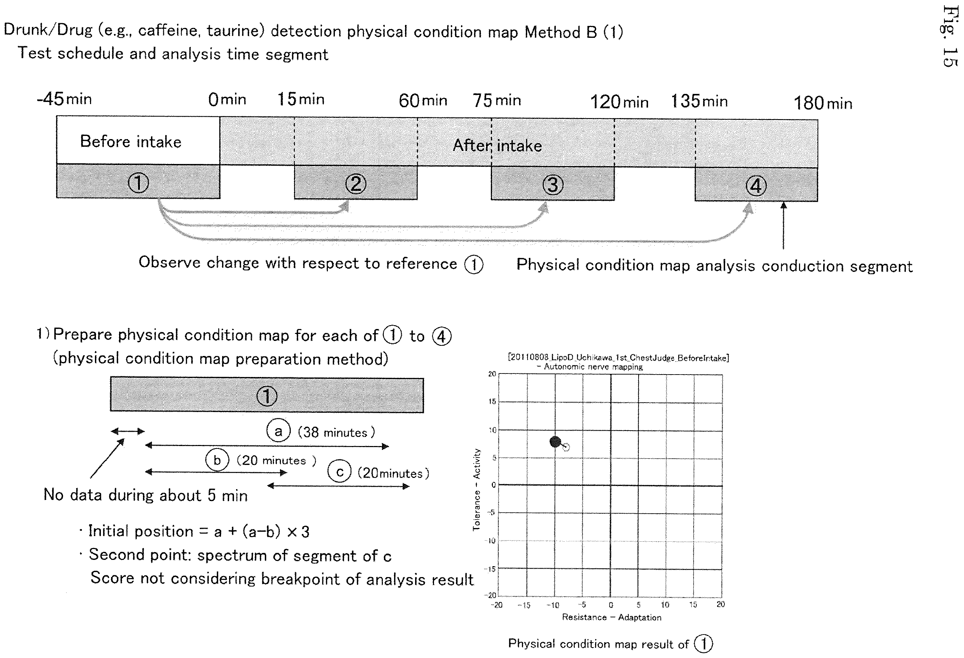

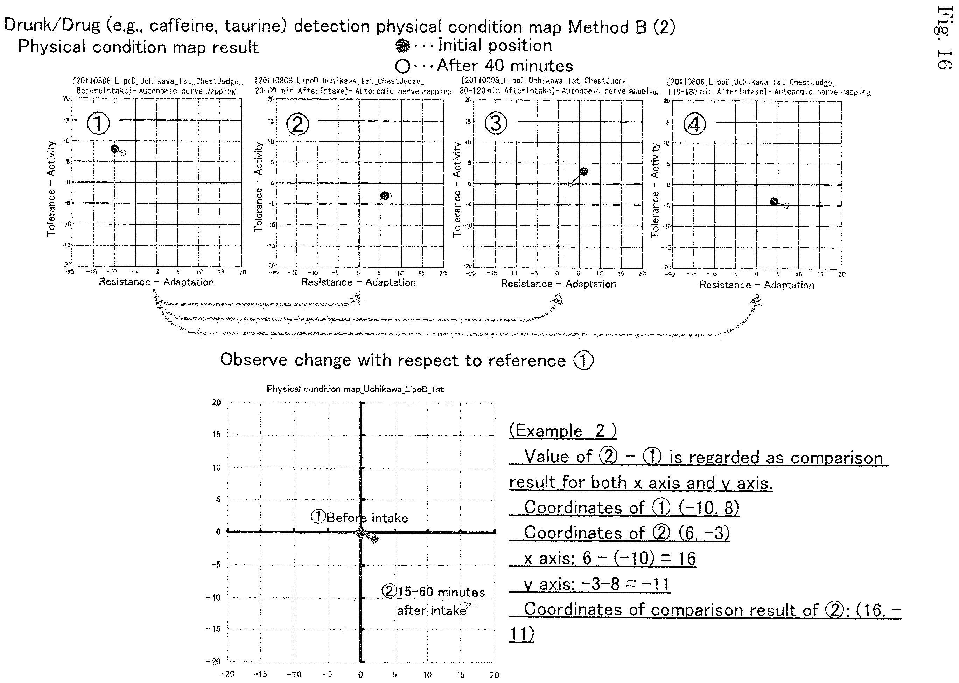

The analysis determination means B estimates the physical condition at a predetermined analysis time after a lapse of a predetermined time after a predetermined change factor of biological state is added, as a physical condition state at the time of analysis from the degree of change in the fluctuation waveform. The physical condition at the time of analysis changes with the lapse of time as a result of production of acetaldehyde due to decomposition of alcohol, and this changed physical condition at the time of analysis is retrieved as "physical condition state at the time of analysis". Concretely, the analysis determination means B is a means that determines changes on the coordinate system of a plurality of analysis time segments to be analyzed with reference to the reference analysis time segment, and analyzes a sensory state from their positions on the coordinate system as shown in Fig. 15 and Fig. 16, and achieves that concretely in the following procedure.

### (Procedure B1)

The means determines a coordinate point of each analysis time segment by using differences between analysis times having different analysis times in each analysis time segment, compares the respective coordinate points of the analysis time segments with the coordinate point of the reference analysis time segment, and estimates respective sensory states of the analysis time segments from the separation distances. First, in one analysis time segment, by grasping difference between a Time segment b ranging from the starting point (excluding the time where there is no data) to a predetermined time, and a Time segment a (entire measurement time) ranging from the starting point to the end point, it is possible to grasp the state change within one analysis time segment.

As shown in Fig. 15, by comparison of an analysis waveform obtained from the frequency-gradient time-series waveform of about 38 minutes which is almost the entire range of the analysis time segment (Time segment a), and an analysis waveform obtained from the frequency-gradient time-series waveform of the first 20 minutes (Time segment b), a sensory score is obtained according to the following equation: a + (a - b) × 3, and the obtained sensory score is used.

### (Procedure B2)

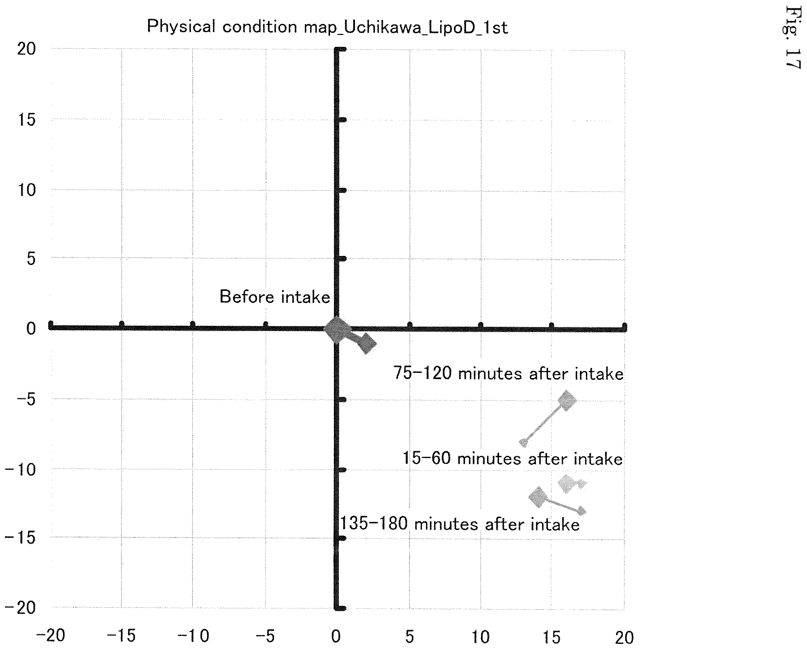

Fig. 17 is a diagram plotting sensory points from the respective Time segment a and Time segment b of the first analysis time segment, the second analysis time segment, the third analysis time segment, and the fourth analysis time segment (big square black mark in the diagram) for the same data as in the aforementioned analysis determination means A (data when Nutrient Drink A having a high taurine content is taken). At this time, the coordinates of the respective analysis time segments are plotted while the coordinates of the initial position of the reference analysis time segment are taken as the origin (0, 0) of the coordinates.

For example, as shown in Fig. 16, since the sensory score coordinates from the Time segment a and Time segment b of the second analysis time segment is (6, -3), in contrast to the sensory score coordinates (-10, 8) from the Time segment a and Time segment b of the first analysis time segment with reference to the first analysis time segment, the sensory score coordinates from the Time segment a and Time segment b of the second analysis time segment will be (16, -11) when the sensory score coordinates from the Time segment a and Time segment b of the first analysis time segment are taken as the origin. Similarly, sensory score coordinates from the respective Time segment a and Time segment b of the third analysis time segment and the fourth analysis time segment are individually compared with the sensory score coordinates from the Time segment a and Time segment b of the first analysis time segment, and relative variations are determined. Fig. 17 is a diagram illustrating the results, and what sensory state is established in each analysis time segment compared with the state before intake of Nutrient Drink A by drinking, namely, to what sensory state the subject has transited from the state of the initial position in that analysis time segment (analysis time) can be grasped.

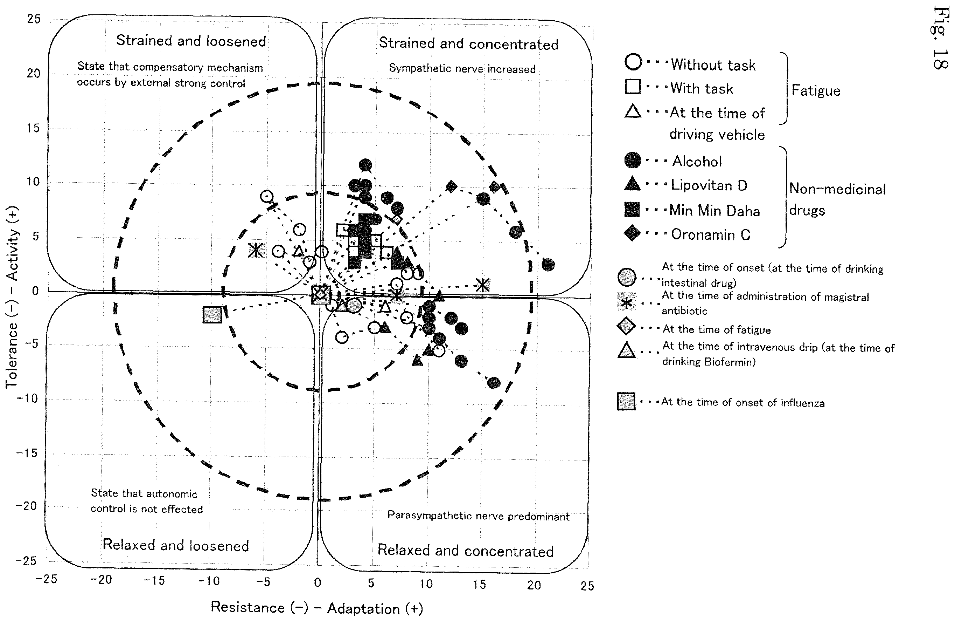

Here, in the coordinate system obtained by the analysis determination means A and the analysis determination means B, since the fluctuation waveforms obtained by the zero-cross detection means are plotted on the X-axis coordinate, and the fluctuation waveforms obtained by the peak detection means are plotted on the Y-axis coordinate, the farther in the positive direction on the horizontal axis, the higher the predominance of the sympathetic nerve activity, namely in a highly concentrated state, and the farther in the negative direction on the horizontal axis, the lower the concentration, namely in a loosened state, and the farther in the negative direction on the vertical axis, the higher the predominance of the parasympathetic nerve activity, namely in a relaxed state, and the farther in the positive direction on the vertical axis, the less the relaxation, namely in a strained state. This is simply illustrated in Fig. 18 wherein the fourth quadrant corresponds to the state where both the concentration and the degree of relaxation are high, the second quadrant corresponds to an opposite state, the first quadrant corresponds to the concentrated and strained state, and the third quadrant corresponds to a relaxed and loosened state. The respective states of these quadrants are based on the definition of movement of a coordinate point taking the horizontal axis as an index of sympathetic nerve activity, and the vertical axis as an index of parasympathetic nerve activity, and according to the indexes assigned to the horizontal axis and the vertical axis and the manner of movement of a coordinate point, for example, the first quadrant can be set as the state where both the concentration and the degree of relaxation are high.

### (Test Example 1)

For a plurality of subjects, biological signals were collected in various physical conditions by the biological signal measuring means 1. The collected biological signals were subjected to a filtering processing for removing body motion components, and the resultant time-series waveforms were processed by the frequency-gradient time-series analysis and computation means 70 to determine frequency-gradient time-series waveforms, and then state estimation was conducted by the frequency analysis means 80, the fluctuation waveform analyzing means 90, and the state estimation means 95. In Test Example 1, the processing was conducted by the analysis determination means A of the first analysis determination means 951 of the state estimation means 95. Fig. 18 to Fig. 25 illustrate the analysis results.

Of the test conditions, "Fatigue", "Without task" represents the state that a subject just sits on a chair for 60 minutes, "Fatigue", "With task" represents the case where a subject plays a computer game for 60 minutes in a sitting posture, and "Fatigue", "Driving" represents the state where a subject drives a vehicle. "Alcohol" represents the case where a subject drinks one 500 ml can of beer (alcohol concentration 5%) (Subjects 01 to 04), and the case where a subject drinks 180 ml of shochu (alcohol concentration 17%) (Subject: Uchikawa). As other cases, analysis was conducted for the cases including the case of taking Nutrient Drink A having a high taurine content (ingredient: taurine 1000 mg and so on, "Lipovitan D" (registered trademark)), the case of taking Nutrient Drink B having a high Vitamin C content (ingredient: Vitamin C 220 mg and so on, Oronamin C (registered trademark)), and the case of taking Nutrient Drink C having a high caffeine content ("Min Min Daha" (registered trademark)) as non-medicinal drugs. Also the case where a subject takes an intestinal drug at the time of occurrence of a disease (diarrhea/stomachache condition), the case where a magistral antibiotic is administered, the state at the time of intravenous drip (also taking Biofermin (registered trademark)), and the state at the time of occurrence of influenza were analyzed.

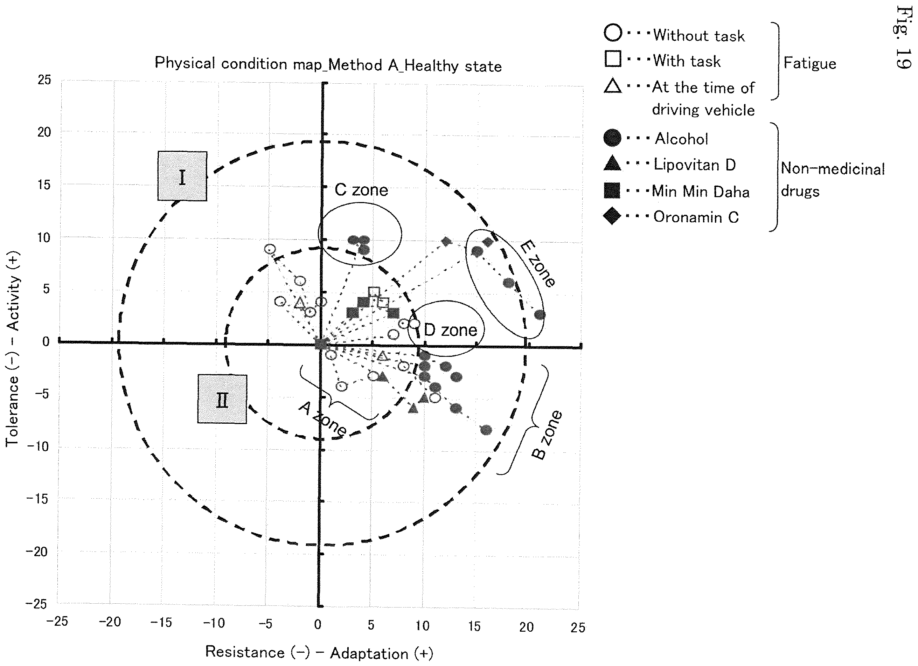

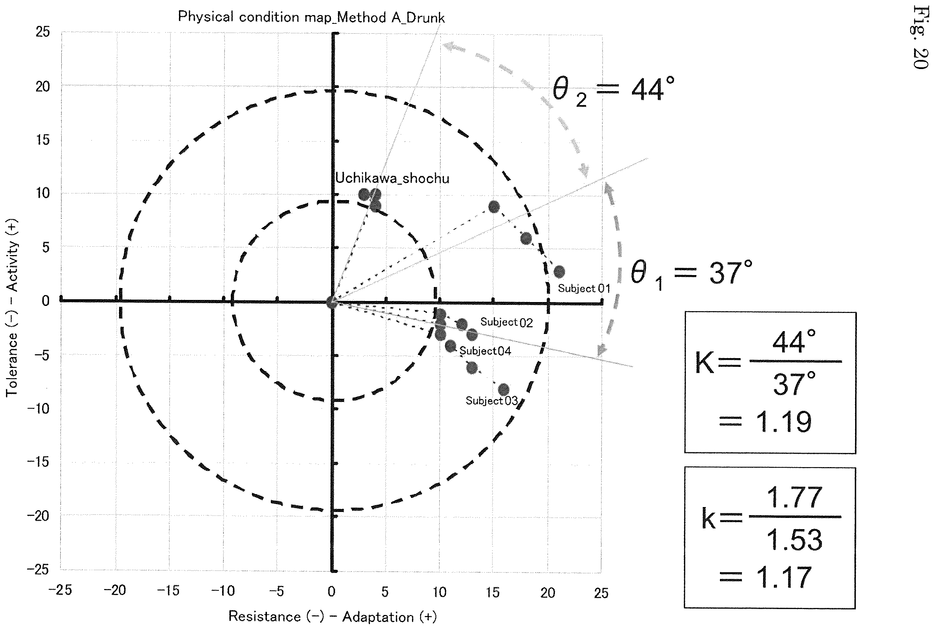

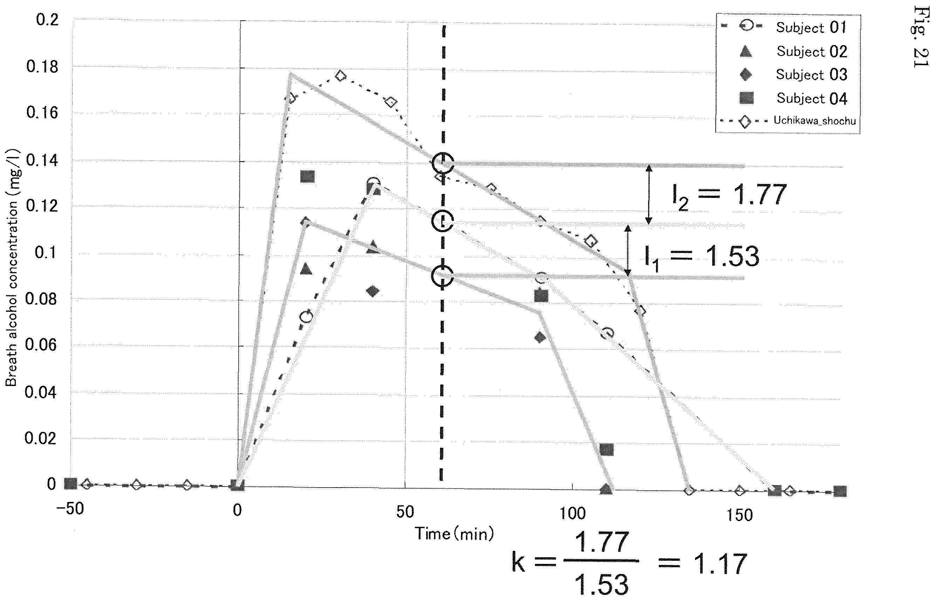

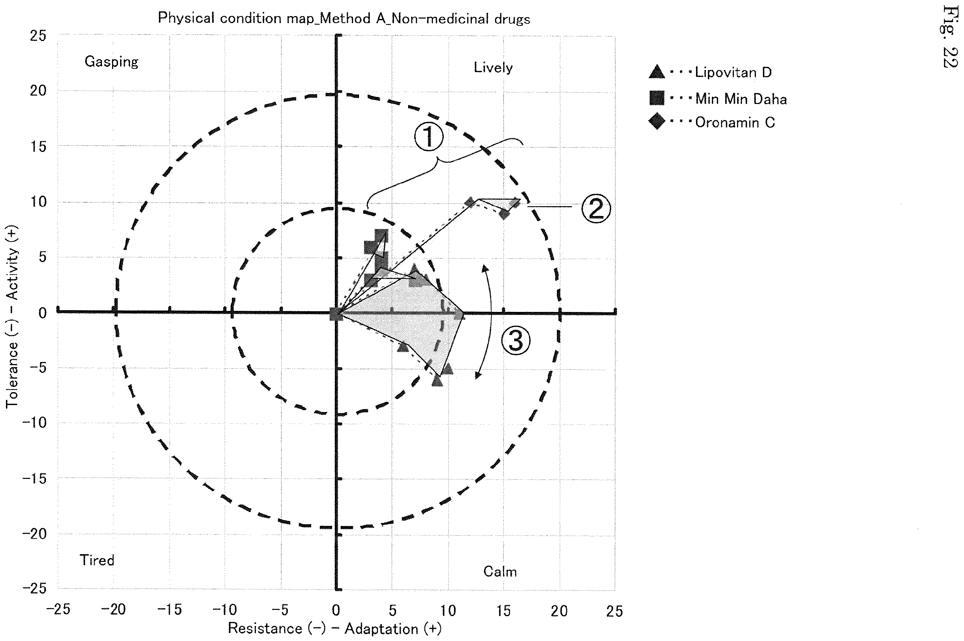

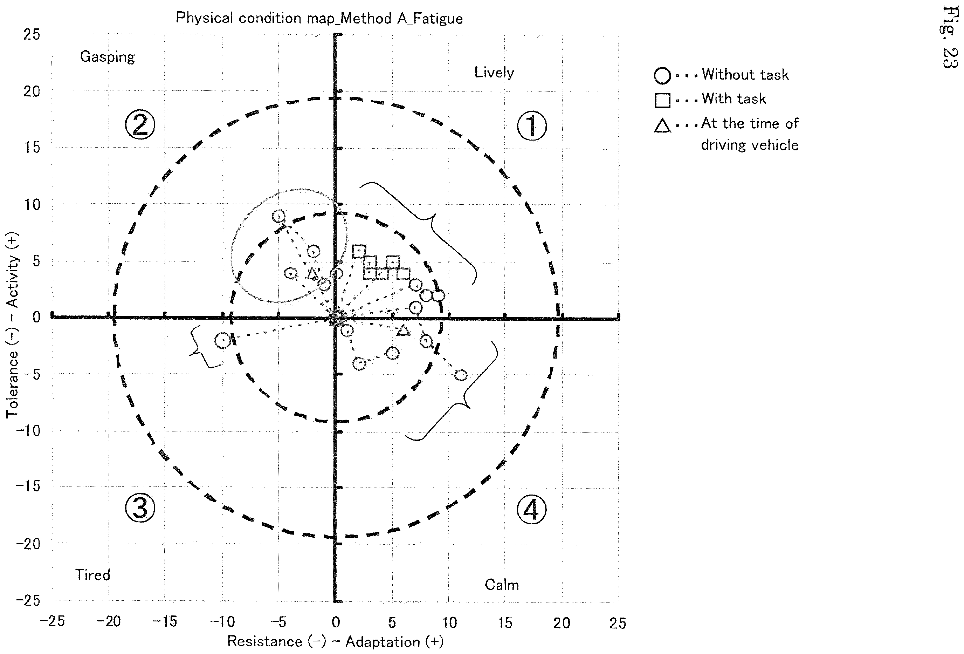

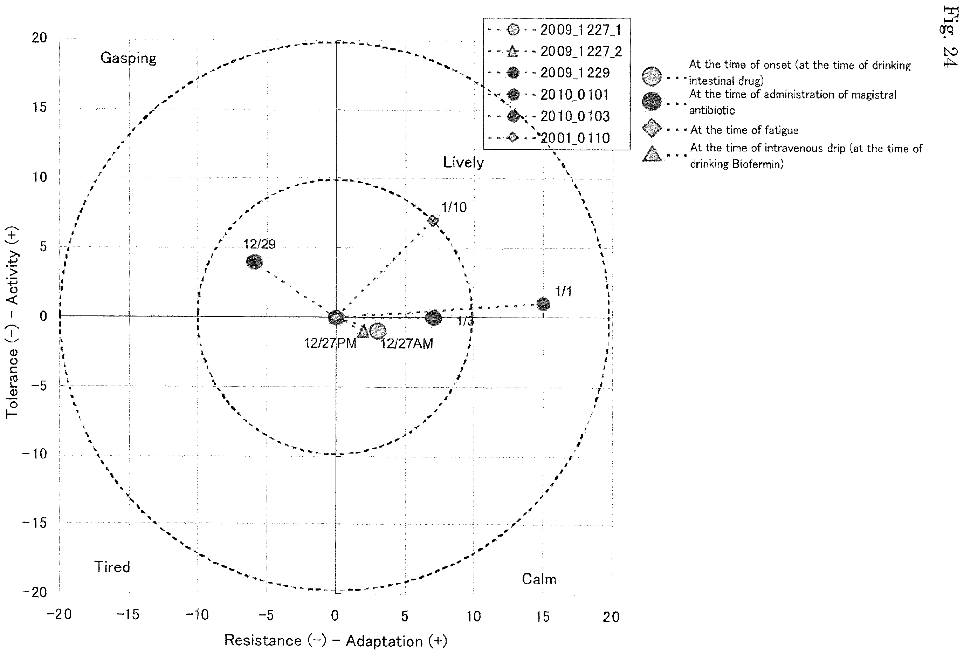

Fig. 18 is a diagram illustrating the analysis results on the same coordinates, Fig. 19 is a diagram selectively illustrating the analysis results for the cases of fatigue and non-medicinal drugs including alcohol, and Fig. 20 is a diagram selectively illustrating the state at the time of drinking (alcohol intake), and breath-alcohol concentration (measured every 15 minutes) of each subject was 0.11 to 0.18 mg/l as illustrated in Fig. 21, corresponding to the range of a refresh state in drunkenness degree classification. The "refresh state" in the present invention also includes "slight intoxication refresh state" corresponding to the breath-alcohol concentration ranging from 0.05 to 0.25 mg/l (0.01 to 0.05% by blood alcohol concentration). Fig. 22 is a diagram selectively illustrating the state of taking non-medical products other than alcohol, Fig. 23 is a diagram selectively illustrating the fatigued state, and Fig. 24 is a diagram selectively illustrating the states at the time of occurrence of diarrhea/stomachache, and at the time of occurrence of influenza.

The inner circle of the donut-shaped region depicted in these diagrams has a radius of a separation distance from the origin (first separation distance) of 10, and the outer circle has a radius of a separation distance from the origin (second separation distance) of 20. Since the first separation distance and the second separation distance forming the donut-shaped region enclosed by the inner circle and the outer circle are determined from the range determinable as the alcohol intake state based on the results of Test Examples, these will be explained after description of the Test Examples.

First, presence or absence of alcohol intake corresponding to a refresh state in drunkenness degree classification will be examined. Distributions of coordinate points at the time of intake alcohol are shown in C zone, E zone, and B zone as shown in Fig. 19, and coordinate points in the second analysis time segment are plotted within the donut-shaped region in any zone. In other words, it can be set that when the separation distance of the coordinate point in the second analysis time segment from the origin is within the range of 10 to 20, the analysis determination means A determines as alcohol intake corresponding to a refresh state in drunkenness degree classification.

Subjects whose coordinate points are plotted in E zone are conscious of slow sobering up from alcohol, and from this fact, it can be said that the separation distance is longer compared with those for other subjects when the sobering up is slow. On the other hand, subjects plotted in C zone are conscious of fast sobering up after alcohol intake, and actually the separation distance closely resembles that of fatigue in normal case "Without task" as shown by D zone. While in the case where Nutrient Drink C is taken, the separation distance of the second analysis time segment is plotted within the donut-shaped region, and it will be estimated as alcohol intake just by the analysis determination means A. So, for achieving more accurate determination of alcohol intake, it is preferred to eventually determine as "alcohol intake state" only when presence of alcohol intake is determined also by the analysis determination means B, namely only when presence of alcohol intake is determined both in the analysis determination means A and B. This point will be described in more detail in the section describing the analysis determination means B.

The fourth quadrant of the coordinates is a zone of parasympathetic nerve predominant, relaxed and highly concentrated state, and in this zone, comparatively relaxed states without little stress are exhibited in the case of the subject having transited toward A zone under fatigue without task, and in the case of intake of Nutrient Drink A, and in the case of a subject of alcohol intake having changed toward B.

For examining the accuracy of the above determination of drinking (alcohol intake), the manner of change was compared with that of the breath-alcohol concentration. First, as illustrated in Fig. 20, based on the change direction from the origin, the subjects were separated into three groups of "Uchikawa-shochu", "Subject 01", and "Subjects 02, 03, 04", and for these, approximation lines from the origin are drawn. Letting the angle formed by the respective approximation lines of "Subject 01" and "Subjects 02, 03, 04" as 01, and the angle formed by the respective approximation lines of "Uchikawa-shochu" and "Subject 01" as 02, the ratio of these is K = 44 degrees / 37 degrees = 1.19.

On the other hand, in Fig. 21 illustrating breath-alcohol concentration, an approximation line to a peak point, an approximation line from the peak point to the point where a sudden change occurs on and after 90 minutes, and an approximation line to the point where the value of the vertical axis is 0 on and after 90 minutes are drawn. And, a ratio of distances between intersections of these approximation lines after a lapse of 60 minutes is determined. As a result, distance 11 on the scale between intersections between the time axis of 60 minutes and the respective approximation lines of "Subject 01" and "Subjects 02, 03, 04" is 1.53, and distance 12 on the scale between intersections between the time axis of 60 minutes and the respective approximation lines of "Uchikawa-shochu" and "Subject 01" is 1.77, and the ratio between these is k = 1.77/1.53 = 1.17. Therefore, these ratios are substantially coincident, revealing that the graph that illustrates the influence of alcohol intake shown in Fig. 20 has very high relationship with the breath-alcohol concentration.

In the approximation lines of breath-alcohol concentration in Fig. 21, when the gradient is large on and after 90 minutes, a rebound by alcohol intake is large, and the condition gets worse by alcohol intake. In this respect, the same conclusion can be drawn by determining that the rebound is larger as the change is closer to I line in Fig. 19.

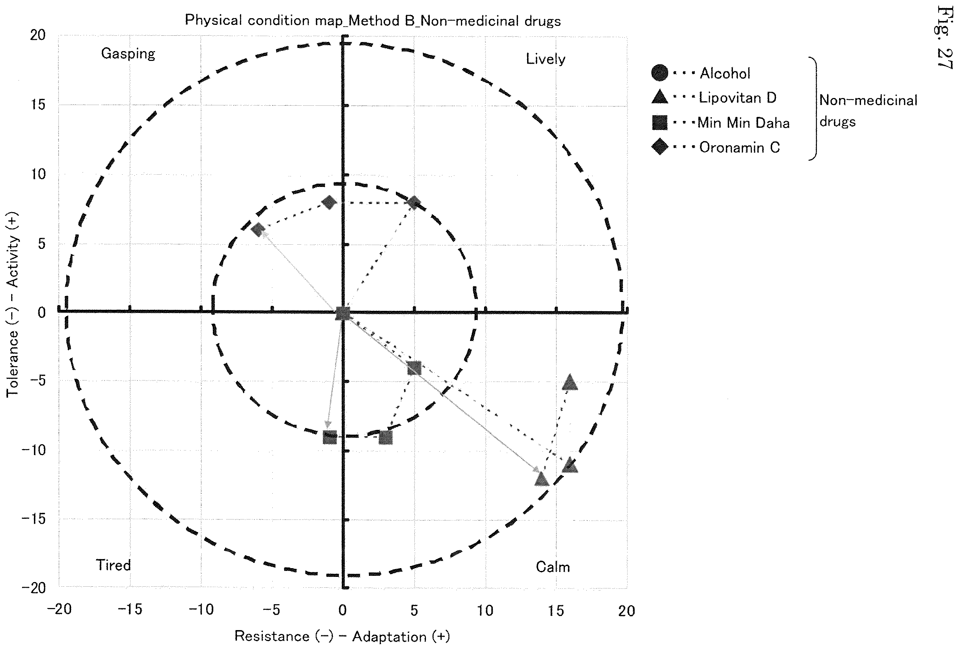

Fig. 22 is a diagram illustrating physical condition changes due to intake of Nutrient Drink A, B, or C. The separation distances in the second analysis time segments of Nutrient Drinks A, C are both within the inner circle. As will be later described, in this test example, when there is a coordinate point inside the inner circle of the donut-shaped region, it is basically determined as a general fatigued state (normal state) (slump state when it is plotted within a smaller circle), and when there is a coordinate point outside the outer circle, it is basically determined as a slump state. When a nutrient drink is taken, it is supposed that the function of keeping a healthy and ordinary state is increased owing to the effect of the nutrient drink, and when the effect of the nutrient drink functions successfully, the separation distance from the origin in the second analysis time segment will be inside the inner circle. In the case of Nutrient Drink B, the separation distance from the origin in the second analysis time segment is large, and sympathetic nerves are increased. In the case of Nutrient Drink B, the coordinate point falls within the inner circle by the analysis determination means B shown in Fig. 27 as will be described later, and is distinguishable from the case of alcohol intake. In the case of Nutrient Drink A, the rebound is small, suggesting that it has the effect of modulating the parasympathetic nerve system. In the case of Nutrient Drink C, since the coordinate point is located in the region where the sympathetic nerves are increased in the first quadrant, it has the effect of stimulating the sympathetic nerve system; however, comparison with data of other subjects reveals that the effect greatly varies from subject to subject, and is likely to be influenced by goodness of the physical condition of each subject.

Regarding the changes in fatigue in Fig. 23, it can be realized that the separation distances from the origin to the coordinate points in the second analysis time segment are small, and most of the coordinate points are plotted inside the inner circle. When a healthy subject is fatigued, the separation distance to the second analysis time segment is small, and is clearly distinguishable from the aforementioned alcohol intake state.

Here, there are three stages of degree of fatigue. The first stage is a state of not feeling a sense of fatigue, and the second stage is a state of not feeling a sense of fatigue owing to a sympathetic compensatory mechanism, and the next third stage is a state of strongly feeling a sense of fatigue accompanied by a human error which can lead to chronic fatigue. For this reason, as the vertical axis, the one detecting the functions of both the parasympathetic nerve system and the sympathetic nerve system by the peak detection means is used. Therefore, it is realized that in the case of the region "1" (first quadrant), the sympathetic nerves are increased, and the subject often feels no sense of fatigue owing to the sympathetic compensatory mechanism during handling the task. However, as the sympathetic compensatory mechanism continues, a rebounding backlash naturally occurs, and the subject often feels a sense of fatigue. In the case of the region "4" (fourth quadrant), the parasympathetic nerve system is predominant, and it is a mode close to a rest in a relaxed state although control of sympathetic nerves is added. The regions indicated by "2" and "3" (second and third quadrants) are assumed to have a chronic sense of fatigue due to bad physical condition or shortage of sleep, and a pathologic sense of fatigue. In the case of the second quadrant, it is assumed that the subject is with fight or filled with a sense of mission, and in the case of the third quadrant, it is assumed that the subject feels a sense of fatigue in a state that involvement of the sympathetic nerves is limited, and the parasympathetic nerves are relatively predominant, or in other words, in a state without fight.

Referring to the data of bad physical condition in Fig. 24 (data of one subject after onset of a disease), the separation distance from the origin is very small although the coordinate point is located inside the inner circle at the time of onset of the disease and at the time of intravenous drip on December 27. From this fact, it is estimated that when the separation distance is very small as described above (1/2 or less, or 1/3 or less of the inner circle), physical condition is bad even if the coordinate point is located inside the inner circle. The bad physical condition is a state of resisting the factor of the bad condition due to the onset of a disease. On December 29, a drug (antibiotic) as a bad condition factor eliminating means is effective, and even though the coordinate point is still inside the inner circle, there is a certain degree of separation distance from the origin in comparison with the coordinate point on December 27, so that it can be said that the state is closer to a calm state in the bad physical condition. Both on January 1 and January 3, the separation distance was large, revealing the effectiveness of the drug administration. Also as a result of the drug administration, the coordinate point moved from the negative region to the positive region on January 1, compared with December 29, and the positive value was much larger on January 3. Therefore, it can be said that the physical condition gradually recovers by the drug administration. Also it is realized that on January 10, the coordinate point moves to inside the inner circle and the state is closer to the normal state (normal fatigued state in a healthy state where fatigues gradually accumulate by actions).

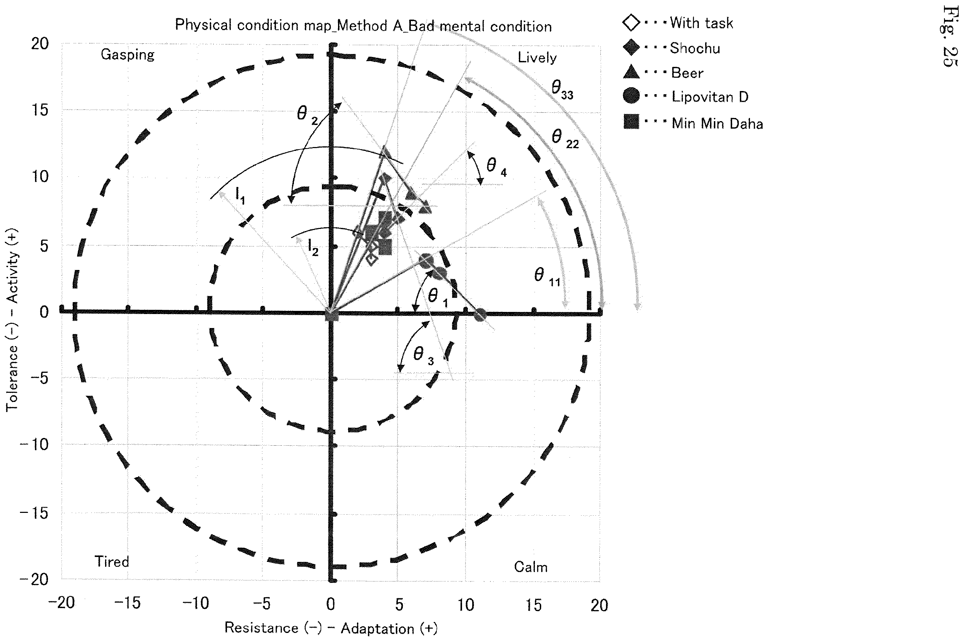

Fig. 25 is a diagram illustrating the analysis results of biological signals of Subject K being in bad mental condition for respective cases. Also in the case of this Subject K, when the subject drinks beer or shochu, the coordinate points in the second analysis time segment are plotted within the donut-shaped region. In other cases, all the coordinate points in the second analysis time segment are plotted inside the inner circle. Therefore, also in the case where the subject is in bad mental condition as is the case with Subject K, it will be appropriate to estimate alcohol intake according to whether the coordinate point is located inside the donut-shaped region as a basic estimation result. Of course, it is required that the coordinate point finally falls within the donut-shaped region also in the analysis determination means B as described above.

When this diagram is viewed in detail, the degree of intensity of the sympathetic compensatory mechanism is suggested by differences between θ11, θ22, and 033. Nutrient Drink A exhibits the most relaxed and very ideal rest mode. θ11 is small, and θ1 of -45 degrees is close to 1/f fluctuation. Among game, shochu and beer, beer looks most effective to relaxation. Beer corresponds to the coordinate point represented by 11 of the separation distance in the second analysis time segment, and also in the subsequent change, θ2 of -45 degrees is close to 1/f, which can be regarded as pleasantness. On the other hand, regarding the shochu, although the direction is negative, θ3 is large, and further, θ4 is positive direction, and a sense of resistance arises. As to the game which is a task, it can be seen that 12 is small, and there is a trend of atrophy. As to the subsequence, slight sense of resistance and sense of fatigue are suggested not so much as by shochu. Further, as to Nutrient Drink C, strong resistance against suppression is exhibited. This can be speculated from the locus exhibited by Nutrient Drink C.

### (Test Example 2)

For the data used in Test Example 1, a processing was conducted by the analysis determination means B among the first analysis determination means 951 of the state estimation means 95 in the present Test Example 2.

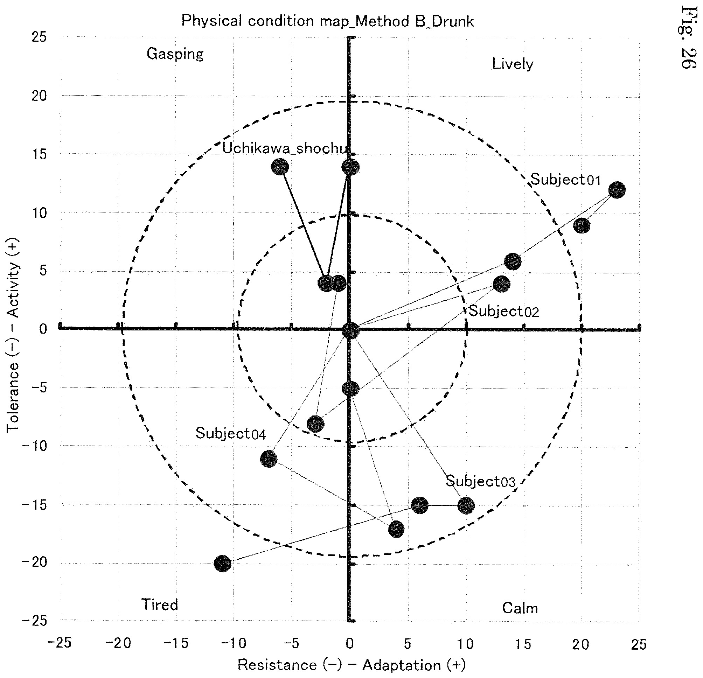

The analysis results at the time of drinking are illustrated in Fig. 26. Fig. 26 reveals that in any subject cases, the coordinate points in the second analysis time segment are plotted within the donut-shaped region formed by the inner circle and the outer circle respectively having a first separation distance "10" and a second separation distance "20" with respect to the origin.

Reviewing the states of respective subjects based on the estimation results by drinking in Fig. 26, it can be realized that in Subject 01, the plotted coordinate points extend substantially linearly in the strained and concentrated region (lively region) in which the sympathetic nerve system is increased although there is a slight rebound. In Subject 02, the coordinate points are distributed over all the quadrants, and the subject seems to be in a rest state. Subject 03 enters the relaxed and concentrated region (calm region) where the parasympathetic nerves are predominant midway, but then enters the relaxed and loosened region of the third quadrant (tired region) where the concentration is low in spite of the relax trend. It can be seen that "Uchikawa-shochu" falls within the strained and loosened region of the second quadrant (gasping region) where the sympathetic compensatory mechanism occurs although it has shown a temporary rebound.

Fig. 27 is a diagram illustrating the results at the time of taking Nutrient Drinks A to C. From Fig. 27, as for Nutrient Drinks B, C, since the position of the coordinate point in the second analysis time segment falls within the inner circle, and it is in an alcohol non-intake state, and the separation distance is larger than or equal to a half of the inner circle from the origin, it can be determined as a normal state rather than a slump state. On the other hand, in the case of Nutrient Drink A, the inner circle is exceeded. However, when the result in Fig. 22 of the analysis determination means A and the result in Fig. 27 of the analysis determination means B are combined, in Nutrient Drinks A to C, at least either one result is inside the inner circle, and the health state when taking any of these nutrient drinks can be determined as a normal state.

Hereinafter, the cases of drinking Nutrient Drinks A to C in Fig. 27 will be individually reviewed. It can be seen that Nutrient Drink A transits within the same quadrant, and has a relaxed trend although there is more or less fluctuation. It can be seen that Nutrient Drink B falling within the inner threshold will little influence on the body, and sensuously transits to the gasping region through temporary vigor. Nutrient Drink C has a trend of being wakeful, but a sense of fatigue arises from the temporary rebound phenomenon.

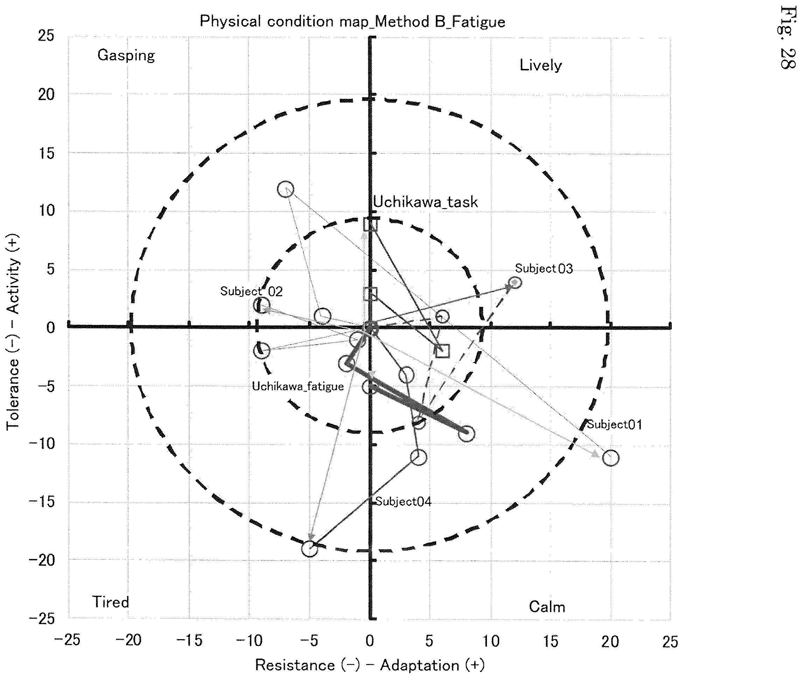

Fig. 28 is a diagram illustrating the analysis results during in a fatigue state. The coordinate points in the second analysis time segment in a fatigue state are plotted inside the inner circle for every subject. Therefore, it is estimated that in every subject, change due to fatigue occurs within the range of a normal state which is not a bad condition, in an alcohol non-intake state. These will be individually reviewed. It can be determined that Subject 01 is in a good state because there is a relaxed trend where the parasympathetic nerve system is predominant although a tired behavior is exhibited midway. Subject 02 is forced to become strained, and is not in a good state at all. Subject 03 has a trend of shifting toward the lively region, and is in a good state. In Subject 04, the sense of fatigue steadily advanced. It is in a relaxed state, but the final state is not good. "Uchikawa-Task" got tired midway, and the state tends to be worse, but has not reached the limit. "Uchikawa-Fatigue" has a trend of quietening down, and the state tends to be good.

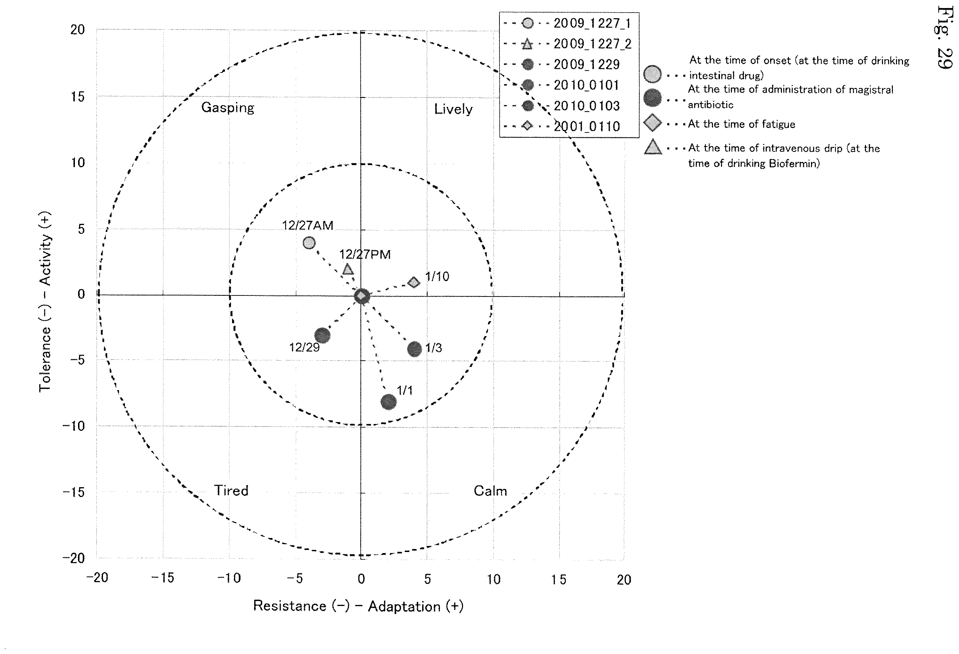

Fig. 29 is a diagram illustrating bad physical conditions. While every coordinate point falls within the inner circle, the separation distance is less than or equal to a half of the radius of the inner circle at the time of onset, and it is a state of resisting in a slump state. Then by the effect of the magistral antibiotic, the separation distance gets close to the first separation distance of the inner circle, and it is in a state that calm state is kept in a slump state. According to the results of the analysis determination means B illustrated in Fig. 29, at the time of onset, coordinate points are unevenly distributed in the second quadrant (gasping region) and third quadrant (tired region) in the negative direction; however, it tends to shift to the fourth quadrant (calm region) and the first quadrant (lively region) by drug administration and intravenous drip, and it seems to have a trend of gradually recovering in a slump state.

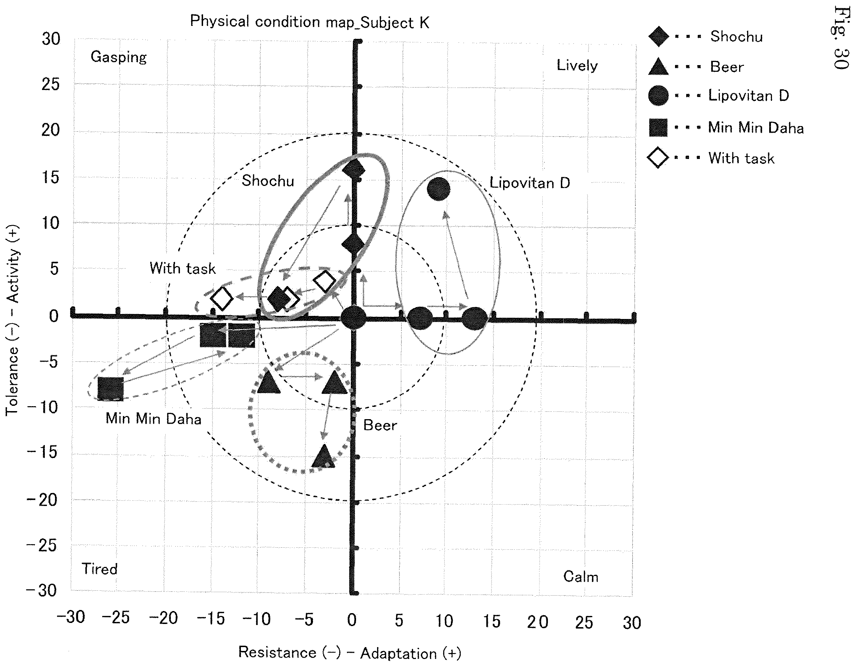

Fig. 30 is a diagram illustrating the results of the biological signals of Subject K being in a bad mental condition analyzed for each case by the analysis determination means B. When the subject drinks Nutrient Drink A, in a fatigued state having conducted a task (game), every coordinate point in the second analysis time segment is located within the inner circle having a radius equivalent to the first separation distance "10". In the case of Nutrient Drink C, it is between the first separation distance "10" and the second separation distance "20", and by the analysis determination means A of Fig. 25, every coordinate point is located inside the inner circle, and either one is within the inner circle. Therefore, in these cases, it can be estimated as a state change in a normal state in terms of the physical condition although the subject basically has a bad mental condition. In the case of beer, the plotted points are on the line of the first separation distance "10", and alcohol intake can be estimated by combining the determination result by the analysis determination means A in Fig. 25. In contrast to this, in the case of shochu, coordinate points in the second analysis time segment are located inside the inner circle having a radius equivalent to the first separation distance "10". In the case of shochu, while the coordinate points fall within the donut-shaped region by the analysis determination means A of Fig. 25, they are out of the donut-shaped region by the analysis determination means B in the present test example, and the example of shochu in subject K being in a bad mental condition is only one exception. All states of alcohol intake other than this exception are plotted in the donut-shaped region both by the analysis determination means A and B.

### (Test Example 3)

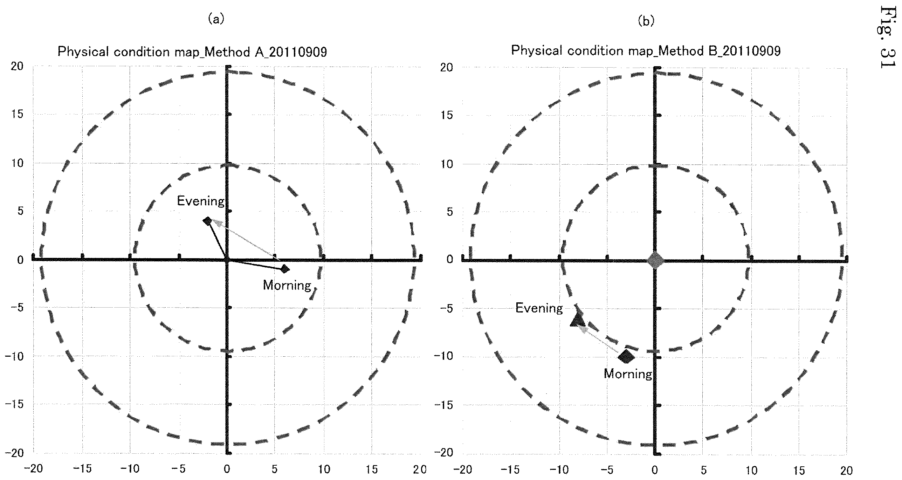

The state of Subject JY during driving was analyzed by the analysis determination means A and the analysis determination means B, and the analysis results are shown in Figs. 31(a), (b), respectively. The subject drove a predetermined time in the morning and in the evening respectively on the same day (September 9, 2011). During the driving time, data of the first half was assigned to the origin of the reference analysis time segment, and the data of the second half was determined as an analysis time segment to be compared.

Referring to Figs. 31(a), (b), in Fig. 31(a), the coordinate points are located inside the inner circle both in the morning and in the evening, so that the basic state of this subject is determined as a normal state. More specifically, Fig. 31(a) demonstrates that the coordinate point transits to the gasping region of the second quadrant wherein the sympathetic compensatory mechanism functions in the evening, while the coordinate point transits in a relaxing trend in the morning. As can be seen from Fig. 31(b), while any coordinate points are located in the tired region of the third quadrant, the coordinate point is close to the calm region of the fourth quadrant in the morning, and the coordinate point is close to the gasping region of the second quadrant where the sympathetic compensatory mechanism functions in the evening. Actually, during the test, Subject JY did not feel a sense of fatigue and was wakeful in the morning, and felt strong sense of fatigue in the evening, and felt drowsiness and stress (on stop-and-go driving of the anterior vehicle) in the second half. Therefore, the results are similar to the actual feelings of the subject.

### (Test Example 4)

Analysis was conducted by using biological signal data in real vehicle driving tests conducted by Subjects A, B, C on different days. In each measurement time on each day, analysis time segments for comparing the second half with reference to the first half were determined by the analysis determination means A, B.

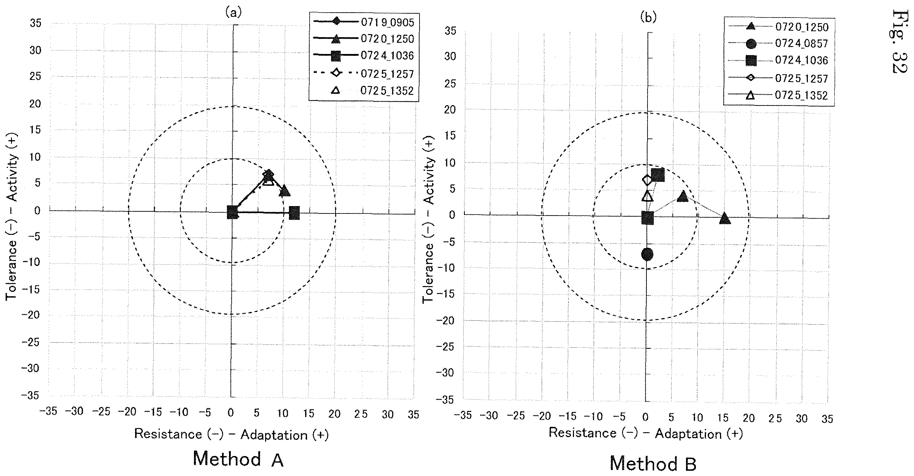

Subject A is a person showing significant ups and downs in the good condition and the bad condition, but in either of Figs. 32(a), (b), the coordinate points are located inside the inner circle. Therefore, in all days, advance of fatigue in a normal state can be observed.

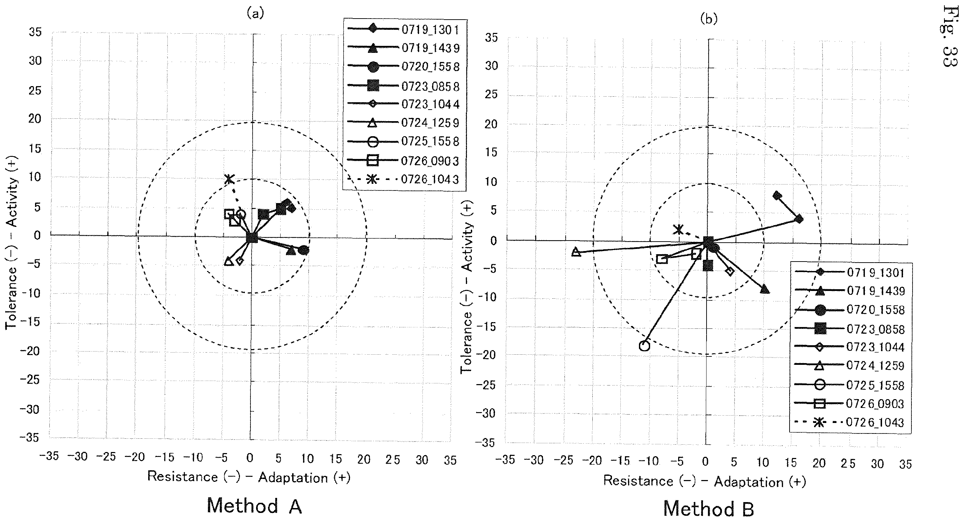

Subject B is a person of a basically healthy type. In the result of the analysis determination means A in Fig. 33(a), coordinate points other than the data of "0726 1043" are located inside the inner circle, and it can be determined that the result is within the range of ordinary advance state of fatigue, and there is no significant physical abnormality. In the analysis determination means B in Fig. 33(b), data of "0726 1043" is also located inside the inner circle. Therefore, in at least one of the analysis determination means A and the analysis determination means B, the data is located inside the inner circle in all days, so that it is estimated as advance of fatigue in a normal state.

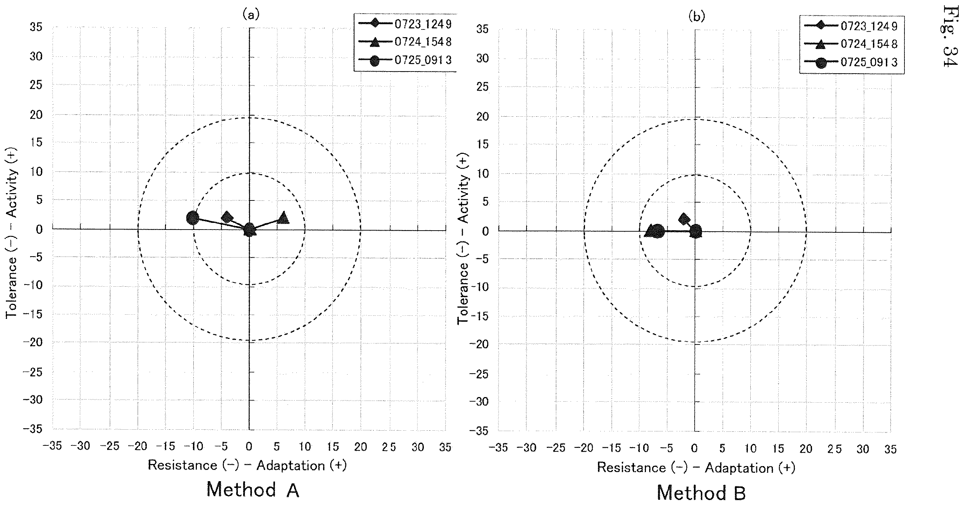

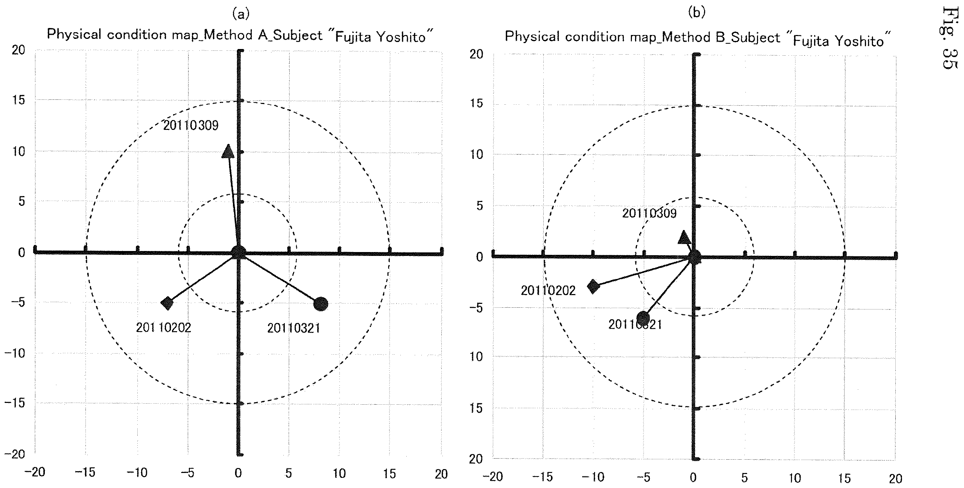

Subject C is a person who is basically positive and physically strong. Both the analysis determination means A in Fig. 34(a) and the analysis determination means B in Fig. 35(b) show similar trends, and the separation distances thereof fall within the inner circle. Since the data of "0724 1548" and "0725 0913" are separated to near the inner circle, it can be determined that the fatigue degree is within the range of the normal state. However, as for the data of "0723 1249", since there are coordinate points whose separation distances from the origin are less than or equal to 1/2 of the inner circle in both of the analysis determination means A and B, the state is estimated as resisting in a slump state.

### (Test Example 5)

A test was conducted for examining differences in analysis results in different measurement postures: the measurement posture wherein the activity of parasympathetic nerves is relatively predominant (recumbent posture), and the measurement posture wherein good balance of sympathetic and parasympathetic nerve activities is easy to be achieved (sitting posture), and in different conditions of sick or not. For any data, data of 40 minutes of the first half in a predetermined measurement time (about 1 hour) on the measurement day was taken as a reference, and data of the analysis time segment of 40 minutes of the second half was picked up as the analysis time segment to be compared.

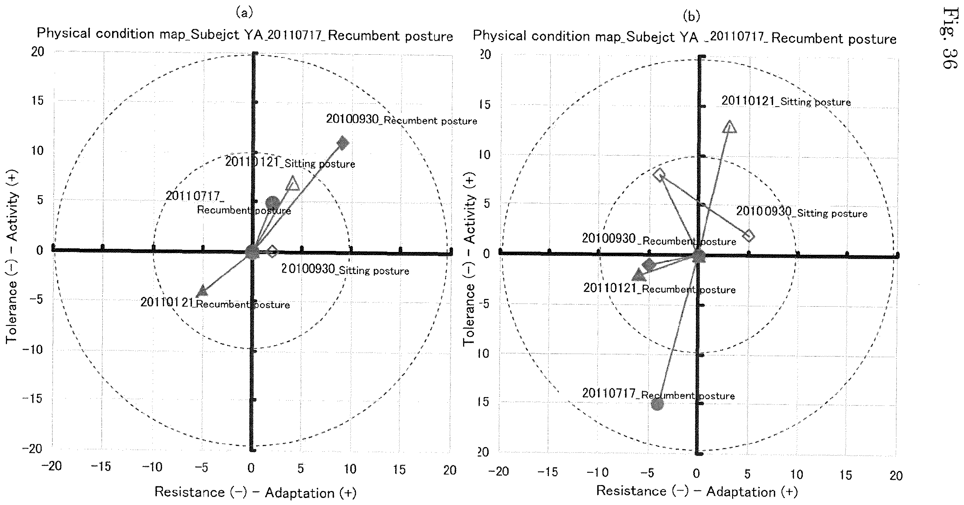

Data of Mr. Fujita Yoshito (86 years old at the time of data measurement) in Fig. 35 is collected in a recumbent posture. Since it was revealed from the present data and data of Fig. 36 to Fig. 38 that changes in the first separation distance and the second separation distance tend to reduce when the posture is a recumbent posture and the parasympathetic nerves are relatively predominant compared with the case of a sitting posture, the first separation distance was set at the position of "6" from the origin, and the second separation distance was set at the position of "15" from the origin. The data of 20110202 is data in the state that the subject has recovered to such an extent that he is able to walk in the house and is able to have a meal in a sitting posture after having underwent partial resection of the large intestine due to cancer. On the other hand, the data of 20110309 is data collected in the condition that ascites and pleural effusion accumulated at re-hospitalization, and the data of 20110321 is data when the ascites were removed and he felt better physically.

Referring the data of 20100309, the coordinate points are located inside the inner circle in the result of the analysis determination means B. However, it can be seen that the distance from the origin is very short. The data of 20110309 was collected in such a situation that ascites and pleural effusion accumulated at re-hospitalization, and thus the subject endured in a slump state, and this data reflects such a situation. On the other hand, as for 20100202 and 20100321, the subject was originally in a slump state, and the coordinate points thereof could be located outside the outer circle because the body was resisting; however, the coordinate positions move inside the outer circle and the state transits to a calm state by a drug. Here, the horizontal axis is taken as an index of the state of the aorta, and the vertical axis is taken as an index of the state of the heart and the aorta. The decrease in function could occur in the course from the stage of 20100202 to the stage of 20100321. The separation distance of 20100202 indicates the function of the heart in the condition that the function of the aorta is reduced, and is presumed to indicate the postoperative state. Also the degree of change in the analysis determination means B is large, and it is presumable that he was in an easily-get-tired state. On the other hand, in the state of 20100309, the change rate of the physical condition in the test is small, but the state greatly changes. In other words, it is presumable that the compensatory mechanism of the sympathetic nerves does not greatly act, and the burden on the heart increases. As for 20100321, the compensatory mechanism of the sympathetic nerves acts, and it is presumable that this state is the best among these three cases. It is presumable that the present subject has a strong heart function because high stability is exhibited in various situations.

Fig. 36 is data of Subject YA (62 years old at the time of data measurement), and in the case of Subject YA, the measurement is conducted both in a sitting posture and in a recumbent posture. 20100930 is the data at the time when lung metastasis was found after an operation of a thyroid cancer. Although the sitting posture data of 20100930 falls within the inner circle in the analysis determination means A, the separation distance from the origin is very short, revealing the state of enduring in a slump state. In the analysis determination means B, although the sitting posture data of 20100930 falls within the inner circle, large change is observed, revealing the state that he is easily get out of condition.

Although not illustrated in the drawing, the recumbent posture data of 20100930 is estimated with reference to the inner circle having a radius of the first separation distance "6" and the outer circle having a radius of the second separation distance "15". In the analysis determination means A, the data reaches near the outer circle, and the separation distance is long, so that it can be estimated that the state is near the state resisting in a slump state.

The data 20110121 and 20110717 were collected on the days lacking administration of magistral drugs, and can be said to be a state enduring in a slump state, and a long separation distance is observed in the analysis determination means B for recumbent posture data of 20110717. This can be estimated as a state close to the state of resisting in a slump state.

The subject in Fig. 36 sleeps during the measurement in the recumbent posture data of 20110717. In other states, the subject is awake. In comparison between data of a recumbent posture, the data of sleep in the analysis determination means B shows a longer separation distance. This suggests the possibility of estimating whether the state is sleep or wakeful, and the quality of sleep in the sleeping state by examining by making the settings of the first separation distance and the second separation distance different between a recumbent posture and a sitting posture. It is estimated that the present subject keeps stability as a result of occurrence of the sympathetic compensatory mechanism in various situations rather than by the strong function of his heart. This can also be estimated by large fluctuation in change rate rather than by the degree of change in the state.

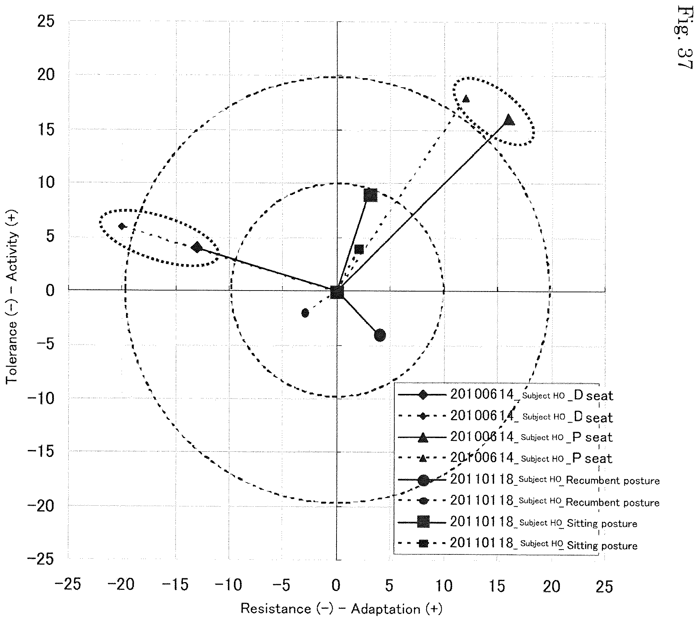

In Fig. 37, the analysis results by the analysis determination means A, B for Subject HO are shown together. The solid line represents the analysis result by the analysis determination means A, and the dotted line represents the analysis result by the analysis determination means B. Subject HO is 89 years old, and the data of 20100614 includes coordinate points located outside the outer circle, suggesting that a bad physical condition can sometimes occur. In the data of 20110118, there is a coordinate point at a separation distance of 1/2 or less of the inner circle, revealing the state enduring in a slump state. The present subject shows a decreasing trend of chaos, and it is estimated that the bad physical condition advances gradually. In the reducing trend of the change rate, an increased state of the sympathetic nerve system is observed for a long time, suggesting the possibility of occurrence of a problem in perpetuity of the sympathetic compensatory mechanism. However, decrease in cardiac function is not observed.

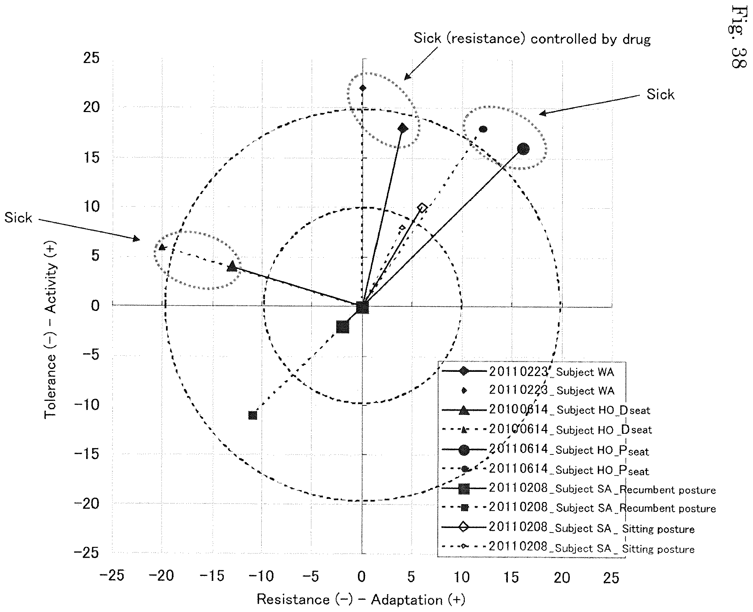

Fig. 38 is a diagram illustrating the analysis results for Subjects WA and SA in addition to the subject in Fig. 37. Also Subject WA shows a separation distance reaching outside the outer circle, and it is suggested that Subject WA is in a slump state and is resisting the factor of the bad condition. On the other hand, Subject SA shows a separation distance of 1/2 or less of the inner circle in the analysis result by the analysis determination means A, revealing the existence of the state enduring in a slump state. Among these three persons, it can be said that Subject SA is healthy. This is presumably because, for example, sympathetic function is increased in a sitting posture, and decrease in sympathetic function is observed in a recumbent posture, and a so-called chaos is well reserved, and the degree of advance of the bad physical condition is small.

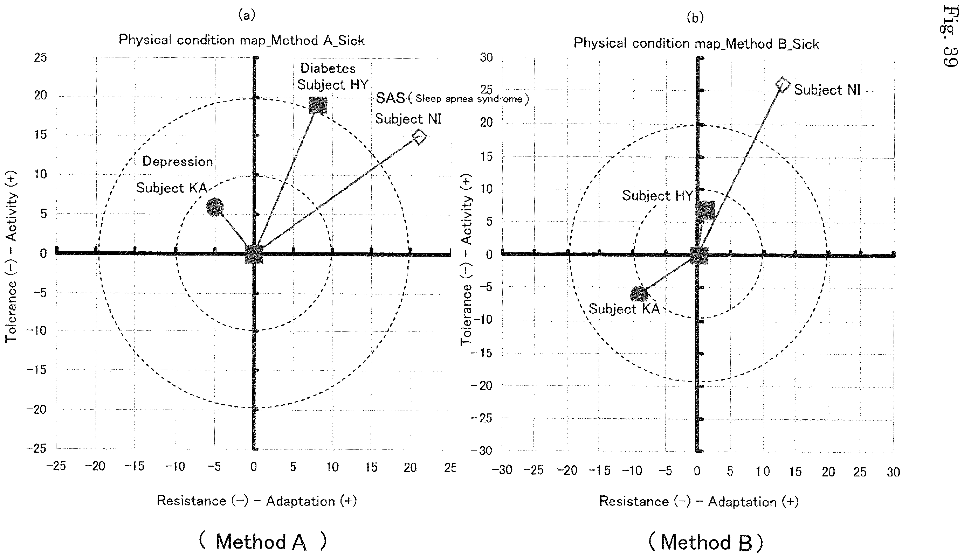

Fig. 39 is a diagram illustrating the analysis results by the analysis determination means A, B for Subject KA suffering from depression, Subject HY suffering from diabetes, and Subject NI suffering from SAS (sleep apnea syndrome). As illustrated in this diagram, in the case of Subjects HY and NI, the coordinate points project outside the outer circle in at least either one of the analysis determination means A, B, and this indicates that they are resisting in a slump state. In the case of Subject KA suffering from depression, the subject is not determined as being sick by the separation distances in the analysis determination means A, B alone, but determined as being in a normal state. The subject shows a displacement in the negative direction of the left half of Fig. 39, namely the second and the third quadrants. Therefore, both in the analysis determination means A, B, even when the separation direction is the negative direction, it is possible to estimate that the subject is in a state suffering from a disease such as depression. As to the cardiac function, Subject HY shows significant increase in function of the sympathetic nerve system, so that the subject is presumed to also suffer from hypertension. The aforementioned Subject HO shows a similar tendency. Both subjects suffer from diabetes. Subject NI, a patient suffering from SAS, shows a fatigued state that is different from that of a healthy person even during the daytime because of insufficient sleep at night. The continuing increased state of the sympathetic function promotes a sense of fatigue. Also the fluctuation rate to the external stress is high and chaos is high. It can be recognized that the autonomic nervous function vigorously in the high chaos. It seems that the blood pressure is naturally high.

### (Test Example 6)

The analysis determination means A determines a physical condition change score by "determination criterial score of subsequent time range (second point) + (determination criterial score of subsequent time range (second point) - determination criterial score of previous time range (initial position)) × n, (wherein, n is correction coefficient) as described above (in the above, n (correction coefficient) = 3).

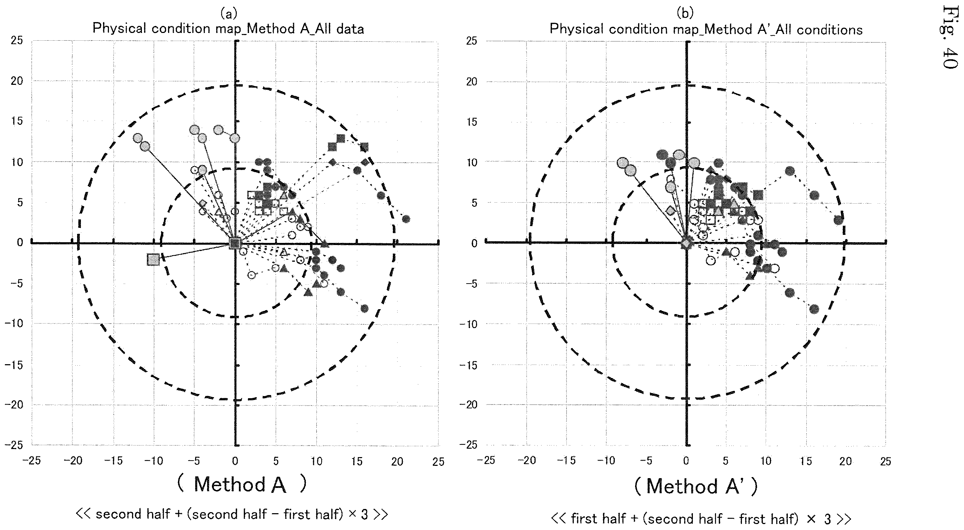

As a result, it is possible to emphasize the change in the subsequent time range with reference to the previous time range; however, just for comparison of these, change in the previous time range with reference to the subsequent time range may be adequate. Fig. 40(a) is a diagram illustrating the analysis results by the afore-mentioned analysis determination means A determined by "determination criterial score of subsequent time range (second point) + (determination criterial score of subsequent time range (second point) - determination criterial score of previous time range (initial position)) × 3", and Fig. 40(b) is a diagram illustrating the analysis results determined by "determination criterial score of previous time range (initial position) + (determination criterial score of subsequent time range (second point) - determination criterial score of previous time range (initial position)) × 3". The data employed herein is as same as the data of Fig. 18 shown in Test Example 1.

From this result, it is possible to emphasize the change in Fig. 40(a), or it is possible to easily grasp the state change by emphasizing abnormal signals occurring in 0.0033 to 0.04 Hz, in particular, 0.01 to 0.04 Hz of the ultralow-frequency band. Contrarily, in Fig. 40(b), change is not emphasized so much, and the state is difficult to be grasped. In other words, it can be recognized that the change is much emphasized by putting a change by function fluctuation on the subsequent state occurring by the function fluctuation rather than by putting a change by the function fluctuation on the previous state. Therefore, in the analysis determination means A, it can be realized that determination is conducted in the manner as illustrated in Fig. 40(a).

### (Test Example 7)

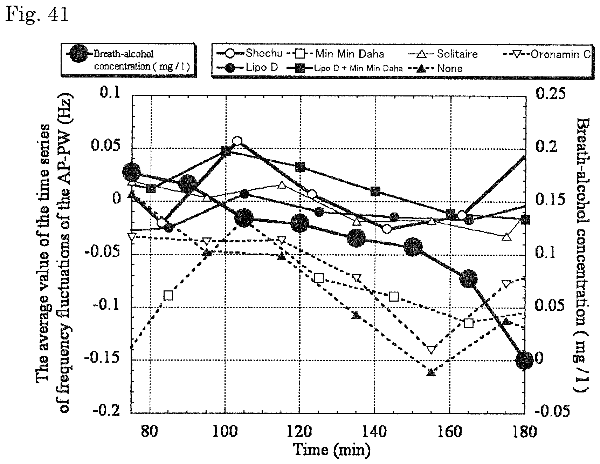

Frequencies of data of "Uchikawa-shochu", data of drinking Nutrient Drinks A, B, C, and data in a fatigued condition of Subject Uchikawa in Test Examples 1 and 2 were analyzed to obtain time-series waveforms of dominant frequencies (see Fig. 41). Also, time transition of the breath-alcohol concentration measured simultaneously with collection of data of "Uchikawa-shochu" is shown in the same data.

By determining the time-series waveforms of the dominant frequencies, it is possible to provisionally determine the case where the change occurs above the time transition of the breath-alcohol concentration as being "drunk" of a predetermined amount (amount corresponding to refresh state in drunkenness degree classification), and the case where the change occurs below as "other states including non-drunk". This is based on the fact that the change in dominant frequency is correlated with the change in fluctuation of the ultralow-frequency band. However, in Fig. 41, not only the data of shochu, but also the data of drinking Nutrient Drink A, the data of drinking both Nutrient Drinks A, C, and the data of playing a game show similar results above the time transition of the breath-alcohol concentration, and it is difficult to distinguish the drunk state from non-drunk state among these set of data. As described above, regarding the dominant frequencies of biological signals in the transition states of drunk, drowsiness, imminent sleeping phenomenon and the like, since the biological signals show irregular vibration waveforms, they are peaks of power spectra of the irregular vibration waveforms. Therefore, they are unlikely to be such clear peaks as a resonance curve for harmonic input occurring in a relaxed or strained state.

On the contrary, according to the analysis determination means A, B of the present invention, since change in fluctuation of the ultralow-frequency band having a correlation with change in dominant frequency is scored according to a predetermined criterion, and the scores are plotted as coordinate points and represented by vector, it is possible to grasp the biological state corresponding to change in fluctuation of the ultralow-frequency band more accurately, and the estimation result is more suited to the sense of a human being. Therefore, in both of the analysis determination means A, B, determining the case where a coordinate point is plotted in the donut-shaped region enclosed by the inner circle and the outer circle as being presence of "alcohol intake" of a predetermined amount (amount corresponding to refresh state in drunkenness degree classification) will give a determination result suited to the state of a human being.

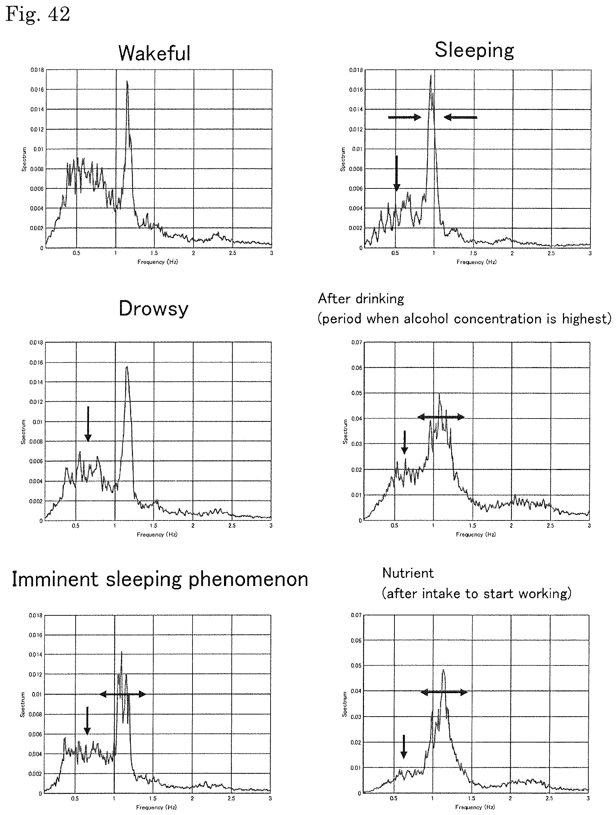

The above points will be further described based on Fig. 42 to Fig. 45. Fig. 42 is a diagram illustrating representative cases of FFT analysis results of original waveforms of aortic pulse waves (APW) in a wakeful state, drowsy state, imminent sleeping phenomenon, sleeping state, after drinking (the time when the alcohol concentration is highest), nutrient (after intake or beginning to take effect). As can be seen in this diagram, comparatively clear peaks appear in a wakeful state, drowsy state, sleeping state, nutrient (after intake or beginning to take effect), whereas irregular vibrations and a large number of peaks appear in the imminent sleeping phenomenon, and after drinking (the time when the alcohol concentration is highest). The appearances of the irregular vibrations allow to estimate that some external factors such as an imminent sleeping phenomenon or drinking have acted. The most predominant peak among the irregular vibration peaks is a dominant frequency, and it is also presumable that the dominant frequency differs depending on the external factor. However, it is difficult to identify the external factor according to the difference in dominant frequency by Fig. 42 alone. The nutrient as the external factor is not so perpetual as drinking, and the effect thereof vanishes in about several tens of minutes. In other words, the fluctuation score is smaller in either of the analysis determination means A and the analysis determination means B, and a different behavior from that of drunk is exhibited.

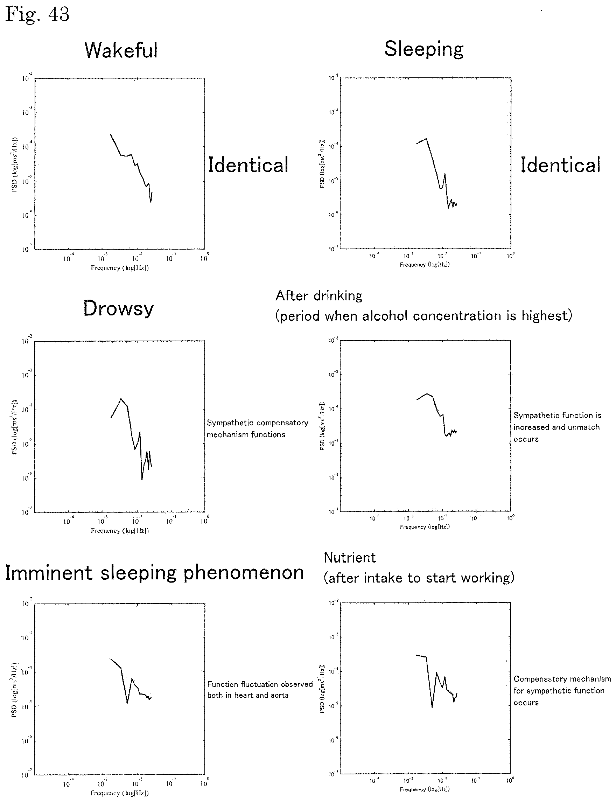

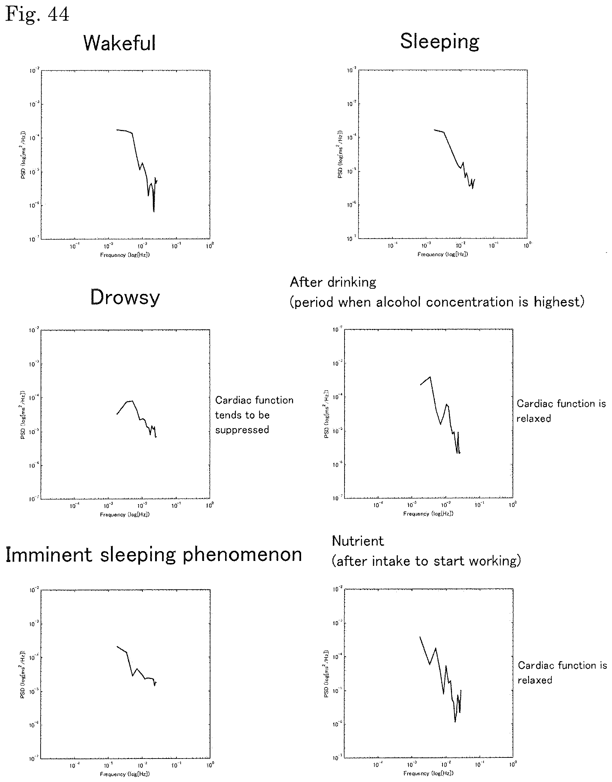

Fig. 43 is a diagram illustrating log-log graphs obtained by further analyzing the frequencies of frequency-gradient time-series waveforms obtained by the zero-cross detection means processing the original APW waveforms used in Fig. 42, and represents the state of the aorta or the state of the sympathetic nerves. Fig. 44 is a diagram illustrating log-log graphs obtained by further analyzing the frequencies of frequency-gradient time-series waveforms obtained by the peak detection means processing the original APW waveforms used in Fig. 42, and represents the state of the heart and the aorta or the state of the sympathetic and parasympathetic nerve systems. In these diagrams, since the change in fluctuation in the ultralow-frequency band of 0.001 to 0.04 Hz, particularly 0.01 to 0.04 Hz exhibits different waveforms according to change in the state of a human being, it is possible to grasp the difference in state more clearly than by Fig. 42.

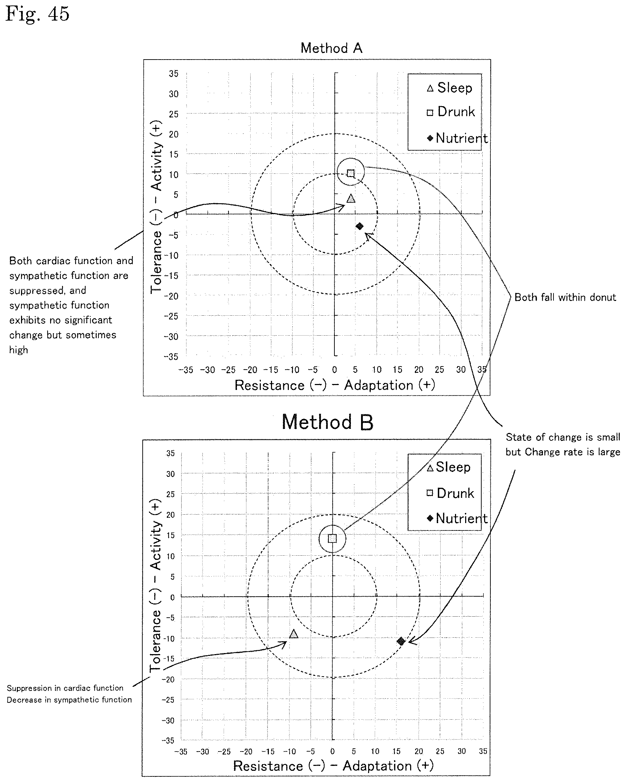

Fig. 45 is a diagram illustrating analysis results obtained by the analysis determination means A (Method A) and the analysis determination means B (Method B) using the data illustrated in Fig. 43 and Fig. 44. As can be seen from Fig. 45, as to the drinking, the plotted points fall within the donut-shaped region between the inner circle and the outer circle both in the analysis determination means A and the analysis determination means B. Regarding the case of drinking a nutrient, the basic change condition is small from the result of the analysis determination means A, but it is determinable that the rate of change is large from the result of the analysis determination means B. Regarding the sleeping, the analysis determination means A reveals that both the cardiac function and the sympathetic function are suppressed, and it can be said that the sympathetic function does not significantly change, and suppression of the cardiac function and the decrease in the sympathetic function are observed from the analysis determination means B. These results reveal that the analysis determination means A (Method A) and the analysis determination means B (Method B) make it possible to make determination while enlarging the change in dominant frequency correlated with the change in fluctuation of the ultralow-frequency band.

### (Determination of first separation distance and second separation distance)

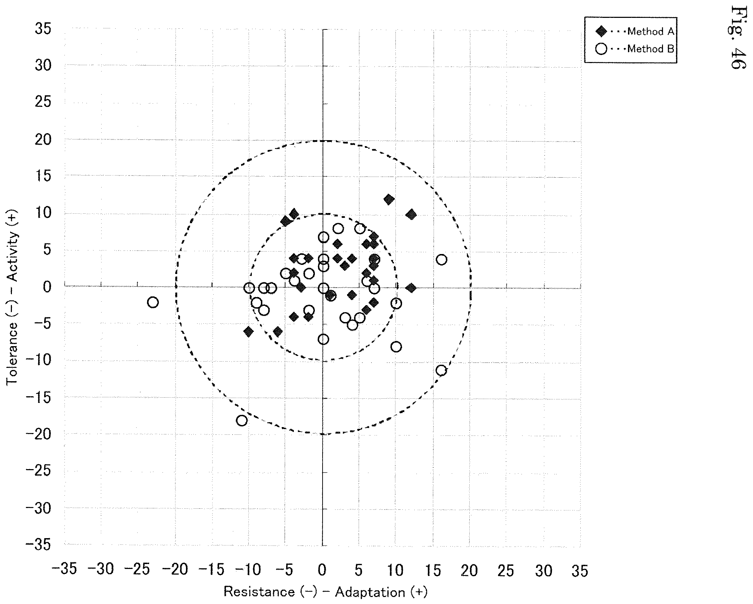

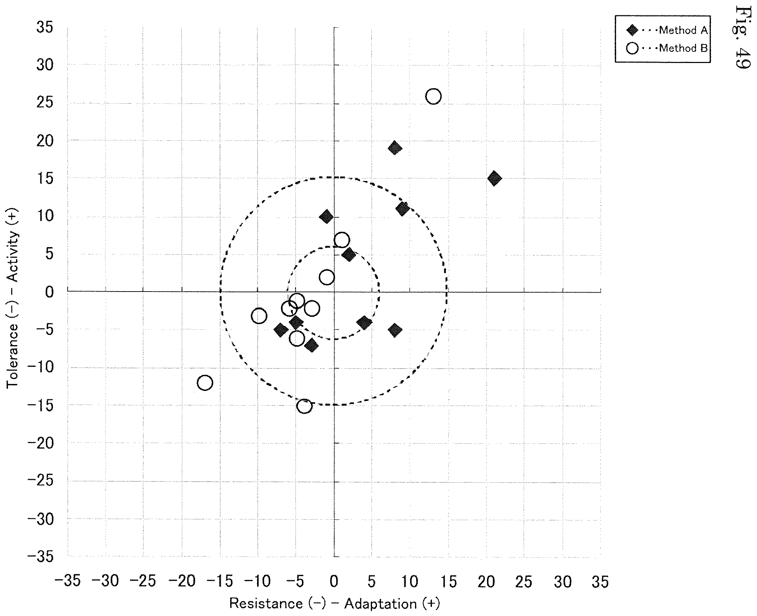

Fig. 46 to Fig. 49 summarize the results of the above Test Examples, and in these diagrams, coordinate points in the second analysis time segment obtained by the analysis determination means A (Method A) and the analysis determination means B (Method B) are plotted for each subject. Fig. 46 is a diagram illustrating coordinate points for each subject obtained from the data measured in an automobile drive test when the subjects drink Nutrient Drinks A to C in Fig. 22, Fig. 23, Fig. 27, Fig. 28, and Fig. 32 to Fig. 34; Fig. 47 is a diagram illustrating coordinate points for each subject obtained from the data at the time of alcohol intake in Fig. 20, Fig. 25, Fig. 27, and Fig. 30; and Fig. 48 is a diagram illustrating the coordinate points for each subject obtained from the data in sitting postures in a slump state (including the sick state) in Fig. 24, Fig. 29, Fig. 36, Fig. 37, and Fig. 38. Fig. 49 is a diagram illustrating coordinate points in the second analysis time segment obtained in recumbent postures from the healthy subjects and the sick subjects in Fig. 35, Fig. 36, Fig. 37, and Fig. 38.

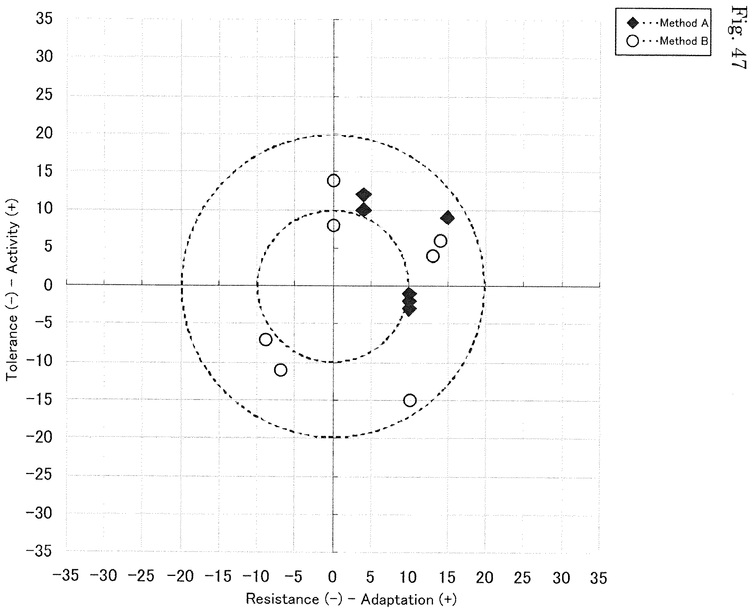

Among these, data of Fig. 46 to Fig. 48 were obtained by measurements in sitting postures. At first, these data are compared. Fig. 47 shows that most of the coordinate points are located within the donut-shaped region situated between the separation distances of about 10 and about 20 from the origin in the case of alcohol intake of a predetermined amount (amount corresponding to a refresh state in drunkenness degree classification). By superimposing Fig. 42 and Fig. 44 in reference to this, it is possible to clearly distinguish from the case of alcohol intake of a predetermined amount (amount corresponding to a refresh state in drunkenness degree classification) with the inner circle having a radius corresponding to the first separation distance "10" and the outer circle having a radius corresponding to the second separation distance "20" as boundaries. In this manner, it is possible to determine the first separation distance and the second separation distance that form the donut-shaped region centering on the distribution region of the coordinate points concentrated in the case of alcohol intake of a predetermined amount (amount corresponding to a refresh state in drunkenness degree classification). For example, the point where more than or equal to a predetermined percentage, for example, 80% or more, preferably 90% or more, and more preferably 95% or more of the coordinate points in the case of alcohol intake are distributed in the donut-shaped region can be calculated and determined from the distribution state.

In the present embodiment, by conducting tests for the normal state (general fatigued state), the slump state (including illness), and the moderate (amount corresponding to a refresh state in drunkenness degree classification) alcohol intake state (the state realized by a function recovery means of moderate alcohol) in a certain number of subjects, it is possible to estimate each state according to whether or not it is an alcohol intake state of a predetermined amount (amount corresponding to a refresh state in drunkenness degree classification) using the first separation distance and the second separation distance determined in the above. However, since the numerical values of the first separation distance and the second separation distance differ between individuals, for example, for each subject, data regarding the normal state, data regarding the slump state, and data regarding the alcohol intake state of a predetermined amount (amount corresponding to a refresh state in drunkenness degree classification) are acquired in advance, and the actual state may be estimated by comparison with the individual data. For example, a long-haul truck management company may store data of individual drivers in advance as database in a computer, and analyze data obtained by the biological signal measuring means collected at the end of driving individually, and compare with the registered data of the driver to determine presence of alcohol intake. Of course, the aforementioned first separation distance "10" and the second separation distance "20" may be used as setting values.

Since a large resistance occurs between 0.001 and 0.04 Hz when the basic state turns into a sudden change state, by determining the position where the large resistance occurs by calculation, it is of course possible to determine the separation distance.

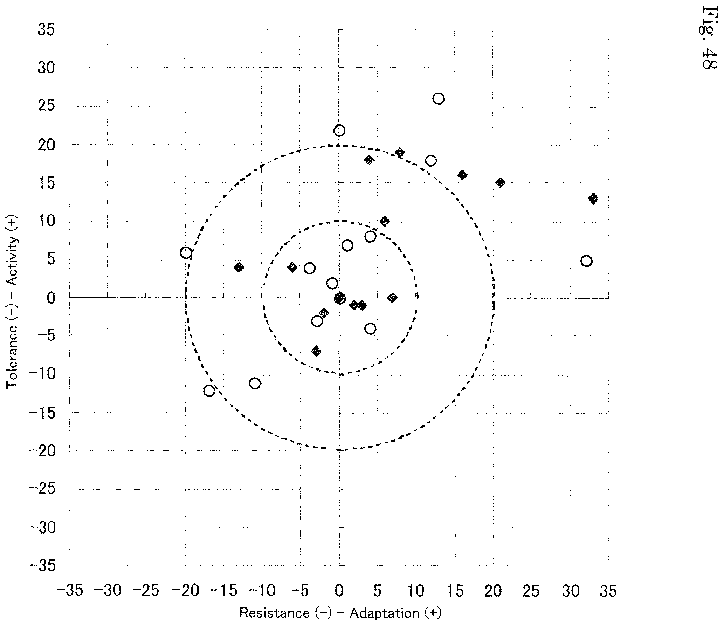

As described above, by determining the first separation distance and the second separation distance and setting the donut-shaped region, the case of Fig. 46 will be determined as the normal state as described in the above test example because coordinates points in either one of the analysis determination means A (Method A) and the analysis determination means B (Method B) are located inside the inner circle. Fig. 47 is determined as the alcohol intake state of a predetermined amount (amount corresponding to a refresh state in drunkenness degree classification) because the coordinate points are plotted within the donut-shaped region. In the case of Fig. 48, the coordinate points are distributed inside the inner circle and outside the outer circle. In the case of outside the outer circle, it can be determined as the state resisting in a slump state, but in the case of inside the inner circle, it may be difficult to be distinguished from the normal state of Fig. 42. So, in the case of inside the inner circle, it may be set so that the state is determined as a state enduring a bad condition factor in a slump state when the distance from the origin is less than or equal to a predetermined distance, for example, 1/2 or less of the inner circle, or 1/3 or less of the inner circle. If the distinction from the normal state is still unclear, it is preferred to use the estimation result by the second analysis determination means 952 as will be described later together.

On the other hand, in the case of a recumbent posture as shown in Fig. 49, many of the coordinate points are located inside the inner circle when the donut-shaped region of 10 to 20 determined in sitting postures is applied. This is ascribable to the fact that in a recumbent posture, the influences of the heart and the aorta are basically minimal, and the parasympathetic nerve system tends to be predominant and the basic condition is in a good state, so that the change is small. Therefore, in determination of a recumbent posture, the first separation distance is set at 6 and the second separation distance is set at 15 with reference to the data of Subject HO who was healthy among the measurement subjects in recumbent postures. In other words, it is necessary to make determination according to different criterial settings for a recumbent posture and a sitting posture.

However, in the case of a recumbent posture, it was also found that in a subject in a state of resisting (struggling against) a disease, the coordinate points largely project outside the outer circle and the change is large. On the other hand, in the period when the symptom is controlled by the action of the drug, the coordinate points shift to inside the outer circle or inside the inner circle, and the change tends to be small as the state gets close to the calm state. However, when a subject not being aware of his/her illness undergoes the measurement, there is a risk of being determined as the alcohol intake state, for example, when the coordinate points are distributed within the donut-shaped region. Also in this case, it is possible to estimate the state more accurately by using the estimation result by the later-described second analysis determination means 952 together.

### (Second analysis determination means 952)

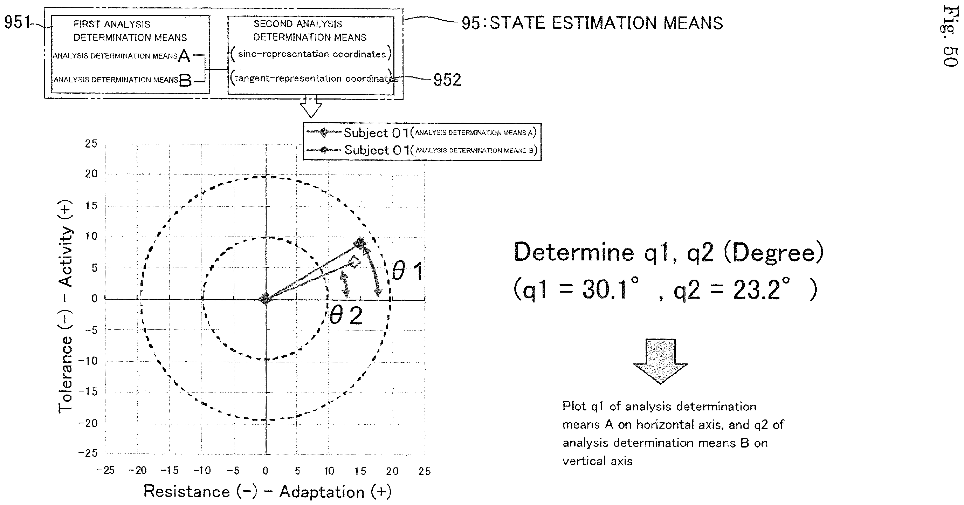

Next, the second analysis determination means 952 that is established in the state estimation means 95 together with the first analysis determination means 951 (analysis determination means A, B) will be described. The second analysis determination means 952 converts positions on the coordinate system of the coordinate points in the analysis time segment to be analyzed plotted by the analysis determination means A and B into a trigonometric function display, and replots them on a new coordinate system, and estimates a biological state based on the positions of the replotted coordinate points. The purpose of this trigonometric function display is to take a difference (| Peak-0x |) of the analysis results obtained by the peak detection means (Peak) and the zero-cross detection means (0x) by representing the coordinates obtained by the peak detection means and the zero-cross detection means in a trigonometric function display, to thereby check the data in which sensitivity of the cardiac function is improved by eliminating the influence of the aorta and the data in which sensitivity of the aorta function is improved. In other words, the rates of contribution of the aorta and the heart which are dependent variables can be grasped by an angle. Naturally, a gray zone where the rates of contribution of these compete with each other is shown. However, by checking both the first analysis determination means 951 (analysis determination means A, B) and the second analysis determination means 952, a test that is sensitive to the heart is achieved. In other words, for the plotted coordinate points, determination is made by checking the combination of a radius component centering on the origin and an angular component. Concretely, as shown in Fig. 50, for each of the coordinate points obtained by analysis determination means A and the coordinate points obtained by the analysis determination means B, an angle corresponding to a trigonometric function display is determined. And the angle corresponding to a trigonometric function display of each coordinate point obtained in the analysis determination means A is plotted on one axis, and the angle corresponding to a trigonometric function display of each coordinate point obtained in the analysis determination means B is plotted on the other axis, to prepare trigonometric function display coordinates.

To be more specific, the second analysis determination means 952 of the present embodiment has means for preparing sine-representation coordinates plotting an angle of sin of each coordinate point obtained in the analysis determination means A on one axis (horizontal axis in the present embodiment) and an angle of sin of each coordinate point obtained in the analysis determination means B on the other axis (vertical axis in the present embodiment), and means for preparing tangent-representation coordinates plotting an angle of tan of each coordinate point obtained in the analysis determination means A on one axis (horizontal axis in the present embodiment) and an angle of tan of each coordinate point obtained in the analysis determination means B on the other axis (vertical axis in the present embodiment), and estimates a biological state based on the positions of each coordinate point in the sine-representation coordinates and in the tangent-representation coordinates.

The second analysis determination means 952 analyzes the coordinate points by the analysis determination means A and B of the first analysis determination means 951 from different directions by making determination while the coordinates are converted into a trigonometric function display. By using both of the results by the first analysis determination means 951 and the second analysis determination means 952, it is possible to estimate the state more accurately.

### (Estimation of inadequate-to-drive state (difficult-to-perform task state) due to a slump state)

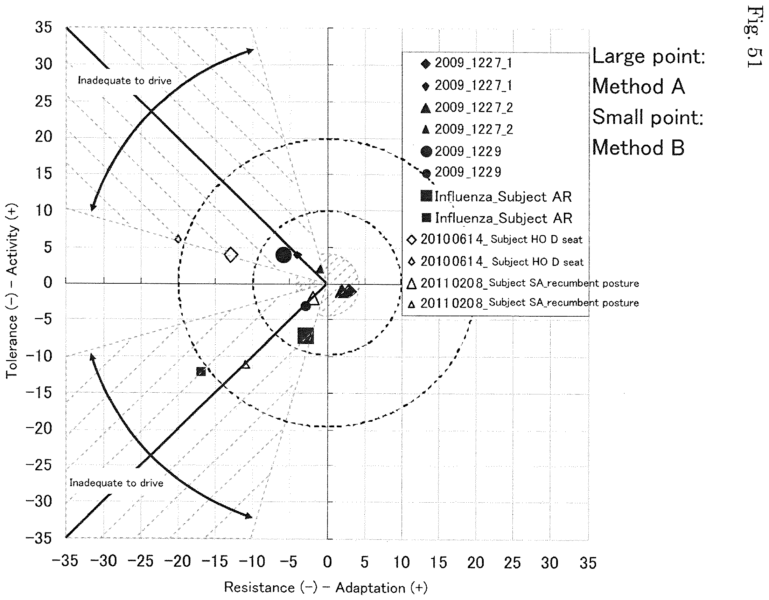

Fig. 51 is a diagram illustrating part of data of the analysis determination means A (Method A) and the analysis determination means B (Method B) of Subject JY in a bad physical condition of Fig. 24 and Fig. 29, data of the analysis determination means A (Method A) and the analysis determination means B (Method B) in slump (sick) states of Subjects HO and SA in Fig. 38, and data of the analysis determination means A (Method A) and the analysis determination means B (Method B) of Subject AR suffering from influenza.

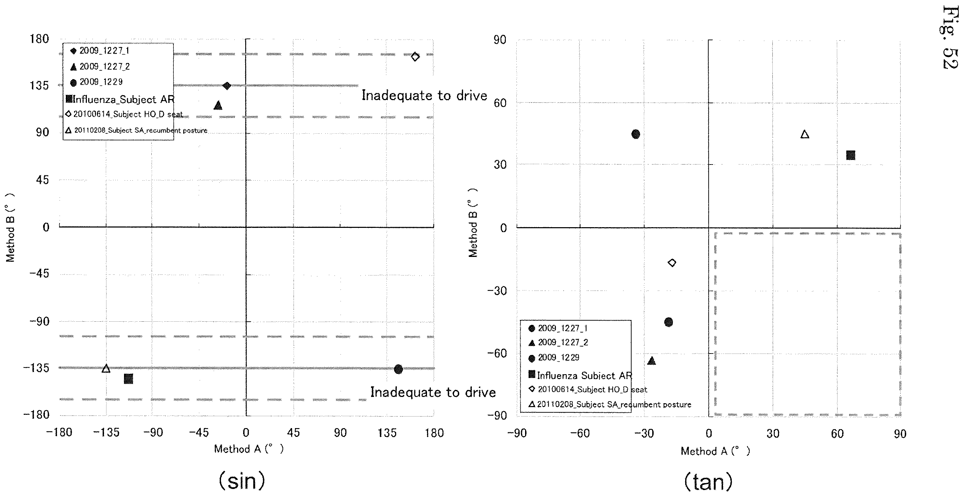

For each coordinate point of the analysis determination means A and B in Fig. 51, sin angles and tan angles are determined and sine-representation coordinates and tangent-representation coordinates are prepared, and shown in Fig. 52. The state of the subject in each test example is compared with the angle of sin, and when the plotted point is located within a predetermined angular range in the sine-representation coordinates (the range of ± 30 degrees centering on +135 degrees, and the range of ± 30 degrees centering on - 135 in the present embodiment), it is determined as "state lacking both physical strength and spirit". Similarly, when the plotted point is located exclusively in the first to third quadrants in the tangent-representation coordinates (when the plotted point is not located in the fourth quadrant), it is determined as "state lacking physical strength but raising spirit".

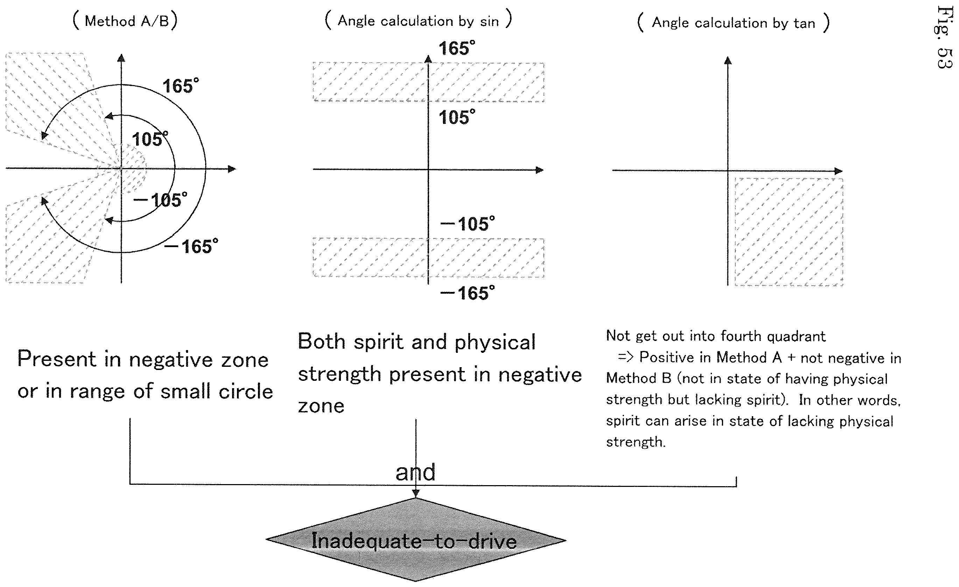

On the other hand, in the analysis determination means A and B of Fig. 51, the state of enduring the slump state corresponds to the region of a very short separation distance from the origin, for example, of 1/2 or less of the radius of the inner circle as described above. In Fig. 51, when there is a coordinate point that satisfies all of the requirements: a requirement that the coordinate point is plotted within the region covering this region and the second and the third quadrants which are negative zones of the analysis determination means A and B, a requirement that the coordinate point is plotted in the zone corresponding to the "state lacking both physical strength and spirit" in the sine-representation coordinates shown in Fig. 52, and a requirement that the coordinate point is not plotted in the fourth quadrant shown by the tangent-representation coordinates in Fig. 52, it is determined that the subject corresponding to the coordinate point is in a state of being difficult to perform a task such as driving, and being difficult to be controlled by autonomic nerves at the point of time when the biological signal data is collected as shown in Fig. 53. Here, taking driving as an example, determination of "inadequate-to-drive state" is made when the above three requirements are satisfied. This is just an example, and it may be determined as "difficult-to-perform task state" indicating difficulty in performing various tasks of a degree of difficulty comparable to driving when the above three requirements are satisfied.

### (Estimation of inadequate-to-drive state (difficult-to-perform task state) by drinking)

Using data of subjects at the time of drinking in Fig. 20, Fig. 21, and Fig. 26, and data of Subject KA at the time of drinking in Fig. 30, analysis by the second analysis determination means 952 was conducted (the analysis was conducted for the biological signals after 15 to 60 minutes from drinking).

It goes without saying that the subject is in the inadequate-to-drive state regardless of the amount when he/she is drunk, and the subject cannot drive when the coordinate point is plotted in the donut-shaped region in the analysis determination means A, B, namely when it is determined as drunk. On the other hand, the penalty for "drunk-driving" is imposed in the case of 0.15 mg/l or higher in terms of breath-alcohol concentration, and it is desired to be able to strictly check the drunk-driving after drinking the amount corresponding to this concentration.

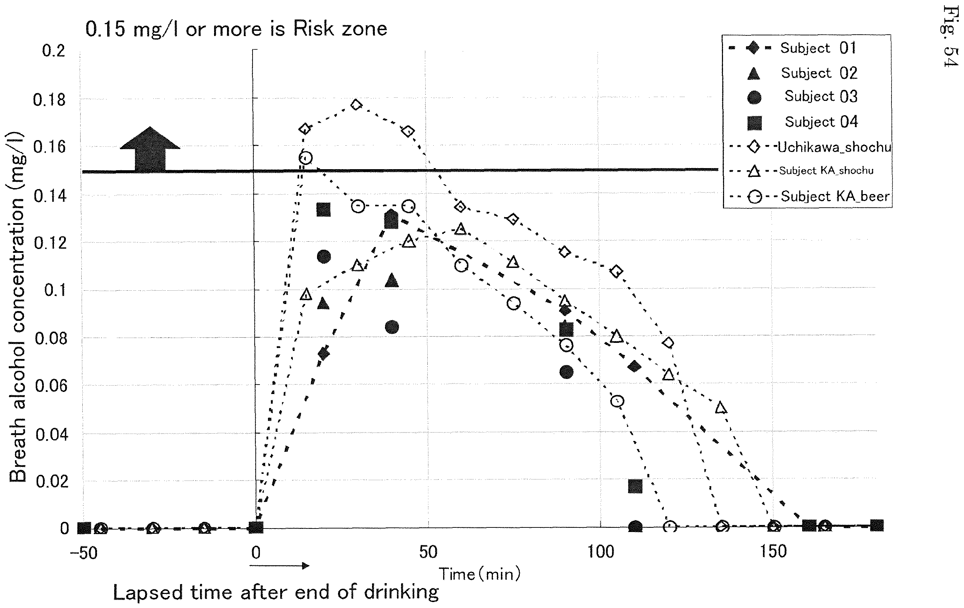

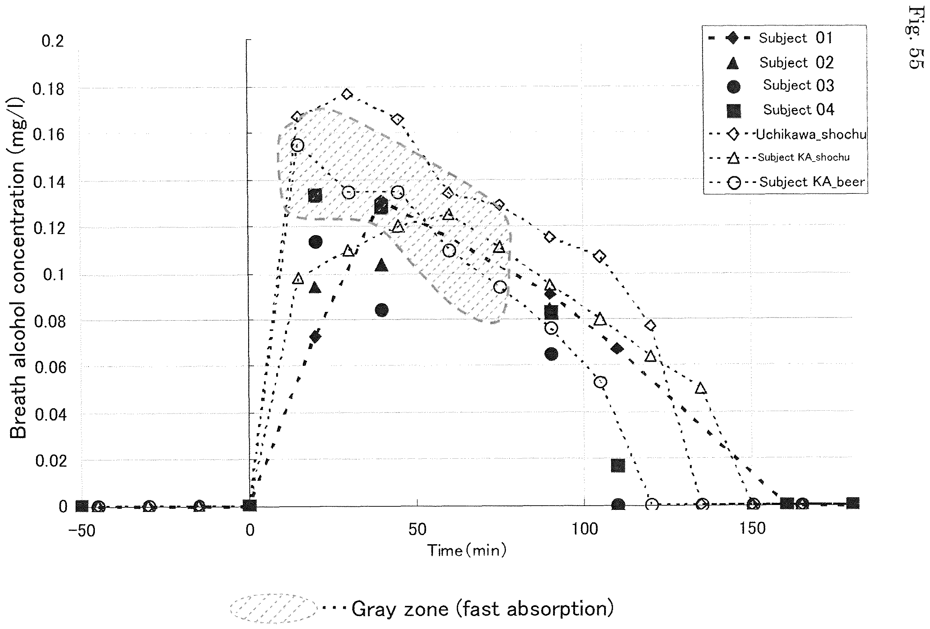

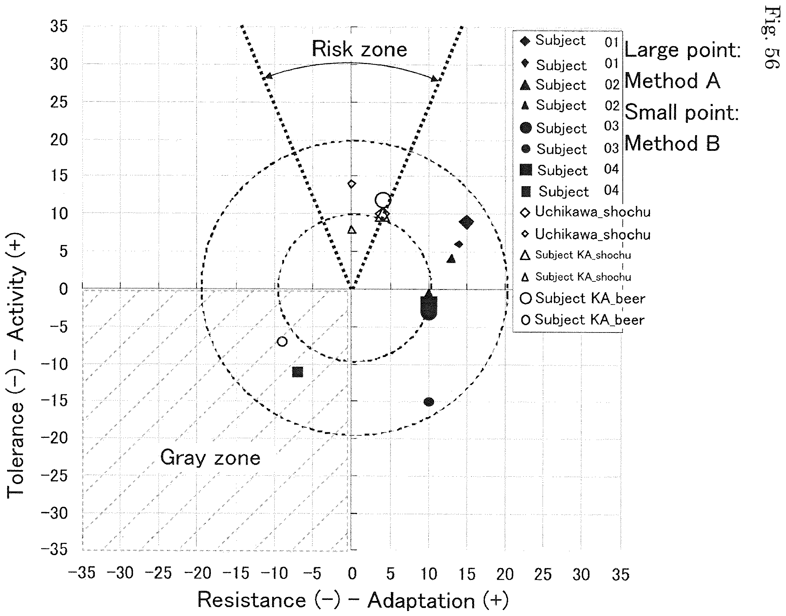

First, the breath-alcohol concentration of 0.15 mg/l or more of each subject shown in Fig. 54 is assigned to a risk region, and the shaded zone shown in Fig. 55 is assigned to a gray zone because there is a tendency of quick absorption of alcohol. Fig. 56 illustrate the risk zone and the gray zone correspondingly in the coordinate systems of the analysis determination means A and B.

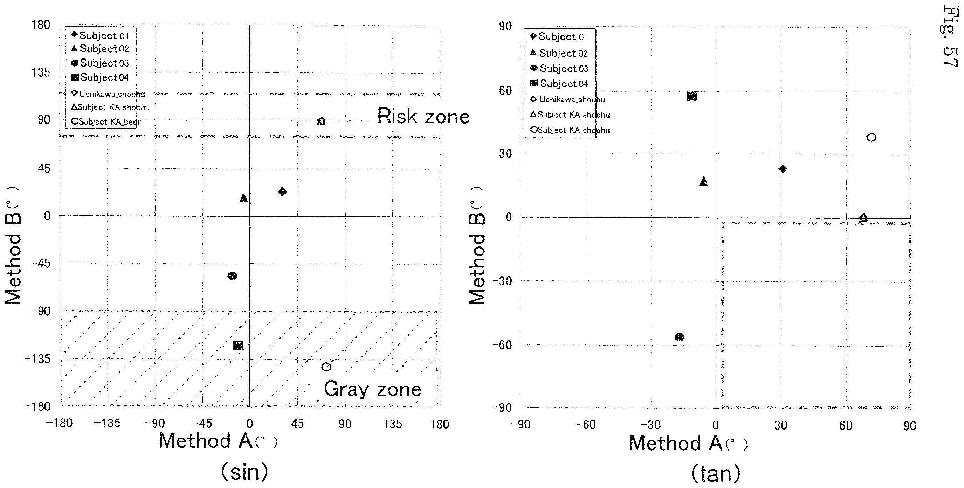

Fig. 57 is a diagram illustrating the sine-representation coordinates and the tangent-representation coordinates of all coordinate points plotted in Fig. 56. In the sine-representation coordinates, the risk region and the region corresponding to the gray zone in Fig. 56 are shown together.

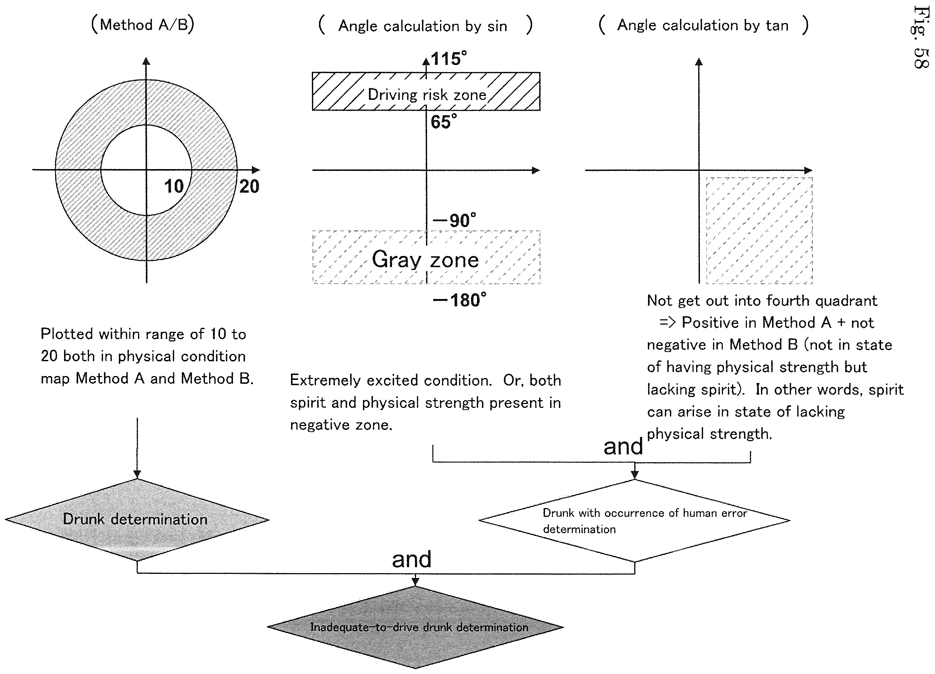

Fig. 58 is a diagram illustrating a determination method using the analysis results of the first analysis determination means 951 and the second analysis determination means 952 as described above. First, when there is a coordinate point plotted in the donut-shaped region by the analysis determination means A and B of the first analysis determination means 951, it is determined as drunk (alcohol intake amount corresponding to a refresh state). Next, when there is a coordinate point that satisfies both of the requirement that it is contained in the risk region (extreme excitement state) and the gray zone (negative zone) on the sine-representation coordinates, and the requirement that it is not plotted in the fourth quadrant on the tangent-representation coordinates, the subject corresponding to the coordinate point is determined as drunk to such an extent that a human error occurs at the time of sampling of the biological signal data. When all of the above three requirements are satisfied, "inadequate-to-drive drunk determination" corresponding to drunk-driving stipulated by the Road Traffic Law is made.

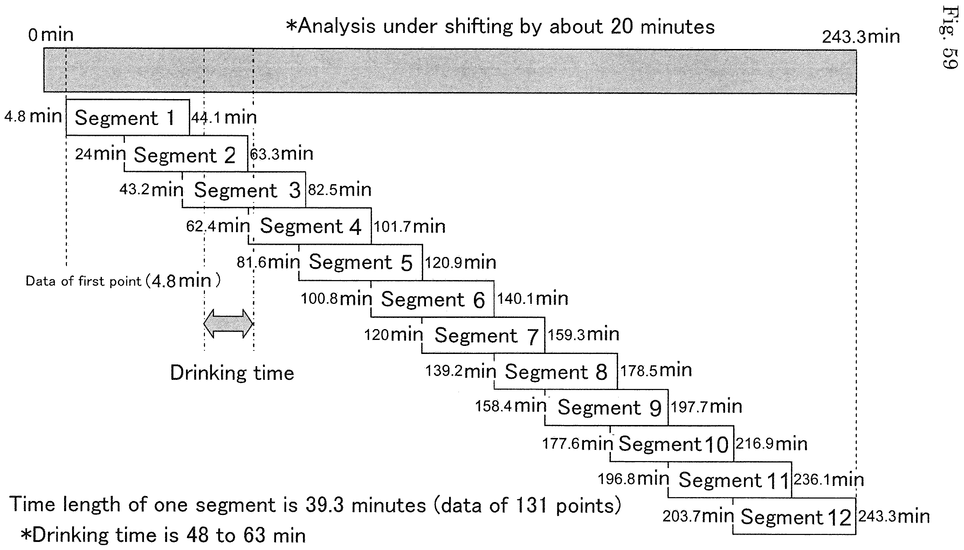

Fig. 59 to Fig. 63 illustrate the results of more detailed analyses of the data of Subject Uchikawa among the foregoing. Analysis time segments 1 to 12 set by sliding every about 20 minutes in the period from the start of the test (before drinking liquor) to the end of the test (after 243.3 minutes) were set as illustrated in Fig. 59 and analysis was conducted. During the period between 48 minutes and 63 minutes after start of the test, the subject drank liquor (1 go̅ (= about 0.181) of shochu).

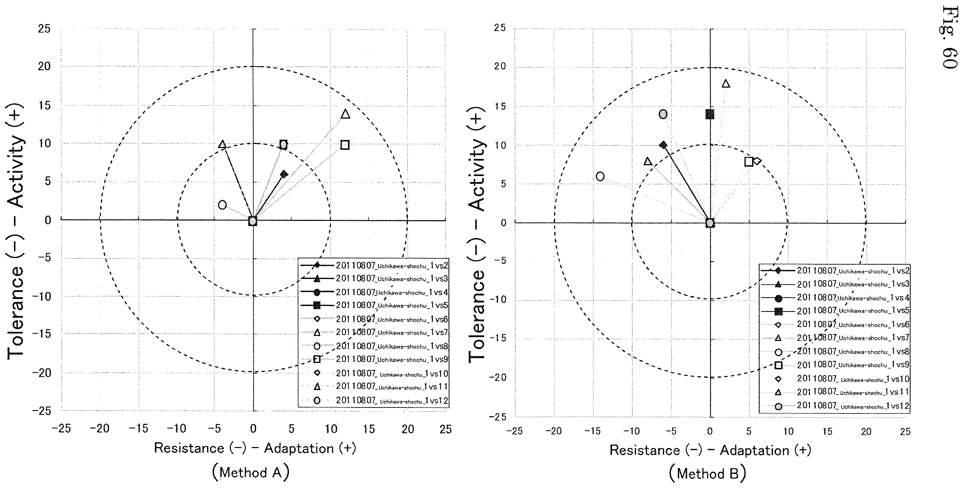

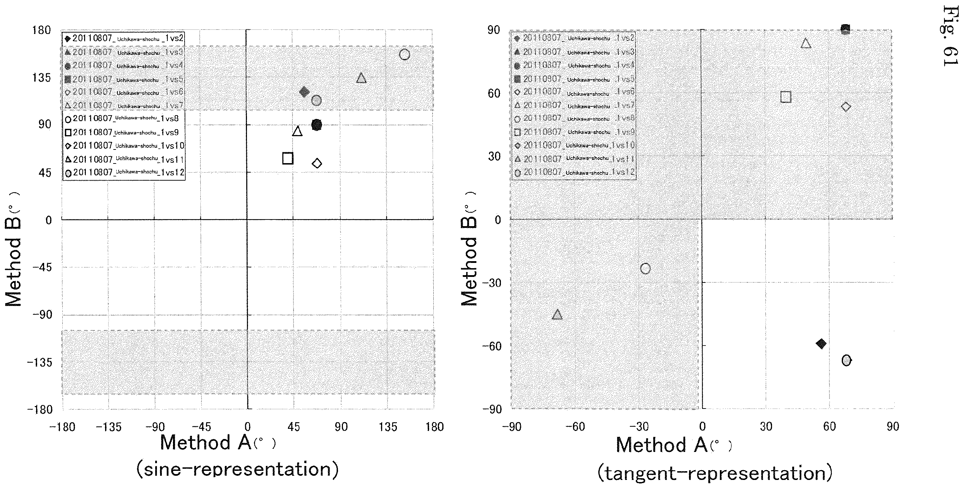

Fig. 60 is a diagram illustrating data of the analysis determination means A (Method A) and the analysis determination means B (Method B) for all the analysis time segments, and Fig. 61 is a diagram illustrating the sine-representation coordinates and the tangent-representation coordinates of all the coordinate points plotted in Fig. 60. This reveals that there is an analysis time segment corresponding to "inadequate-to-drive drunk determination" that satisfies all of the three requirements in Fig. 58.

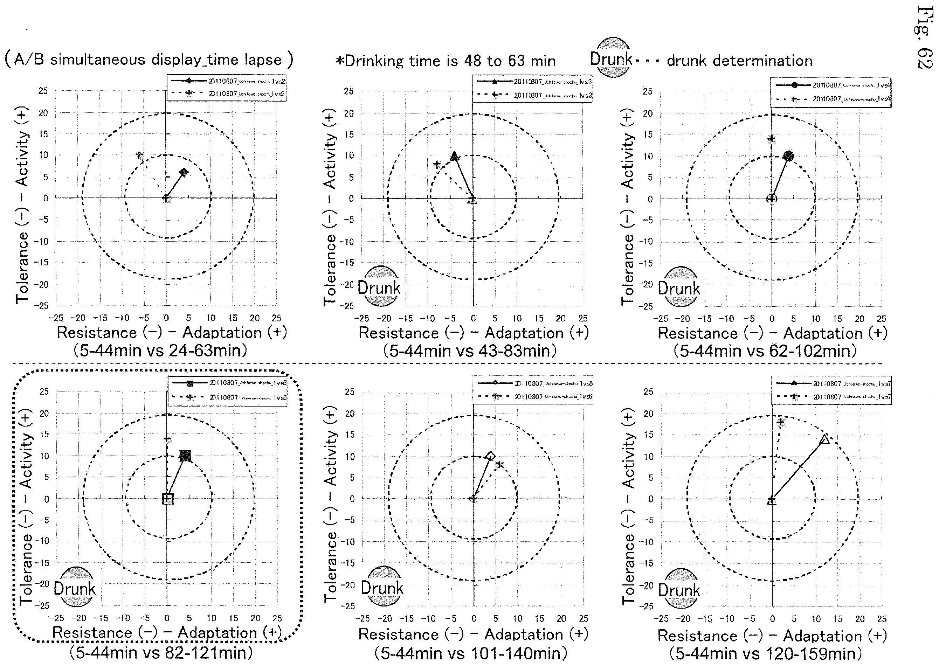

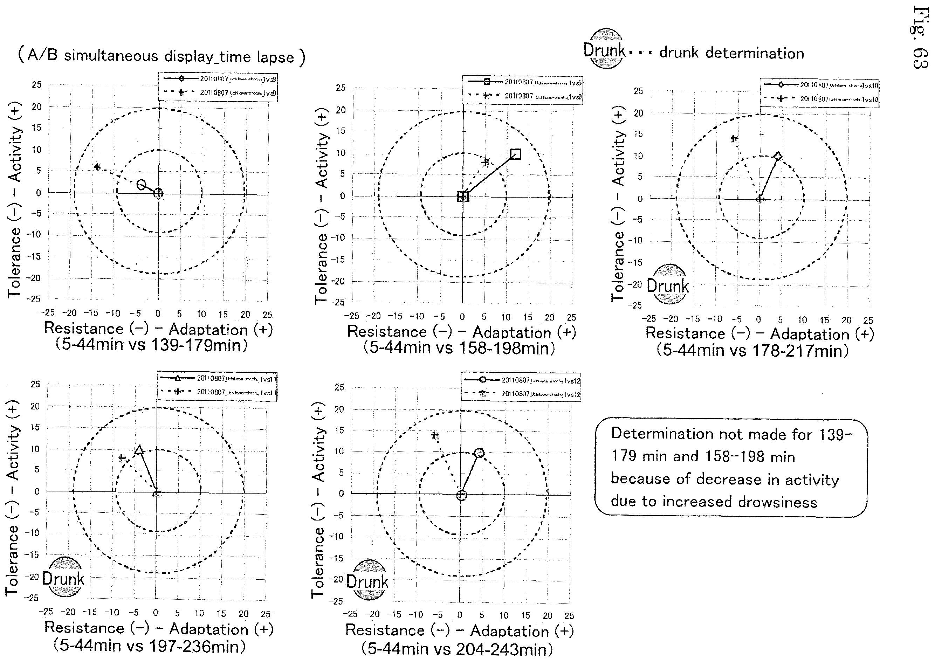

The results displaying the data of the analysis determination means A (Method A) and the analysis determination means B (Method B) together for each analysis time segment are illustrated in Fig. 62 and Fig. 63. After drinking during the period of 48 to 63 minutes, data of both Method A and Method B are contained in the donut-shaped region in most of the analysis time segments, and it is determined as "drunk". However, in the analysis time segment of 137-179 minutes, and in the analysis time segment of 158-198 minutes, it is not determined as "drunk". This determination would be wrong because it was determined as "drunk" in a later analysis time segment, and it is deemed that the cause of this error is decrease in activity due to increase in drowsiness of the subject.

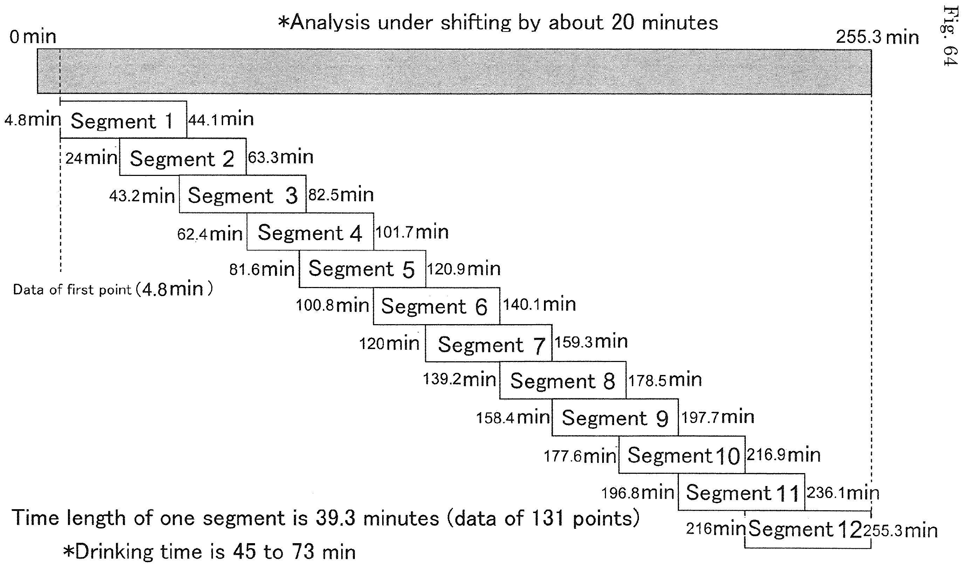

Fig. 64 to Fig. 69 illustrate the analysis results of Subject MA who is a small man in 20's and gets drunk very easily. The analysis time segments are as shown in Fig. 64, and analysis was conducted while setting the analysis time segments 1 to 12 by sliding every about 20 minutes in the period from the start of the test (before drinking liquor) to the end of the test (after 255.3 minutes). During the period between 45 minutes and 73 minutes after start of the test, he drank liquor (one bottle of beer).

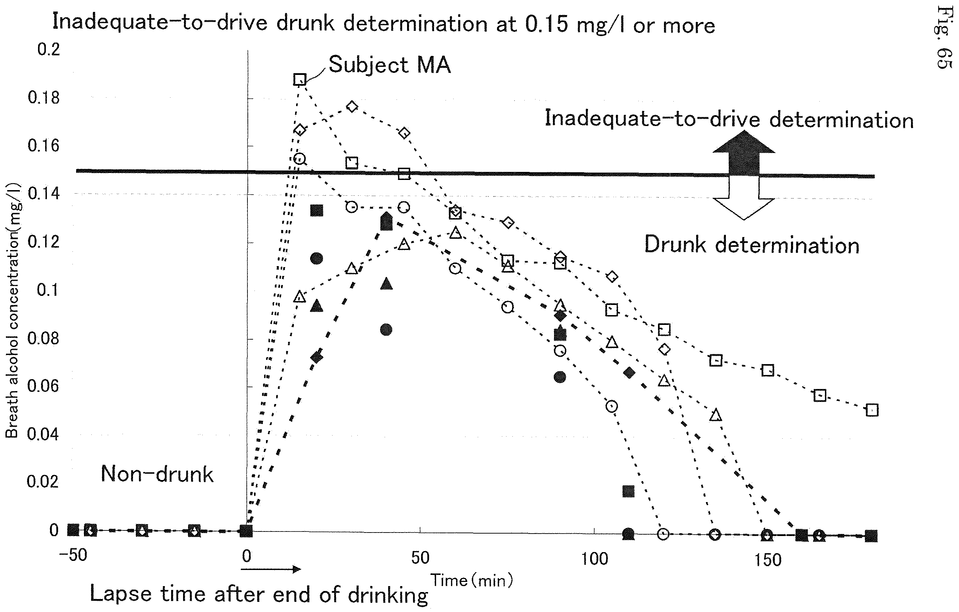

Fig. 65 is a diagram illustrating the breath-alcohol concentration of Subject MA, and as illustrated in this diagram, the highest value of the breath-alcohol concentration was 0.19 mg/l. This is near the upper limit of the range of a refresh state in drunkenness degree classification, and the state would be close to the drunkenness state because he was subjectively slightly intoxicated because he got easily drunk, and often closed his eyes during the test.

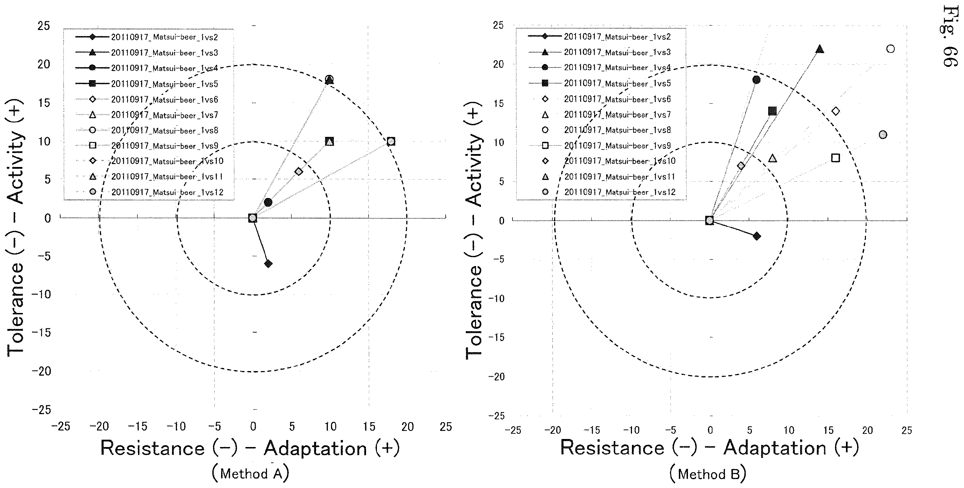

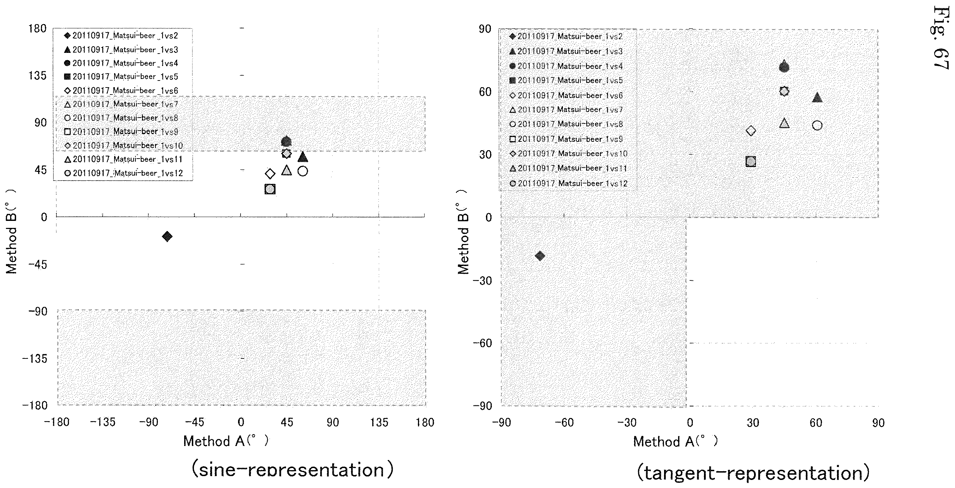

Fig. 66 is a diagram illustrating the data of the analysis determination means A (Method A) and the analysis determination means B (Method B) of all the analysis time segments, and Fig. 67 is a diagram illustrating the sine-representation coordinates and the tangent-representation coordinates of all the coordinate points plotted in Fig. 66. This reveals that there is an analysis time segment corresponding to "inadequate-to-drive drunk determination" that satisfies all of the three requirements in Fig. 58.

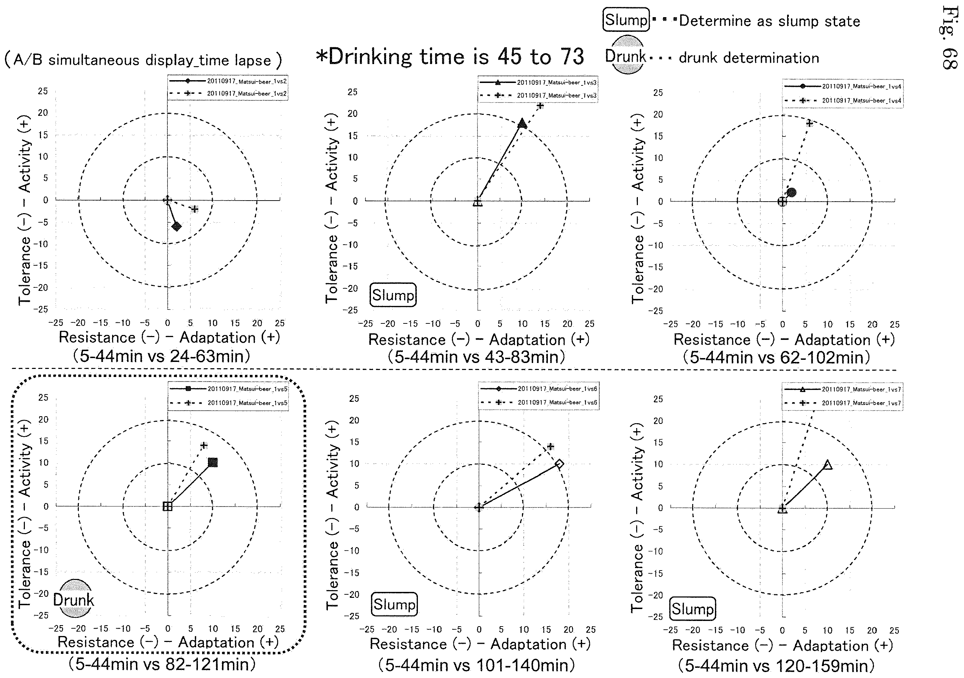

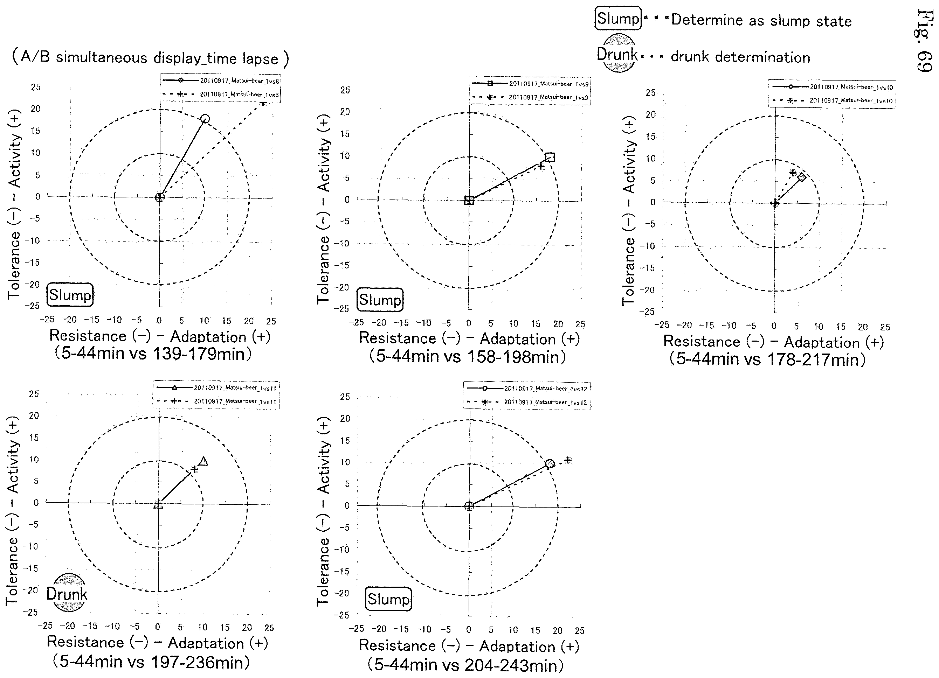

Fig. 68 and Fig. 69 illustrate the results displaying the data of the analysis determination means A (Method A) and the analysis determination means B (Method B) together for each analysis time segment. In the case of Subject MA, in the analysis time segment of 82-121 minutes and in the analysis time segment of 179-236 minutes, data of both of the Method A and the Method B is contained in the donut-shaped region, and it is determined as "drunk"; however, in the analysis time segment of 43-83 minutes, in the analysis time segment of 101-140 minutes, in the analysis time segment of 120-159 minutes, in the analysis time segment of 139-179 minutes, in the analysis time segment of 159-198 minutes, and in the analysis time segment of 204-243 minutes, data of either one or both of the Method A and the Method B is plotted outside the outer circle. Further, any plotted positions are located in the first quadrant. This indicates that as a result of drinking, Subject MA has reached the state exceeding the refresh state, namely the slump state due to excess drinking for Subject MA. The coordinate points plotted in the first quadrant, and the large separation distance from the origin indicate that the activity of the sympathetic nerve system is greatly affected by stimulation by the alcohol. Thus, according to the analysis determination means A and B of the present invention, it is possible to estimate that the subject has reached the slump state by a drinking amount exceeding the refresh state (of course, it differs between individuals). In each of the analysis time segments of 60-102 minutes and 178-217 minutes, the separation distance from the origin is short. This is ascribable to decrease in activity due to increase in drowsiness of the subject.

### (Estimation of fatigued state in normal state (neither the slump state, nor the alcohol intake state))

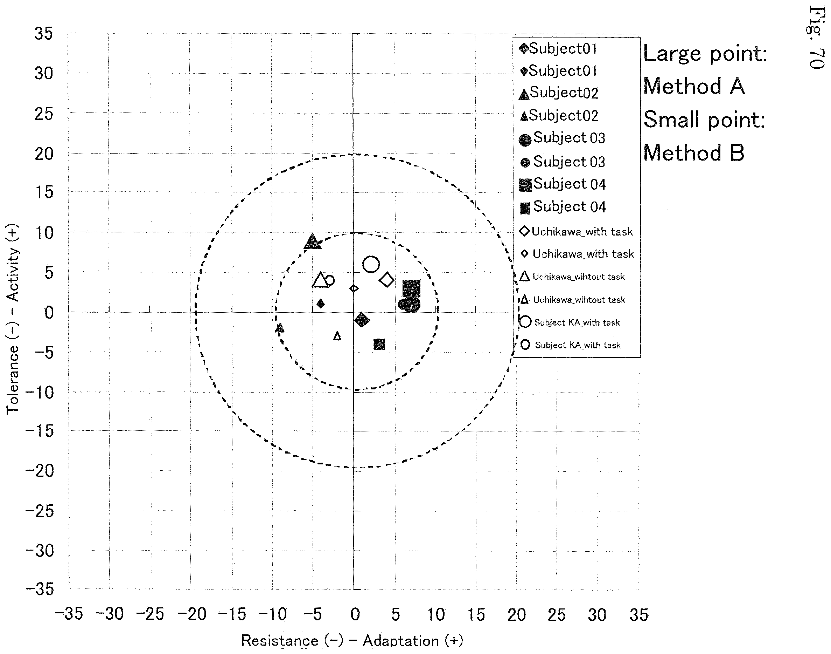

Fig. 70 is a diagram illustrating the data of the analysis determination means A (Method A) and the analysis determination means B (Method B) of Subjects 01 to 04, Subject Uchikawa (with task, without task) in the condition that they feel fatigue due to a lapse of time in a normal state in Fig. 23 and Fig. 28, and the data of the analysis determination means A (Method A) and the analysis determination means B (Method B) of Subject KA (with task) in Fig. 30.

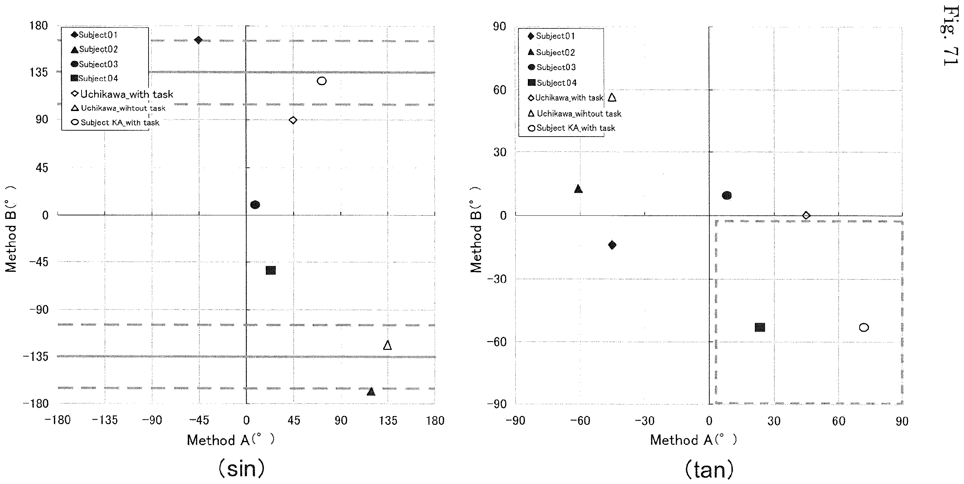

For each coordinate point of the analysis determination means A and B in Fig. 70, sin angle and tan angle were determined, and sine-representation coordinates and tangent-representation coordinates were prepared, and shown in Fig. 71. Comparison between the state of the subject in each test example and the sin angle revealed that it is appropriate to determine whether it is fatigue in a normal state or not in the following manner by using the first analysis determination means 951 and the second analysis determination means 952.

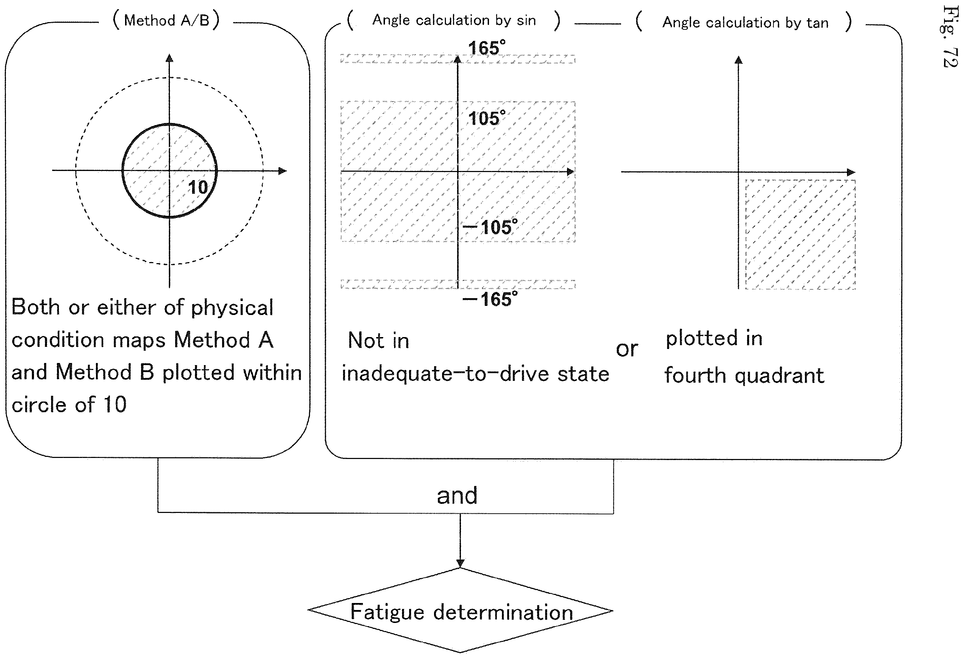

That is, as shown in Fig. 72, it is essential to satisfy the requirement that the coordinate point is plotted inside the inner circle in the analysis determination means A and B. In addition to this, it is necessary to satisfy either one of the requirement that the predetermined range in the sine-representation coordinates is not a case plotted in the range of ± 30 degrees centering on +135 degrees or in the range of ± 30 degrees centering on -135 degrees corresponding to "inadequate-to-drive" in Fig. 53 ("state lacking both physical strength and spirit"), and the requirement that in the tangent-representation coordinates, the plotted points are located not only in the first to third quadrants but also in the fourth quadrant.

Now, data of Subject A in Fig. 32, data of Subject B in Fig. 33, and data of Subject C in Fig. 34 will be verified based on the criterion whether or not the above two requirements are satisfied.

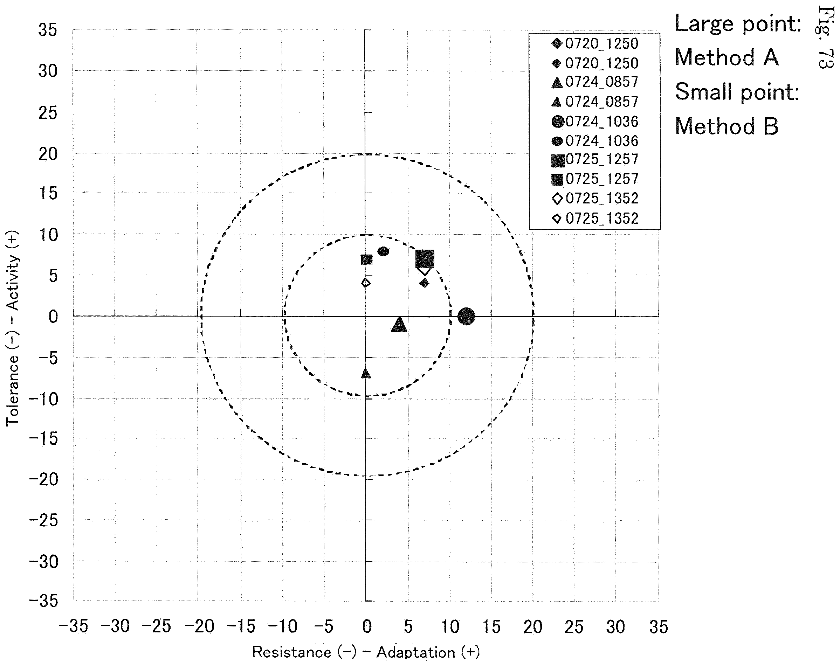

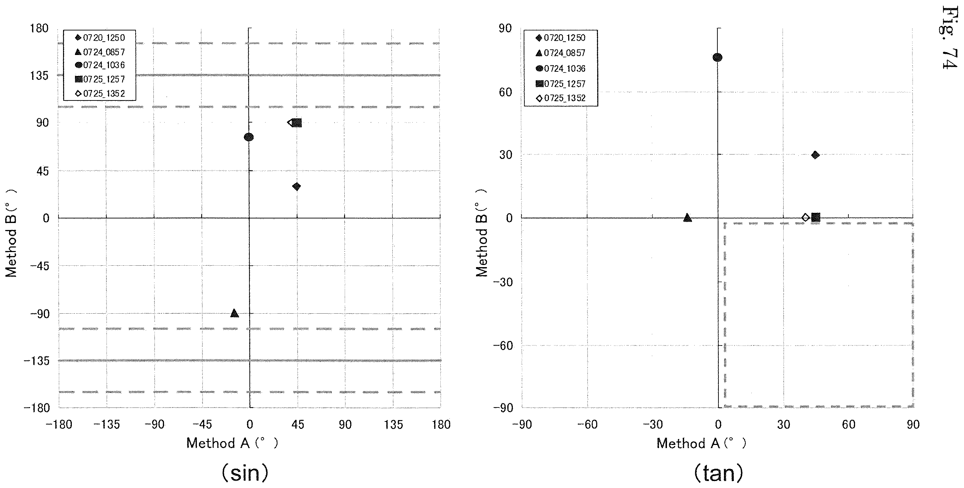

Fig. 73 is a diagram illustrating the data of second analysis time segment of both the analysis determination means A and B of Subject A in Fig. 32, and Fig. 74 is a diagram illustrating sine-representation coordinates and tangent-representation coordinates processed by the second analysis determination means 952. In Fig. 73, coordinate points of Subject A by either one of the methods are plotted inside the inner circle. On the other hand, in the tangent-representation coordinates of Fig. 74, no coordinate point is plotted in the fourth quadrant; however, in the sine-representation coordinates, no coordinate point is plotted in the range of ±30 degrees centering on +135 degrees or in the range of ±30 degrees centering on -135 degrees corresponding to "inadequate-to-drive". Thus, since the above two requirements are satisfied, it can be realized that the physical condition of Subject A during the test period (one week) is a normal state, and the subject exhibits fatigue due to normal activity in a normal state.

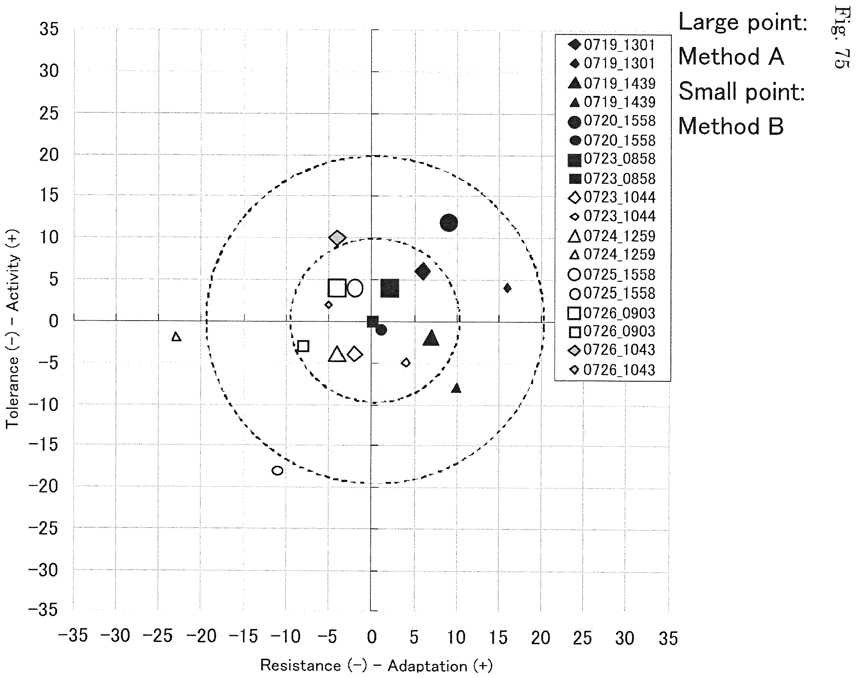

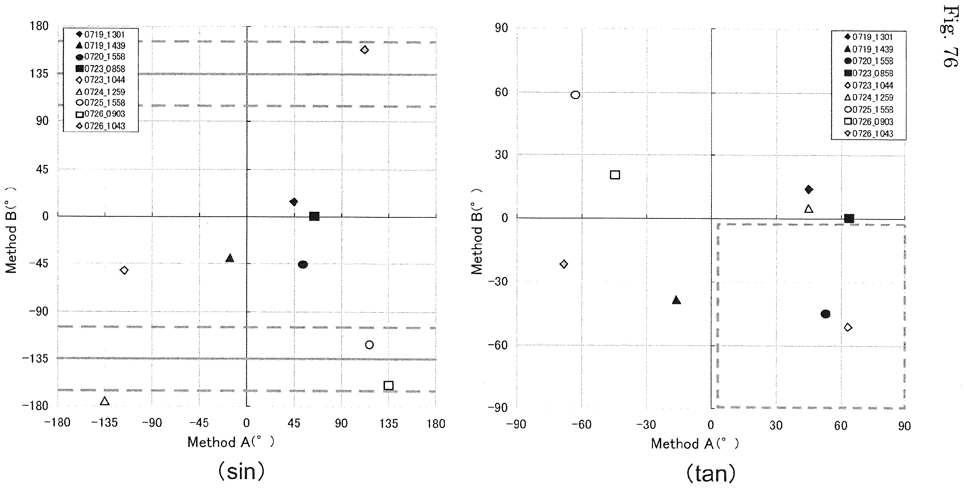

Fig. 75 is a diagram illustrating the data of second analysis time segment of both the analysis determination means A and B of Subject B in Fig. 33, and Fig. 76 is a diagram illustrating sine-representation coordinates and tangent-representation coordinates processed by the second analysis determination means 952. In Fig. 75, coordinate points of Subject A by either one of the methods are plotted inside the inner circle. On the other hand, in the sine-representation coordinates of Fig. 76, there is a coordinate point plotted in the range of ± 30 degrees centering on +135 degrees or in the range of ± 30 degrees centering on -135 degrees corresponding to "inadequate-to-drive"; however, in the tangent-representation coordinates, there is a coordinate point plotted in the fourth quadrant. Thus, since the above two requirements are satisfied, it can be realized that the physical condition of Subject B during the test period (one week) is a normal state, and the subject exhibits fatigue due to normal activity in a normal state.

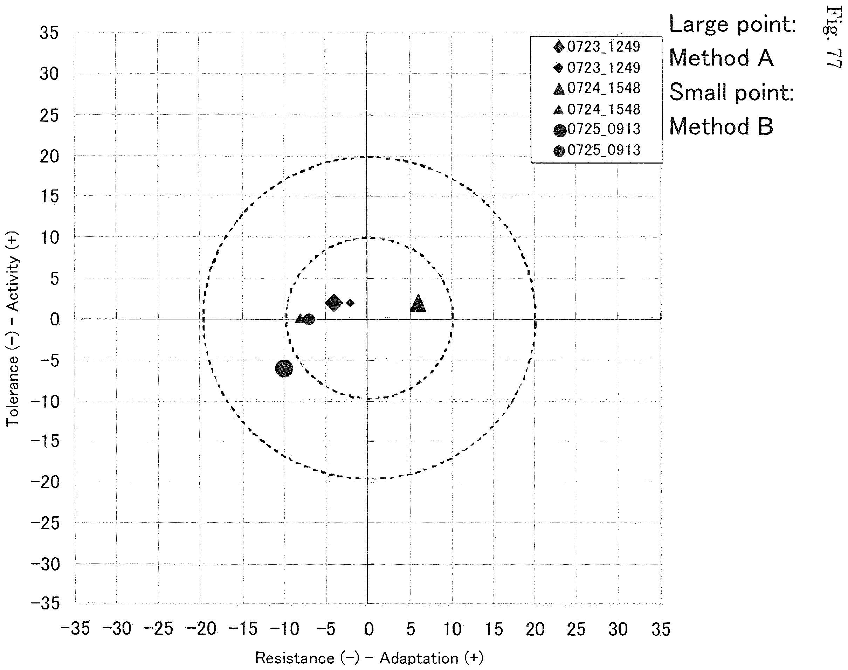

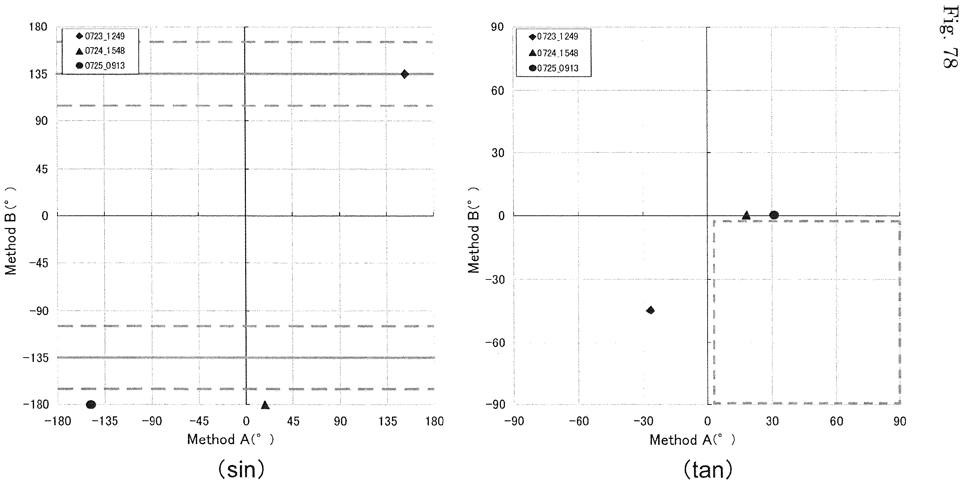

Fig. 77 is a diagram illustrating the data of second analysis time segment of both the analysis determination means A and B of Subject C in Fig. 34, and Fig. 78 is a diagram illustrating sine-representation coordinates and tangent-representation coordinates processed by the second analysis determination means 952. In Fig. 77, coordinate points of Subject A by either one of the methods are plotted inside the inner circle. On the other hand, in the sine-representation coordinates of Fig. 78, there is a coordinate point plotted in the range of ± 30 degrees centering on +135 degrees or in the range of ± 30 degrees centering on -135 degrees corresponding to "inadequate-to-drive", and in the tangent-representation coordinates, there is no coordinate point plotted in the fourth quadrant. Thus, since the above two requirements are not satisfied, it is determined that Subject C was sometimes in poor physical condition during the test period (one week).

### (Estimation of slump state (individual case))

Whether the subject is in a state of a bad physical condition caused by a disease or the like (in particular, corresponding to the above difficult-to-perform task state in a pathological condition) was analyzed individually by the second analysis determination means 952.

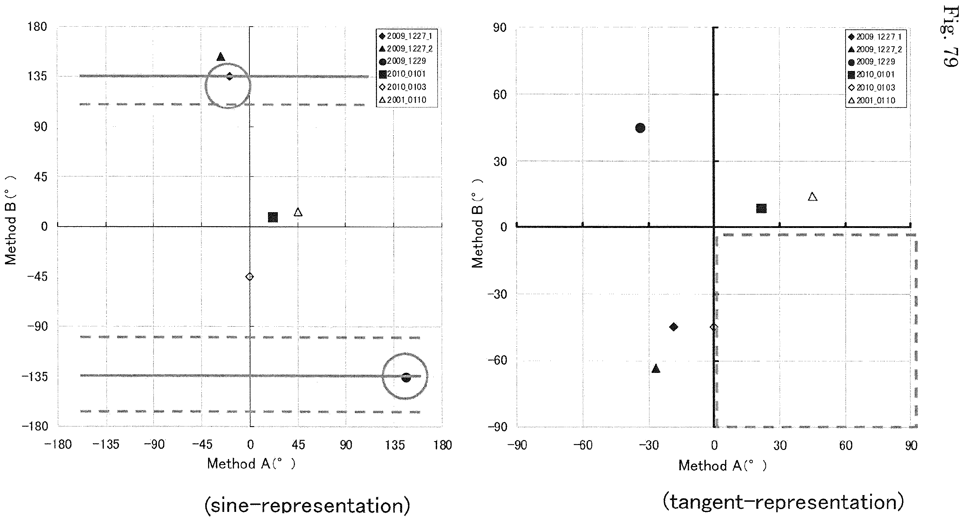

Fig. 79 is a diagram illustrating the processing results of the data of Subject JY of Fig. 24 and Fig. 29. Since there is a coordinate point plotted near +135 degrees and -135 degrees in the sine-representation, and there is no coordinate point plotted in the fourth quadrant in the tangent-representation, this state can be determined as corresponding to a pathological state. Therefore, the processing results coincide with the analysis results of Fig. 24 and Fig. 29.

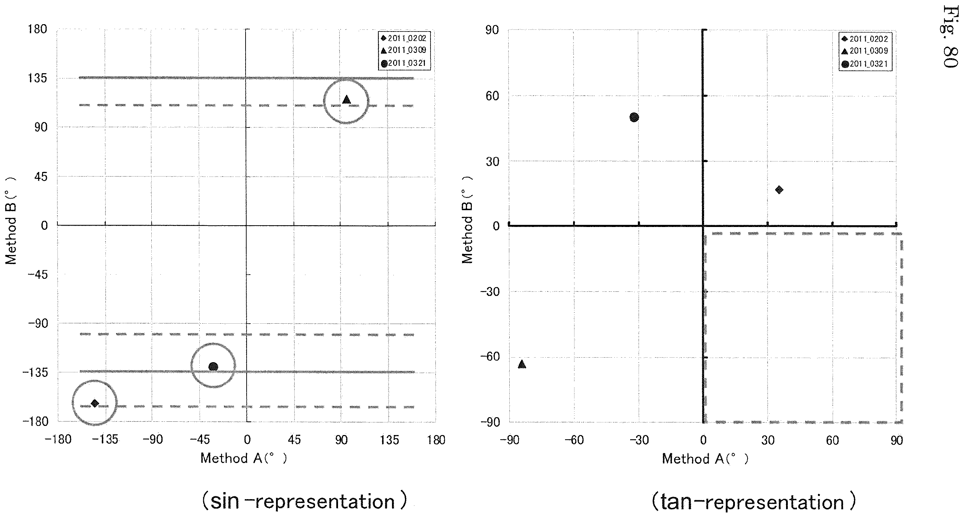

Fig. 80 is a diagram illustrating the processing results of the data of Subject "Fujita Yoshito" of Fig. 35. Since there is a coordinate point plotted near +135 degrees and -135 degrees in the sine-representation, and there is no coordinate point plotted in the fourth quadrant in the tangent-representation, this state can be determined as corresponding to a pathological state.

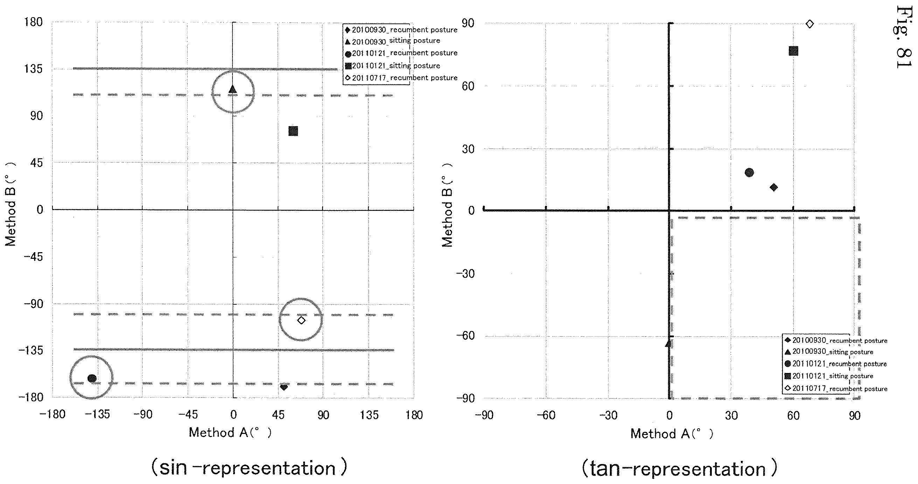

Fig. 81 is a diagram illustrating the processing results of the data of Subject YA of Fig. 36. Since there is a coordinate point plotted near +135 degrees and -135 degrees in the sine-representation, and there is no coordinate point plotted in the fourth quadrant in the tangent-representation , this state can be determined as corresponding to a pathological state.

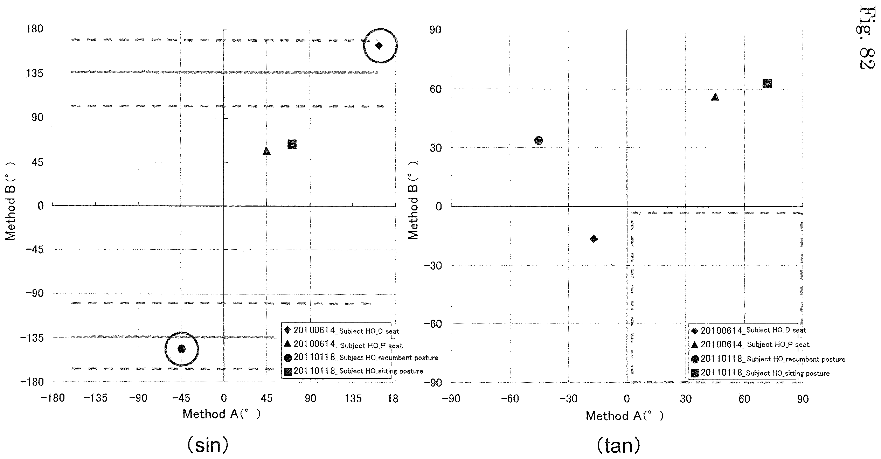

Fig. 82 is a diagram illustrating the processing results of the data of Subject HO of Fig. 37. Since there is a coordinate point plotted near +135 degrees and -135 degrees in the sine-representation, and there is no coordinate point plotted in the fourth quadrant in the tangent-representation , this state can be determined as corresponding to a pathological state.

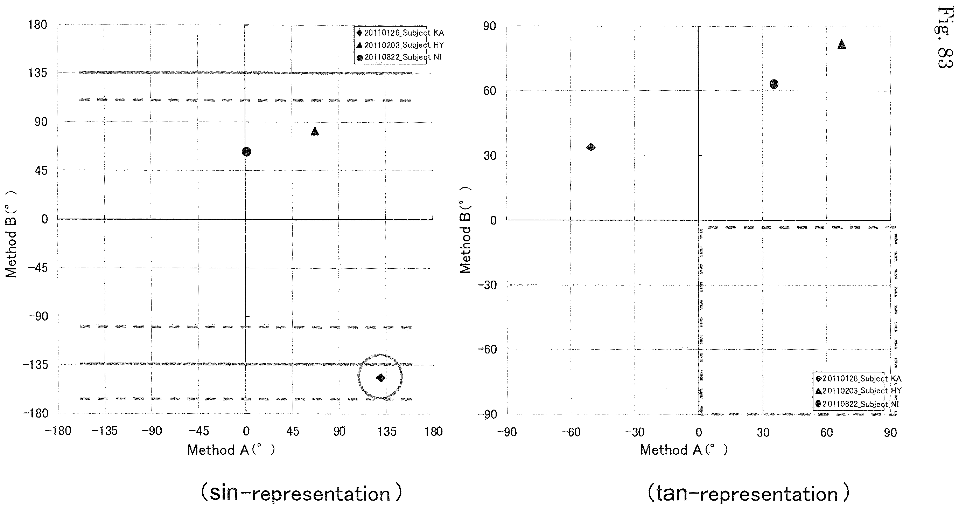

Fig. 83 is a diagram illustrating the processing results of the data of Subjects KA (depression), HY (diabetes), and NI (SAS (sleep apnea syndrome) of Fig. 39. As to Subject KA, since there is a coordinate point plotted near -135 degrees in the sine-representation, and there is no coordinate point plotted in the fourth quadrant in the tangent-representation, this state can be determined as corresponding to a pathological state. As to Subject HY, and Subject NI, since there is no coordinate point plotted near +135 degrees and -135 degrees in the sine-representation, this state can be determined as not corresponding to a pathological state.

Even when it is difficult to determine the state only by the separation distances of the coordinate points by the analysis determination means A, B of the first analysis determination means 951, more accurate state estimation is achieved by combining the second analysis determination means 952 in estimation.

### (Physical condition measurement case during driving)

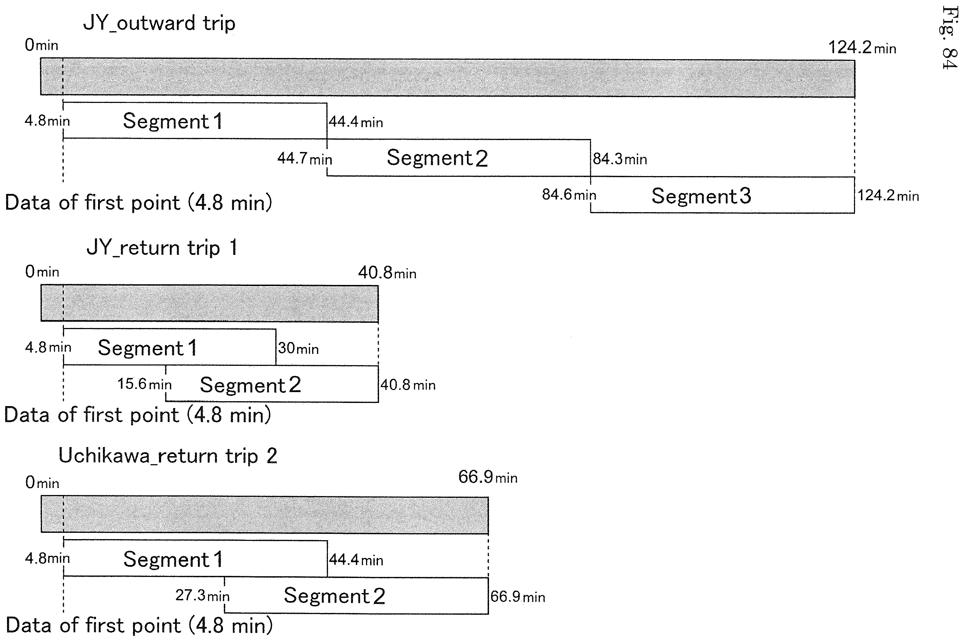

As illustrated in Fig. 84, APWs in about 2-hour driving to a predetermined point were collected, and analyzed in the same manner as descried above. In an outward trip, Subject JY drove all the way, and the data during 124.2 minutes was analyzed. As to a return trip, data of 40.8 minutes during which Subject JY drove, and data of 66.9 minutes during which Subject Uchikawa drove was analyzed.

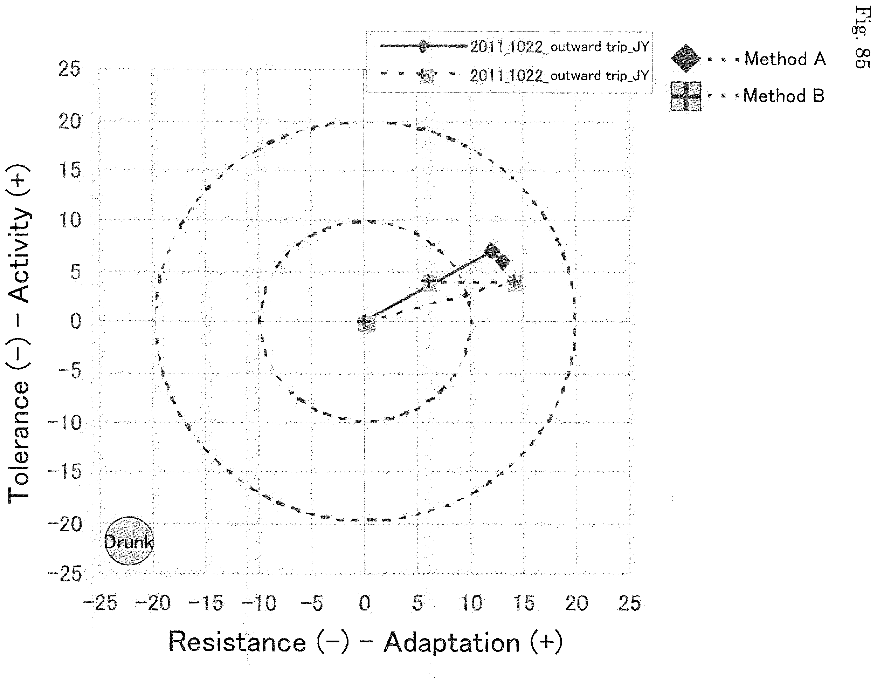

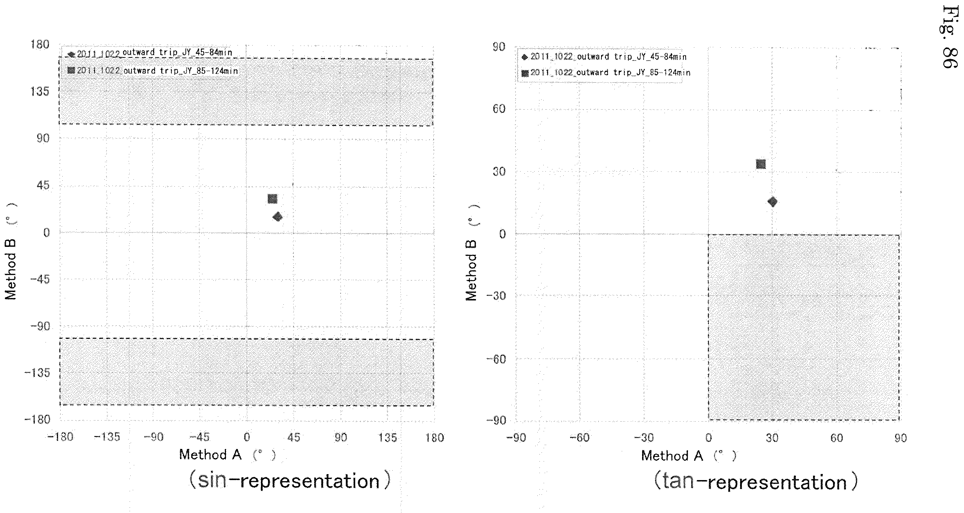

Fig. 85 is a diagram illustrating the analysis results obtained by the analysis determination means A (Method A) and the analysis determination means B (Method B) of Subject JY in the outward trip, and Fig. 86 is a diagram illustrating sine-representation coordinates and tangent-representation coordinates of the coordinate points plotted in Fig. 85. In the outward trip, Subject JY continuously drank coffee intermittently every 20 minutes. This condition was set because the time during which the effect of caffeine appears is about 20 minutes. Fig. 85 reveals that there is a coordinate point plotted in the donut-shaped region both in the Method A and the Method B, and it will be determined as drunk if the determination is made only by the Method A and the Method B. This is ascribable to the fact that absorption and degradation of caffeine, in particular, by drinking coffee undergo a course similar to that for absorption and degradation of alcohol. However, in the sine-representation coordinates of Fig. 86, there is no coordinate point contained in the risk zone (extremely excited state) and the gray zone (negative zone). Therefore, by using the second analysis determination means 952 together, the second analysis determination means 952 determines as "non-drunk" even if the first analysis determination means 951 (analysis determination means A and B) determines as "drunk", so that determination as the inadequate-to-drive state due to drunk as described above will not be made. Therefore, in this case, it is estimated that pharmacological effects of drug components other than alcohol (caffeine, in this case) strongly act in a non-drunk state.

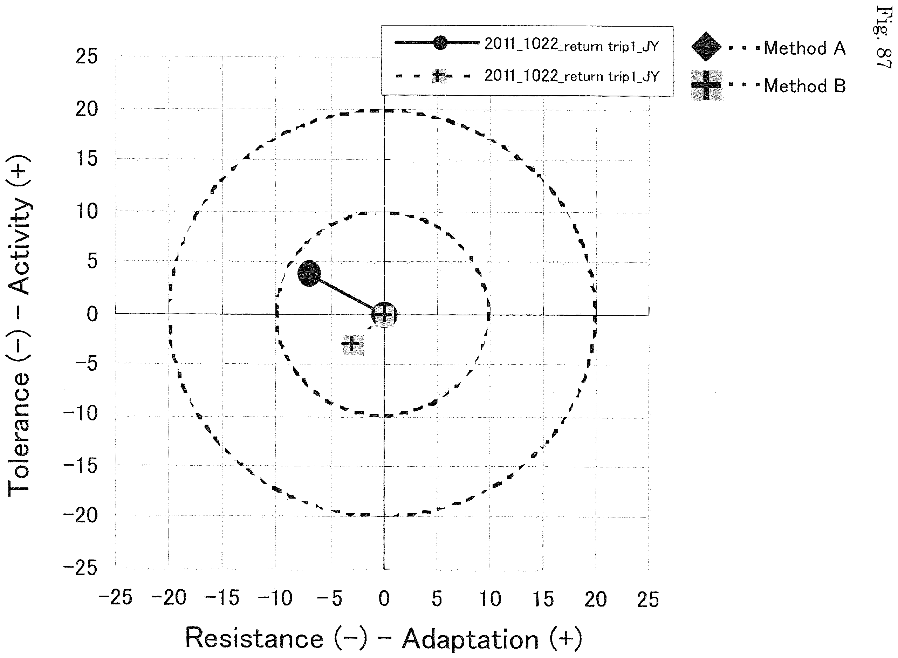

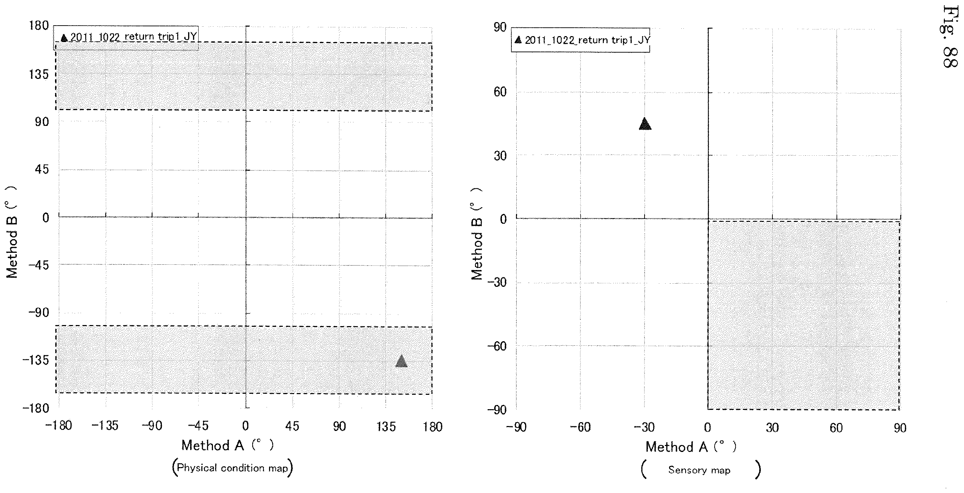

Fig. 87 is a diagram illustrating the analysis results obtained by the analysis determination means A (Method A) and the analysis determination means B (Method B) of Subject JY in the return trip, and Fig. 88 is a diagram illustrating sine-representation coordinates and tangent-representation coordinates of the coordinate points plotted in Fig. 87. In Fig. 87, there is a coordinate point plotted inside the inner circle both in the Method A and the Method B, and there is no coordinate point plotted in the fourth quadrant in tangent-representation coordinates of Fig. 88, so that it can be estimated as a state undergoing a normal fatigue course.

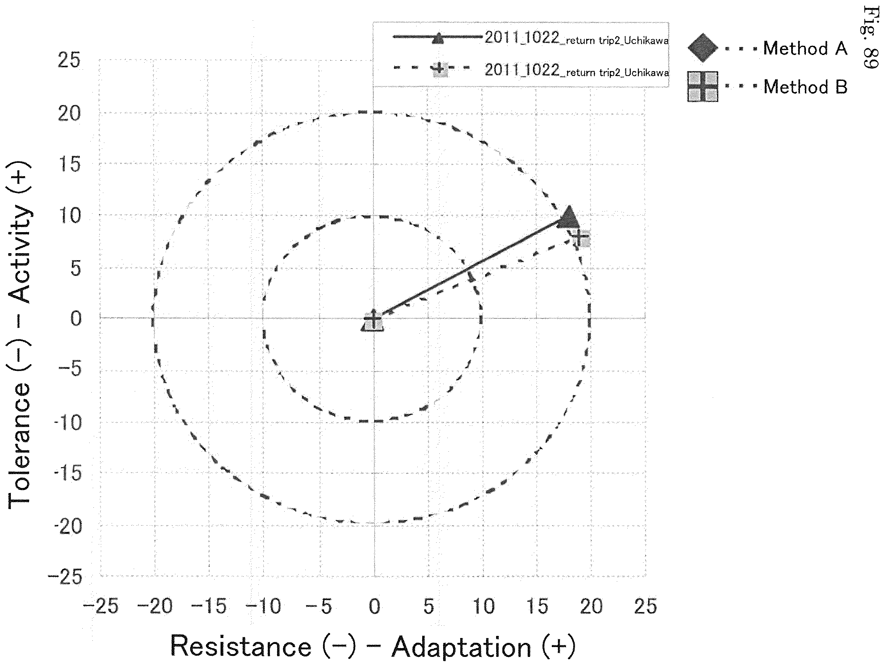

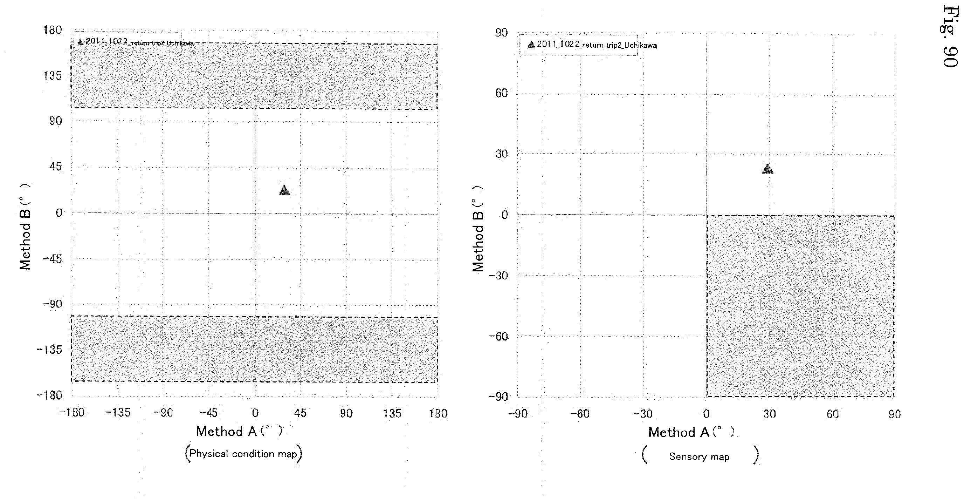

On the other hand, as to the analysis result of Subject Uchikawa who took over the driving halfway in the return trip, there is a coordinate point plotted outside the outer circle both in the Method A and the Method B as seen in Fig. 89. In the sine-representation coordinates of Fig. 90, there is no coordinate point plotted in the range of ± 30 degrees centering on +135 degrees and ± 30 degrees centering on -135 degrees illustrated in Fig. 53 for which determination of "state lacking both physical strength and spirit" is made. In both of the sine-representation coordinates and tangent-representation coordinates in Fig. 90, there is a coordinate point plotted in the first quadrant although the requirement of "drunk" is not satisfied. From this, it is estimated that although the cardiac function tends to decrease, a sudden state change arises, namely a sympathetic nerves compensatory mechanism functions due to increase in the sympathetic nerve system.

### (Sleep Quality Estimation 1)

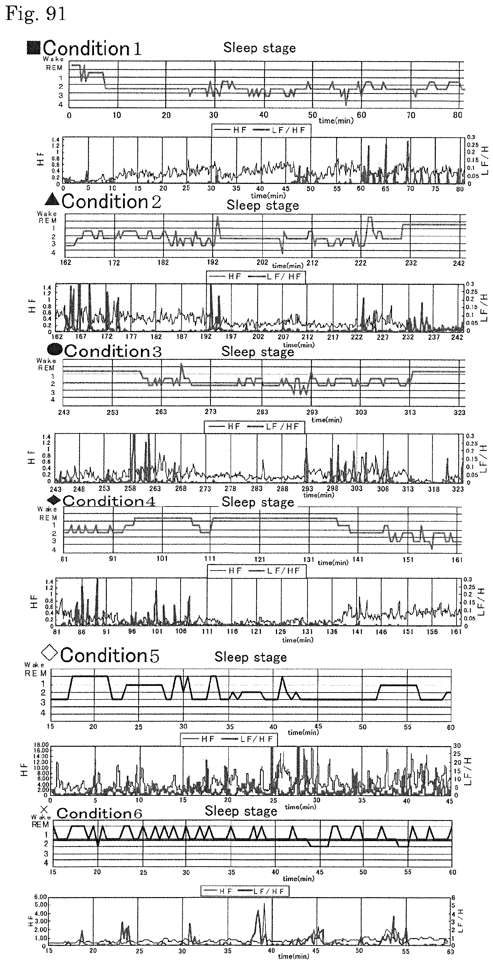

Healthy male subjects in 20's, 30's and 80's were subjected to a sleep test in recumbent postures (sleep time: nocturnal sleep). For the test, the subjects wore a simplified electroencephalograph, a finger plethysmograph, and a sensor mat for APW measurement, and were classified by the conditions for respective sleep states determined from sleep polygraphs, and analyzed for each condition, and displayed on sine-representation coordinates. The conditions determined from sleep polygraphs are as follows. Fig. 91 is a diagram illustrating sleep polygraphs and time-series waveforms of HF and LF/HF in the following conditions 1 to 6.
(1) Condition 1: the case where it is determined from the sleep polygraph that the state transits in the manner of wakeful state → sleep stages 1 → 2 → 3 → 4 → 4 → 2 → 1.
   The state of autonomic nervous function estimated from the time-series waveforms of HF and LF/HF obtained by analyzing the finger plethysmograms is as follows.
   In a falling asleep time corresponding to wakeful state → sleep stage 1, the sympathetic nerve function decreased. The period corresponding to sleep stages 2 → 3 → 4 is a non-REM sleep transition period wherein the parasympathetic nerves are increased. Since a slow wave sleep most frequently appears, the frequency of occurrence of sympathetic nerve action is lower than that in a wakeful state, and decreases to half compared with that in a wakeful state. The slow wave sleep is a rest of brain, and is important for relaxation of mental strain. When the sleep stage transits in the manner of 4 → 3 → 2 → 1, the sympathetic nerve action frequently occurs while the parasympathetic nerves are kept increased. Therefore, the sympathetic nerve function during sleep and the depth of sleep do not correlate with each other. On the other hand, correlation with increase in parasympathetic nerves is high, and it is closely related with the feeling of deep sleep. It can be interpreted that the function of parasympathetic nerves are increased during the non-REM sleep, and the function of the sympathetic nerve system are increased in the REM sleep period.
(2) Condition 2: the case where it is determined from the sleep polygraph that the state transits in the manner of sleep stages 3 → 2 → 1 → 3 → 2 → intermediate awakening → 2 → 4 → 1 → 2 → 3 → 2 → intermediate awakening → 1 → 2 → REM.
   In a general view from the autonomic nervous function, it can be interpreted that the action of the sympathetic nerve function is increased due to frequent occurrence of the sympathetic nerve action, and the sleep transits from deep sleep of non-REM sleep to shallow sleep, and then the sleep is swung back to deeper sleep by increased parasympathetic nerves. It is thought that the subject is forced to wake up by external stimulation, and it is thought that significant autonomic nervous system response such as palpitation occurs. It is thought that the action of the sympathetic nerves is temporarily increased by such external stimulation or internal stimulation. Although it is unclear that this is caused by increase in the sympathetic nerves or the decrease in the parasympathetic nerve function, it can be interpreted that the state did not transit to the wakeful state and the deep sleep continued regardless of the increase in the heart rate. However, from around this point of time, it seems that the feeling of deep sleep decreases. It seems that the action of the parasympathetic nerve system decreased, the activity of the sympathetic nerves increased and the sleep state transited to REM sleep.
(3) Condition 3, Condition 4: Both of these are the cases that the sleep transited to deep sleep in a similar way to that in Condition 2, and Condition 3 is determined as the case where REM sleep transits to non-REM sleep, and REM → 1 → 2 → 3 → 2 → 1 → 2 → 1 is repeated, and Condition 4 is determined as the case where depth of sleep is increased as the parasympathetic nerves are increased in the manner of REM → 1 → 2 → 3 → 4 → 2 → 3.
   In other words, the sympathetic nerve function during sleep and the depth of sleep do not correlate with each other. On the other hand, the correlation with the increase in parasympathetic nerves is high, and there is a close relation with the feeling of deep sleep. That is, the function of the parasympathetic nerves is increased during non-REM sleep, and the function of the sympathetic nerve system is increased in REM sleep period.
(4) Condition 5: the case where after repetition of REM and non-REM, the sleep transited to non-REM in the last half. Generally, the action of the parasympathetic nerves is high, but the frequency of occurrence of sympathetic nerves is as same as that in a wakeful state. In other words, the function of the parasympathetic nerves is increased during non-REM sleep, and the function of the sympathetic nerve system is increased in REM sleep period.
(5) Condition 6: the case where REM and non-REM are continuously repeated. Decrease in the parasympathetic nerve function and elevation in the sympathetic nerves occurred, and the heart rate increased. Activity of consciousness as represented by sleep-awakening rhythm is controlled by the autonomic nervous system, and this condition corresponds to the situation that the sympathetic nerve system functions predominantly, and the function of the parasympathetic nerves decreases during awakening.

In contrast to Condition 1, REM-sleep and intermediate awakening often occurred and the feeling of deep sleep was poor in Conditions 2 to 4. In Condition 1, a rest by sleeping is taken, and this functions as a preparatory sleep for wakeup. Condition 5 is considered as sleep of poor quality because REM sleep is mixed with non-REM sleep. Condition 6 is also sleep of poor quality because the subject seems to keep dozing off.

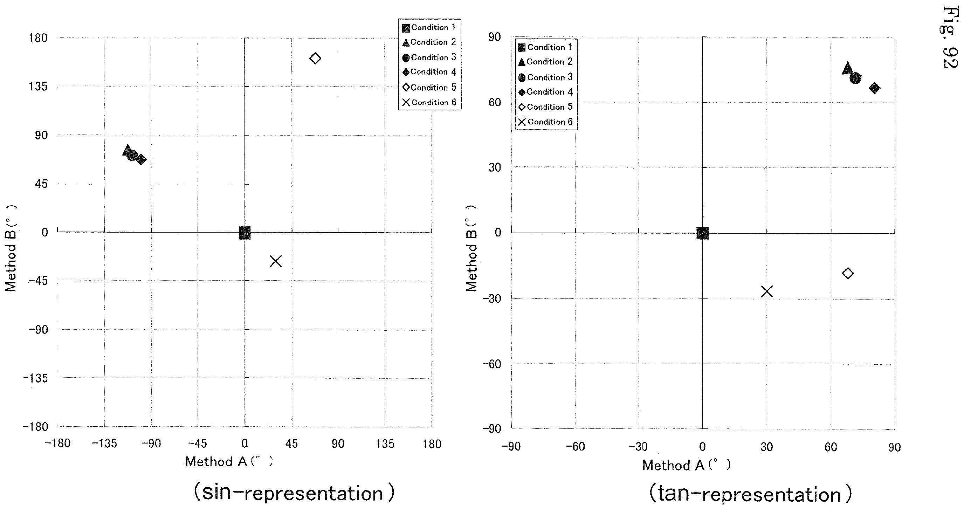

Fig. 92 illustrates sine-representation coordinates and tangent-representation coordinates determined for Conditions 1 to 6 analyzed by the first analysis determination means 951 and the second analysis determination means 952. While coordinate points of Condition 1 are plotted at the origin, coordinate points of the Condition 2, Condition 3 and Condition 4 are plotted in the position relatively closer to the second quadrant in the sine-representation coordinates, and to the first quadrant in the tangent-representation coordinates. On the other hand, sleeps of poor quality in Condition 5 and Condition 6 are plotted in positions different from those of Conditions 2 to 4, and it can be realized that plotted positions in the sine-representation coordinates and the tangent-representation coordinates can be distinguished according to the quality of sleep. In particular, the difference is significant in the sine-representation coordinates, and it can be realized that in sleep of good quality in Conditions 1 to 4, the plotted points tend to be unevenly distributed on the left side of the vertical axis (on the side of the second and third quadrants), and in sleep of poor quality in Conditions 5 to 6, the plotted points tend to be unevenly distributed on the right side of the vertical axis (on the side of the first and fourth quadrants).

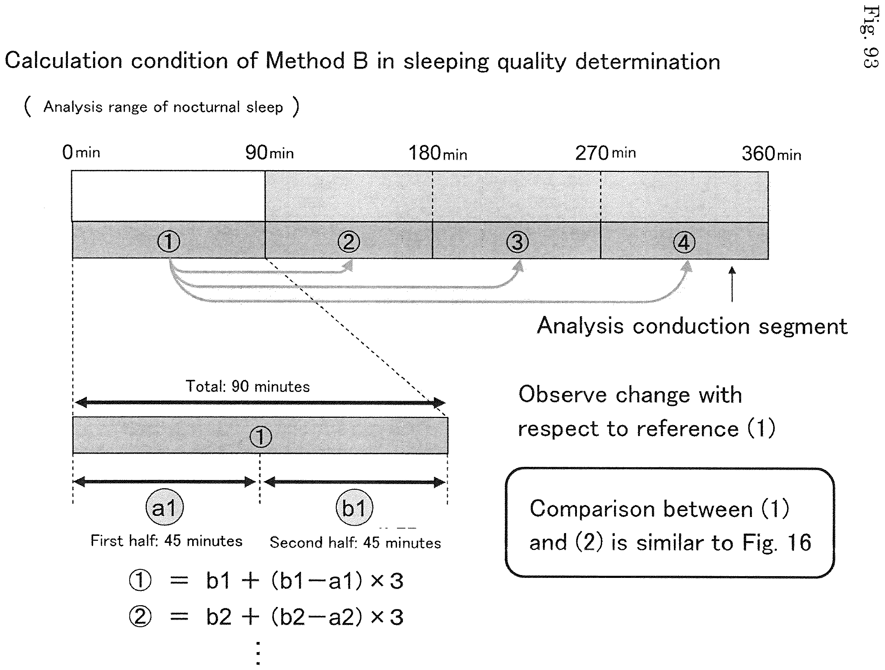

For analysis of nocturnal sleep, when the computation of the analysis determination means A (Method A) of the first analysis determination means 951 is conducted, the period of analysis time segment is set at about 90 minutes rather than about 45 minutes as is the case of the wakeful state shown in Fig. 12. Also in conducting the computation of the analysis determination means B (Method B) in the first analysis determination means 951, as shown in Fig. 93, each analysis time segment of about 90 minutes is divided into a first half (45 minutes (a1) and a second half (45 minutes (b1)), and a score determined by the expression: b1 + (b1 - a1) × 3 by comparison between the analysis waveform in a log-log graph in the first half and the analysis waveform in a log-log graph in the second half is used, rather than conducting a computation by comparison between the analysis waveform (Time segment a) in about 38 minutes which is the almost entire range of the analysis time segment and the analysis waveform of the initial 20 minutes (Time segment b), as shown in Fig. 15. In the case of nocturnal sleep, REM sleep and non-REM sleep are repeated in a cycle of about 90 minutes on average (about 80 minutes to about 110 minutes depending on the individual). Therefore, in grasping the state during nocturnal sleep, it is difficult to grasp the correct state change only by detecting the first half part in the entirety as is the case with the wakeful state and during daytime napping (during short sleep). It is necessary to divide the analysis time segment of about 90 minutes into the first half and the second half according to the cycle of the sleep, and determines how the sleep state changes therein.

Once scores of these analysis time segments have been determined, the scores are plotted with the score of the first analysis time segment which is a reference being the origin of the coordinates, and differences between scores of the second to fourth analysis time segments and the score of the first analysis time segment are determined, and plotted on the coordinates. This procedure is similar to the case where the analysis means B (Method B) is used in a wakeful state as described in Fig. 16.

In the following description, the analysis determination means B (Method B) applied in the nocturnal sleep test is determined by the computation conditions dividing each analysis time segment of about 90 minutes shown in Fig. 93 into a first half and a second half.

### (Sleep Quality Estimation 2)

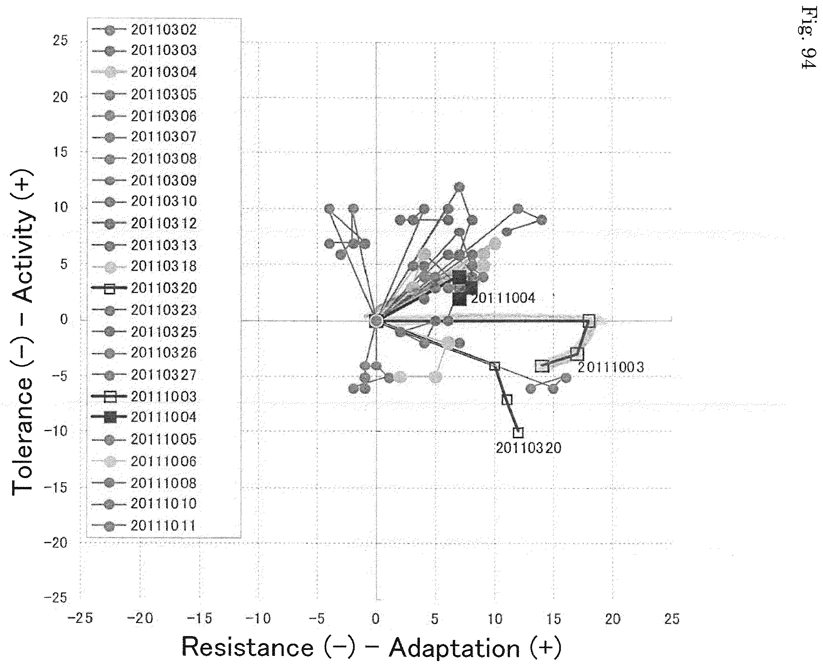

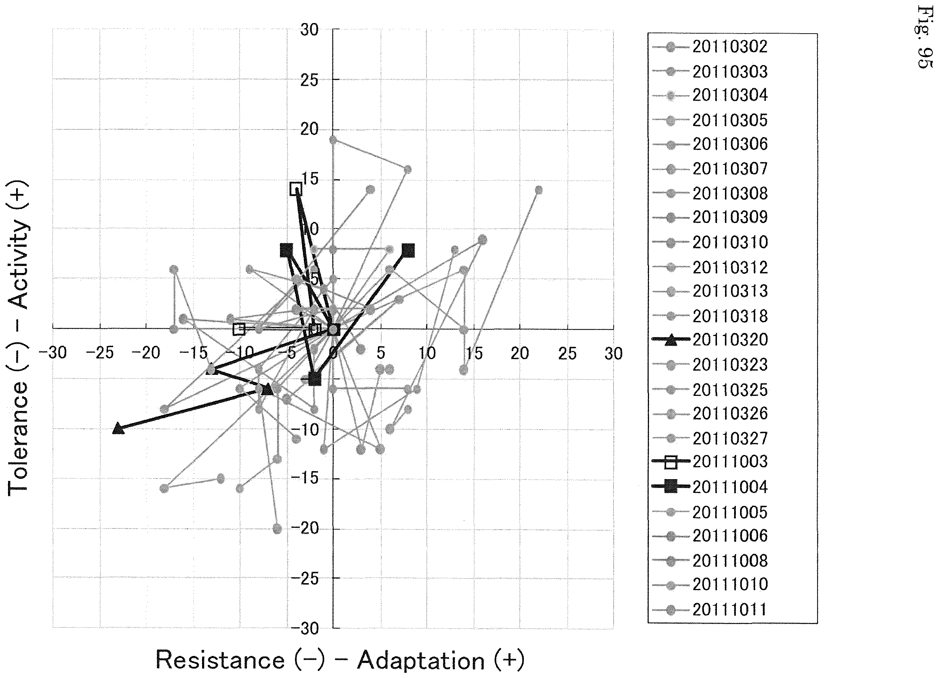

For a female Subject OG in 30's, biological signal measuring means was set on her bedding, and biological signals (APW) were collected during ordinary nocturnal sleep. The test was conducted over one month. Fig. 94 and Fig. 95 illustrate the analysis results by the analysis determination means A and B. In Fig. 94, 20111003 and 20110320 are the dates when the subject was significantly aware of having taken enough sleep. In data of these dates, the coordinate points in the second analysis time segment are separated from the original point by 10 or more. Therefore, as one criterion to determine whether one takes a sleep of good quality, presence of a coordinate point plotted outside the inner circle having a radius of 10 is recited. Referring to the analysis determination means A of Fig. 94; the coordinate points are generally unevenly distributed on the side of the first and fourth quadrants. The first quadrant is the region where the activity of the sympathetic nerves is predominant to the parasympathetic nerves, and thus the subject is concentrated but not relaxed, and the fourth quadrant is the region where the sympathetic nerves and the parasympathetic nerves are well balanced, and thus the subject is relaxed in a concentrated state. In other words, in the case of this subject, the direction of transition of the overall physical condition change determinable from the analysis determination means A is the direction of high concentration, and displacement to the first quadrant rather than the fourth quadrant occurs in many days, so that it is estimated that she often falls asleep in an unrelaxed state. On the other hand, referring to the result of the analysis determination means B of Fig. 95, large displacement on and after the first analysis time segment is observed in many days, and the coordinate points are dispersed in the first to fourth quadrants as a whole, and the state where concentration is high in a relaxed condition (fourth quadrant), the state where concentration is low and loosened in a relaxed condition (third quadrant), the state where concentration is high in an unrelaxed condition (first quadrant), and the state where she is loosened in an unrelaxed condition (second quadrant) appeared without any deviation, and it is realized that this subject succeeds in having a sleep of high quality. In other words, this subject succeeds in having a good sleep as function recovery means.

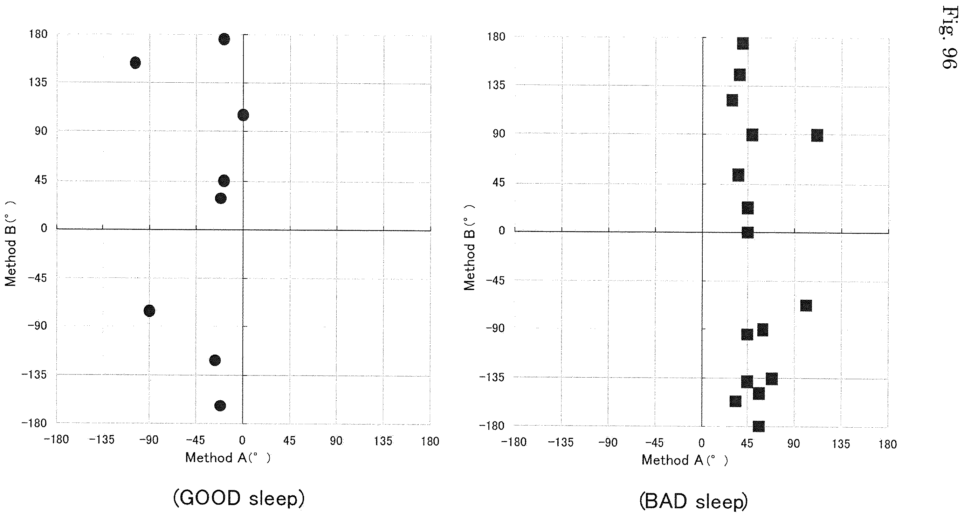

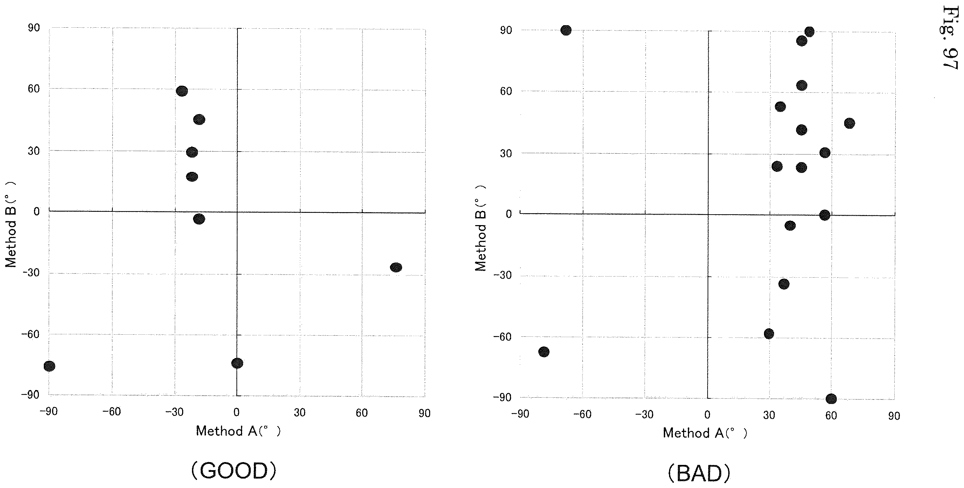

Fig. 96 illustrates sine-representation coordinates determined by the second analysis determination means 952, and Fig. 97 illustrates tangent-representation coordinates determined by the second analysis determination means 952. In these diagrams, "GOOD" represents the analysis result for the sleep wherein feeling of deep sleep is subjectively high, and intermediate awakening is not contained, and "BAD" represents the analysis result for the sleep wherein feeling of deep sleep is low and intermediate awakening is contained. As illustrated in Fig. 96 and Fig. 97, both in sine-representation coordinates and tangent-representation coordinates, the coordinate points tend to be dispersed in the second and the third quadrants in the case where there is no intermediate awakening, and they tend to be dispersed in the first and the fourth quadrants in the case where there is an intermediate awakening. In particular, in sine-representation coordinates of Fig. 96, the tendency is significant. This point is similar to the above test of (Sleep Quality Estimation 1), and the quality of sleep can be clearly determined according to the quadrant where the point is plotted, in particular, in sine-representation coordinates.

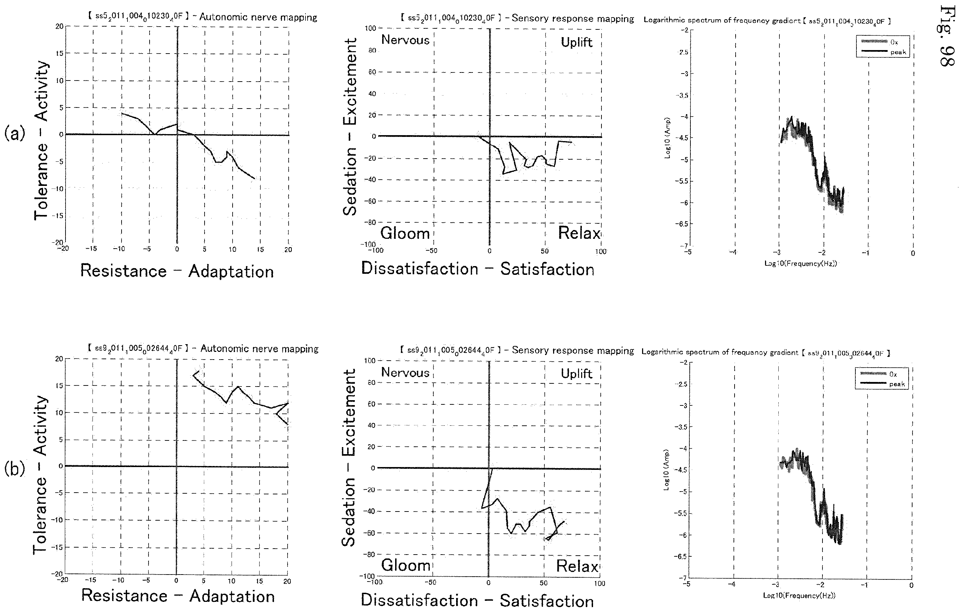

Fig. 98(a) is a diagram illustrating a physical condition map and a sensory map obtained by analyzing the data of 20111003 having had a sleep of high quality without intermediate awakenings, and a log-log graph of power spectral density obtained by frequency analysis of the frequency-gradient time-series waveform. In the physical condition map, the time-series variation line traces a locus that is steadily declining by approximately 45 degrees. In the sensory map, the displacements are substantially equal in the upper region and the lower region bounded by a virtual line corresponding to a value of the vertical axis of -20, and transition substantially parallel to the horizontal axis is observed. Therefore, these physical condition map and sensory map also show that 20111003 had a sleep of very good quality. On the other hand, Fig. 98(b) is a diagram illustrating a physical condition map, a sensory map, a log-log graph of power spectral density obtained from the measurement results of 20111004. The data of 20111004 also looks good from Fig. 94. While the time-series variation line is steadily declining in the physical condition map, the coordinate points are unevenly distributed in the first quadrant, so that the state is a non-relaxed state. Also, the sensory map shows that the transition substantially parallel with the horizontal axis, but is disarranged in comparison with the data of 20111003. Therefore, it can be said that 20111004 is an acceptable sleep but is a highly strained and unrelaxed sleep in comparison with 20111003.

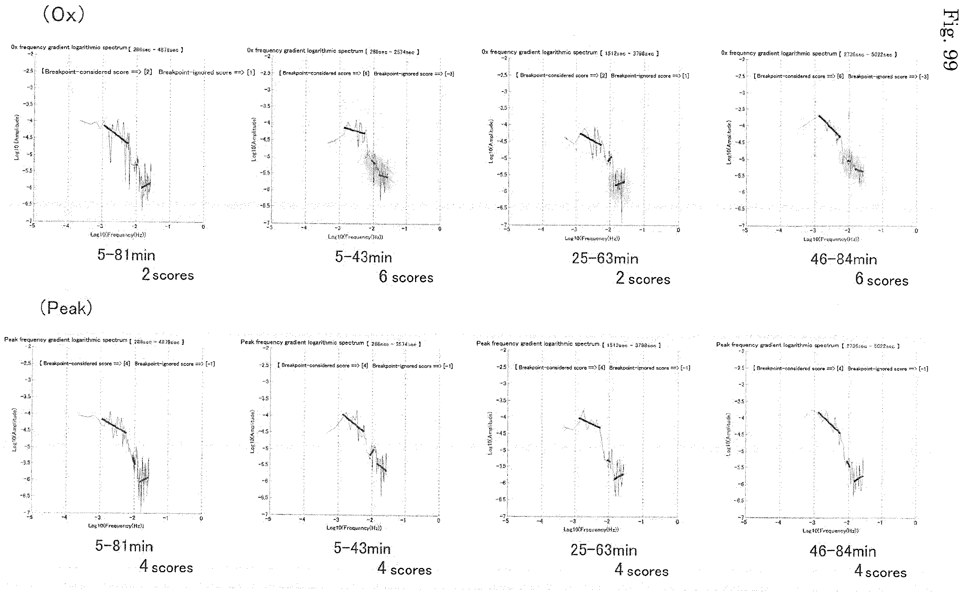

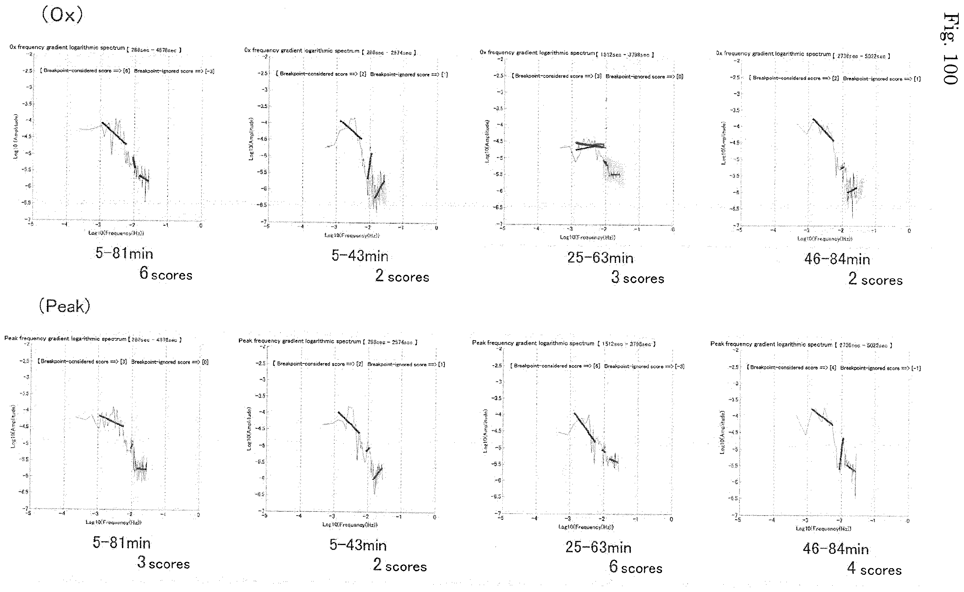

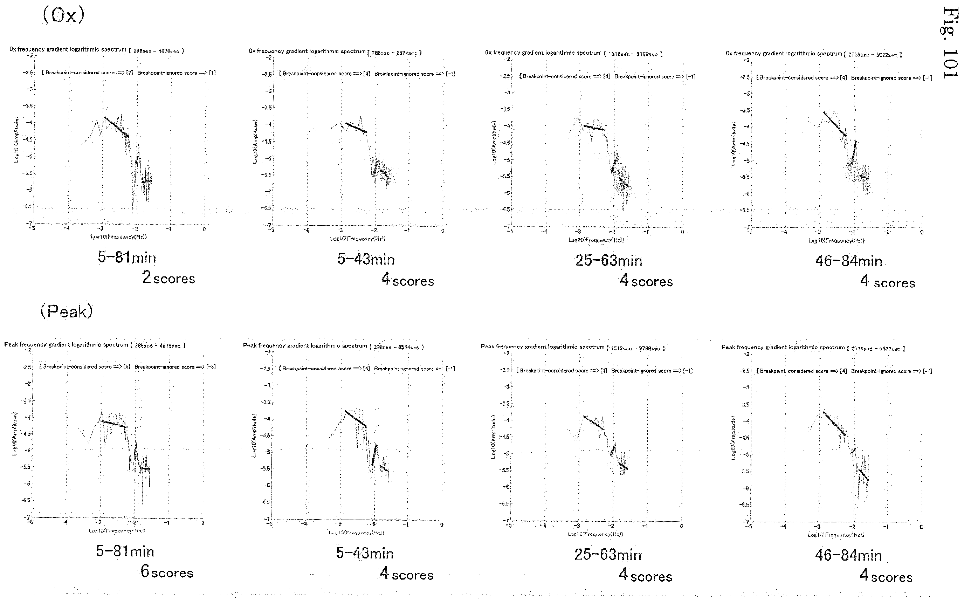

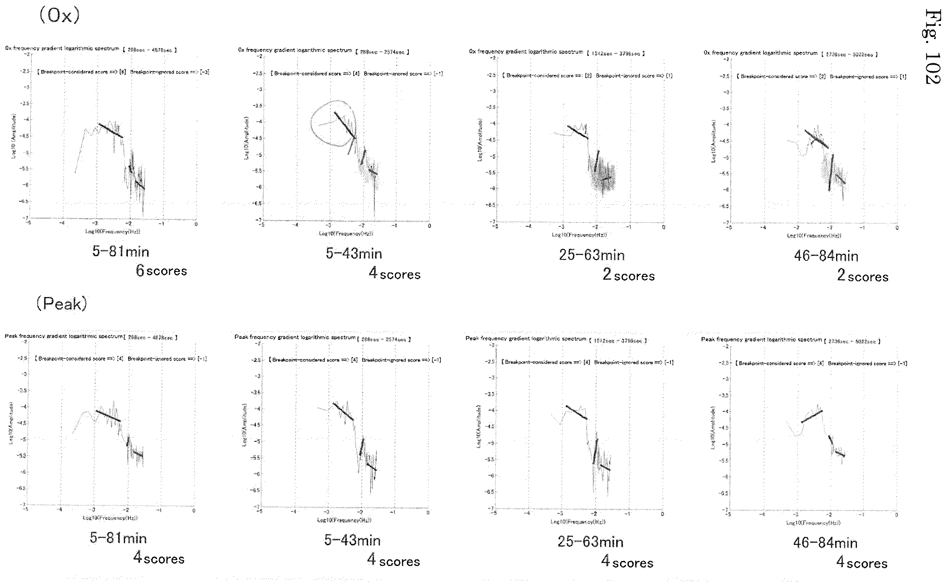

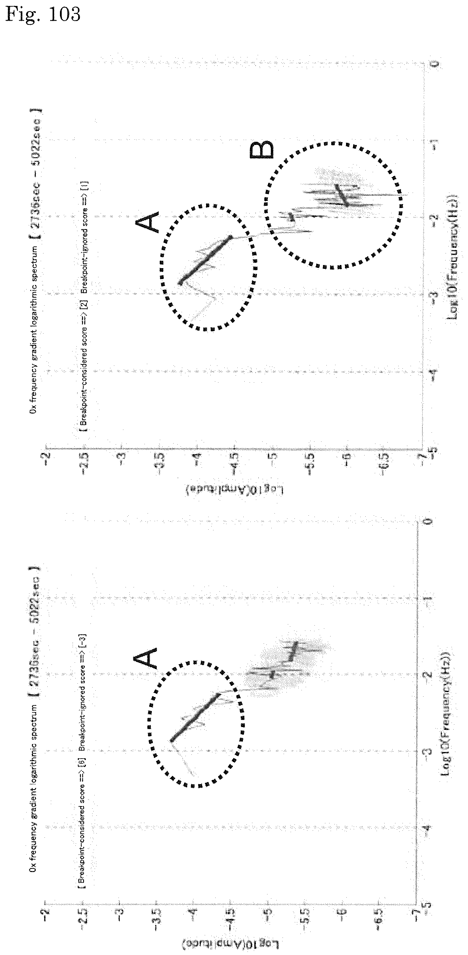

In 20111003 having had a sleep of good quality without intermediate awakening, the frequencies of the frequency-gradient time-series waveforms are analyzed every predetermined time by the frequency analysis means 80, and the results are displayed in a log-log graph plotting the frequency on the horizontal axis and the power spectral density on the vertical axis, and additionally, regression lines are determined by the regression line computation means 901 of the fluctuation waveform analyzing means 90, and the determination criterial scores are determined by the determination criterial score calculation means 902, and the results are shown in Fig. 99 to Fig. 102 (analysis time segments are segments of 90 minutes, Fig. 99 is a diagram illustrating the first analysis time segment, Fig. 100 is a diagram illustrating the second analysis time segment, Fig. 101 is a diagram illustrating the third analysis time segment, and Fig. 102 is a diagram illustrating the fourth analysis time segment). Focusing on the gradients of the respective regression lines in the long-cyclic region, the mid-cyclic region, and the short-cyclic region in these drawings, it can be seen that the gradients of the regression lines in the long-cyclic region are negative in most cases. On the other hand, the gradients of the regression lines in the mid-cyclic region and the short-cyclic region are sometimes positive and sometimes negative. Fig. 103 is a diagram illustrating a representative example thereof.

This reveals that during a sleep, a gradient of a regression line in the long-cyclic region (region of A in Fig. 103) is basically negative, and a gradient of a regression line in the mid-cyclic region and the short-cyclic region (region of B in Fig. 103) often turns to positive even during a sleep when the compensatory mechanism of the sympathetic nerves arises due to increased sympathetic nerves. Therefore, the state estimation means 95 is able to determine the quality of sleep regarding whether or not the activity of the sympathetic nerves is increased during the sleep, by determining whether the gradients of the regression lines in the mid-cyclic region and the short-cyclic region are positive or negative in the state that the gradient in the long-cyclic region is negative during the sleep.

### (Sleep Quality Estimation 3)

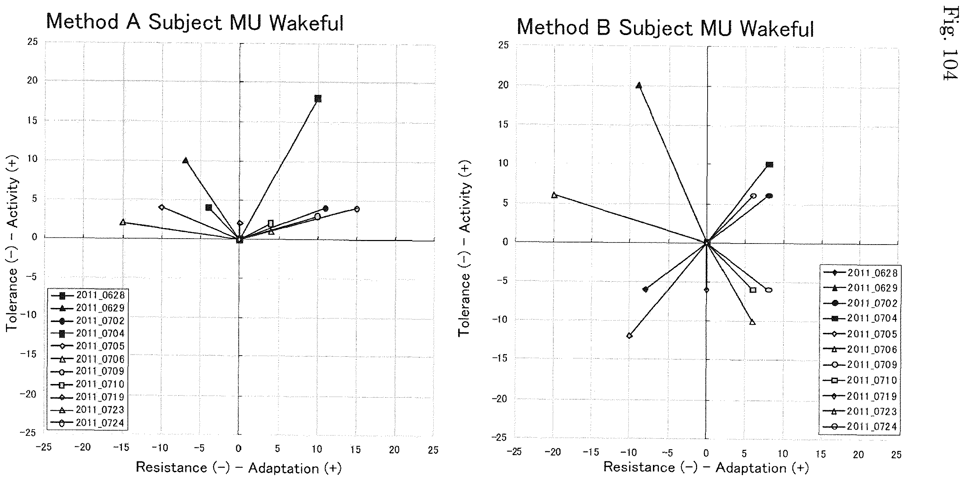

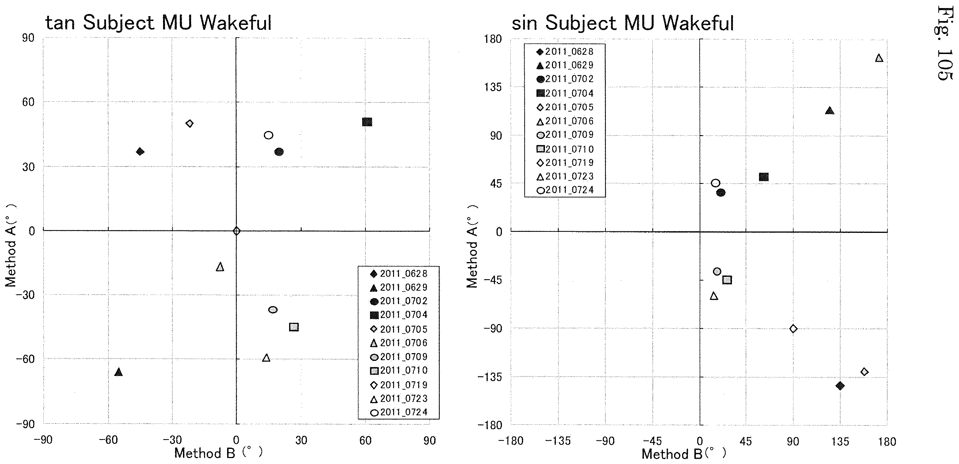

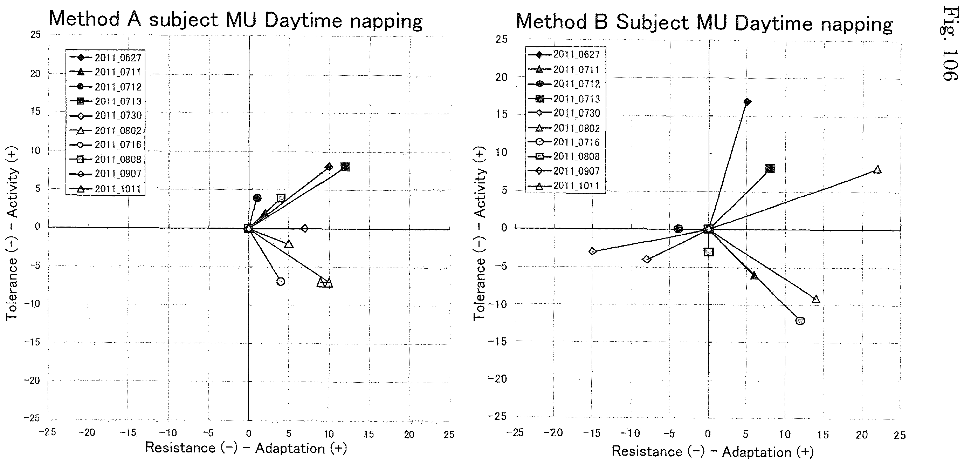

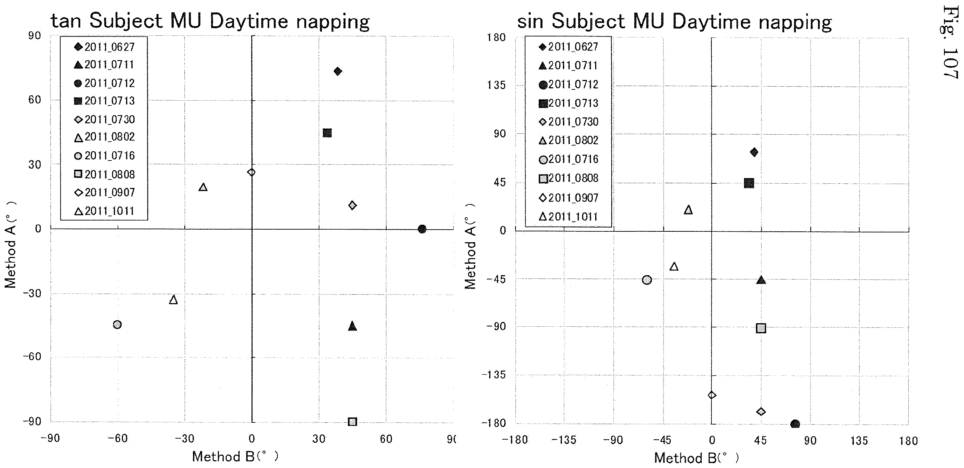

Fig. 104 to Fig. 109 illustrate the analysis results of female Subject MU in 20's. Fig. 104 and Fig. 105 represent the analysis results in a wakeful state, and Fig. 106 and Fig. 107 represent the analysis results when she took a daytime nap (short sleep) of about 30 minutes as function recovery means. Subject MU showed signs of dysfunction of the autonomic nervous system at the time of measurement.

Referring to the result of analysis determination means A (Method A) in Fig. 104, it can be seen that coordinate points are unevenly distributed in the first and the second quadrants. This indicates that she is in a basically unrelaxed state, and the physical condition greatly transits as the concentrated (strained) state (first quadrant) and the loosened state (second quadrant) are repeated therein. Viewing the separation distance from the origin, there is a measurement day on which the measured point is a half or less of the separation distance 10 corresponding to the inner circle, indicating a slump. It can be said that the analysis determination means A determines a condition of a human being macroscopically, and the analysis determination means B (Method B) determines a physical condition relatively microscopically for indicating the physical condition at the time of analysis. In this sense, from the microscopic view, although uneven distribution into the third (relaxed and loosened state) and the fourth quadrant (relaxed and concentrated state) is observed, distribution into the first and the second quadrants is also observed, and in particular, from the fact that the separation distance is long in the second quadrant and short in the fourth quadrant, it can be realized that the activity of the parasympathetic nerves is weak and there is a strong tendency of not being relaxed.

In the sine-representation coordinates and tangent-representation coordinates of Fig. 105, uneven distribution into the first and the fourth quadrants is observed although the coordinate points are distributed entirely. Exhibition of such uneven distribution is also indicative of bad basic physical condition of the subject.

Also in the analysis determination means A (Method A) of Fig. 106, uneven distribution in the first quadrant and the fourth quadrant is observed. Although there is a day on which the coordinate point transits to the fourth quadrant by a rest effect by daytime napping, large displacement in the first quadrant is observed on more days, and there is a high tendency that the subject cannot be relaxed even by daytime napping and fatigues accumulate. Also in the analysis determination means B (Method B), large separation distance in the first quadrant is observed on many days, indicating that there is a day on which the subject cannot be relaxed.

The coordinate points are distributed in the entire sine-representation coordinates of Fig. 107; however, they are relatively distributed unevenly in the first quadrant in the tangent-representation coordinates. Therefore, also from this, it can be seen that the subject is in a strained state and cannot be relaxed on many days.

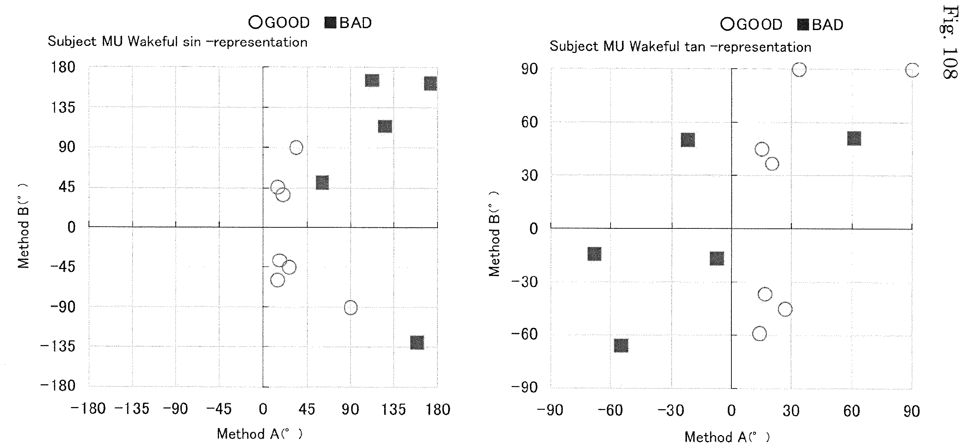

Fig. 108 to Fig. 109 are diagrams illustrating comparison between in a wakeful state and during daytime napping of Subject MU. In the diagrams, "GOOD" indicated by a white circle represents the analysis result of the analysis time segment when the physical condition is subjectively good, and "BAD" indicated by a black square represents the analysis result of the analysis time segment when the physical condition is subjectively bad. In the wakeful state, the coordinate points are unevenly distributed in the first and the fourth quadrants in a good physical condition both in the sine-representation coordinates and the tangent-representation coordinates as shown in Fig. 108. Since many coordinate points are distributed in the first quadrant, it can be seen that the degree of strain is high even when the physical condition is good.

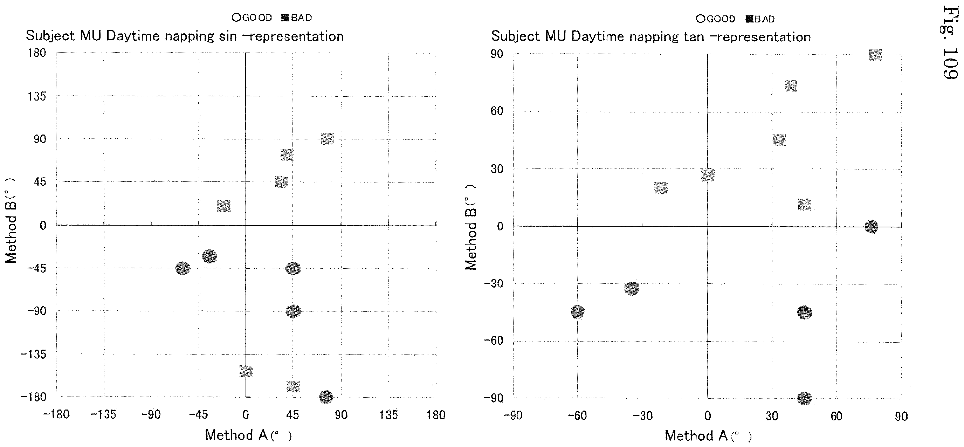

During daytime napping, as shown in Fig. 109, the tendency of dispersing along the horizontal axis corresponding to the analysis determination means B (Method B) is observed both in the sine-representation coordinates and the tangent-representation coordinates. In other words, Subject MU is of such a type that, when she takes a daytime nap, she recovers according to the change rate after change in the biological state, and is more susceptible to the disturbance in comparison with the process of recovering with the change in the biological state, and her sleep seems to tend to be shallow, and has a strong tendency of having such a sleep that gives awakening in a relatively short time, or a sluggish sleep.

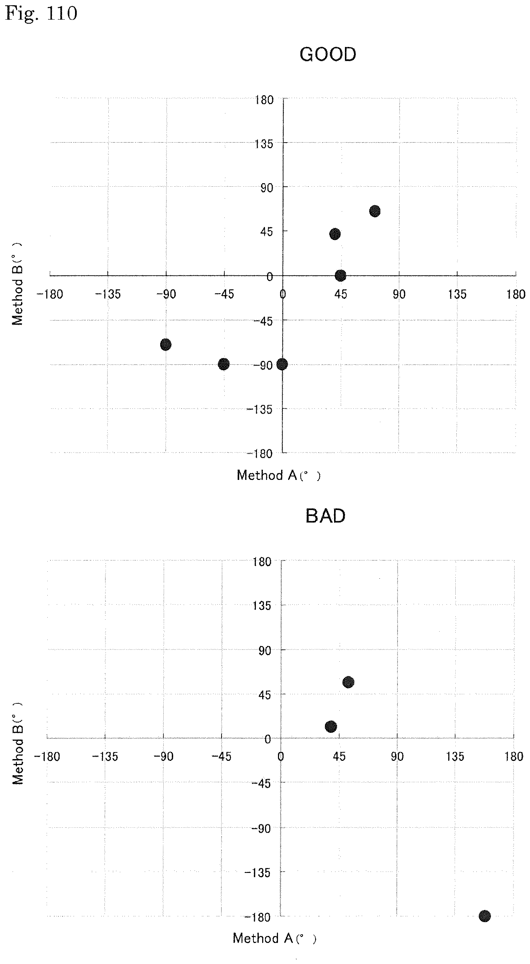

Fig. 110 is a diagram illustrating the results of the nocturnal sleep test of Subject MU on sine-representation coordinates. The data of Subject OG shown in Fig. 96 tends to be dispersed on the side of the second and the third quadrants in the case of "GOOD" not accompanied by intermediate awakening, and tends to be dispersed on the side of the first and the forth quadrants in the case of "BAD" accompanied by intermediate awakening. On the other hand, in the case of Subject MU, the coordinate points tend to be dispersed on the side of the first and the forth quadrants in the case of "BAD" accompanied by intermediate awakening; however, in the case of the "GOOD" that is believed not to be accompanied by intermediate awakening, some coordinate points are dispersed on the side of the second and the third quadrants and other coordinate points are dispersed on the side of the first and the fourth quadrants. Since Subject MU shows signs of dysfunction of the autonomic nervous system, discord between the subjective symptom and the analysis result arises. In other words, even if Subject MU is not aware of intermediate awakening while subjectively having a deep sleep feeling, the result indicates that in the nocturnal sleep for which the coordinate points are plotted on the side of the first and the fourth quadrant, the sleep is accompanied by intermediate awakening and thus the sleep is of poor quality. In other words, Subject MU does not usually take a good sleep from the objective view. Therefore, even if the subject subjectively feels "GOOD", the feeling is relative feeling to her usual sleep of not so high quality, when the coordinate points are plotted on the side of the first and the fourth quadrant. Hence, according to the determination method of the present invention, this case is objectively determined as "BAD". On the other hand, the case where the coordinate points are dispersed on the side of the second and the third quadrants among the cases of "GOOD" results from recovery of the function of Subject MU, and it can be said that the determination method of the present invention can be an objective determination method that will not depend on the subjectivity of the subject.

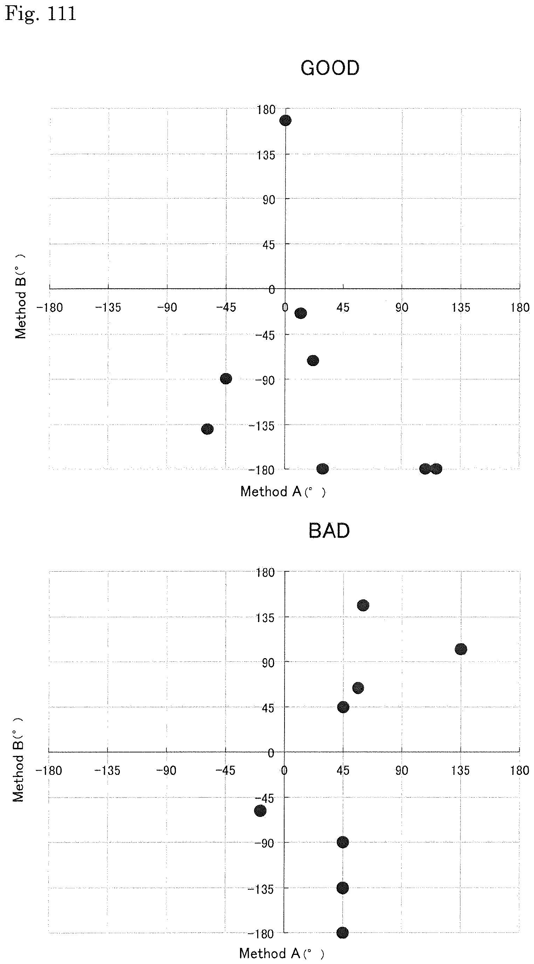

Fig. 111 is a diagram illustrating the analysis result of Subject KT in 20's. Subject KT also has signs of dysfunction of the autonomic nervous system. Therefore, on the measurement day of subjectively "BAD", the coordinate points are dispersed on the side of the first and the fourth quadrants, but on the measurement day of subjectively "GOOD", discord between the data and the analysis result arises as is the case with the above subject. Likewise Subject MU, in this Subject KT, the case where the coordinate points are dispersed on the side of the second and the third quadrants among the cases of "GOOD" comes into coincident with the objective determination as a result of recovery of the function.

### (Sleep Quality Estimation 4)

Next, male Subject YG in 20's took a nocturnal sleep of about 6.5 hours on three different Beds A, B and C over three days, and the biological signals (APW) collected in these sleeps were analyzed to select a bed suited for Subject YG. Subject YG is a so-called short sleeper whose hours of sleep are about 4 hours on average.

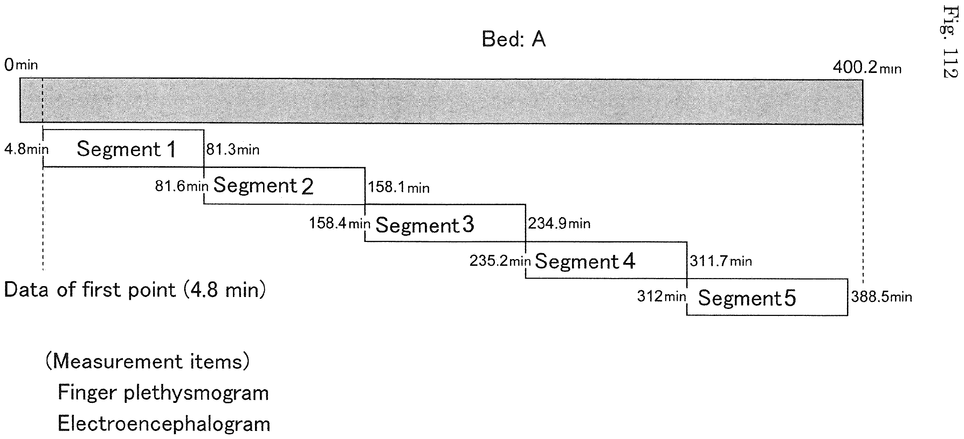

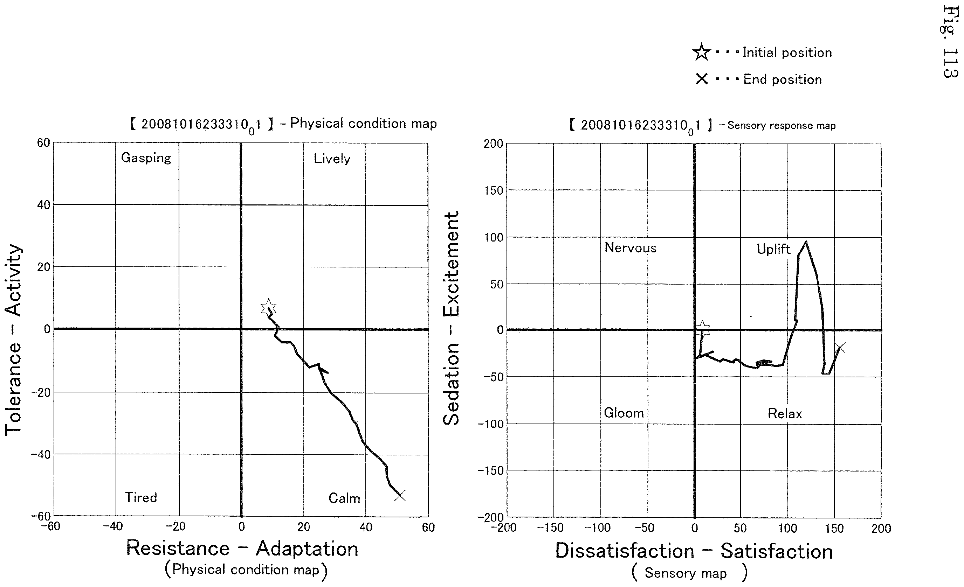

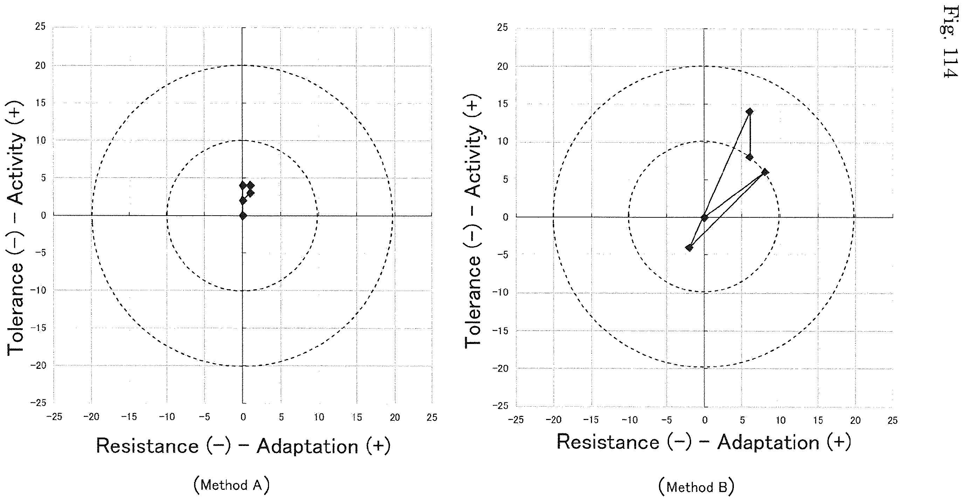

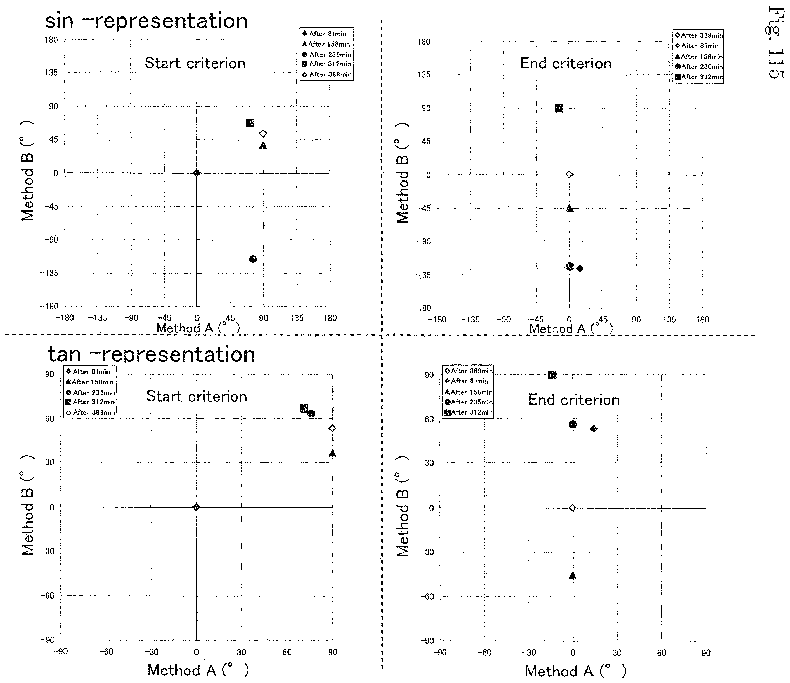

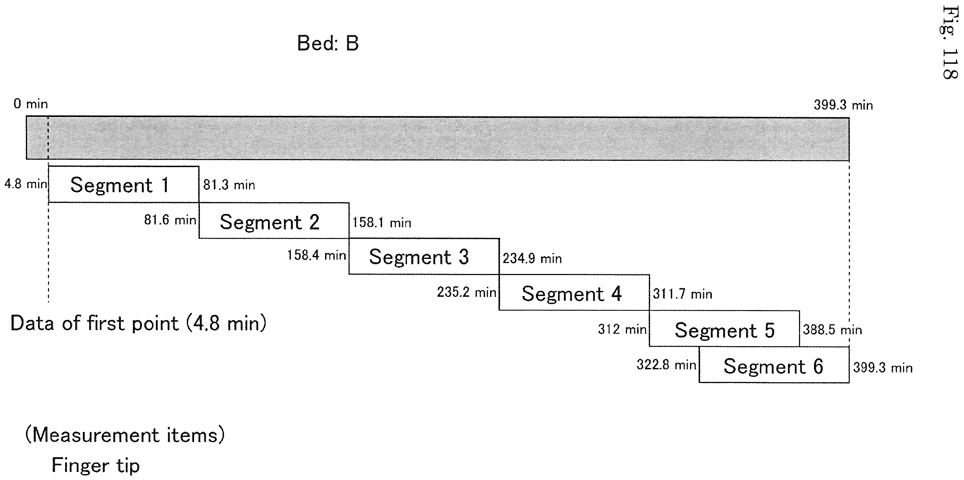

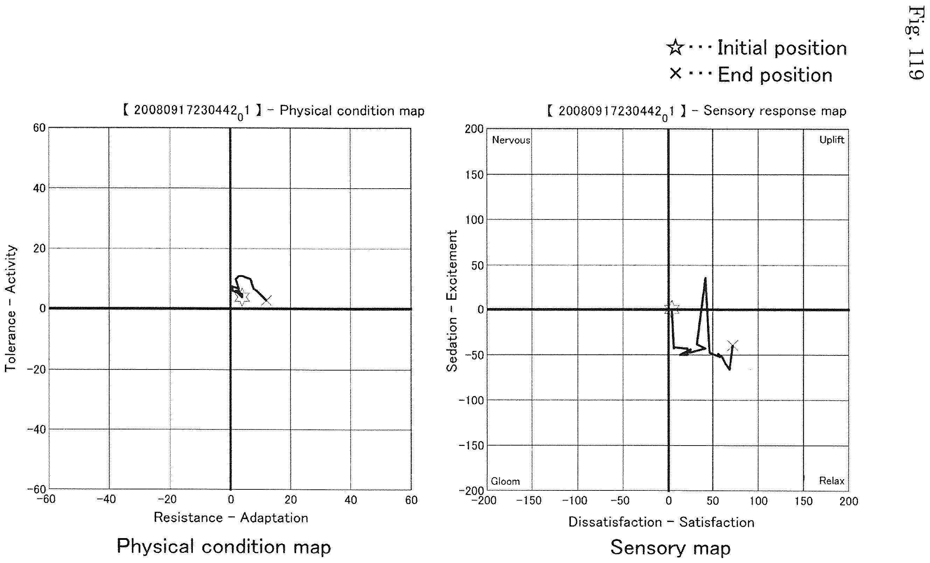

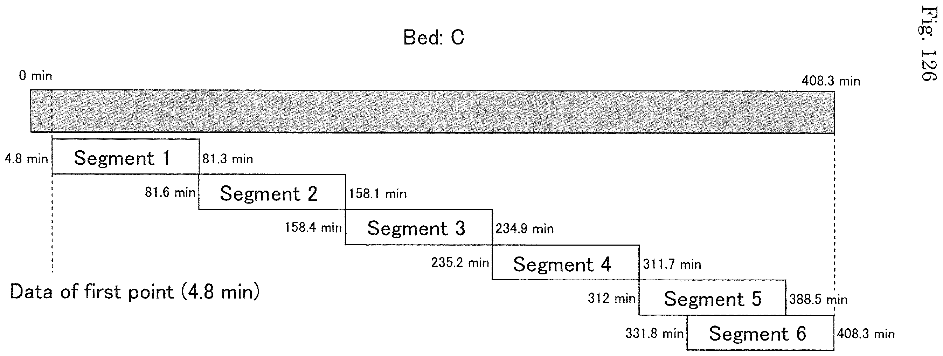

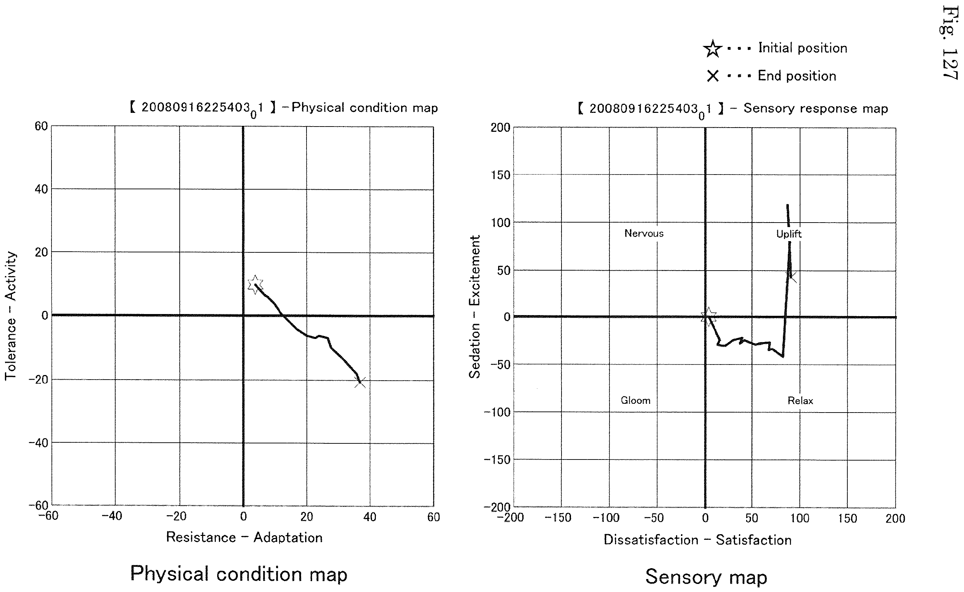

Fig. 112, Fig. 118 and Fig. 126 illustrate the analysis time segments (analysis time zones) during sleeping on Bed A, B, and C. Fig. 113, Fig. 119 and Fig. 127 illustrate a physical condition map and a sensory map, Fig. 114, Fig. 120 and Fig. 128 illustrate the analysis results obtained by the analysis determination means A (Method A) and the analysis determination means B (Method B) in the first analysis determination means 951, and Fig. 115, Fig. 121 and Fig. 129 illustrate the analysis results of the second analysis determination means 952.

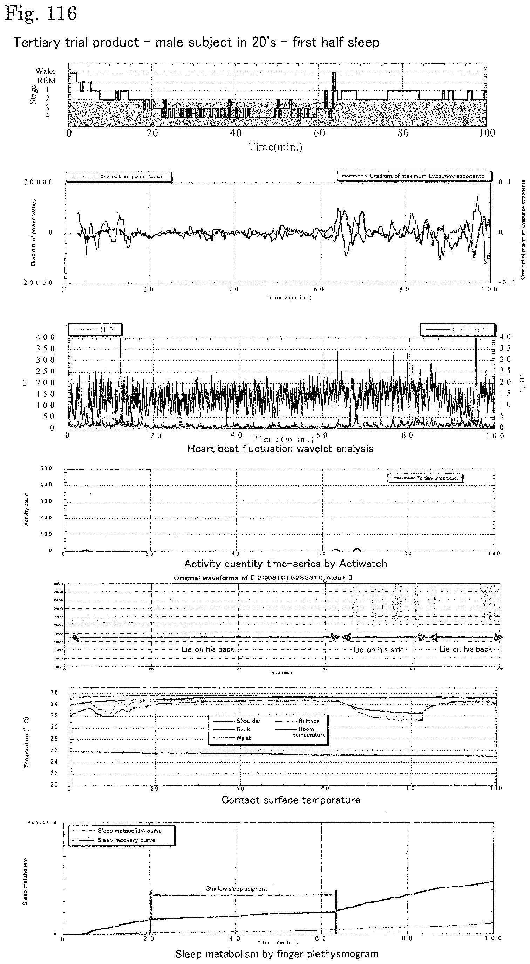

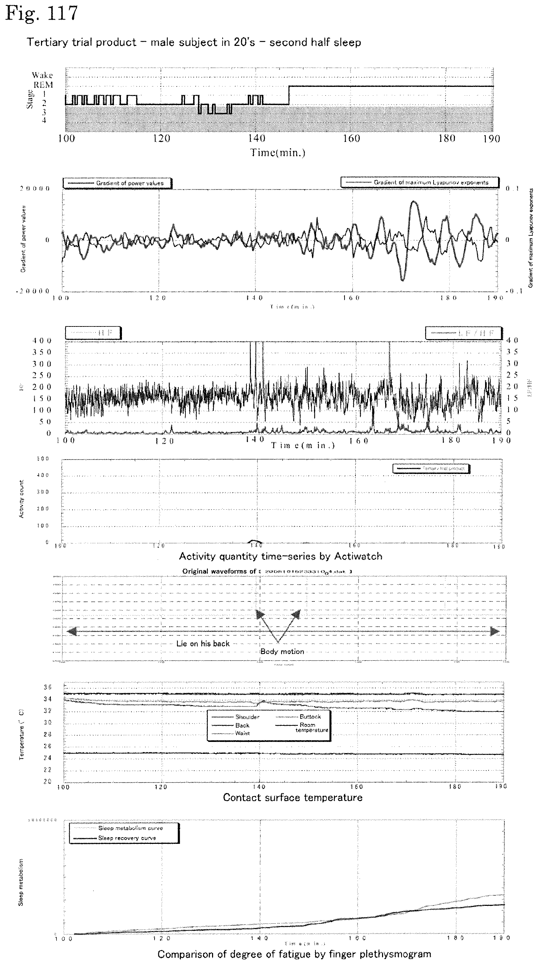

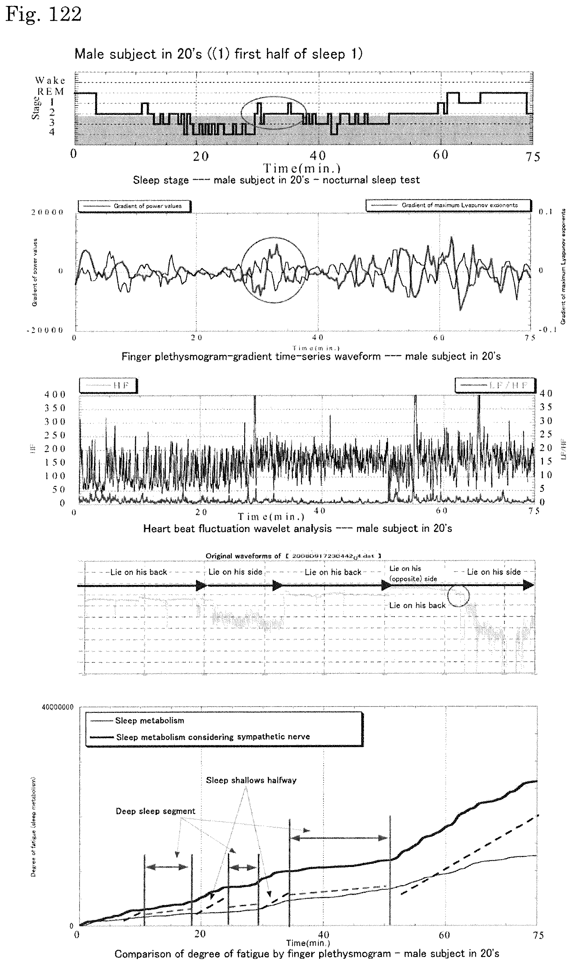

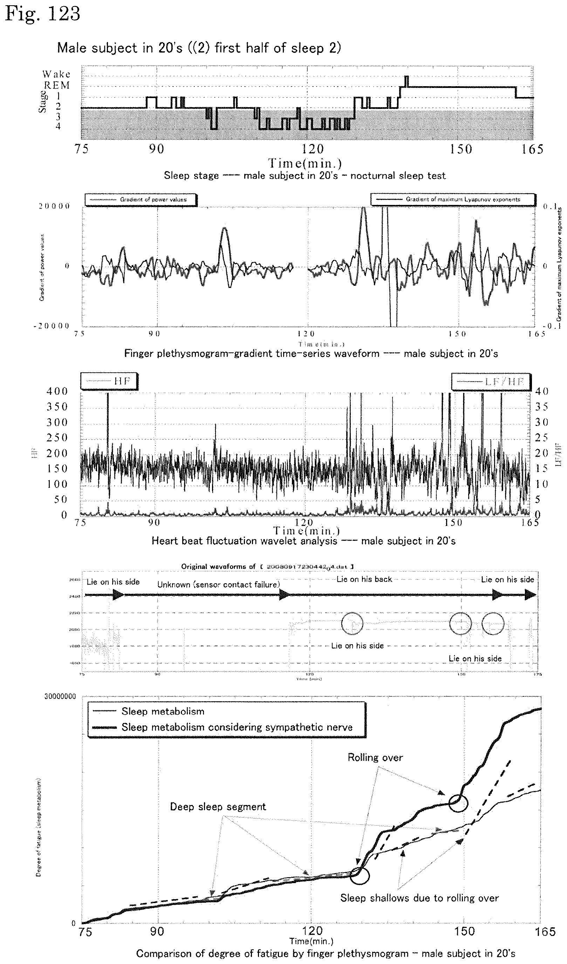

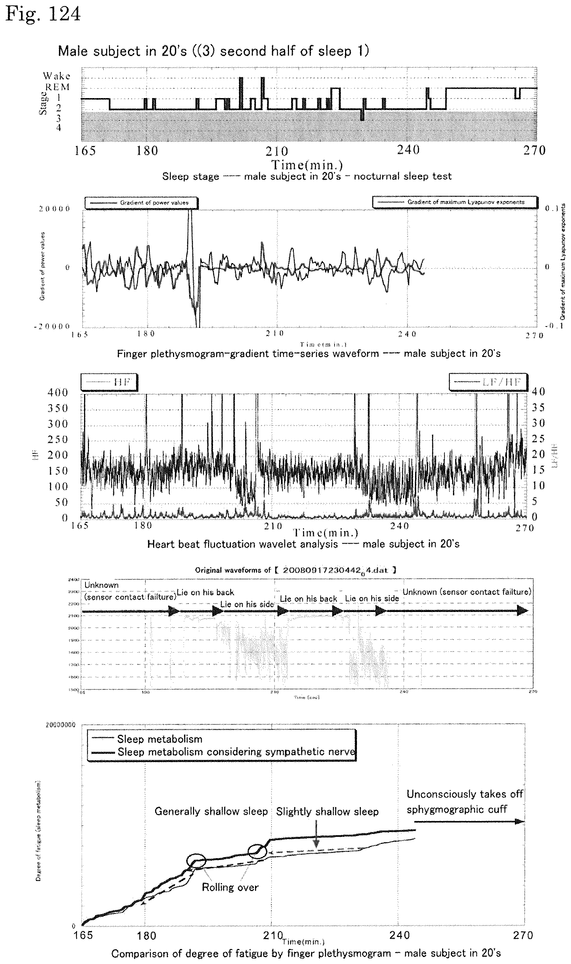

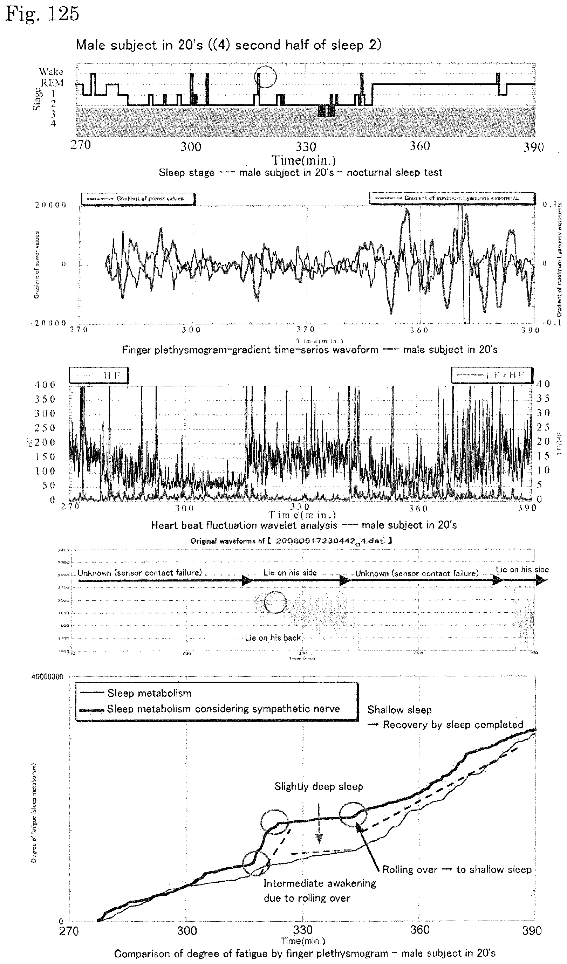

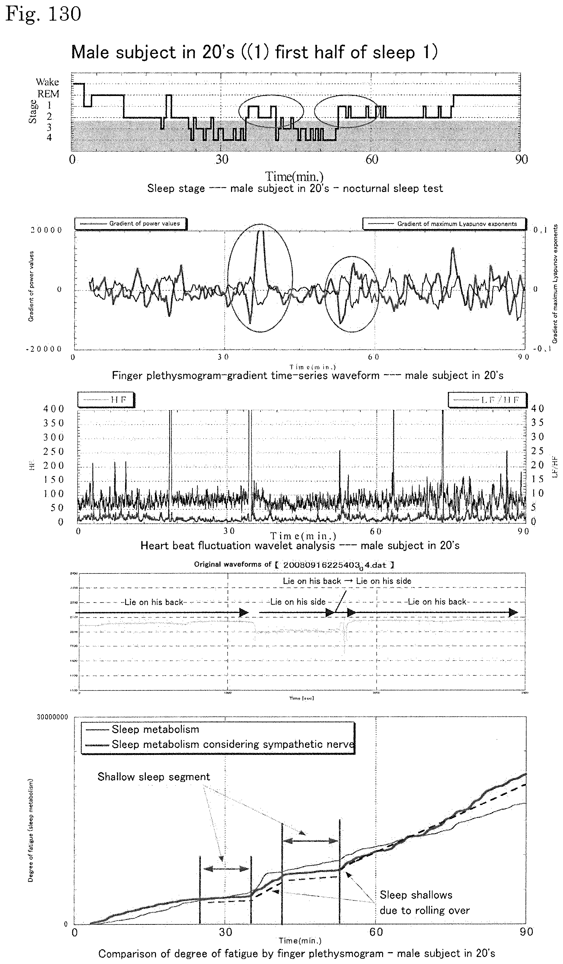

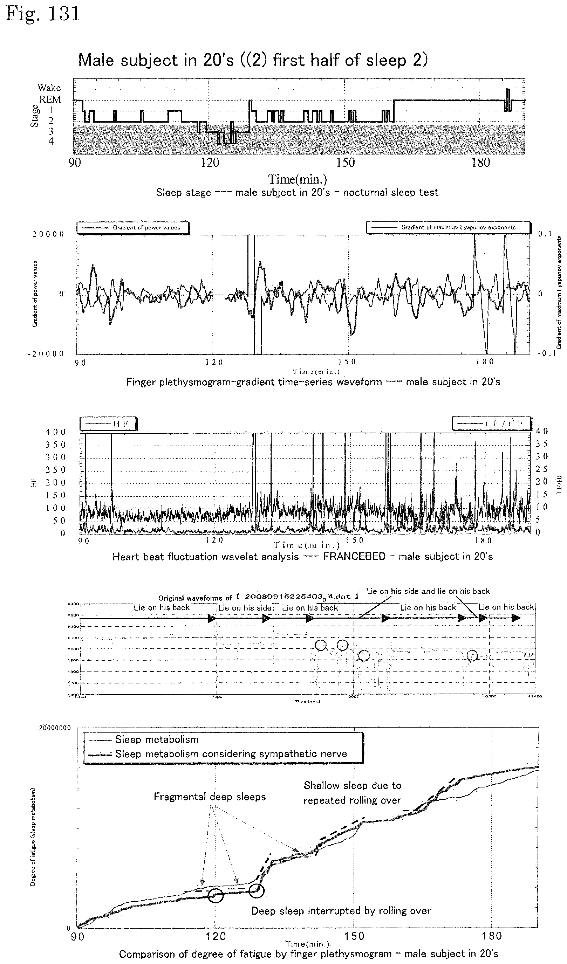

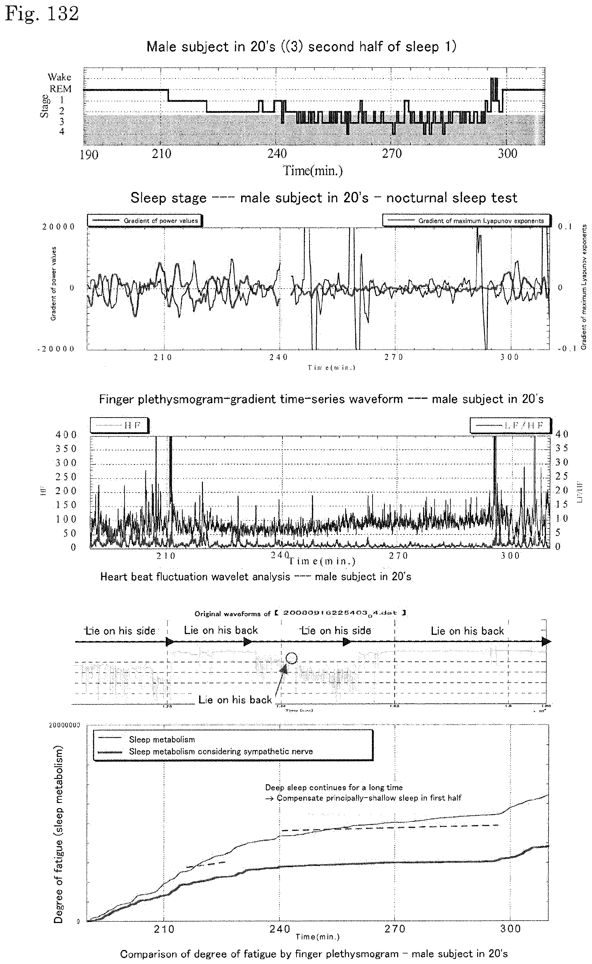

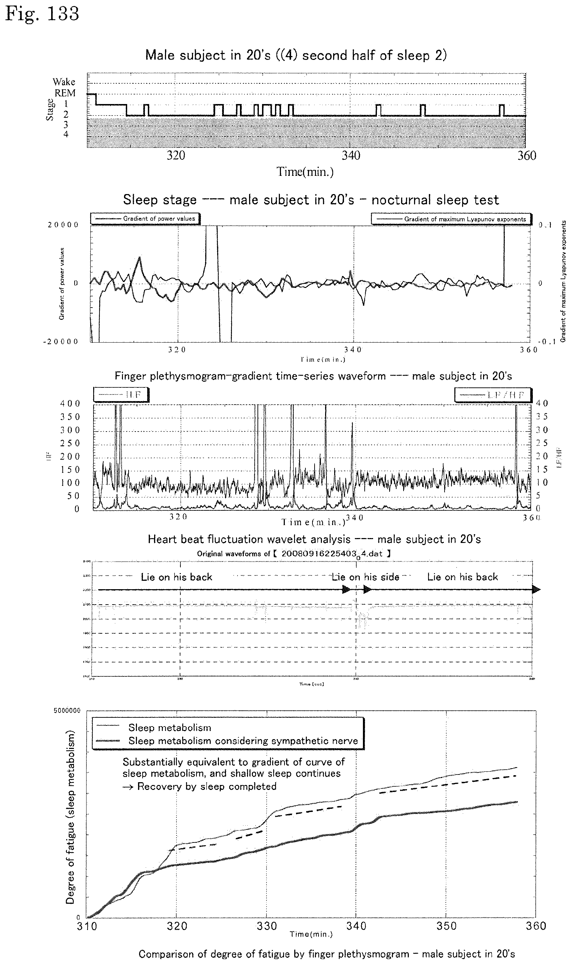

Here, Fig. 116 to Fig. 117 illustrate the measurement results by the means for measuring an existent condition of a human being during a sleep measured during a sleep on Bed A, and illustrate, in the order from top to bottom, the determination result of depth of sleep by electroencephalogram, the time-series waveforms of gradients of power and maximum Lyapunov exponents of finger plethysmograms, time-series waveforms of HF and LF/HF obtained by a wavelet analysis of the waveforms of the electrocardiogram, the time-series waveform of activity quantity by activity meter (Actiwatch), the transition of posture condition, the transition of temperature of the contact surface with Bed A, and the transition of sleep metabolism determined from the finger plethysmogram. Fig. 122 to Fig. 125 illustrate the measurement results by the means for measuring an existent sleep condition (quality) measured during a sleep on Bed B, and illustrate, in the order from top to bottom, the determination result of depth of sleep by electroencephalogram, the time-series waveforms of gradients of power and maximum Lyapunov exponents of finger plethysmograms, time-series waveforms of HF and LF/HF obtained by a wavelet analysis of the waveforms of the electrocardiogram, the transition of posture condition, and the transition of sleep metabolism determined from the finger plethysmogram. Fig. 130 to Fig. 133 illustrate the measurement results by the means for measuring an existent sleep condition (quality) measured during a sleep on Bed C, and illustrate, in the order from top to bottom, the determination result of depth of sleep by electroencephalogram, the time-series waveforms of gradients of power and maximum Lyapunov exponents of finger plethysmograms, time-series waveforms of HF and LF/HF obtained by a wavelet analysis of the waveforms of the electrocardiogram, the transition of posture condition, and the transition of sleep metabolism determined from the finger plethysmogram.

In general, a sleep in which REM sleep and non-REM sleep are regularly repeated on an approximately 90-minute cycle, and no intermediate awakening is contained is regarded as a sleep of high quality, and one can feel refreshed when he/she wakes up during REM sleep. This point is determined according to the existent indexes of Fig. 116 to Fig. 117, Fig. 122 to Fig. 125 and Fig. 130 to Fig. 133. As the determination result, Subject YG can take a sleep of higher quality than prescribed on any of Beds A, B and C, and the quality of sleep is highest in Bed A, followed by Bed B and Bed C.

Then the determination results are compared with the methods of the present invention. In comparison among the physical condition maps of Fig. 113, Fig. 119 and Fig. 127, the time-series variation line transits mainly in the fourth quadrant on a gradient of 1/f (45 degrees) in Bed A. In Bed B, the time-series variation line transits in the first quadrant on a gradient of 1/f. In Bed C, the time-series variation line transits across the first quadrant and the fourth quadrant on a gradient of 1/f. In the sensory map of Bed A, the time-series variation line temporarily shifts below within the fourth quadrant, and then transits substantially parallel with the horizontal axis. The curve jumps on the side of the first quadrant midway, and this reveals temporary emotional uplift. Also in Bed B and Bed C, after the time-series variation line temporarily drops, it transits substantially parallel with the horizontal axis, and also there is temporary emotional uplift midway. The width until the line returns to the former line that is substantially parallel with the horizontal axis after occurrence of temporary emotional uplift is largest in Bed A, followed by Bed B and Bed C.

These reveal that in the physical condition map, a sleep of higher quality is taken when the line transits on a gradient of 1/f (45 degrees), and in particular, the longer the transition time in the fourth quadrant which is a relax region, the more preferable the quality of the sleep. In the sensory map, it is desired that the line transits parallel with the horizontal axis and stably with little vertical fluctuation in terms of the quality of the sleep, and when emotional uplift occurs midway, it is preferred that the line gradually rises and gradually returns rather than changing suddenly for high stability. The temporary drop indicates the course of falling asleep, and when there is no such a dropping course, and the line extends along the horizontal axis from the beginning, it meant that the subject has immediately fallen asleep.

Here, the physical condition map is prepared by sequentially determining coordinate points for each analysis time segment according to a predetermined criterion by using difference between the analysis times that are set differentially within the analysis time segment, and plotting the results as the time-series variation line, and the map represents the time-series change of physical condition within the analysis time segment. This is prepared by the physical condition map preparation means which is a computer program set in the state estimation means 95. Concretely, by the procedure similar to Procedures A1, A2 of the analysis determination means A as descried above, analysis times in each analysis time segment is segmented more finely, and the coordinate points are sequentially plotted from the first analysis time segment to the last analysis time segment without taking the initial position as the origin, and thus, the time-series variation line is prepared.

The sensory map is prepared by sequentially determining coordinate points for each analysis time segment according to a criterion different from that of the physical condition map preparation means by using difference between the analysis times that are set differentially within the analysis time segment, and plotting the results as the time-series variation curve, and represents the time-series change of sense within the analysis time segment. This is prepared by the sensory map preparation means which is a computer program set in the state estimation means 95. Concretely, the physical condition change score obtained by frequency analysis of the zero-cross detection means is represented on the horizontal axis, and the variation (gradient) of graph determined from the time-series waveform of frequency fluctuation obtained by the peak detection means is represented on the vertical axis. The time-series waveform of frequency fluctuation is given by conducting a slide-calculation for determining a mean value of frequency for each of predetermined time windows set with a predetermined overlap period in the time-series waveform obtained by the frequency computation means 710 as described above, and outputting the physical condition change scores of the mean value of frequency obtained for each time window as the frequency fluctuation time-series waveform. It is determined by the frequency fluctuation computation means that is executed by the frequency fluctuation computation procedure which is a computer program of the biological state estimation device of the present invention. Since the time-series waveform of frequency fluctuation by the peak detection means is linked with the frequency fluctuation of the heart rate, it is possible to easily determine whether the heart rate is increased, decreased or stagnated with high sensitivity according to whether the variation (gradient) of the time-series waveform of frequency fluctuation is increased, decreased or stagnated, and it will be an index that reflects the perception that a person has (because the perception markedly reflects the increase or decrease of the heart rate) at the time more directly.

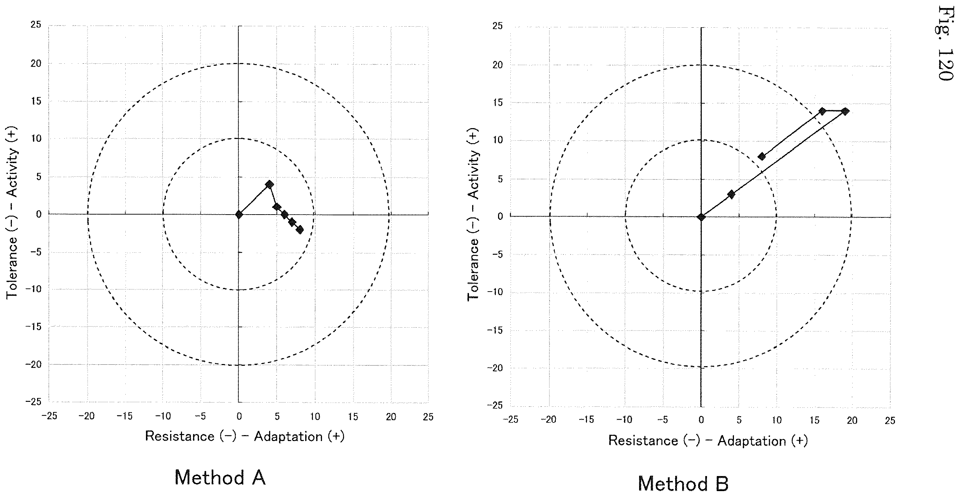

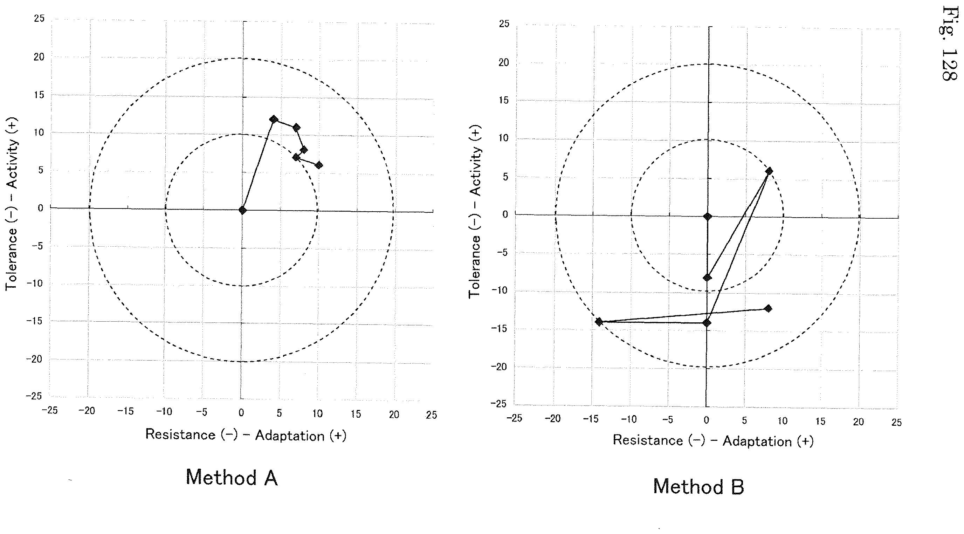

Next, the analysis results obtained by the analysis determination means A (Method A) and the analysis determination means B (Method B) in the first analysis determination means 951 illustrated in Fig. 114, Fig. 120 and Fig. 128 will be compared with the existent indexes as described above. First, in Fig. 114, the data of the second point is largely displaced from the origin to near the inner circle in Method B. In Fig. 120, the data of the third point is largely displaced from the origin to the position exceeding the inner circle in Method B. In Fig. 128, the data of the second point is largely displaced from the origin to the position exceeding the inner circle in Method A, and the data of the third point is largely displaced from the origin to near the inner circle in Method B. From the above, exhibition of large displacement of coordinate point at least in either of the analysis determination means A (Method A) and the analysis determination means B (Method B) in an initial stage of sleep is a criterion for determination of a sleep of good quality. In other words, taking a normal nocturnal sleep properly is a function recovery action that is important for a human being to live, and the importance thereof is much larger than that of a temporary sleep such as a daytime nap. Therefore, the physical conditions (conditions of organs including the heart) during a sleep of a predetermined time or longer for function recovery (normal nocturnal sleep) is presumed to be largely different from the conditions in a wakeful state where fatigue advances. Therefore, as the state changes to a sleep state, such large displacement with respect to the wakeful state (origin) would be exhibited.

Next, the analysis results of the second analysis determination means 952 illustrated in Fig. 115, Fig. 121 and Fig. 129 will be compared with the existent indexes as described above. In each drawing, the sine-representation coordinates and the tangent-representation coordinates on the left-hand side of the drawing are given for comparing by plotting the data from start of falling asleep to the first analysis time segment as the origin (start criterion), and the sine-representation coordinates and the tangent-representation coordinates on the right-hand side of the drawing are given for comparing by plotting the data of the analysis time segment directly before wakeup (the last analysis time segment of the test) as the origin (end criterion). Since particularly significant difference appears in the sine-representation coordinates, comparison will be made between the respective sine-representation coordinates. This subject is a short sleeper as described above, and tends to wake up in a shorter time compared with ordinary people. Therefore, determination as a sleep of good quality not accompanied by intermediate awakening is less likely to be made in such a long-time sleep as is the present test. Therefore, in these drawings, the coordinate points plotted on the side of the second and the third quadrants tend to be basically few in this subject.

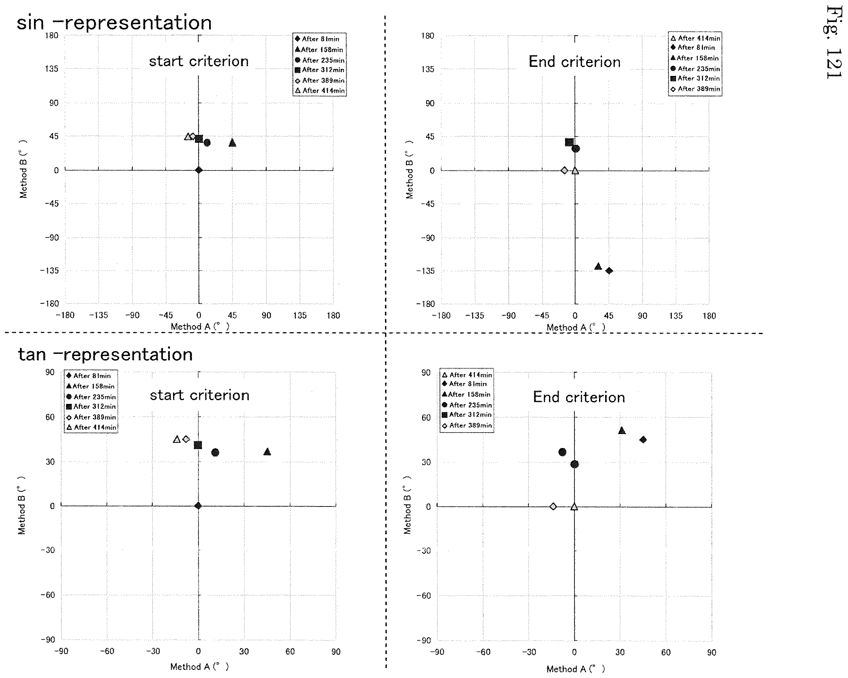

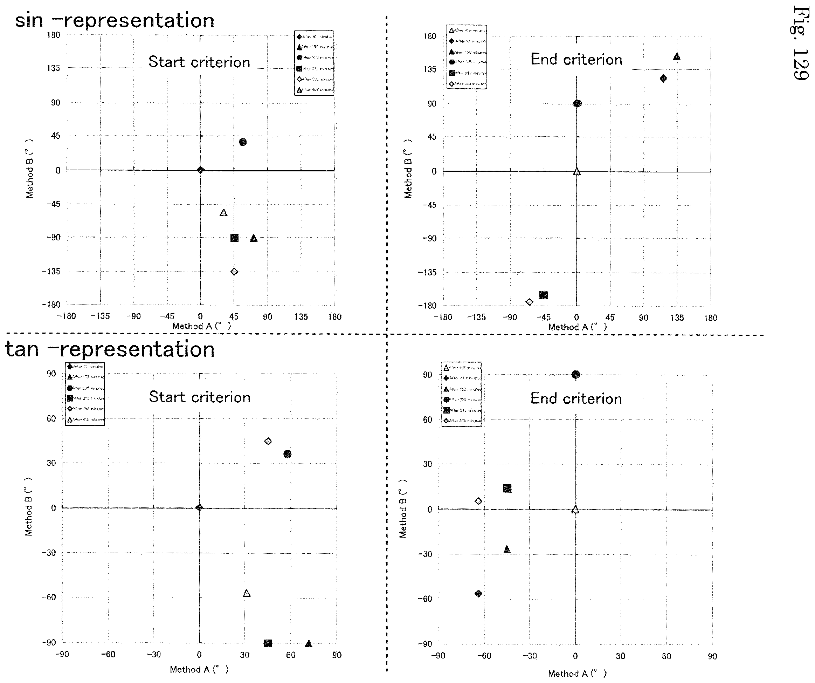

Among these, in Fig. 129, the coordinate points of the first half are unevenly distributed on the side of the first and the fourth quadrants as is clearly shown in the end criterion, and it is realized that the sleep in the first half is not deep. In the case of Fig. 121, change is small and fluctuation is small in any case of start criterion and end criterion. This indicates that there is no difference between REM sleep and non-REM sleep that is required for a sleep of good quality, so that the sleep cannot be said to be a sleep of high quality. On the other hand, in Fig. 115, the coordinate points are more dispersed compared with the case of Fig. 121, and as to the end criteria, the data generally tends to be distributed near the second and the third quadrants compared with other cases. Therefore, the relative comparison reveals that Bed A is suited for Subject YG.

### (Examination of peak/zero-cross detection means 1)

Data of drinking tests and fatigue tests were analyzed. The analyses of the drinking tests were conducted using data of five male subjects in Fig. 21 and additionally data of one female subject. The average age of the subjects was 22.8 ± 4.5, and all the subjects were active in an alcohol patch test conducted before the test. The intake alcohol was one can of 500 ml-canned beer (alcohol concentration 5%) (Subjects 01 to 04, 06), or 180 ml of shochu (alcohol concentration 17%) (Subjects: 05, 06) as described above. The alcohol intake per body weight was 0.36 ± 0.03 g/kg.

The time zone to be analyzed is after 35-45 minutes from start of the test in an undrunk state, and after 10-20 minutes after end of drinking. The detected APW was converted into a time-series waveform by using the peak detection means and the zero-cross detection means, and a gradient time-series waveform regarding frequency fluctuation was determined. This time-series waveform was subjected to a spectrum analysis, and the result was displayed on a log-log graph, and an approximation line (hereinafter, referred to as fractal analysis result) was determined.

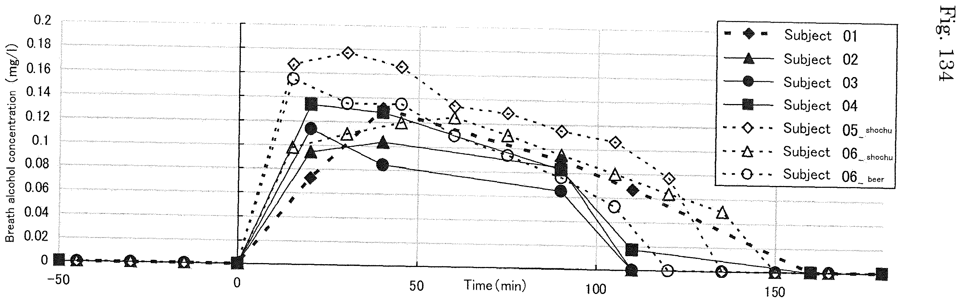

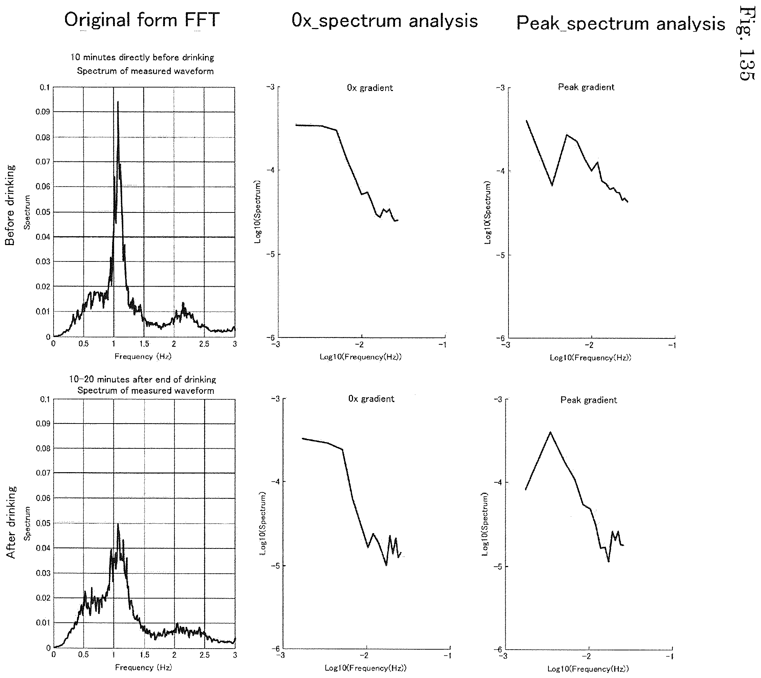

Fig. 134 is a diagram illustrating breath-alcohol concentrations. All the subjects exhibited 0.11 to 0.18 mg/l, which corresponds to a slight intoxication refresh state. Fig. 135 is a diagram illustrating the results of Fourier transformation for APW before and after drinking, and the fractal analysis results. In contrast to before drinking, the peak frequencies in the vicinity of 1 Hz are dispersed after drinking, and this demonstrates that the heart rate is disrupted, and the heart turns into an irregular vibration system as a result of drinking. In the fractal analysis result, since changes in the components of 0.01 to 0.04 Hz are larger in the waveform by the peak detection means than in the waveform by the zero-cross detection means, it can be realized that the control of the heart changes.

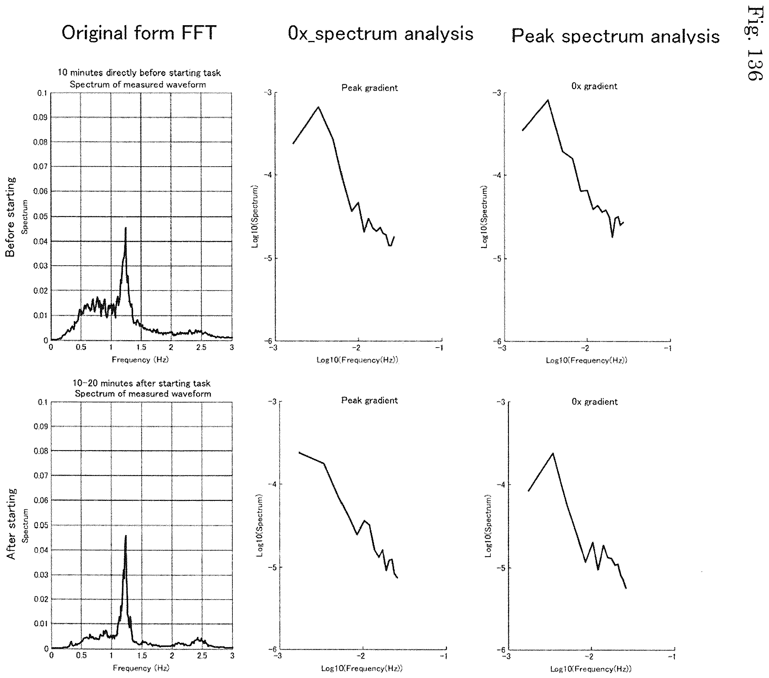

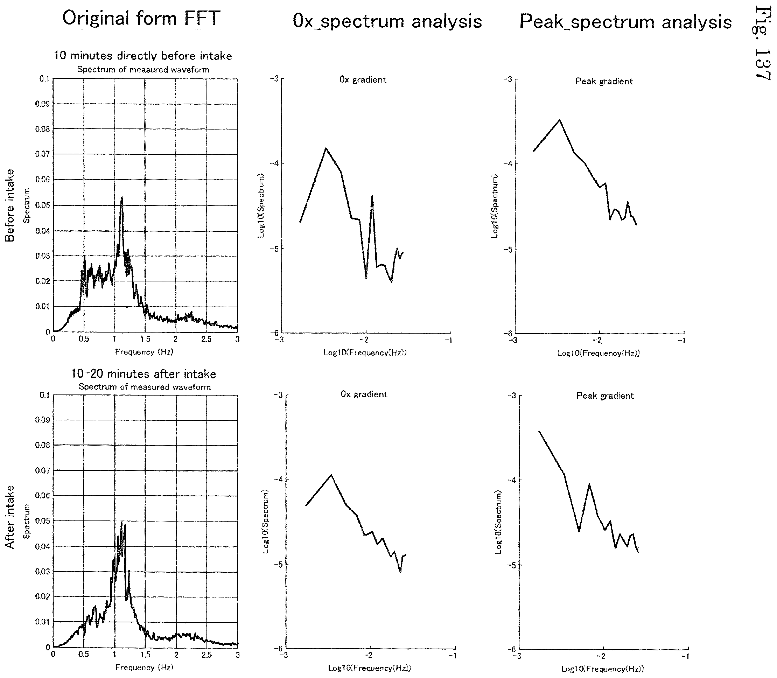

Fig. 136 is a diagram illustrating the case of playing a computer game (task) for 60 minutes in sitting postures, and the results of Fourier transformation for APW before and after starting of the task, and the fractal analysis results. Change in the frequency band in the vicinity of 1 Hz is small, but change in the range between 0.5 Hz and 1 Hz is large. In the fractal analysis, changes in the components of 0.001 to 0.0053 Hz are larger in the waveform by the zero-cross detection means than in the waveform by the peak detection means. Fig. 137 illustrates the case of drinking Nutrient Drink A having a high taurine content, and a similar tendency as the case of charging the task in Fig. 136 is exhibited. Although the power spectrum of the 0.5th component decreases after intake, the change is not so large, and is such a level that decrease is observed, and the 1st component slightly exhibits the behavior of irregular vibration system.

In other words, in the data of fatigue (with task) and the data of Nutrient Drink A intake, the spectrum change of the original APW waveform exhibits a smaller spectrum of the 0.5th component after starting of the task or after intake, than before starting of the task or before intake. The spectrum of the 1st component exhibits harmonic oscillation accompanied by little change in center frequency. Comparison between before drinking and after drinking reveals that the spectrum of the 1st component largely decreases, and turns into irregular vibration. The center frequency of the fluctuation of heart rate shifts from 1.2 Hz to 1.0 Hz, and decrease in heart rate due to sleep is observed.

On the other hand, the gradients determined from the zero-cross detection means and the peak detection means little change between before and after starting of the task or between before and after intake in the data of fatigue (with task) and the data of Nutrient Drink A intake, and is nearly 1/f, and it seems to be a state where the sympathetic nerves and the parasympathetic nerves are well balanced. On the other hand, in the data of drinking, while the gradient is nearly 1/f before drinking, a bifurcation phenomenon occurs in the vicinity of 0.01 Hz both in the zero-cross detection means and the peak detection means after drinking. This is ascribable to the aforementioned irregular vibration due to disturbance of heart rate fluctuation. From this case, it is proved that APW is able to detect a rough change of physical condition, and the extremely or ultra low frequency of APW is able to detect reactions of autonomic nervous systems.

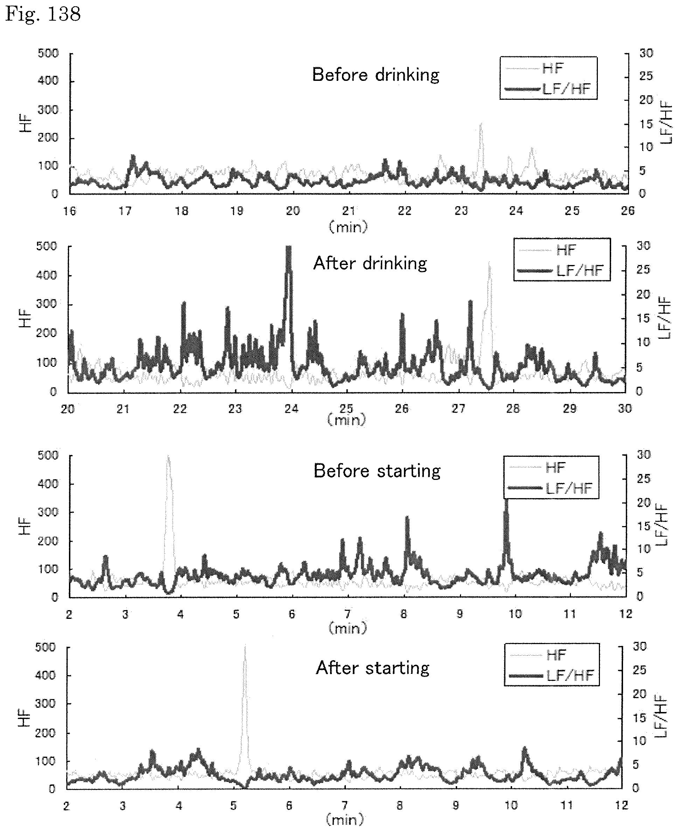

Fig. 138 is a diagram illustrating the results of HF and LF/HF determined from the finger plethysmograms. Under the drinking condition, both the sympathetic nerve system and the parasympathetic nerve system are increased from about 20 minutes after drinking. It can be seen that the function of the sympathetic nerve system decreases under the sitting fatigue condition. These results are similar to the results of APW as shown in Fig. 135. As shown in Fig. 134, regardless of whether the alcohol concentration exceeds 0.15 mg/l at which drunk-driving determination is made, change in the irregular variation system occurred in the dynamic state of the heart as shown in Fig. 135, and change in the time-series waveform obtained from the peak detection means occurred. Drinking causes change in the activity level of the heart such as elevation of heart rate, and fall in blood pressure as an acute action of alcohol is also reported. Here, it is suggested that drinking causes increase in the cardiac autonomic nervous system and decrease in the sympathetic nerve system activity. However, in the slight intoxication refresh state, increase in the autonomic nervous system is problematic, and in comparison with a rest state, it seems that a fractal analysis method by the peak detection means is effective for the influence by drinking. The results of Fig. 135 and Fig. 136 suggested that the peak detection means indicates the action of the sympathetic nerve and parasympathetic nerve systems, and the zero-cross detection means indicates the action of the sympathetic nerve system.

These suggest the possibility of estimating whether the subject is drunk or not by utilizing the fractal analysis result of the gradient time-series waveform determined from the APW. Since change is observed in the range of 0.01 to 0.04 Hz also in zero-cross detection means, it will be more desirable to take both components into account.

### (Examination of peak/zero-cross detection means 2)

In the above description, the analysis is conducted using the frequency time-series waveform determined by the zero-cross detection means and the peak detection means. Here, the time-series waveform of frequency obtained by the zero-cross detection means corresponds to the action of the sympathetic nerve system as described above, and the time-series waveform of frequency obtained by the peak detection means corresponds to the action including actions of both the sympathetic nerves and the parasympathetic nerves, namely the action of the parasympathetic nerve system controlled by the action of the sympathetic nerves (parasympathetic nerve activity in which sympathetic nerve compensatory action is included). Hence, by determining the index corresponding to only the parasympathetic nerve activity in which an index for the sympathetic nerves is not included, it can be possible to grasp the degree of activity of the sympathetic nerves and the parasympathetic nerves more clearly.

Here, in the biological state estimation device of the present invention, the peak/zero-cross detection means may be set as the means for grasping only the activity of the parasympathetic nerves. The peak/zero-cross detection means divides the data of the time-series waveform of frequency using the peak point in the peak detection means by the data of the time-series waveform of frequency using the zero-cross point in the zero-cross detection means, and determines the time-series waveform of frequency using the obtained peak/zero-cross values.

The frequency-gradient time-series waveform analysis and computation means determines frequency-gradient time-series waveforms from each of the time-series waveforms of frequency respectively obtained from the zero-cross detection means and the peak/zero-cross detection means, and the fluctuation waveform analyzing means determines a first determination criterial score based on the fluctuation waveforms obtained from the time-series waveforms of frequency using the zero-cross detection means, and a second determination criterial score based on the fluctuation waveforms obtained from the time-series waveforms of frequency using the peak/zero-cross detection means. The state estimation means determines a coordinate point on the coordinate system while taking the first determination criterial score as an index of one axis, and the second determination criterial score as an index of the other axis.

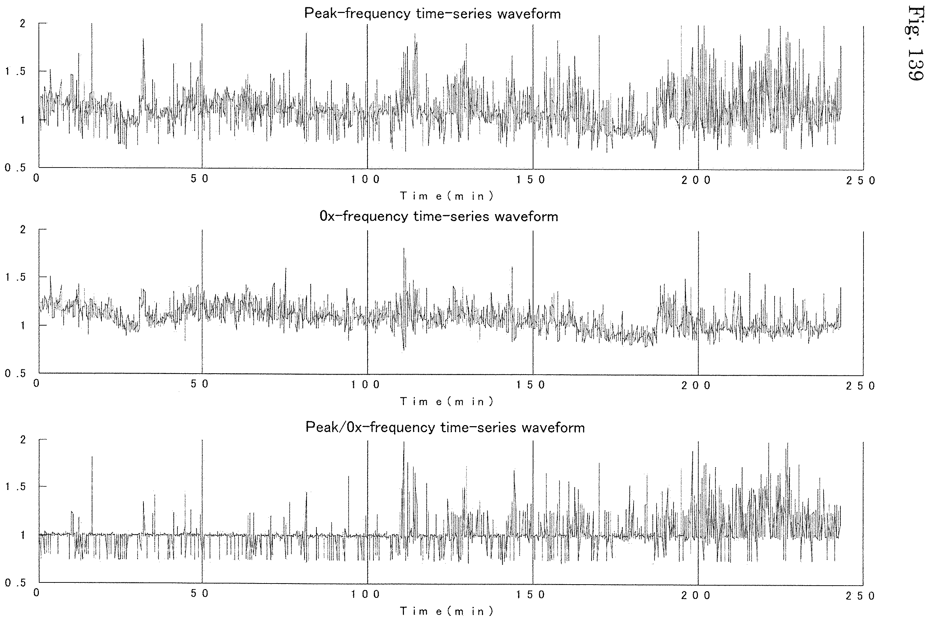

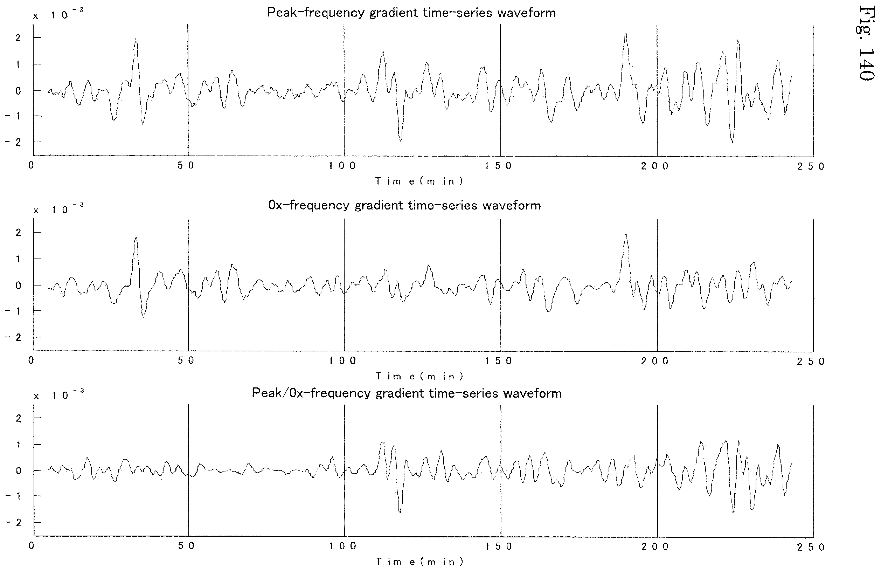

Fig. 139 is a diagram illustrating a frequency time-series waveform using the peak detection means (peak-frequency time-series waveform), a frequency time-series waveform using the zero-cross detection means (0x-frequency time-series waveform), and a frequency time-series waveform using the peak/zero-cross detection means (peak/0x-frequency time-series waveform) of the data of Subject Uchikawa analyzed in Fig. 59 to Fig. 63. Fig. 140 is a diagram illustrating frequency-gradient time-series waveforms obtained from the frequency time-series waveforms (peak-frequency time-series waveform, 0x-frequency time-series waveform, peak/0x-frequency time-series waveform) of Fig. 139.

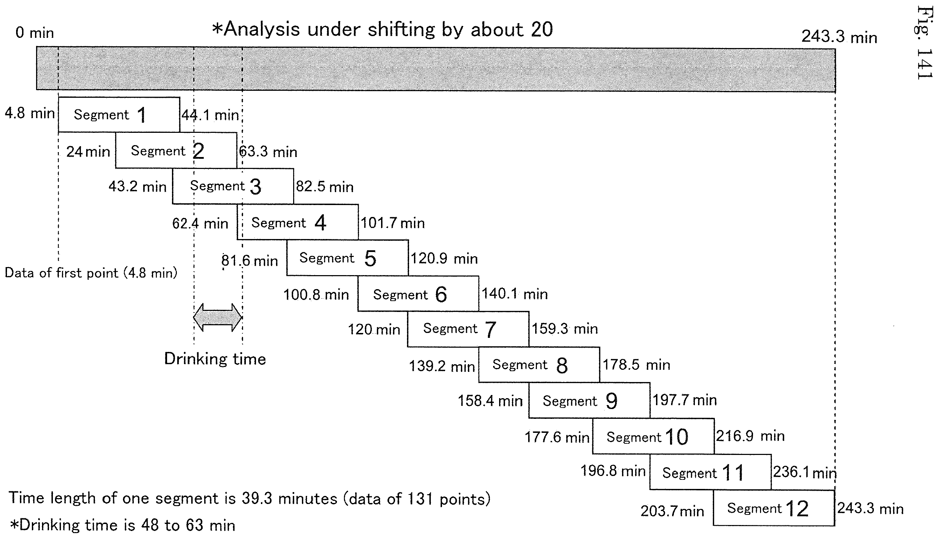

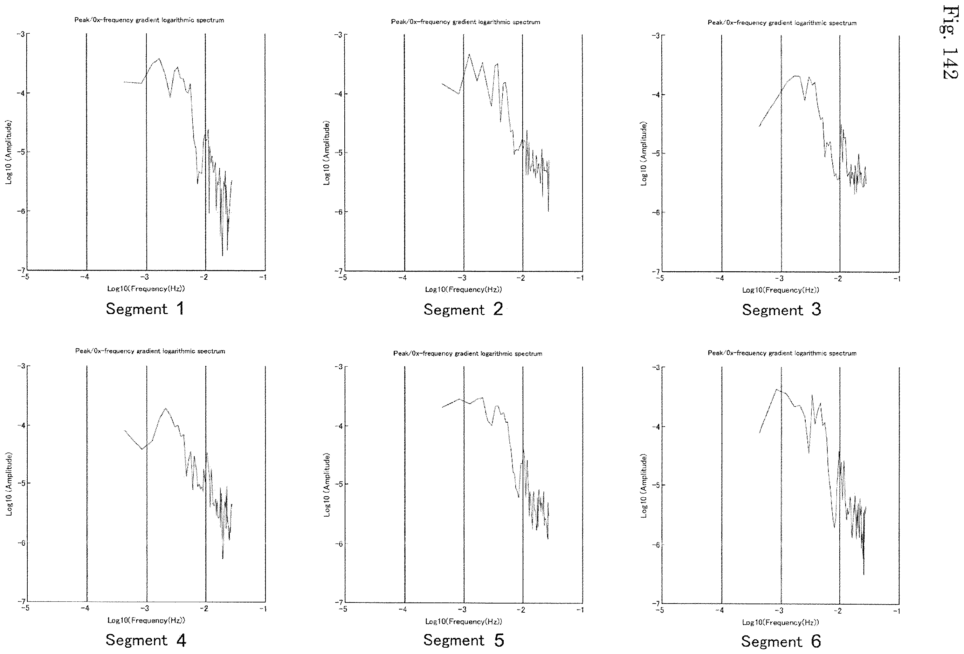

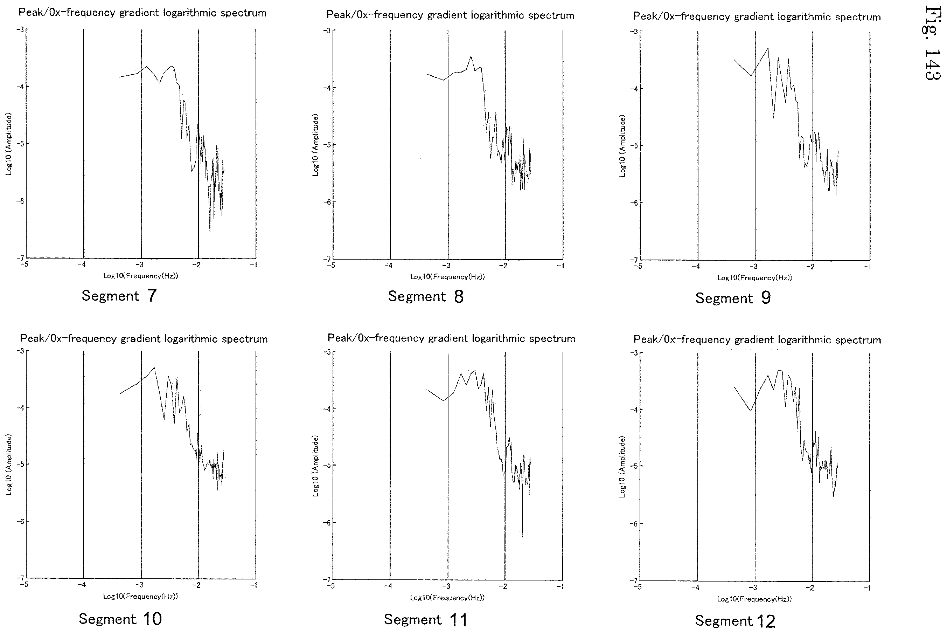

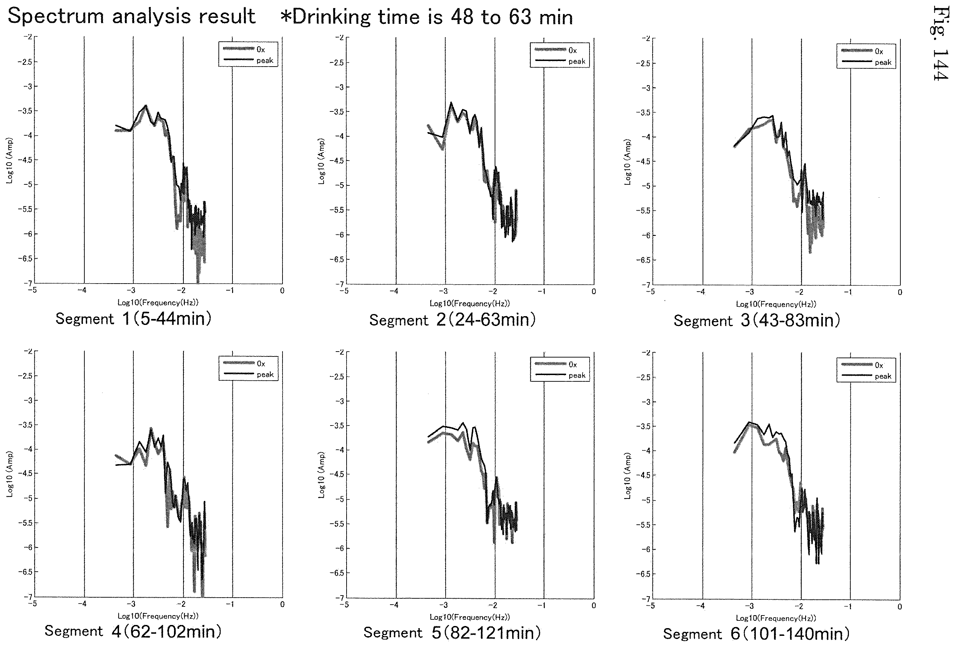

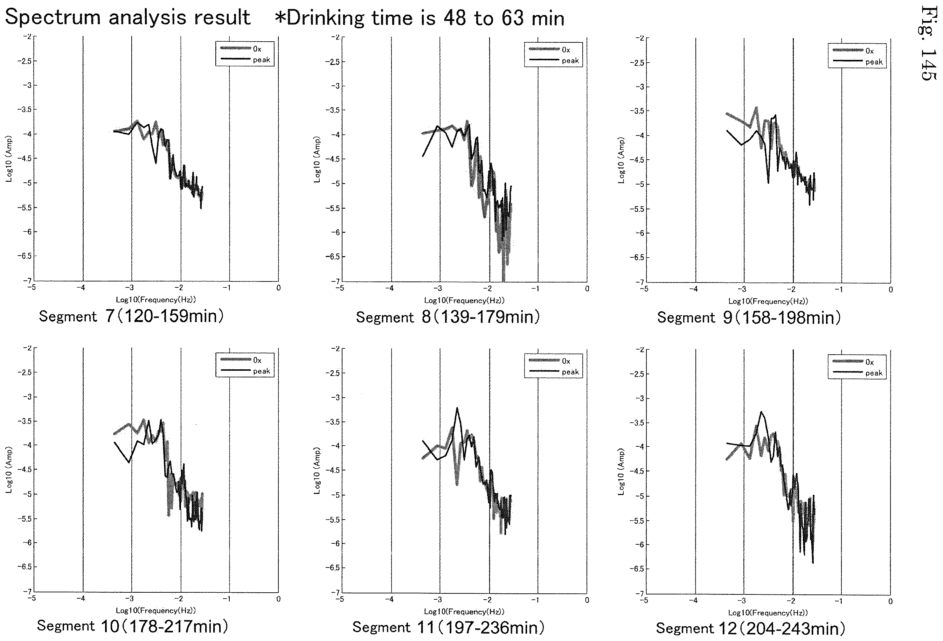

Fig. 141 is a diagram illustrating analysis time segments used for analyzing 0x-frequency-gradient time-series waveforms and peak/0x-frequency-gradient time-series waveforms obtained in the above. Fig. 142 and Fig. 143 are diagrams illustrating the fluctuation waveforms of log-log graphs in respective analysis time segments of peak/Ox-frequency-gradient time-series waveforms obtained by frequency analysis. Fig. 144 and Fig. 145 are diagrams illustrating the fluctuation waveforms of log-log graphs in respective analysis time segments of 0x-frequency-gradient time-series waveforms and peak-frequency-gradient time-series waveforms obtained by frequency analysis. By comparing Fig. 142 and Fig. 143, with Fig. 144 and Fig. 145, it is possible to find that the fluctuation waveforms of log-log graphs of peak/0x-frequency-gradient time-series waveforms differ from the fluctuation waveforms of log-log graphs of peak-frequency-gradient time-series waveforms.

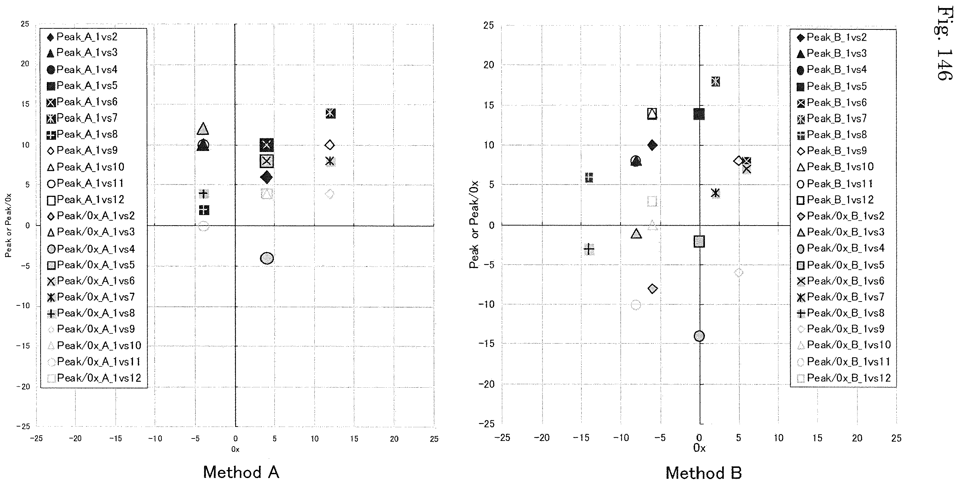

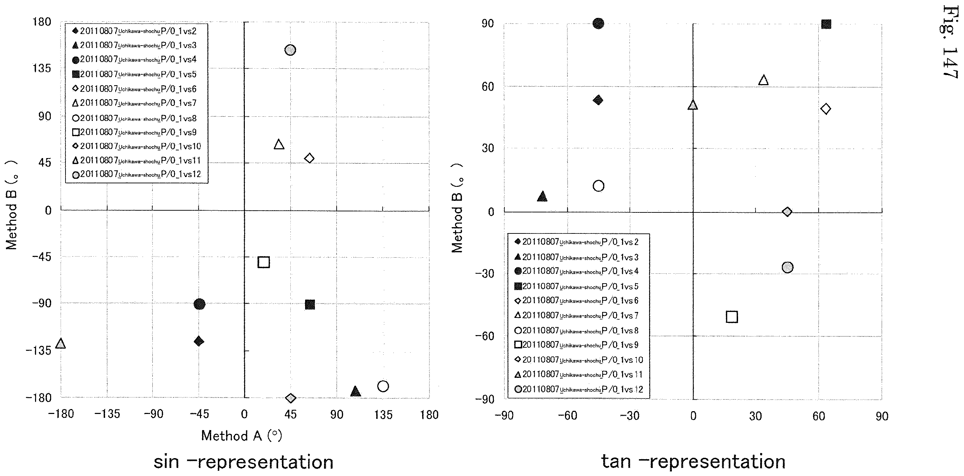

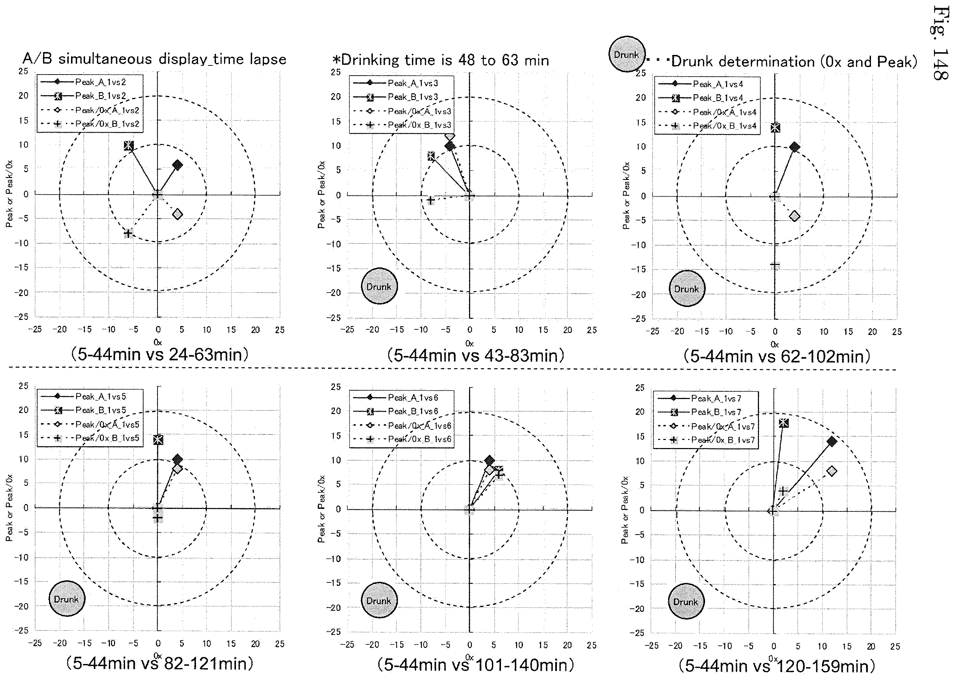

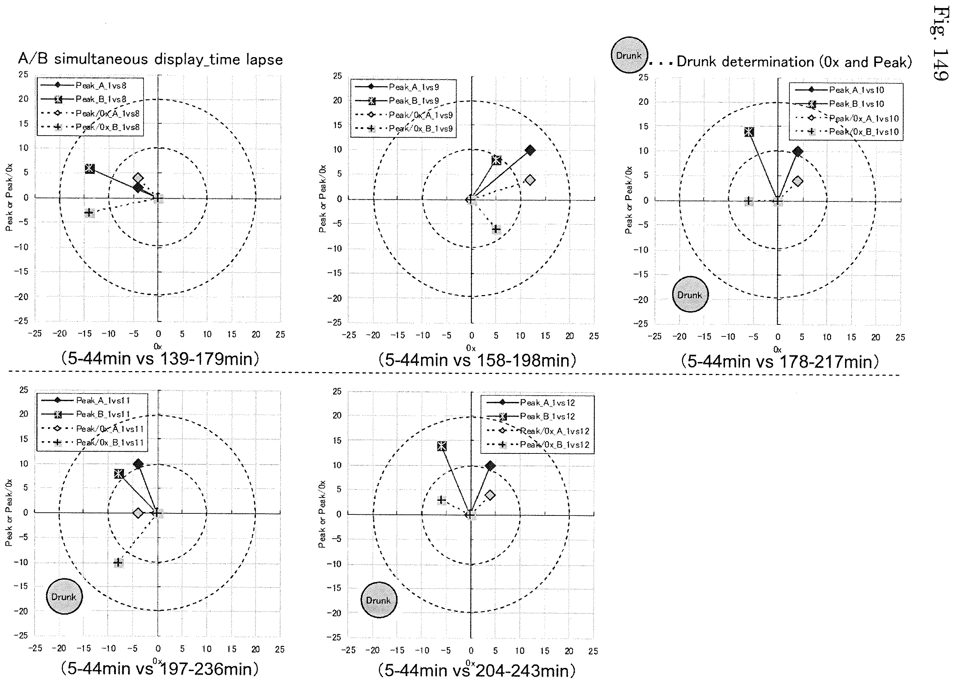

Fig. 146 is a diagram illustrating the analysis results obtained by the analysis determination means A (Method A) and the analysis determination means B (Method B) using the above fluctuation waveforms. The horizontal axis is set by using the determination criterial score determined from the fluctuation waveforms of the 0x-frequency-gradient time-series waveforms, and the vertical axis is set by using the determination criterial score determined from the fluctuation waveforms of the peak/0x-frequency-gradient time-series waveforms. For comparison, the results obtained by using the peak-frequency time-series waveforms of Fig. 60 are also shown. Fig. 147 is a diagram illustrating the sine-representation coordinates and the tangent-representation coordinates obtained for the coordinate points plotted among the coordinate points of Fig. 146 using the determination criterial score of the peak/0x-frequency-gradient time-series waveforms as a vertical axis. Fig. 148 and Fig. 149 are diagrams illustrating the display results in respective analysis time segments of the data obtained by the analysis determination means A (Method A) and the analysis determination means B (Method B) illustrated in Fig. 146 likewise Fig. 62 and Fig. 63. In Fig. 148 and Fig. 149, the results obtained by determining the vertical axes illustrated in Fig. 62 and Fig. 63 from the peak-frequency time-series waveforms are also illustrated (in the drawings, "Peak A" and "Peak B" represent data of vertical axes obtained from the peak-frequency time-series waveforms, and "Peak/0x A" and "Peak/0x B" represent data of vertical axes obtained from the peak/0x-frequency-gradient time-series waveforms). The display of "Drunk" indicates the analysis time segment for which "drunk" determination is made based on the results of the vertical axes of Fig. 62 and Fig. 63 from the peak-frequency time-series waveforms.

In the results of the analysis determination means A and B of Fig. 146, the coordinate points obtained by using the peak/0x-frequency-gradient time-series waveforms are plotted while they are entirely deviated in the direction of the third quadrant and the fourth quadrants in comparison with the results obtained by using the peak-frequency time-series waveform. As to each analysis time segment of Fig. 148 and Fig. 149, it can be seen that the analysis time segment for which "drunk" determination is made by the result using the peak-frequency time-series waveforms does not correspond to "drunk" determination in the result using the peak/0x-frequency-gradient time-series waveforms. On the other hand, in the result using the peak/0x-frequency-gradient time-series waveforms, there is a strong trend that the number of coordinate points plotted in the third and the fourth quadrants increases. It is deemed that when the peak/0x- frequency-gradient time-series waveforms which sensitively reflects the state of the parasympathetic nerves are used, the information on being relaxed by drinking acutely appears, and the correlation with the physical condition increases. On the other hand, the information about influence of the drinking on the sympathetic compensatory mechanism is no longer considered, so that the cases where data of both the analysis determination means A and B are plotted in the donut-shaped region is reduced, and it can be said that it is more appropriate to use the peak-frequency time-series waveforms considering the sympathetic compensatory mechanism for "drunk" determination.

Here, Fig. 144 is a diagram illustrating a physical condition map and a sensory map prepared by using the index based on the peak-frequency time-series waveforms as a vertical axis, and Fig. 145 is a diagram illustrating a physical condition map and a sensory map prepared by using the index based on the peak/0x- frequency-gradient time-series waveforms as a vertical axis. Both of the physical condition maps have a gradient of nearly 1/f; however, the change in physical condition from the point at which subject is slightly strained in the initial stage of the test to a relaxed state by alcohol intake is more properly shown in Fig. 145. On the other hand, as to the sensory map, since the consciousness of stabilizing the feeling worked by the sympathetic compensatory mechanism even under alcohol intake, it can be said that the tendency appears more strongly in Fig. 144.

From these facts, when the peak/zero-cross detection means is used, the number of dependent variables reduces in comparison with the case where data obtained from the peak detection means is used as the vertical axis, and the sensitivity increases, so that the aspect of control of the autonomic nervous system is grasped more appropriately. However, there is a possibility that the gap with the sense recognizing the state accompanied by the sympathetic compensatory mechanism as a basic state can extend because of the acuteness and the increased sensitivity, but it is deemed that the correlation with the physical condition is improved. In other words, it can be said that the present method is appropriate for identifying the one that is purely controlled by the sympathetic and the parasympathetic functions.

### (Relation between APW and finger plethysmogram)

Figs. 150 are diagrams illustrating comparison between a verification finger plethysmogram and a surface pulse wave (APW) obtained by removing noise components of low-frequency band and high-frequency band such as breath and body motion of an original waveform of an output signal obtained from the sensing mechanism unit 230 of the biological signal measuring means 1, and then emphasizing and filtering the fluctuation components in respective beats. Fig. 150(i) is a diagram illustrating comparison by time-series waveforms. The peak time of the surface pulse wave coincides with that of the finger plethysmogram, and a good result was obtained. Fig. 150(ii) is a diagram illustrating a spectrum of finger plethysmogram. The peak at 1.35 Hz represents a basic component which is a pulse rate, and the peaks at 2.7 Hz and 4.05 Hz represent the second and the third harmonic components, respectively. Here, the peak at 2.7 Hz which is the second harmonic component is a reflected wave from the blood occurring every beat called a notch. Fig. 150(iii) is a diagram illustrating a spectrum of a pressure fluctuation waveform from the sensor unit. The peak band of less than 1 Hz is a component containing body motion and breath, and occupies the maximum component in the waveform. Also a basic component of pulse and a notch are detected; however, the amplitude levels thereof are equal to each other. Fig. 150(iv) is a diagram illustrating a spectrum of a surface pulse wave component obtained by a signal processing. Likewise the finger plethysmogram, a basic component and a notch were detected accurately. The bands of less than 1 Hz and more than or equal to 4 Hz shown in Fig. 150(iii) are blocked, and a good result wherein the basic component is emphasized compared with the notch component is obtained.

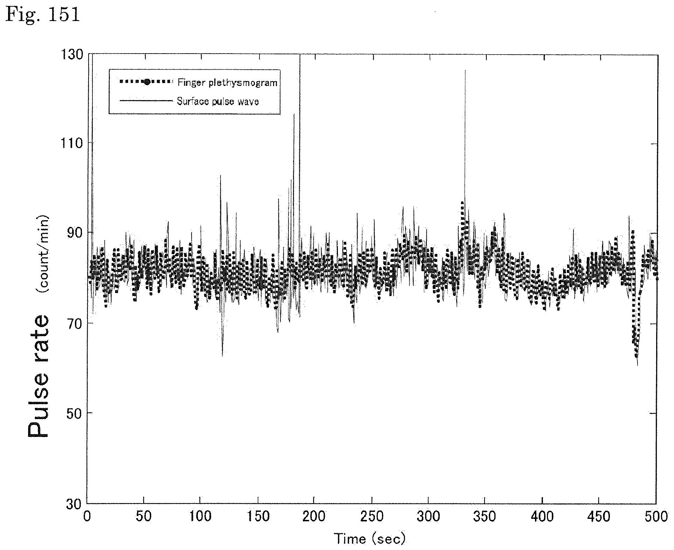

Fig. 151 is a diagram illustrating comparison between a surface pulse wave and a heart rate waveform calculated from the finger plethysmogram. The vertical axis represents a pulse rate per one minute. Both the surface pulse wave and the finger plethysmogram fluctuate centering on a pulse rate of about 80 times per minute, and the degrees of fluctuation almost coincide with each other. It is generally known that autonomic fluctuation can be grasped by a wavelet analysis of fluctuation of pulse. This suggests that autonomic fluctuation can also be grasped from a surface pulse wave.

### (Relation between APW and response of autonomic nervous system)

### (1) Test method

In a laboratory, subjects in sitting postures underwent a sleep induction test starting in a wakeful state and ending in a sleeping state. The subjects are 29 healthy male persons in 20's to 50's. The measurement items include APW, finger plethysmogram, electroencephalogram, and electrocardiogram.

### (2) Test results and Discussion

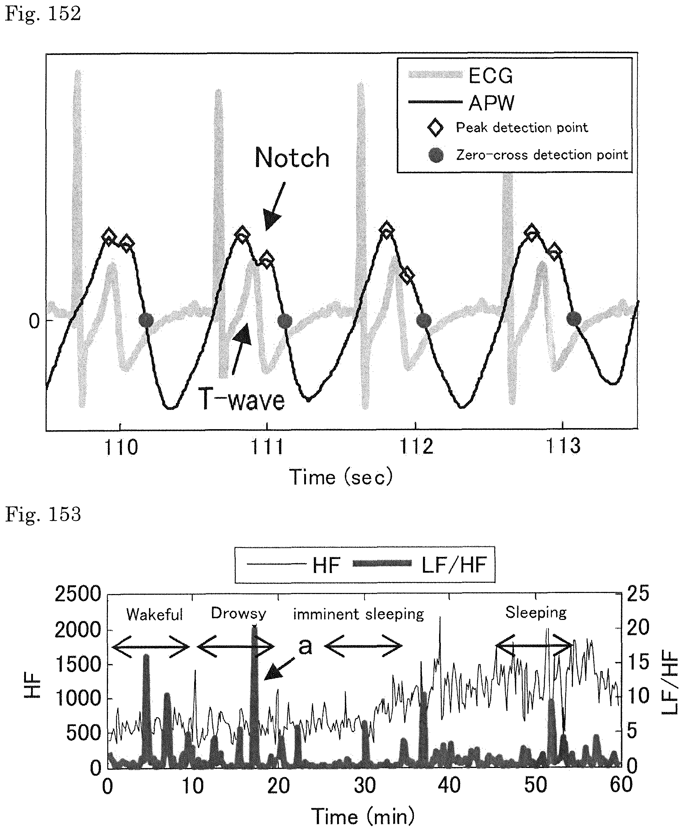

Fig. 152 is a diagram illustrating APW and electrocardiogram (ECG) of a male subject in 20's. The points shown in the drawing represent zero-cross detection points and peak detection points obtained by the zero-cross detection means and the peak detection means, respectively. The position of a notch of APW is nearly coincident with a T wave of ECG that appears in an ejection period when the semilunar value of the heart closes and the cardiac output stops. Therefore, the zero-cross detection means picks up data of a diastolic phase of the blood, and the peak detection means picks up data of both a diastolic phase and a systolic phase. In other words, the zero-cross detection means seems to grasp the action of the sympathetic nerve system by the data regarding elasticity of the aorta itself. The peak detection means seems to grasp actions of both the aorta and the heart, namely actions of both the parasympathetic nerve system and the sympathetic nerve system. If the state was estimated by using data containing both components regarding the extensibility of the aorta and the beat of the heart, the variation would large and it would be difficult to target. So, by separating information on the aorta and information on the heart from APW by using both the zero-cross detection means and the peak detection means as described above, it can be possible to estimate the state accurately by response of the autonomic nervous system.

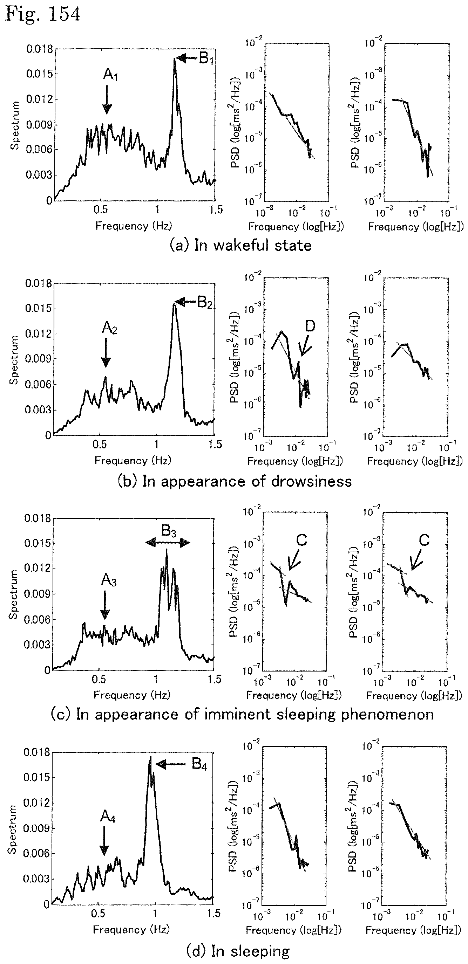

Fig. 153 is a diagram illustrating the active levels of the sympathetic nerves and the parasympathetic nerves in the sleep induction test determined from the finger plethysmogram of a male subject in 20's. Figs. 154 are diagrams illustrating log-log graphs of the frequency analysis results for ten minutes of an original APW wave and the frequency analysis results of a time-series waveform obtained by the zero-cross detection method and the peak detection method in respective states (wakeful, drowsy, imminent sleeping phenomenon sleep). Fig. 154(a) is a diagram illustrating the result in a wakeful state, Fig. 154(b) is a diagram illustrating the result at occurrence of drowsiness, Fig. 154(c) is a diagram illustrating the result at occurrence of an imminent sleeping phenomenon, and Fig. 154(d) is a diagram illustrating the result in a sleep state. Fig. 153 is determined from the finger plethysmogram by a wavelet analysis.

In the spectrum change of an original APW wave, the spectrum of 0.5th component changes from the arrow A1 to the arrow A4, and decreases as the state changes in the order of drowsiness → imminent sleeping phenomenon → sleep when they are compared by using the wakeful state as a reference. It can be seen that the spectrum of the 1st component changes from the arrow B1 to the arrow B4, and it changes from harmonic oscillation to irregular vibration and then to harmonic oscillation. The center frequency of the heart rate fluctuation shifts from 1.2 Hz to 1.0 Hz, and decrease in heart rate due to sleep is observed. Influence of the irregular vibration indicated by the arrow B3 at the time of occurrence of the imminent sleeping phenomenon appeared as a bifurcation phenomenon in the vicinity of 0.0053 Hz indicated by the arrow C in the zero-cross detection means and the peak detection means. The imminent sleeping phenomenon is a state being about to fall asleep, and it is deemed that the strong drowsiness and the disturbance in the heartbeat fluctuation appear as the irregular vibration. On the other hand, in a wakeful state, at the time of occurrence of drowsiness, and in the sleeping state, the gradients determined from the zero-cross detection means and the peak detection means are 1/f, and it is deemed that the sympathetic nerves and the parasympathetic nerves are well balanced. At the time of occurrence of drowsiness, the arrow D part indicated by the zero-cross detection means largely fluctuates in the vicinity of 0.01 Hz; however, this fluctuation is not observed in the peak detection means. This represents that the cardiac function tends to be relaxed, and in the autonomic nervous system, the sympathetic compensatory mechanism occurs due to occurrence of drowsiness. This corresponds to the change of the arrow a part in Fig. 153, and seems to correspond to the change in the 0.5th component indicated by the arrows A1 to A4 in Fig. 154 in the original waveform spectrum. It is deemed that the autonomic nervous system, in particular, the function of the sympathetic nerve system largely contributes on the fluctuation in the vicinity of 0.0053 Hz in the zero-cross detection means and the peak detection means at the time of occurrence of the imminent sleeping phenomenon. In the sleeping state, both the zero-cross detection method and the peak detection method are close to 1/f, and it is deemed that both the cardiac function and the autonomic function are well balanced, and the subject is in a relaxed state. The aspects of changes shown in Fig. 153 and Fig. 154 are well coincident with the estimated states. From these facts, it is suggested that APW is able to grasp a rough change of physical condition, and the extremely or ultra low frequency of APW is able to grasp reactions of autonomic nervous systems.

### (Analysis using frequency fluctuation computation means)

As the frequency analysis means 80, the means that analyzes frequencies of the frequency fluctuation time-series waveforms determined by the frequency fluctuation computation means, and outputs the fluctuation waveforms in log-log graphs of frequency and power spectral density may be employed. The frequency fluctuation computation means determines frequency fluctuation time-series waveforms from respective time-series waveforms of frequency obtained from each of the zero-cross detection means and the peak detection means.

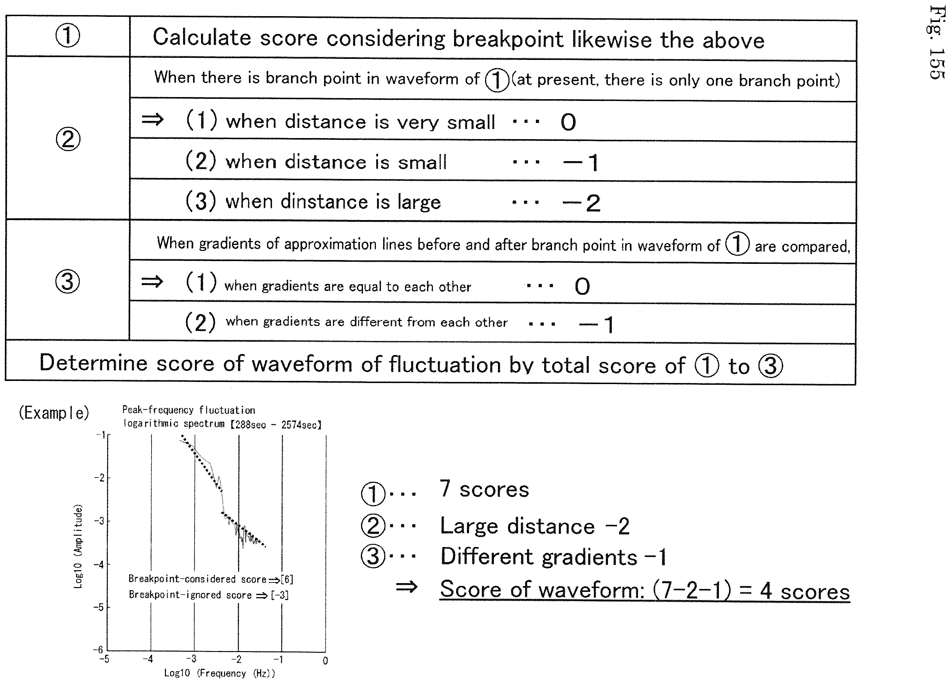

When a log-log graph as shown in the lower chart of Fig. 155 is determined by the frequency analysis means 80, identification is made based on the rule as shown in the upper chart of Fig. 155, for example. In this chart, "distance" means difference between the terminal end of a regression line and the leading end of the next regression line, and "gradient" means a gradient of each regression line. Determination of "very short distance", "short distance" or "large distance", and determination of "same gradient" or "different gradients" are made by arbitrarily setting thresholds respectively, and scoring according to correspondence thereto.

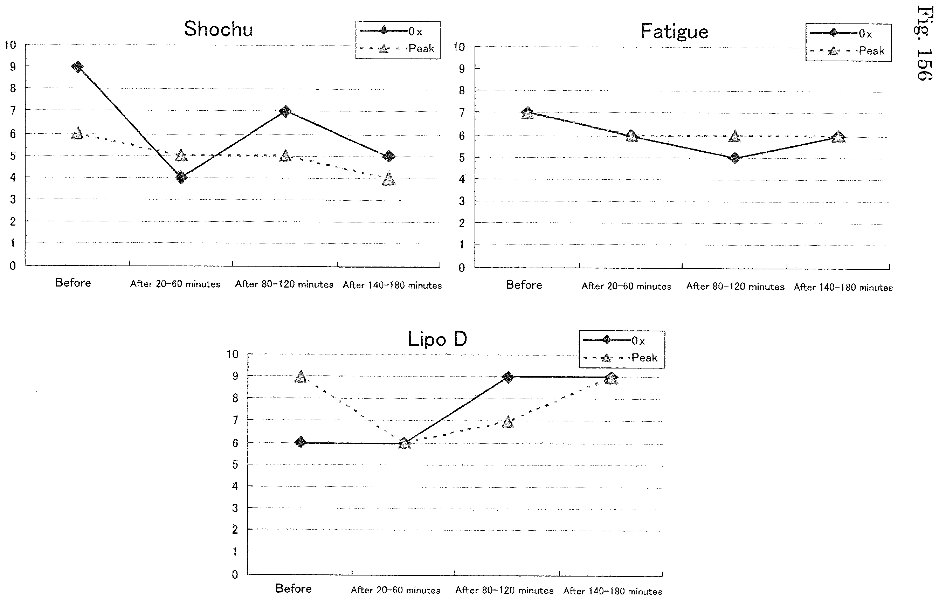

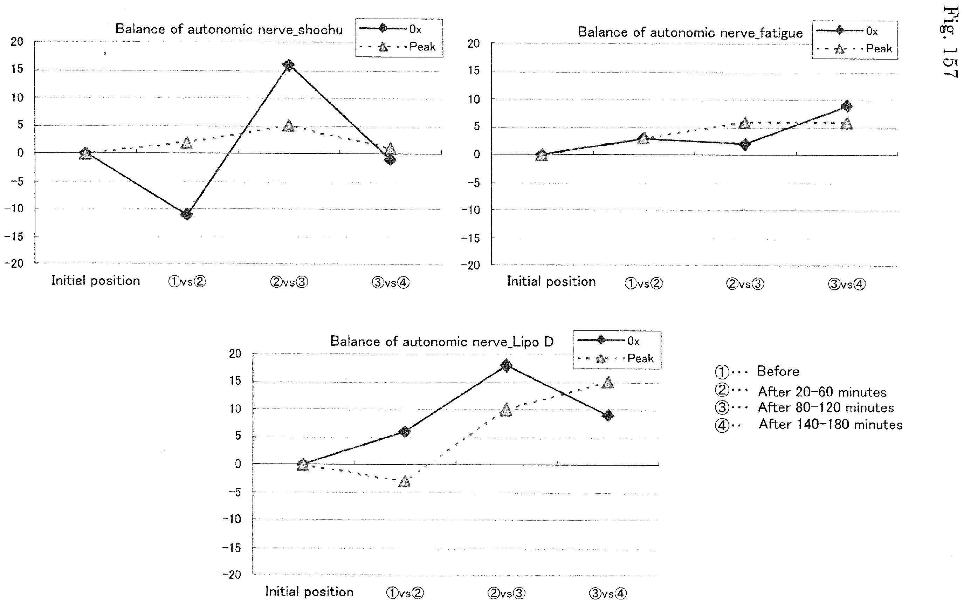

Fig. 156 is a diagram illustrating the scores obtained based on the above rule in a time-series order (analysis time segment order) of the data shown in Fig. 134 to Fig. 137. Fig. 157 is a diagram illustrating transitions of the balance of the autonomic nervous system, obtained by calculating a difference in scores between adjacent analysis time segments when the score of an initial analysis time segment is "zero". In both cases, in "drunk" in contrast with "fatigue', fluctuation with large fluctuation occurs and the feature of an irregular vibration system appears. On the other hand, in "fatigue", change is small and the feature of a harmonic oscillation system appears. In the case of drinking "Nutrient Drink A (Lipo D)", the change has a rising trend, and a behavior of an irregular vibration system is exhibited initially, and difference between the score by the zero-cross detection means and the score by the peak detection means gradually reduces, and the feature of a harmonic oscillation system is exhibited after the momentary irregular vibration unlike the case of drunk.

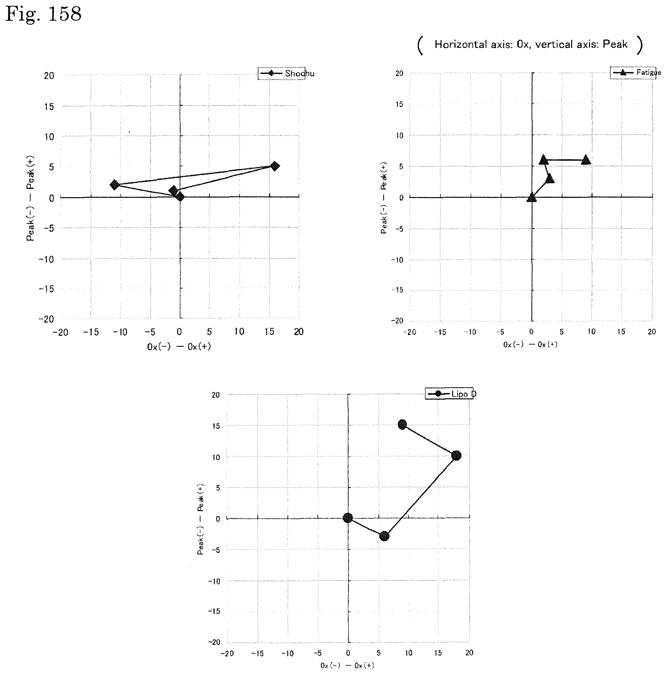

Fig. 158 illustrates four-quadrant coordinates in which the score calculated by a zero-cross detection mean is on the horizontal axis, and the score calculated by a peak detection means is on the vertical axis. These coordinates also reveal that different changes are exhibited between drunk and other conditions. Accordingly, analysis using the frequency fluctuation time-series waveforms makes it possible to estimate the condition of a person by determining difference between the harmonic oscillation system and the irregular vibration system more clearly.

### Reference Signs List

- 1: biological signal measuring means
- 201: back support cushion member
- 210: bag-shaped member
- 220: base cushion member
- 230: sensing mechanism unit
- 233: sensor
- 240: pelvis and waist supporting member
- 60: biological state estimation device
- 70: frequency-gradient time-series analysis and computation means
- 710: frequency computation means
- 720: gradient time-series computation means
- 80: frequency analysis means
- 90: fluctuation waveform analyzing means
- 901: regression line computation means
- 902: determination criterial score calculation means
- 95: state estimation means
- 98: pathological state discriminating means

## Claims

1. A biological state estimation device (60) that estimates a biological state using a biological signal of an autonomic nervous system, collected by a biological signal measuring means (1), the biological state estimation device (60) comprising:
a frequency analysis means (80) that analyzes frequencies of the biological signal to obtain a fluctuation waveform in a ultra-low-frequency band of 0.001 Hz to 0.04 Hz; and a state estimation means (95) that substitutes and displays the fluctuation waveform obtained by the frequency analysis means (80) with index values regarding a sympathetic nerve and a parasympathetic nerve based on predetermined criteria to estimate the biological state based on a change with time in the index values,
wherein the state estimation means (95) is a means that obtains the fluctuation waveform obtained by the frequency analysis means (80) as coordinate points on a four-quadrant coordinate system in which respective indices regarding the sympathetic nerve and the parasympathetic nerve are illustrated on vertical and horizontal axes based on the predetermined criteria to display vectors and estimates the biological state based on a change with time of the coordinate points.

2. The biological state estimation device (60) according to claim 1, wherein the state estimation means (95) includes a first analysis determination means (951) that estimates whether the biological state is a normal fatigued state where fatigue accumulates due to activities, a slump state, or a function recovery state where a predetermined function recovery means is performed based on a position of a coordinate point in a target analysis time segment in relation to a coordinate point in a reference analysis time segment.

3. The biological state estimation device (60) according to claim 2, wherein the first analysis determination means (951) includes at least one of:
an analysis determination means A that estimates a transition direction of an overall change in physical conditions after a change factor of a predetermined biological state is added in a reference analysis time segment based on the degree of change in the fluctuation waveform as a physical condition change trend; and an analysis determination means B that estimates a physical condition state in a predetermined analysis period when a predetermined period has passed after a change factor of the predetermined biological state is added based on the degree of change of the fluctuation waveform as an analysis physical condition state.

4. The biological state estimation device (60) according to claim 2, wherein the state estimation means (95) further includes a second analysis determination means (952) that substitutes the positions on the coordinate system of the coordinate points in the target analysis time segment with trigonometric representations to plot the positions again in a new coordinate system and estimates the biological state based on the replotted positions of the coordinate points.

5. The biological state estimation device (60) according to claim 4, wherein the second analysis determination means (952) includes a means that creates trigonometric representation coordinates with respect to each of the respective coordinate points obtained by the analysis determination means A and B of the first analysis determination means (951), the trigonometric representation coordinates being plotted using an angle corresponding to the trigonometric representations of the coordinate points obtained by the analysis determination means A as one axis and an angle corresponding to the trigonometric representations of the coordinate points obtained by the analysis determination means B as the other axis, and the second analysis determination means (952) estimates the biological state based on the positions of the coordinate points of the trigonometric representation coordinates.

6. The biological state estimation device (60) according to claim 5, the second analysis determination means (952) includes:
a means that obtains a sine angle of each of the respective coordinate points obtained by the analysis determination means A and B of the first analysis determination means (951) to create sine-representation coordinates plotted using the sine angle of the respective coordinate points obtained by the analysis determination means A as one axis and the sine angle of the respective coordinate points obtained by the analysis determination means B as the other axis; and a means that obtains a tangent angle of the respective coordinate points obtained by the analysis determination means A and B of the first analysis determination means (951) to create tangent-representation coordinates plotted using the tangent angle of the respective coordinate points obtained by the analysis determination means A as one axis and the tangent angle of the respective coordinate points obtained by the analysis determination means B as the other axis, and the second analysis determination means (952) estimates the biological state based on the positions of the coordinate points of the sine-representation coordinates and the tangent-representation coordinates.

7. The biological state estimation device (60) according to claim 4, wherein the state estimation means (95) further includes a sleep quality estimation means that estimates the quality of sleep as the function recovery means.

8. The biological state estimation device (60) according to claim 7, wherein the state estimation means (95) further includes:
a physical condition map creation means that sequentially obtains coordinate points based on predetermined criteria using a difference between analysis periods which are different in respective analysis time segments to create a time-series change line indicating a time-series change in physical conditions in the analysis time segment; and a sensory map creation means that sequentially obtains coordinate points based on criteria different from those of the physical condition map creation means using a difference between analysis periods that are different in respective analysis time segments to create a time-series change line indicating a time-series change in senses in the analysis time segment, and the sleep quality estimation means estimates the quality of sleep by taking a transition trend of the respective time-series change lines of the physical condition map creation means and the sensory map creation means.

9. A computer program set in a biological state estimation device (60) that estimates a biological state using a biological signal of an autonomic nervous system, collected by a biological signal measuring means (1), the computer program causing a computer to execute:
a frequency analysis procedure that analyzes frequencies of the biological signal to obtain a fluctuation waveform in a ultra-low-frequency band of 0.001 Hz to 0.04 Hz; and a state estimation procedure that substitutes and displays the fluctuation waveform obtained by the frequency analysis procedure with index values regarding a sympathetic nerve and a parasympathetic nerve based on predetermined criteria to estimate the biological state based on a change with time in the index values,
wherein the state estimation procedure is a procedure that obtains the fluctuation waveform obtained by the frequency analysis means (80) as coordinate points on a four-quadrant coordinate system in which respective indices regarding the sympathetic nerve and the parasympathetic nerve are illustrated on vertical and horizontal axes based on the predetermined criteria to display vectors and estimating the biological state based on a change with time of the coordinate points.

10. The computer program according to claim 9, wherein
the state estimation procedure includes a first analysis determination procedure that estimates whether the biological state is a normal fatigued state where fatigue accumulates due to activities, a slump state, or a function recovery state where a predetermined function recovery procedure is performed based on a position of a coordinate point in a target analysis time segment in relation to a coordinate point in a reference analysis time segment.

11. The computer program according to claim 10, wherein the first analysis determination procedure includes at least one of:
an analysis determination procedure A that estimates a transition direction of an overall change in physical conditions after a change factor of a predetermined biological state is added in a reference analysis time segment based on the degree of change in the fluctuation waveform as a physical condition change trend; and an analysis determination procedure B that estimates a physical condition state in a predetermined analysis period when a predetermined period has passed after a change factor of the predetermined biological state is added based on the degree of change of the fluctuation waveform as an analysis physical condition state.

12. The computer program according to claim 9, wherein the state estimation procedure further includes a second analysis determination procedure that substitutes the positions on the coordinate system of the coordinate points in the target analysis time segment with trigonometric representations to plot the positions again in a new coordinate system and estimates the biological state based on the replotted positions of the coordinate points.

13. The computer program according to claim 12, wherein the state estimation procedure further includes:
a physical condition map creation procedure that sequentially obtains coordinate points based on predetermined criteria using a difference between analysis periods which are different in respective analysis time segments to create a time-series change line indicating a time-series change in physical conditions in the analysis time segment; and a sensory map creation procedure that sequentially obtains coordinate points based on criteria different from those of the physical condition map creation procedure using a difference between analysis periods that are different in respective analysis time segments to create a time-series change line indicating a time-series change in senses in the analysis time segment, and the sleep quality estimation procedure estimates the quality of sleep by taking a transition trend of the respective time-series change lines of the physical condition map creation procedure and the sensory map creation procedure.

## Patentansprüche

1. Vorrichtung zur Einschätzung des biologischen Zustands (60), die einen biologischen Zustand einschätzt unter Verwendung eines biologischen Signals eines autonomen Nervensystems, das von einem Mittel zum Messen von biologischen Signalen (1) eingefangen wird, wobei die Vorrichtung zur Einschätzung des biologischen Zustands (60) umfasst:
ein Frequenzanalysemittel (80), das Frequenzen des biologischen Signals analysiert, um eine Fluktuationswellenform in einem Ultraniederfrequenzband von 0,001 Hz bis 0,04 Hz zu erhalten; und ein Mittel zur Zustandseinschätzung (95), das die vom Frequenzanalysemittel (80) erhaltene Fluktuationswellenform ersetzt und anzeigt mit Indexwerten in Bezug auf einen sympathischen Nerv und einen parasympathischen Nerv auf der Grundlage von vorbestimmten Kriterien, um den biologischen Zustand auf der Grundlage einer Veränderung in den Indexwerten im Verlauf der Zeit einzuschätzen,
wobei das Mittel zur Zustandseinschätzung (95) ein Mittel ist, das die vom Frequenzanalysemittel (80) erhaltene Fluktuationswellenform als Koordinatenpunkte in einem Vierquadranten-Koordinatensystem erhält, in dem jeweilige Indizes in Bezug auf den sympathischen Nerv und den parasympathischen Nerv auf vertikalen und horizontalen Achsen auf der Grundlage der vorbestimmten Kriterien abgebildet sind, um Vektoren anzuzeigen, und den biologischen Zustand auf der Grundlage einer Veränderung der Koordinatenpunkte im Verlauf der Zeit einschätzt.

2. Vorrichtung zur Einschätzung des biologischen Zustands (60) nach Anspruch 1, wobei das Mittel zur Zustandseinschätzung (95) ein erstes Analysebestimmungsmittel (951) enthält, das einschätzt, ob der biologische Zustand ein normaler ermüdeter Zustand, in dem sich Ermüdung infolge von Aktivitäten ansammelt, ein Einbruchszustand oder ein Zustand der Funktionswiederherstellung ist, in dem ein vorbestimmtes Funktionswiederherstellungsmittel ausgeführt wird auf der Grundlage einer Position eines Koordinatenpunkts in einem Zeitsegment zur Zielanalyse im Verhältnis zu einem Koordinatenpunkt in einem Zeitsegment zur Referenzanalyse.

3. Vorrichtung zur Einschätzung des biologischen Zustands (60) nach Anspruch 2, wobei das erste Analysebestimmungsmittel (951) mindestens eines enthält von:
einem Analysebestimmungsmittel A, das eine Übergangsrichtung einer generellen Veränderung der körperlichen Bedingungen einschätzt, nachdem ein Veränderungsfaktor eines vorbestimmten biologischen Zustands in ein Zeitsegment zur Referenzanalyse auf der Grundlage des Grads der Veränderung der Fluktuationswellenform als ein Trend der Veränderung der körperlichen Bedingung hinzugefügt wurde; und einem Analysebestimmungsmittel B, das einen körperlichen Bedingungszustand in einer vorbestimmten Analysezeitspanne einschätzt, wenn eine vorbestimmte Zeitspanne verstrichen ist, nachdem ein Veränderungsfaktor des vorbestimmten biologischen Zustands auf der Grundlage des Grads von Veränderung der Fluktuationswellenform als eine Analyse des körperlichen Bedingungszustands hinzugefügt wurde.

4. Vorrichtung zur Einschätzung des biologischen Zustands (60) nach Anspruch 2, wobei das Mittel zur Zustandseinschätzung (95) weiter ein zweites Analysebestimmungsmittel (952) enthält, das die Positionen der Koordinatenpunkte im Koordinatensystem im Zeitsegment zur Zielanalyse durch trigonometrische Darstellungen ersetzt, um die Positionen erneut in ein neues Koordinatensystem einzuzeichnen, und den biologischen Zustand auf der Grundlage der neu eingezeichneten Positionen der Koordinatenpunkte einschätzt.

5. Vorrichtung zur Einschätzung des biologischen Zustands (60) nach Anspruch 4, wobei das zweite Analysebestimmungsmittel (952) ein Mittel enthält, das trigonometrische Darstellungskoordinaten bezüglich eines jeden der jeweiligen durch die Analysebestimmungsmittel A und B des ersten Analysebestimmungsmittels (951) erhaltenen Koordinatenpunkte erzeugt, wobei die trigonometrischen Darstellungskoordinaten unter Verwendung eines Winkels, der den trigonometrischen Darstellungen der von dem Analysebestimmungsmittel A erhaltenen Koordinatenpunkten entspricht, als eine Achse, und eines Winkels, der den trigonometrischen Darstellungen der vom Analysebestimmungsmittel B erhaltenen Koordinatenpunkte entspricht, als die andere Achse eingezeichnet werden, und wobei das zweite Analysebestimmungsmittel (952) den biologischen Zustand auf der Grundlage der Positionen der Koordinatenpunkte der trigonometrischen Darstellungskoordinaten einschätzt.

6. Vorrichtung zur Einschätzung des biologischen Zustands (60) nach Anspruch 5, wobei das zweite Analysebestimmungsmittel (952) enthält:
ein Mittel, das einen Sinuswinkel von jedem der jeweiligen von den Analysebestimmungsmitteln A und B des ersten Analysebestimmungsmittels (951) erhaltenen Koordinatenpunkte erhält, um Sinus-Darstellungskoordinaten zu erzeugen, die unter Verwendung des Sinuswinkels der jeweiligen vom Analysebestimmungsmittel A erhaltenen Koordinatenpunkte als eine Achse und des Sinuswinkels der jeweiligen vom Analysebestimmungsmittel B erhaltenen Koordinatenpunkte als die andere Achse eingezeichnet werden; und ein Mittel, das einen Tangentenwinkel der jeweiligen von den Analysebestimmungsmitteln A und B des ersten Analysebestimmungsmittels (951) erhaltenen Koordinatenpunkte erhält, um Tangent-Darstellungskoordinaten zu erstellen, die unter Verwendung des Tangentenwinkels der jeweiligen vom Analysebestimmungsmittel A erhaltenen Koordinatenpunkte als eine Achse und des Tangentenwinkels der jeweiligen vom Analysebestimmungsmittel B erhaltenen Koordinatenpunkte als die andere Achse eingezeichnet werden, und das zweite Analysebestimmungsmittel (952) den biologischen Zustand auf der Grundlage der Positionen der Koordinatenpunkte der Sinus-Darstellungskoordinaten und der Tangenten-Darstellungskoordinaten einschätzt.

7. Vorrichtung zur Einschätzung des biologischen Zustands (60) nach Anspruch 4, wobei das Mittel zur Zustandseinschätzung (95) weiter ein Mittel zur Einschätzung der Schlafqualität enthält, das die Qualität des Schlafs als das Funktionswiederherstellungsmittel einschätzt.

8. Vorrichtung zur Einschätzung des biologischen Zustands (60) nach Anspruch 7, wobei das Mittel zur Zustandseinschätzung (95) weiter enthält:
ein Mittel zur Erstellung einer Karte von körperlichen Bedingungen, das sequentiell Koordinatenpunkte erhält auf der Grundlage von vorbestimmten Kriterien unter Verwendung einer Differenz zwischen Analysezeitspannen, die in jeweiligen Analysezeitsegmenten unterschiedlich sind, um eine Veränderungslinie von Zeitserien zu erstellen, die eine Veränderung von Zeitserien der körperlichen Bedingungen im Analysezeitsegment anzeigt; und ein Mittel zur Erstellung einer sensorischen Karte, die sequentiell Koordinatenpunkte auf der Grundlage von Kriterien erhält, die unterschiedlich sind von denen des Mittels zur Erstellung einer Karte von körperlichen Bedingungen, unter Verwendung einer Differenz zwischen Analysezeitspannen, die in jeweiligen Analysezeitsegmenten unterschiedlich sind, um eine Veränderungslinie von Zeitserien zu erstellen, die eine Veränderung von Zeitserien der Sinne im Analysezeitsegment anzeigt, und wobei das Mittel zur Einschätzung der Schlafqualität die Qualität des Schlafs einschätzt durch Nehmen eines Übergangstrends der jeweiligen Veränderungslinie von Zeitserien des Mittels zur Erstellung einer Karte von körperlichen Bedingungen und des Mittels zur Erstellung einer sensorischen Karte.

9. Computerprogramm, eingerichtet in einer Vorrichtung zur Einschätzung des biologischen Zustands (60), die einen biologischen Zustand einschätzt unter Verwendung eines biologischen Signals eines autonomen Nervensystems, das von einem Mittel zum Messen von biologischen Signalen (1) eingefangen wird, wobei das Computerprogramm veranlasst, dass ein Computer vornimmt:
ein Frequenzanalyseverfahren, das Frequenzen des biologischen Signals analysiert, um eine Fluktuationswellenform in einem Ultraniederfrequenzband von 0,001 Hz bis 0,04 Hz zu erhalten; und ein Zustandseinschätzungsverfahren, das die vom Frequenzanalyseverfahren erhaltene Fluktuationswellenform ersetzt und anzeigt mit Indexwerten in Bezug auf einen sympathischen Nerv und einen parasympathischen Nerv auf der Grundlage von vorbestimmten Kriterien, um den biologischen Zustand auf der Grundlage einer Veränderung in den Indexwerten im Verlauf der Zeit einzuschätzen,
wobei das Zustandseinschätzungsverfahren ein Verfahren ist, das die vom Frequenzanalysemittel (80) erhaltene Fluktuationswellenform als Koordinatenpunkte in einem Vierquadranten-Koordinatensystem erhält, in dem jeweilige Indizes in Bezug auf den sympathischen Nerv und den parasympathischen Nerv auf vertikalen und horizontalen Achsen auf der Grundlage der vorbestimmten Kriterien abgebildet werden, um Vektoren anzuzeigen, und zum Einschätzen des biologischen Zustands auf der Grundlage einer Veränderung der Koordinatenpunkte im Verlauf der Zeit.

10. Computerprogramm nach Anspruch 9, wobei
das Zustandseinschätzungsverfahren ein erstes Analysebestimmungsverfahren enthält, das einschätzt, ob der biologische Zustand ein normaler ermüdeter Zustand, in dem sich Ermüdung infolge von Aktivitäten ansammelt, ein Einbruchszustand oder ein Zustand der Funktionswiederherstellung ist, in dem ein vorbestimmtes Funktionswiederherstellungsverfahren ausgeführt wird auf der Grundlage einer Position eines Koordinatenpunkts in einem Zeitsegment zur Zielanalyse im Verhältnis zu einem Koordinatenpunkt in einem Zeitsegment zur Referenzanalyse.

11. Computerprogramm nach Anspruch 10, wobei das erste Analysebestimmungsverfahren mindestens eines enthält von:
einem Analysebestimmungsverfahren A, das eine Übergangsrichtung einer generellen Veränderung der körperlichen Bedingungen einschätzt, nachdem ein Veränderungsfaktor eines vorbestimmten biologischen Zustands in ein Zeitsegment zur Referenzanalyse auf der Grundlage des Grads der Veränderung der Fluktuationswellenform als ein Trend der Veränderung der körperlichen Bedingung hinzugefügt wurde; und einem Analysebestimmungsverfahren B, das einen körperlichen Bedingungszustand in einer vorbestimmten Analysezeitspanne einschätzt, wenn eine vorbestimmten Zeitspanne verstrichen ist, nachdem ein Veränderungsfaktor des vorbestimmten biologischen Zustands auf der Grundlage des Grads von Veränderung der Fluktuationswellenform als eine Analyse des körperlichen Bedingungszustands hinzugefügt wurde.

12. Computerprogramm nach Anspruch 9, wobei das Zustandseinschätzungsverfahren weiter ein zweites Analysebestimmungsverfahren enthält, das die Positionen der Koordinatenpunkte im Koordinatensystem im Zeitsegment zur Zielanalyse durch trigonometrische Darstellungen ersetzt, um die Positionen erneut in ein neues Koordinatensystem einzuzeichnen, und den biologischen Zustand auf der Grundlage der neu eingezeichneten Positionen der Koordinatenpunkte einschätzt.

13. Computerprogramm nach Anspruch 12, wobei das Zustandseinschätzungsverfahren weiter enthält:
ein Verfahren zur Erstellung einer Karte von körperlichen Bedingungen, das sequentiell Koordinatenpunkte erhält auf der Grundlage von vorbestimmten Kriterien unter Verwendung einer Differenz zwischen Analysezeitspannen, die in jeweiligen Analysezeitsegmenten unterschiedlich sind, um eine Veränderungslinie von Zeitserien zu erstellen, die eine Veränderung von Zeitserien der körperlichen Bedingungen im Analysezeitsegment anzeigt; und ein Verfahren zur Erstellung einer sensorischen Karte, die sequentiell Koordinatenpunkte auf der Grundlage von Kriterien erhält, die unterschiedlich sind von denen des Verfahrens zur Erstellung einer Karte von körperlichen Bedingungen unter Verwendung einer Differenz zwischen Analysezeitspannen, die in jeweiligen Analysezeitsegmenten unterschiedlich sind, um eine Veränderungslinie von Zeitserien zu erstellen, die eine Veränderung von Zeitserien der Sinne im Analysezeitsegment anzeigt, und wobei das Verfahren zur Einschätzung der Schlafqualität die Qualität des Schlafs einschätzt durch Nehmen eines Übergangstrends der jeweiligen Veränderungslinie von Zeitserien des Verfahrens zur Erstellung einer Karte von körperlichen Bedingungen und des Verfahrens zur Erstellung einer sensorischen Karte.

## Revendications

1. Dispositif d'estimation d'état biologique (60) qui estime un état biologique en utilisant un signal biologique d'un système nerveux autonome, collecté par un moyen de mesure de signal biologique (1), le dispositif d'estimation d'état biologique (60) comprenant :
un moyen d'analyse de fréquence (80) qui analyse les fréquences du signal biologique pour obtenir une forme d'onde de fluctuation dans une bande à ultrabasse fréquence de 0,001 Hz à 0,04 Hz ; et un moyen d'estimation d'état (95) qui substitue et affiche la forme d'onde de fluctuation obtenue par le moyen d'analyse de fréquence (80) avec des valeurs d'indice concernant un nerf sympathique et un nerf parasympathique sur la base de critères prédéterminés pour estimer l'état biologique sur la base d'un changement dans le temps des valeurs d'indice,
dans lequel le moyen d'estimation d'état (95) est un moyen qui obtient la forme d'onde de fluctuation obtenue par le moyen d'analyse de fréquence (80) en tant que points de coordonnées sur un système de coordonnées à quatre quadrants dans lequel des indices respectifs concernant le nerf sympathique et le nerf parasympathique sont illustrés sur des axes vertical et horizontal sur la base des critères prédéterminés pour afficher des vecteurs et estime l'état biologique sur la base d'un changement dans le temps des points de coordonnées.

2. Dispositif d'estimation d'état biologique (60) selon la revendication 1, dans lequel le moyen d'estimation d'état (95) inclut un premier moyen de détermination d'analyse (951) qui estime si l'état biologique est un état fatigué normal où la fatigue s'accumule en raison d'activités, un état d'effondrement, ou un état de récupération de fonction où un moyen de récupération de fonction prédéterminé est effectué sur la base d'une position d'un point de coordonnées dans un segment de temps d'analyse cible par rapport à un point de coordonnées dans un segment de temps d'analyse de référence.

3. Dispositif d'estimation d'état biologique (60) selon la revendication 2, dans lequel le premier moyen de détermination d'analyse (951) inclut au moins un parmi :
un moyen de détermination d'analyse A qui estime une direction de transition d'un changement global de conditions physiques après qu'un facteur de changement d'un état biologique prédéterminé est ajouté dans un segment de temps d'analyse de référence sur la base du degré de changement de la forme d'onde de fluctuation en tant que tendance au changement de condition physique; et un moyen de détermination d'analyse B qui estime un état de condition physique dans une période d'analyse prédéterminée lorsqu'une période prédéterminée a passé après qu'un facteur de changement de l'état biologique prédéterminé est ajouté sur la base du degré de changement de la forme d'onde de fluctuation en tant qu'état de condition physique d'analyse.

4. Dispositif d'estimation d'état biologique (60) selon la revendication 2, dans lequel le moyen d'estimation d'état (95) inclut en outre un deuxième moyen de détermination d'analyse (952) qui substitue les positions sur le système de coordonnées des points de coordonnées dans le segment de temps d'analyse cible par des représentations trigonométriques pour tracer les positions à nouveau dans un nouveau système de coordonnées et estime l'état biologique sur la base des positions retracées des points de coordonnées.

5. Dispositif d'estimation d'état biologique (60) selon la revendication 4, dans lequel le deuxième moyen de détermination d'analyse (952) inclut un moyen qui crée des coordonnées de représentation trigonométrique par rapport à chacun des points de coordonnées respectifs obtenus par les moyens de détermination d'analyse A et B du premier moyen de détermination d'analyse (951), les coordonnées de représentation trigonométrique étant tracées en utilisant un angle correspondant aux représentations trigonométriques des points de coordonnées obtenus par le moyen de détermination d'analyse A en tant qu'axe et un angle correspondant aux représentations trigonométriques des points de coordonnées obtenus par le moyen de détermination d'analyse B en tant qu'autre axe, et le deuxième moyen de détermination d'analyse (952) estime l'état biologique sur la base des positions des points de coordonnées des coordonnées de représentation trigonométrique.

6. Dispositif d'estimation d'état biologique (60) selon la revendication 5, le deuxième moyen de détermination d'analyse (952) inclut :
un moyen qui obtient un angle sinusal de chacun des points de coordonnées respectifs obtenus par les moyens de détermination d'analyse A et B du premier moyen de détermination d'analyse (951) pour créer des coordonnées de représentation sinusale en utilisant l'angle sinusal des points de coordonnées respectifs obtenus par le moyen de détermination d'analyse A en tant qu'un axe et l'angle sinusal des points de coordonnées respectifs obtenus par le moyen de détermination d'analyse B en tant qu'autre axe ; et un moyen qui obtient un angle tangentiel des points de coordonnées respectifs obtenus par les moyens de détermination d'analyse A et B du premier moyen de détermination d'analyse (951) pour créer des coordonnées de représentation tangentielle tracées en utilisant l'angle tangentiel des points de coordonnées respectifs obtenus par le moyen de détermination d'analyse A en tant qu'un axe et l'angle tangentiel des points de coordonnées respectifs obtenus par le moyen de détermination d'analyse B en tant qu'autre axe, et le deuxième moyen de détermination d'analyse (952) estime l'état biologique sur la base des positions des points des coordonnées de représentation sinusale et des coordonnées de représentation tangentielle.

7. Dispositif d'estimation d'état biologique (60) selon la revendication 4, dans lequel le moyen d'estimation d'état (95) inclut en outre un moyen d'estimation de qualité du sommeil qui estime la qualité du sommeil en tant que moyen de récupération de fonction.

8. Dispositif d'estimation d'état biologique (60) selon la revendication 7, dans lequel le moyen d'estimation d'état (95) inclut en outre :
un moyen de création de carte de condition physique qui obtient de manière séquentielle des points de coordonnées sur la base de critères prédéterminés en utilisant une différence entre des périodes d'analyse qui sont différentes dans des segments de temps d'analyse respectifs pour créer une ligne de changement chronologique indiquant un changement chronologique des conditions physiques dans le segment de temps d'analyse ; et un moyen de création de carte sensorielle qui obtient de manière séquentielle des points de coordonnées sur la base de critères différents de ceux du moyen de création de carte de condition physique en utilisant une différence entre des périodes d'analyse qui sont différentes dans des segments de temps d'analyse respectifs pour créer une ligne de changement chronologique indiquant un changement chronologique des sens dans le segment de temps d'analyse, et le moyen d'estimation de qualité du sommeil estime la qualité du sommeil en prenant une tendance à la transition des lignes de changement chronologique respectives du moyen de création de carte de condition physique et du moyen de création de carte sensorielle.

9. Ensemble de programmes informatiques dans un dispositif d'estimation d'état biologique (60) qui estime un état biologique en utilisant un signal biologique d'un système nerveux autonome, collecté par un moyen de mesure de signal biologique (1), le programme informatique amenant un ordinateur à exécuter :
une procédure d'analyse de fréquence qui analyse les fréquences du signal biologique pour obtenir une forme d'onde de fluctuation dans une bande à ultrabasse fréquence de 0,001 Hz à 0,04 Hz ; et une procédure d'estimation d'état qui substitue et affiche la forme d'onde de fluctuation obtenue par la procédure d'analyse de fréquence avec des valeurs d'indice concernant un nerf sympathique et un nerf parasympathique sur la base de critères prédéterminés pour estimer l'état biologique sur la base d'un changement dans le temps des valeurs d'indice,
dans lequel la procédure d'estimation d'état est une procédure qui obtient la forme d'onde de fluctuation obtenue par le moyen d'analyse de fréquence (80) en tant que points de coordonnées sur un système de coordonnées à quatre quadrants dans lequel des indices respectifs concernant le nerf sympathique et le nerf parasympathique sont illustrés sur des axes vertical et horizontal sur la base des critères prédéterminés pour afficher des vecteurs et estimant l'état biologique sur la base d'un changement dans le temps des points de coordonnées.

10. Programme informatique selon la revendication 9, dans lequel
la procédure d'estimation d'état inclut une première procédure de détermination d'analyse qui estime si l'état biologique est dans un état fatigué normal où la fatigue s'accumule en raison d'activités, un état d'effondrement, ou un état de récupération de fonction où une procédure de récupération de fonction prédéterminée est effectuée sur la base d'une position d'un point de coordonnées dans un segment de temps d'analyse cible par rapport à un point de coordonnées dans un segment de temps d'analyse de référence.

11. Programme informatique selon la revendication 10, dans lequel la première procédure de détermination d'analyse inclut au moins une parmi :
une procédure de détermination d'analyse A qui estime une direction de transition d'un changement global des conditions physiques après qu'un facteur de changement d'un état biologique prédéterminé est ajouté dans un segment de temps d'analyse de référence sur la base du degré de changement de la forme d'onde de fluctuation en tant que tendance au changement de condition physique; et une procédure de détermination d'analyse B qui estime un état de condition physique dans une période d'analyse prédéterminée lorsqu'une période prédéterminée a passé après qu'un facteur de changement de l'état biologique prédéterminé est ajouté sur la base du degré de changement de la forme d'onde de fluctuation en tant qu'état de condition physique d'analyse.

12. Programme informatique selon la revendication 9, dans lequel la procédure d'estimation d'état inclut en outre une deuxième procédure de détermination d'analyse qui substitue les positions sur le système de coordonnées des points de coordonnées dans le segment de temps d'analyse cible par des représentations trigonométriques pour tracer les positions à nouveau dans un nouveau système de coordonnées et estime l'état biologique sur la base des positions retracées des points de coordonnées.

13. Programme informatique selon la revendication 12, dans lequel la procédure d'estimation d'état inclut en outre :
une procédure de création de carte de condition physique qui obtient de manière séquentielle des points de coordonnées sur la base de critères prédéterminés en utilisant une différence entre des périodes d'analyse qui sont différentes dans des segments de temps d'analyse respectifs pour créer une ligne de changement chronologique indiquant un changement chronologique de conditions physiques dans le segment d'analyse de temps ; et une procédure de création de carte sensorielle qui obtient de manière séquentielle des points de coordonnées sur la base de critères différents de ceux de la procédure de création de carte de condition physique en utilisant une différence entre des périodes d'analyse qui sont différentes dans des segments de temps d'analyse respectifs pour créer une ligne de changement chronologique indiquant un changement chronologique de sens dans le segment de temps d'analyse, et la procédure d'estimation de qualité du sommeil estime la qualité du sommeil en prenant une tendance à la transition des lignes de changement chronologique respectives de la procédure de création de carte de condition physique et de la procédure de création de carte sensorielle.
